# EUROPEAN PATENT APPLICATION

(11) **EP 4 814 020 A1**
(43) Date of publication of application: **30.09.2026**
(21) Application number: 24893466.3
(22) Date of filing: 20.11.2024
(51) Int. Cl.: C07K 16/46, C07K 19/00, C12N 15/09, C12N 15/13, C12N 15/85

(54) **METHOD FOR PREPARING HETEROLOGOUS MULTIMER BY MEANS OF RECOMBINATION REACTION**

(30) Priority: 20.11.2023 CN 202311544684; 23.01.2024 CN 202410093537
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHAO, Jie, Shanghai 200245 (CN); HE, Feng, Shanghai 200245 (CN); ZHANG, Mingxi, Shanghai 200245 (CN); XUE, Zhendong, Shanghai 200245 (CN); BIAN, Guoyong, Shanghai 200245 (CN)
(74) Representative: Potter Clarkson
(86) International application number: PCT/CN2024/133232
(87) International publication number: WO 2025/108310

(57) **Abstract**

The present disclosure relates to a method for preparing a heterologous multimer by means of a recombination reaction. In particular, the present disclosure relates to a method for preparing the heterologous multimer (especially a multispecific antibody), and changing the amino acid of the CH3 domain to facilitate the formation of the heterologous multimer (especially the multispecific antibody).

## Description

The present application claims priority to Chinese Patent Application No. CN 202311544684.7 filed on November 20, 2023 and Chinese Patent Application No. CN 202410093537.0 filed on January 23, 2024.

### TECHNICAL FIELD

The present disclosure relates to a method for preparing a heteromultimer (especially a multispecific antibody) and altering amino acids of a CH3 domain to promote the formation of a heteromultimer (especially a multispecific antibody).

### BACKGROUND

The statements herein merely provide background information related to the present disclosure and may not necessarily constitute the prior art.

Bispecific antibodies are examples of multispecific antibodies. They are antibody molecules capable of simultaneously and specifically binding to two antigens or two epitopes. Compared to monoclonal antibodies, bispecific antibodies have unique mechanisms of action and have significant advantages.

Among the various bispecific antibody platforms developed by numerous pharmaceutical companies (Brinkmann U, Kontermann R E. The making of bispecific antibodies[C]//MAbs. Taylor & Francis, 2017, 9(2): 182-212), the DuoBody platform has unique antibody engineering mechanisms and advantages.

Human IgGs can be classified into four subtypes: IgG1, IgG2, IgG3, and IgG4, with IgG4 accounting for approximately 5%. IgG4 has unique biological properties and can undergo dynamic heavy chain exchange *in vivo*, also known as Fab-arm exchange (FAE), which can lead to the formation of half-antibody molecules or the formation of a bispecific antibody from two half-antibody molecules (Van Der Neut Kolfschoten M, Schuurman J, Losen M, et al. Anti-inflammatory activity of human IgG4 antibodies by dynamic Fab arm exchange. Science, 2007, 317(5844): 1554-1557). Utilizing the FAE property of IgG4, Genmab has developed bispecific antibodies with controlled FAE. Research by Genmab shows that the dissociation of non-covalent CH3 regions is a key rate-limiting step in FAE, and that the R409 residue of IgG4 reduces the CH3-CH3 interaction. Dutch researchers have found that mutating the lysine at position 409 in IgG1 into arginine (IgG1-K409R) can impart FAE capability to IgG1 (Labrijn A F, Rispens T, Meesters J, et al. Species-specific determinants in the IgG CH3 domain enable Fab-arm exchange by affecting the noncovalent CH3-CH3 interaction strength. The Journal of Immunology, 2011, 187(6): 3238-3246); a corresponding mutation site (L368/K370/D399/F405/Y407) was introduced into another antibody CH3 region, and the FAE results of various combinations show that the combination of IgG1-K409R and IgG1-F405L can improve the efficiency of FAE (Labrijn A F, Meesters J I, de Goeij B E C G, et al. Efficient generation of stable bispecific IgG1 by controlled Fab-arm exchange. Proceedings of the National Academy of Sciences, 2013, 110(13): 5145-5150).

The DuoBody platform offers a relatively simple process for preparing a bispecific antibody: introducing mutation sites K409R and F405L into the CH3 domains of the Fc regions of two IgG1 antibodies, respectively, mixing the two target antibodies in a ratio, and allowing them to undergo FAE under specific reducing conditions to form a bispecific antibody. The DuoBody platform has various advantages: it requires less engineering, as only one site needs to be mutated in each of two parental antibodies; the resulting bispecific antibody closely resembles natural IgG1 in size and conformation, theoretically has similar physicochemical properties and drug developability to a natural monoclonal antibody, involves a low risk of immunogenicity, retains the Fc functions (ADCC, ADCP, CDC, etc.) of natural IgG1, and has a similar pharmacokinetic profile to natural IgG1.

In recent years, three consecutive DuoBody-based bispecific antibodies, including amivantamab, teclistamab, and talquetamab, have been approved by the U.S. FDA. Amivantamab (JNJ-61186372), a bispecific antibody targeting EGFR and c-Met developed based on the DuoBody platform, was approved by the U.S. FDA on May 21, 2021 for the treatment of adult patients with locally advanced or metastatic non-small cell lung cancer carrying EGFR exon 20 insertion mutations whose disease progressed during or after platinum-based chemotherapy. Teclistamab and talquetamab are CD3×BCMA and CD3×GPRC5D bispecific T-cell engagers (TCEs), respectively, and are used for adult patients with relapsed or refractory multiple myeloma (MM) who have previously received at least multiple frontline therapies.

In using Genmab's DuoBody platform to prepare bispecific antibodies, the present disclosure found that some parental antibodies carrying mutation F405L frequently exhibited SEC purity below 90% after Protein A affinity purification. This indicates that F405L may reduce the intrinsic physicochemical stability of the parental antibodies. Moreover, the results of an assessment of recombination efficiency via HPLC-IEC showed that the FAE efficiency of the combination of IgG1-K409R and IgG1-F405L was around 95%, indicating room for improvement in reaction efficiency. In addition, the K409R mutation used in the DuoBody platform is derived from human IgG4, while the F405L mutation is derived from rhesus monkey IgG4. Therefore, in theory, the stability of bispecific antibody molecules produced by this platform remains comparable to that of IgG4 antibodies and is inferior to that of wild-type human IgG1. Bispecific antibodies prepared using the DuoBody platform may not be suitable for the preparation of certain types of antibody-drug conjugates (ADCs). This is because some ADCs employ cysteine site-specific conjugation technology, where two disulfide bonds in the antibody hinge region are reduced and conjugated with drug molecules. If the interaction between the CH3 domains of the antibody Fc region is not strong enough, the ADC molecules may undergo FAE *in vivo*, which may pose risks in clinical applications. The CH3 domains of the bispecific antibodies prepared in the present disclosure may have superior physicochemical stability compared to DuoBody, thereby offering better drug developability and broader application.

### SUMMARY

The present disclosure provides a method for preparing a heteromultimer (e.g., a heterodimer), comprising the following steps:
a) a step of providing a dimer comprising two first polypeptides,
b) a step of providing a dimer comprising two second polypeptides,
c) a step of co-incubating the dimer comprising two first polypeptides and the dimer comprising two second polypeptides under reducing conditions, and
d) obtaining a heteromultimer comprising the first polypeptide and the second polypeptide (e.g., a heterodimer comprising one first polypeptide and one second polypeptide),
wherein the first polypeptide and the second polypeptide each comprise one CH3 domain; the CH3 domain(s) of the first polypeptide and/or the second polypeptide comprises at least one amino acid mutation selected from the group consisting of positions 347, 349, 351, 354, 356, 357, 364, 366, 368, 394, 405, 407, 409, 411, and 439; the CH3 domains of the first polypeptide and the second polypeptide differ by at least one mutation site, and mutation sites in the CH3 domains are indicated by EU numbering.

The present disclosure provides a method for preparing a heteromultimer, comprising the following steps:
a) a step of providing a molecule comprising a first polypeptide homomer,
b) a step of providing a molecule comprising a second polypeptide homomer, and
c) a step of co-incubating the molecule comprising the first polypeptide homomer and the molecule comprising the second polypeptide homomer under reducing conditions,
wherein the first polypeptide and the second polypeptide each comprise one CH3 domain; the CH3 domains of the first polypeptide and the second polypeptide differ by at least one mutation site, and mutation sites in the CH3 domains are indicated by EU numbering.

In some embodiments, the method for preparing a heteromultimer according to any one of the foregoing further comprises d) a step of obtaining a heteromultimer comprising the first polypeptide and the second polypeptide (e.g., a heterodimer comprising one first polypeptide and one second polypeptide). In some embodiments, step c) adding a reductant to the mixture and incubating may result in the dissociation of monomers of the homomers (e.g., the first polypeptide homomer and the second polypeptide homomer) and the recombination of heteromonomers (e.g., one first polypeptide and one second polypeptide), thereby leading to the formation of a heterodimer comprising one dissociated first polypeptide monomer and one dissociated second polypeptide monomer. In some embodiments, provided is the method for preparing a heteromultimer according to any one of the foregoing, wherein the molecule comprising the first polypeptide homomer and the molecule comprising the second polypeptide homomer bind to different antigens or epitopes.

In some embodiments, provided is the method for preparing a heteromultimer according to any one of the foregoing, wherein the CH3 domain(s) of the first polypeptide and/or the second polypeptide comprise(s) at least one amino acid mutation selected from the group consisting of positions 347, 349, 351, 354, 356, 357, 364, 366, 368, 394, 405, 407, 409, 411, and 439.

In some embodiments, provided is the method for preparing a heteromultimer according to any one of the foregoing, wherein the CH3 domain(s) of the first polypeptide and/or the second polypeptide comprise(s) at least one amino acid mutation selected from the group consisting of positions 349, 351, 354, 356, 364, 366, 368, 394, 405, 409, 411, and 439.

In some embodiments, provided is the method for preparing a heteromultimer according to any one of the foregoing, wherein the CH3 domains of the first polypeptide and the second polypeptide each comprise one or more mutations that promote heterologous formation. In some embodiments, the mutations that promote heterologous formation refer to a class of amino acid mutations that can promote the dissociation of a homomer (e.g., a homodimer) into monomers and/or promote the formation of a heteromultimer (e.g., a heterodimer) from two heteromonomers. In some embodiments, the mutations that promote heterologous formation are amino acid mutations at a CH3 interaction interface. In some embodiments, the mutations that promote heterologous formation are charged amino acid mutations at a CH3 interaction interface. In some embodiments, the mutations that promote heterologous formation are oppositely charged amino acid mutations at a CH3 interaction interface. In some embodiments, a hydrogen bond, an electrostatic interaction, or a salt bridge is formed between the CH3 domains of the first polypeptide and the second polypeptide.

In some embodiments, the mutations that promote heterologous formation are any one of the mutations described in the present disclosure. In some embodiments, the mutations that promote heterologous formation are one or more amino acid mutations selected from the group consisting of positions 347, 349, 351, 354, 356, 357, 364, 366, 368, 394, 405, 407, 409, 411, and 439. In some embodiments, the mutations that promote heterologous formation are one or more amino acid mutations selected from the group consisting of positions 349, 351, 354, 356, 364, 366, 368, 394, 405, 409, 411, and 439.

In some embodiments, provided is the method for preparing a heteromultimer according to any one of the foregoing, wherein the CH3 domain(s) of the first polypeptide and/or the second polypeptide comprise(s) at least one amino acid mutation selected from the group consisting of the following combinations:
the amino acid at position 356 is mutated into Lys (K), Arg (R), or His (H); and/or
the amino acid at position 439 is mutated into Glu (E) or Asp (D); and/or
the amino acid at position 349 is mutated into Leu (L), Phe (F), Ser (S), Cys (C), Ala (A), Val (V), Thr (T), or Gly (G); and/or
the amino acid at position 351 is mutated into Cys (C), Val (V), Thr (T), Ile (I), Phe (F), or Met (M); and/or
the amino acid at position 364 is mutated into Ala (A), Val (V), Thr (T), Leu (L), Tyr (Y), or Phe (F); and/or
the amino acid at position 366 is mutated into Gly (G), Ser (S), Ala (A), Val (V), Leu (L), His (H), or Ile (I); and/or
the amino acid at position 368 is mutated into Val (V), Ile (I), Met (M), or Ala (A); and/or
the amino acid at position 394 is mutated into Phe (F), Ala (A), Ser (S), Cys (C), Val (V), or Asn (N); and/or
the amino acid at position 405 is mutated into Thr (T), Leu (L), or Tyr (Y); and/or
the amino acid at position 407 is mutated into Cys (C), Val (V), Leu (L), His (H), or Phe (F); and/or
the amino acid at position 409 is mutated into Gln (Q), Arg (R), or Asp (D); and/or
the amino acid at position 411 is mutated into Asn (N), Tyr (Y), or Leu (L); and/or
the amino acid at position 354 is mutated into Tyr (Y), Cys (C), Phe (F), or Trp (W);
   and/or
the amino acid at position 357 is mutated into Cys (C); and/or
the amino acid at position 347 is mutated into Glu (E).

In some embodiments, provided is the method for preparing a heteromultimer according to any one of the foregoing, wherein the CH3 domain of the first polypeptide comprises an amino acid mutation selected from the group consisting of the following (a1) to (c2):
(a1) amino acid residues at positions 356 and 351 (EU numbering),
(a2) amino acid residues at positions 356 and 364 (EU numbering),
(a3) amino acid residues at positions 356 and 366 (EU numbering),
(a4) amino acid residues at positions 356 and 368 (EU numbering),
(a5) amino acid residues at positions 356 and 394 (EU numbering),
(a6) amino acid residues at positions 356 and 405 (EU numbering),
(a7) amino acid residues at positions 356 and 409 (EU numbering),
(a8) amino acid residues at positions 356 and 411 (EU numbering),
(b1) amino acid residues at positions 356 and 349 (EU numbering),
(c1) an amino acid residue at position 405 (EU numbering), or
(c2) an amino acid residue at position 349 (EU numbering);
the CH3 domain of the second polypeptide comprises an amino acid mutation selected from the group consisting of the following (d1) to (i2):
   (d1) amino acid residues at positions 439 and 351 (EU numbering),
   (d2) amino acid residues at positions 439 and 364 (EU numbering),
   (d3) amino acid residues at positions 439 and 366 (EU numbering),
   (d4) amino acid residues at positions 439 and 368 (EU numbering),
   (d5) amino acid residues at positions 439 and 394 (EU numbering),
   (d6) amino acid residues at positions 439 and 405 (EU numbering),
   (d7) amino acid residues at positions 439 and 409 (EU numbering),
   (d8) amino acid residues at positions 439 and 411 (EU numbering),
   (e1) amino acid residues at positions 439 and 354 (EU numbering),
   (f1) amino acid residues at positions 439, 354, and 351 (EU numbering),
   (f2) amino acid residues at positions 439, 354, and 366 (EU numbering),
   (f3) amino acid residues at positions 439, 354, and 368 (EU numbering),
   (g1) an amino acid residue at position 394 (EU numbering),
   (h) amino acid residues at positions 354 and 364 (EU numbering),
   (i1) an amino acid residue at position 364 (EU numbering), or
   (i2) an amino acid residue at position 354 (EU numbering).

In some embodiments, provided is the method for preparing a heteromultimer according to any one of the foregoing, wherein the CH3 domain of the first polypeptide comprises an amino acid mutation selected from the group consisting of the following (a1) to (c2):
(a1) amino acid residues at positions 356 and 351 (EU numbering),
(a5) amino acid residues at positions 356 and 394 (EU numbering),
(a8) amino acid residues at positions 356 and 411 (EU numbering),
(b1) amino acid residues at positions 356 and 349 (EU numbering), or
(c2) an amino acid residue at position 349 (EU numbering);
the CH3 domain of the second polypeptide comprises an amino acid mutation selected from the group consisting of the following (d1) to (i1):
   (d1) amino acid residues at positions 439 and 351 (EU numbering),
   (d5) amino acid residues at positions 439 and 394 (EU numbering),
   (d8) amino acid residues at positions 439 and 411 (EU numbering),
   (f1) amino acid residues at positions 439, 354, and 351 (EU numbering),
   (h) amino acid residues at positions 354 and 364 (EU numbering), or
   (i1) an amino acid residue at position 364 (EU numbering).

In some embodiments, provided is the method for preparing a heteromultimer according to any one of the foregoing, wherein the CH3 domain of the first polypeptide comprises an amino acid mutation selected from the group consisting of the following (a1) to (c2):
(a1) amino acid residues at positions 356 and 351 (EU numbering),
(b1) amino acid residues at positions 356 and 349 (EU numbering), or
(c2) an amino acid residue at position 349 (EU numbering);
the CH3 domain of the second polypeptide comprises an amino acid mutation selected from the group consisting of the following (d1) to (i1):
   (d1) amino acid residues at positions 439 and 351 (EU numbering),
   (f1) amino acid residues at positions 439, 354, and 351 (EU numbering),
   (h) amino acid residues at positions 354 and 364 (EU numbering), or
   (i1) an amino acid residue at position 364 (EU numbering).

In some embodiments, provided is the method for preparing a heteromultimer according to any one of the foregoing, wherein the CH3 domain of the first polypeptide comprises an amino acid mutation selected from the group consisting of the following (a1) to (c2):
(a1) amino acid residues of 356K and 351I (EU numbering), or
amino acid residues of 356K and 351T (EU numbering),
(a2) amino acid residues of 356K and 364A (EU numbering),
(a3) amino acid residues of 356K and 366A (EU numbering),
(a4) amino acid residues of 356K and 368I (EU numbering),
(a5) amino acid residues of 356K and 394A (EU numbering), or
amino acid residues of 356K and 394S (EU numbering),
(a6) amino acid residues of 356K and 405Y (EU numbering),
(a7) amino acid residues of 356K and 409Q (EU numbering),
(a8) amino acid residues of 356K and 411Y (EU numbering),
(b1) amino acid residues of 356K and 349C (EU numbering), or
amino acid residues of 356K and 349S (EU numbering),
(c1) an amino acid residue of 405T (EU numbering), or
(c2) an amino acid residue of 349C, 349S, or 349G (EU numbering);
the CH3 domain of the second polypeptide comprises an amino acid mutation selected from the group consisting of the following (d1) to (i2):
   (d1) amino acid residues of 439E and 351I (EU numbering), or
   amino acid residues of 439E and 351T (EU numbering),
   (d2) amino acid residues of 439E and 364A (EU numbering),
   (d3) amino acid residues of 439E and 366A (EU numbering),
   (d4) amino acid residues of 439E and 368I (EU numbering),
   (d5) amino acid residues of 439E and 394A (EU numbering), or
   amino acid residues of 439E and 394S (EU numbering),
   (d6) amino acid residues of 439E and 405Y (EU numbering),
   (d7) amino acid residues of 439E and 409Q (EU numbering),
   (d8) amino acid residues of 439E and 411Y (EU numbering),
   (e1) amino acid residues of 439E and 354Y (EU numbering),
   (f1) amino acid residues of 439E, 354C, and 351I (EU numbering),
   (f2) amino acid residues of 439E, 354C, and 366A (EU numbering),
   (f3) amino acid residues of 439E, 354C, and 368I (EU numbering),
   (g1) an amino acid residue of 394F (EU numbering),
   (h) amino acid residues of 354C and 364Y (EU numbering), or
   amino acid residues of 354C and 364F (EU numbering),
   (i1) an amino acid residue of 364Y (EU numbering), or
   (i2) an amino acid residue of 354W (EU numbering).

In some embodiments, provided is the method for preparing a heteromultimer according to any one of the foregoing, wherein the CH3 domain of the first polypeptide comprises an amino acid mutation selected from the group consisting of the following (a1) to (c2):
(a1) amino acid residues of 356K and 351I (EU numbering),
(a5) amino acid residues of 356K and 394S (EU numbering),
(a8) amino acid residues of 356K and 411Y (EU numbering),
(b1) amino acid residues of 356K and 349C (EU numbering), or
(c2) an amino acid residue of 349S or 349C (EU numbering);
the CH3 domain of the second polypeptide comprises an amino acid mutation selected from the group consisting of the following (d1) to (i1):
   (d1) amino acid residues of 439E and 351I (EU numbering),
   (d5) amino acid residues of 439E and 394S (EU numbering),
   (d8) amino acid residues of 439E and 411Y (EU numbering),
   (f1) amino acid residues of 439E, 354C, and 351I (EU numbering),
   (h) amino acid residues of 354C and 364Y (EU numbering), or
   amino acid residues of 354C and 364F (EU numbering),
   (i1) an amino acid residue of 364Y (EU numbering).

In some embodiments, provided is the method for preparing a heteromultimer according to any one of the foregoing, wherein the CH3 domain of the first polypeptide comprises an amino acid mutation selected from the group consisting of the following (a1) to (c2):
(a1) amino acid residues of 356K and 351I (EU numbering),
(a5) amino acid residues of 356K and 349C (EU numbering), or
(c2) an amino acid residue of 349S or 349C (EU numbering);
the CH3 domain of the second polypeptide comprises an amino acid mutation selected from the group consisting of the following (d1) to (i1):
   (d1) amino acid residues of 439E and 351I (EU numbering),
   (f1) amino acid residues of 439E, 354C, and 351I (EU numbering),
   (h) amino acid residues of 354C and 364Y (EU numbering), or
   (i1) an amino acid residue of 364Y (EU numbering).

### Preparation Method for Heteromultimer Comprising First-Class Mutation Combination

In some embodiments, provided is the method for preparing a heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 349, 351, 364, 366, 368, 394, 405, 407, 409, and 411.

In some embodiments, provided is the method for preparing a heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical amino acid mutation selected from the group consisting of positions 351, 364, 366, 368, 394, 405, 409, and 411.

In some embodiments, provided is the method for preparing a heteromultimer according to any one of the foregoing, wherein the CH3 domain of the first polypeptide comprises amino acid mutations at positions 356 and 351, and the CH3 domain of the second polypeptide comprises amino acid mutations at positions 439 and 351.

In some embodiments, provided is the method for preparing a heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of 349L, 349F, 351C, 351V, 351T, 351I, 351F, 351M, 364A, 364V, 364T, 364L, 366G, 366S, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411N, 411L, and 411Y.

In some embodiments, provided is the method for preparing a heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of 349L, 351C, 351V, 351T, 351I, 351F, 351M, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y.

In some embodiments, provided is the method for preparing a heteromultimer according to any one of the foregoing, wherein the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of 349L, 351C, 351V, 351T, 351I, 351F, 351M, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y.

In some embodiments, provided is the method for preparing a heteromultimer according to any one of the foregoing, wherein:
the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of positions 351, 364, 366, 368, 394, 405, 409, and 411, wherein:
the amino acid at position 351 (EU numbering) is mutated into Cys (C), Val (V), Thr (T), Ile (I), Phe (F), or Met (M); or
the amino acid at position 364 (EU numbering) is mutated into Ala (A), Val (V), Thr (T), or Leu (L); or
the amino acid at position 366 (EU numbering) is mutated into Ser (S), Ala (A), Val (V), Leu (L), His (H), or Ile (I); or
the amino acid at position 368 (EU numbering) is mutated into Val (V), Ile (I), Met (M), or Ala (A); or
the amino acid at position 394 (EU numbering) is mutated into Ala (A), Ser (S), Cys (C), Val (V), or Asn (N); or
the amino acid at position 405 (EU numbering) is mutated into Leu (L) or Tyr (Y); or
the amino acid at position 409 (EU numbering) is mutated into Gln (Q); or
the amino acid at position 411 (EU numbering) is mutated into Asn (N), Tyr (Y), or Leu (L).

In some embodiments, provided is the method for preparing a heteromultimer according to any one of the foregoing, wherein:
the CH3 domain of the first polypeptide comprises an amino acid mutation of 356K, and
the CH3 domain of the second polypeptide comprises an amino acid mutation of 439E,
wherein the CH3 domains of the first polypeptide and the second polypeptide each further comprise one identical amino acid mutation selected from the group consisting of positions 351, 364, 366, 368, 394, 405, 409, and 411, wherein:
   position 351 (EU numbering) is mutated into Cys (C), Val (V), Thr (T), Ile (I), or Phe (F); or
   position 364 (EU numbering) is mutated into Ala (A), Val (V), Thr (T), or Leu (L); or
   position 366 (EU numbering) is mutated into Ser (S), Ala (A), Val (V), Leu (L), or His (H); or
   position 368 (EU numbering) is mutated into Val (V), Ile (I), or Met (M); or
   position 394 (EU numbering) is mutated into Ala (A), Ser (S), Cys (C), Val (V), or Asn (N); or
   position 405 (EU numbering) is mutated into Leu (L) or Tyr (Y); or
   position 409 (EU numbering) is mutated into Gln (Q) or Arg (R); or
   position 411 (EU numbering) is mutated into Asn (N) or Tyr (Y).

In some embodiments, provided is the method for preparing a heteromultimer according to any one of the foregoing, wherein the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351T, 351I, 364A, 366A, 368I, 394A, 394S, 405Y, 409Q, and 411Y.

In some embodiments, provided is the method for preparing a heteromultimer according to any one of the foregoing, wherein the CH3 domain of the first polypeptide comprises amino acid mutations of 356K and 351I, and the CH3 domain of the second polypeptide comprises amino acid mutations of 439E and 351I.

In some embodiments, provided is the method for preparing a heteromultimer according to any one of the foregoing, wherein:
the CH3 domain of the first polypeptide comprises amino acid mutations of D/E356K and Y349L, and the CH3 domain of the second polypeptide comprises amino acid mutations of K439E and Y349L; or
the CH3 domain of the first polypeptide comprises amino acid mutations of D/E356K and L351C, and the CH3 domain of the second polypeptide comprises amino acid mutations of K439E and L351C; or
the CH3 domain of the first polypeptide comprises amino acid mutations of D/E356K and L351V, and the CH3 domain of the second polypeptide comprises amino acid mutations of K439E and L351V; or
the CH3 domain of the first polypeptide comprises amino acid mutations of D/E356K and L351T, and the CH3 domain of the second polypeptide comprises amino acid mutations of K439E and L351T; or
the CH3 domain of the first polypeptide comprises amino acid mutations of D/E356K and L351I, and the CH3 domain of the second polypeptide comprises amino acid mutations of K439E and L351I; or
the CH3 domain of the first polypeptide comprises amino acid mutations of D/E356K and L351F, and the CH3 domain of the second polypeptide comprises amino acid mutations of K439E and L351F; or
the CH3 domain of the first polypeptide comprises amino acid mutations of D/E356K and L351M, and the CH3 domain of the second polypeptide comprises amino acid mutations of K439E and L351M; or
the CH3 domain of the first polypeptide comprises amino acid mutations of D/E356K and S364A, and the CH3 domain of the second polypeptide comprises amino acid mutations of K439E and S364A; or
the CH3 domain of the first polypeptide comprises amino acid mutations of D/E356K and S364T, and the CH3 domain of the second polypeptide comprises amino acid mutations of K439E and S364T; or
the CH3 domain of the first polypeptide comprises amino acid mutations of D/E356K and S364L, and the CH3 domain of the second polypeptide comprises amino acid mutations of K439E and S364L; or
the CH3 domain of the first polypeptide comprises amino acid mutations of D/E356K and T366G, and the CH3 domain of the second polypeptide comprises amino acid mutations of K439E and T366G; or
the CH3 domain of the first polypeptide comprises amino acid mutations of D/E356K and T366A, and the CH3 domain of the second polypeptide comprises amino acid mutations of K439E and T366A; or
the CH3 domain of the first polypeptide comprises amino acid mutations of D/E356K and T366V, and the CH3 domain of the second polypeptide comprises amino acid mutations of K439E and T366V; or
the CH3 domain of the first polypeptide comprises amino acid mutations of D/E356K and T366L, and the CH3 domain of the second polypeptide comprises amino acid mutations of K439E and T366L; or
the CH3 domain of the first polypeptide comprises amino acid mutations of D/E356K and T366H, and the CH3 domain of the second polypeptide comprises amino acid mutations of K439E and T366H; or
the CH3 domain of the first polypeptide comprises amino acid mutations of D/E356K and T366I, and the CH3 domain of the second polypeptide comprises amino acid mutations of K439E and T366I; or
the CH3 domain of the first polypeptide comprises amino acid mutations of D/E356K and L368V, and the CH3 domain of the second polypeptide comprises amino acid mutations of K439E and L368V; or
the CH3 domain of the first polypeptide comprises amino acid mutations of D/E356K and L368I, and the CH3 domain of the second polypeptide comprises amino acid mutations of K439E and L368I; or
the CH3 domain of the first polypeptide comprises amino acid mutations of D/E356K and L368A, and the CH3 domain of the second polypeptide comprises amino acid mutations of K439E and L368A; or
the CH3 domain of the first polypeptide comprises amino acid mutations of D/E356K and L368A, and the CH3 domain of the second polypeptide comprises amino acid mutations of K439E and L368A; or
the CH3 domain of the first polypeptide comprises amino acid mutations of D/E356K and T394A, and the CH3 domain of the second polypeptide comprises amino acid mutations of K439E and T394A; or
the CH3 domain of the first polypeptide comprises amino acid mutations of D/E356K and T394S, and the CH3 domain of the second polypeptide comprises amino acid mutations of K439E and T394S; or
the CH3 domain of the first polypeptide comprises amino acid mutations of D/E356K and T394C, and the CH3 domain of the second polypeptide comprises amino acid mutations of K439E and T394C; or
the CH3 domain of the first polypeptide comprises amino acid mutations of D/E356K and T394V, and the CH3 domain of the second polypeptide comprises amino acid mutations of K439E and T394V; or
the CH3 domain of the first polypeptide comprises amino acid mutations of D/E356K and T394N, and the CH3 domain of the second polypeptide comprises amino acid mutations of K439E and T394N; or
the CH3 domain of the first polypeptide comprises amino acid mutations of D/E356K and F405L, and the CH3 domain of the second polypeptide comprises amino acid mutations of K439E and F405L; or
the CH3 domain of the first polypeptide comprises amino acid mutations of D/E356K and F405Y, and the CH3 domain of the second polypeptide comprises amino acid mutations of K439E and F405Y; or
the CH3 domain of the first polypeptide comprises amino acid mutations of D/E356K and Y407C, and the CH3 domain of the second polypeptide comprises amino acid mutations of K439E and Y407C; or
the CH3 domain of the first polypeptide comprises amino acid mutations of D/E356K and Y407V, and the CH3 domain of the second polypeptide comprises amino acid mutations of K439E and Y407V; or
the CH3 domain of the first polypeptide comprises amino acid mutations of D/E356K and Y407L, and the CH3 domain of the second polypeptide comprises amino acid mutations of K439E and Y407L; or
the CH3 domain of the first polypeptide comprises amino acid mutations of D/E356K and Y407H, and the CH3 domain of the second polypeptide comprises amino acid mutations of K439E and Y407H; or
the CH3 domain of the first polypeptide comprises amino acid mutations of D/E356K and Y407F, and the CH3 domain of the second polypeptide comprises amino acid mutations of K439E and Y407F; or
the CH3 domain of the first polypeptide comprises amino acid mutations of D/E356K and K409Q, and the CH3 domain of the second polypeptide comprises amino acid mutations of K439E and K409Q; or
the CH3 domain of the first polypeptide comprises amino acid mutations of D/E356K and K409R, and the CH3 domain of the second polypeptide comprises amino acid mutations of K439E and K409R; or
the CH3 domain of the first polypeptide comprises amino acid mutations of D/E356K and T411L, and the CH3 domain of the second polypeptide comprises amino acid mutations of K439E and T411L; or
the CH3 domain of the first polypeptide comprises amino acid mutations of D/E356K and T411Y, and the CH3 domain of the second polypeptide comprises amino acid mutations of K439E and T411Y.

The present disclosure provides a method for preparing a heteromultimer, comprising the following steps:
a) a step of providing a molecule comprising a first polypeptide homomer, wherein the first polypeptide comprises one CH3 domain, and the CH3 domain of the first polypeptide comprises amino acid mutations of 356K and 351I;
b) a step of providing a molecule comprising a second polypeptide homomer, wherein the second polypeptide comprises one CH3 domain, and the CH3 domain of the second polypeptide comprises amino acid mutations of 439E and 351I; and
c) a step of co-incubating the molecule comprising the first polypeptide homomer and the molecule comprising the second polypeptide homomer under reducing conditions,
wherein mutation sites in the CH3 domains are indicated by EU numbering.

In some embodiments, provided is the method for preparing a heteromultimer according to any one of the foregoing, wherein the CH3 domains of the first polypeptide and the second polypeptide each comprise one charged amino acid mutation (e.g., a positively charged amino acid or a negatively charged amino acid), and the amino acid mutation in the CH3 domain of the first polypeptide and the amino acid mutation in the CH3 domain of the second polypeptide are oppositely charged. Further, the CH3 domains of the first polypeptide and the second polypeptide each comprise one identical amino acid mutation, and the mutations are located at a CH3 interaction interface and can weaken the interaction between the CH3 domains of the first polypeptide homomer and/or the second polypeptide homomer, such that the first polypeptide homomer and/or the second polypeptide homomer more easily dissociate(s) into first polypeptide monomers and second polypeptide monomers under reducing conditions, thereby promoting the production of a heteromultimer comprising one first polypeptide and one second polypeptide.

### Preparation Method for Heterodimer Comprising Second-Class Mutation Combination

In some embodiments, provided is the method for preparing a heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to iv):
i) 349;
ii) 354 and 351;
iii) 354 and 366; and
iv) 354 and 368.

In some embodiments, provided is the method for preparing a heteromultimer according to any one of the foregoing, wherein the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368.

In some embodiments, provided is the method for preparing a heteromultimer according to any one of the foregoing, wherein the CH3 domain of the first polypeptide comprises amino acid mutations at positions 356 and 349, and the CH3 domain of the second polypeptide comprises amino acid mutations at positions 439, 354, and 351.

In some embodiments, provided is the method for preparing a heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to iv):
i) 349C;
ii) 354C and 351I;
iii) 354C and 366A; and
iv) 354C and 368I.

In some embodiments, provided is the method for preparing a heteromultimer according to any one of the foregoing, wherein the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I.

In some embodiments, provided is the method for preparing a heteromultimer according to any one of the foregoing, wherein the CH3 domain of the first polypeptide comprises amino acid mutations of 356K and 349C, and the CH3 domain of the second polypeptide comprises amino acid mutations of 439E, 354C, and 351I.

In some embodiments, provided is the method for preparing a heteromultimer according to any one of the foregoing, wherein:
the CH3 domain of the first polypeptide comprises amino acid mutations of D/E356K and 349C, and the CH3 domain of the second polypeptide comprises amino acid mutations of K439E, S354C, and L351I; or
the CH3 domain of the first polypeptide comprises amino acid mutations of D/E356K and Y349C, and the CH3 domain of the second polypeptide comprises amino acid mutations of K439E, S354C, and T366A; or
the CH3 domain of the first polypeptide comprises amino acid mutations of D/E356K and Y349C, and the CH3 domain of the second polypeptide comprises amino acid mutations of K439E, S354C, and L368I.

The present disclosure provides a method for preparing a heteromultimer, comprising the following steps:
a) a step of providing a molecule comprising a first polypeptide homomer, wherein the first polypeptide comprises one CH3 domain, and the CH3 domain of the first polypeptide comprises amino acid mutations of 356K and 349C;
b) a step of providing a molecule comprising a second polypeptide homomer, wherein the second polypeptide comprises one CH3 domain, and the CH3 domain of the second polypeptide comprises amino acid mutations of 439E, 354C, and 351I; and
c) a step of co-incubating the molecule comprising the first polypeptide homomer and the molecule comprising the second polypeptide homomer under reducing conditions,
wherein mutation sites in the CH3 domains are indicated by EU numbering.

In some embodiments, provided is the method for preparing a heteromultimer according to any one of the foregoing, wherein the CH3 domains of the first polypeptide and the second polypeptide each comprise one charged amino acid mutation (e.g., a positively charged amino acid or a negatively charged amino acid), and the amino acid mutation in the CH3 domain of the first polypeptide and the amino acid mutation in the CH3 domain of the second polypeptide are oppositely charged. Further, the CH3 domains of the first polypeptide and the second polypeptide each further comprise one cysteine mutation, so that a disulfide bond can be formed. Furthermore, the CH3 domain of the first polypeptide or the second polypeptide comprises one amino acid mutation, and the mutation is located at a CH3 interaction interface and can weaken the interaction between the CH3 domains of the first polypeptide homomer or the second polypeptide homomer, such that the first polypeptide homomer or the second polypeptide homomer more easily dissociates into first polypeptide monomers and second polypeptide monomers under reducing conditions, thereby promoting the production of a heteromultimer comprising one first polypeptide and one second polypeptide.

### Preparation Method for Heteromultimer Comprising Third-Class Mutation Combination

In some embodiments, provided is the method for preparing a heteromultimer according to any one of the foregoing, wherein:
the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394; or
the CH3 domain of the first polypeptide comprises an amino acid mutation at position 366, and the CH3 domain of the second polypeptide comprises an amino acid mutation at position 405 or 407.

In some embodiments, provided is the method for preparing a heteromultimer according to any one of the foregoing, wherein:
the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394.

In some embodiments, provided is the method for preparing a heteromultimer according to any one of the foregoing, wherein the CH3 domain of the first polypeptide comprises an amino acid mutation at position 349, and the CH3 domain of the second polypeptide comprises an amino acid mutation at position 364.

In some embodiments, provided is the method for preparing a heteromultimer according to any one of the foregoing, wherein:
the CH3 domain of the first polypeptide comprises an amino acid mutation of 349S, and the CH3 domain of the second polypeptide comprises one amino acid mutation selected from the group consisting of 364Y, 354F, 354W, and 354Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 349A, and the CH3 domain of the second polypeptide comprises one amino acid mutation selected from the group consisting of 354F, 354Y, and 354W; or
the CH3 domain of the first polypeptide comprises an amino acid mutation of 349V, and the CH3 domain of the second polypeptide comprises an amino acid mutation of 354F; or
the CH3 domain of the first polypeptide comprises an amino acid mutation of 349T, and the CH3 domain of the second polypeptide comprises an amino acid mutation of 354F; or
the CH3 domain of the first polypeptide comprises an amino acid mutation of 405T, and the CH3 domain of the second polypeptide comprises an amino acid mutation of 394F; or
the CH3 domain of the first polypeptide comprises an amino acid mutation of 366H, and the CH3 domain of the second polypeptide comprises an amino acid mutation of 405L or 407L.

In some embodiments, provided is the method for preparing a heteromultimer according to any one of the foregoing, wherein:
the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 405T, and the CH3 domain of the second polypeptide comprises an amino acid mutation of 394F.

In some embodiments, provided is the method for preparing a heteromultimer according to any one of the foregoing, wherein the CH3 domain of the first polypeptide comprises an amino acid mutation of 349S, and the CH3 domain of the second polypeptide comprises an amino acid mutation of 364Y.

The present disclosure provides a method for preparing a heteromultimer, comprising the following steps:
a) a step of providing a molecule comprising a first polypeptide homomer, wherein the first polypeptide comprises one CH3 domain, and the CH3 domain of the first polypeptide comprises an amino acid mutation of 349S;
b) a step of providing a molecule comprising a second polypeptide homomer, wherein the second polypeptide comprises one CH3 domain, and the CH3 domain of the second polypeptide comprises an amino acid mutation of 364Y; and
c) a step of co-incubating the molecule comprising the first polypeptide homomer and the molecule comprising the second polypeptide homomer under reducing conditions,
wherein mutation sites in the CH3 domains are indicated by EU numbering.

In some embodiments, provided is the method for preparing a heteromultimer according to any one of the foregoing, wherein:
the CH3 domain of the first polypeptide comprises one amino acid mutation that is a mutation of a small-volume amino acid residue into a large-volume amino acid residue, which can produce a protuberance in the CH3 interface, and the CH3 domain of the second polypeptide comprises one amino acid mutation that is a mutation of a large-volume amino acid residue into a small-volume amino acid residue, which can produce a cavity in the CH3 interface; consequently, the protuberance and the cavity promote the production of a heteromultimer comprising one first polypeptide and one second polypeptide; or
the CH3 domain of the first polypeptide comprises one amino acid mutation that is a mutation of a large-volume amino acid residue into a small-volume amino acid residue, which can produce a cavity in the CH3 interface, and the CH3 domain of the second polypeptide comprises one amino acid mutation that is a mutation of a small-volume amino acid residue into a large-volume amino acid residue, which can produce a protuberance in the CH3 interface; consequently, the cavity and the protuberance promote the production of a heteromultimer comprising one first polypeptide and one second polypeptide.

### Preparation Method for Heteromultimer Comprising Fourth-Class Mutation Combination

In some embodiments, provided is the method for preparing a heteromultimer according to any one of the foregoing, wherein:
the CH3 domain of the first polypeptide comprises an amino acid mutation at position 349, and the CH3 domain of the second polypeptide comprises an amino acid mutation at position 354, wherein the CH3 domain of the second polypeptide further comprises one amino acid mutation selected from the group consisting of positions 351, 364, 366, 368, and 405; or
the CH3 domain of the first polypeptide comprises an amino acid mutation at position 349, and the CH3 domain of the second polypeptide comprises amino acid mutations at positions 347 and 357.

In some embodiments, provided is the method for preparing a heteromultimer according to any one of the foregoing, wherein the CH3 domain of the first polypeptide comprises an amino acid mutation at position 349, and the CH3 domain of the second polypeptide comprises amino acid mutations at positions 354 and 364.

In some embodiments, provided is the method for preparing a heteromultimer according to any one of the foregoing, wherein:
the CH3 domain of the first polypeptide comprises an amino acid mutation of 349C, and the CH3 domain of the second polypeptide comprises an amino acid mutation of 354C, wherein the CH3 domain of the second polypeptide further comprises one amino acid mutation selected from the group consisting of 351I, 364Y, 364F, 366A, 368I, and 405Y; or
the CH3 domain of the first polypeptide comprises an amino acid mutation of 349C, and the CH3 domain of the second polypeptide comprises amino acid mutations of 347E and 357C.

In some embodiments, provided is the method for preparing a heteromultimer according to any one of the foregoing, wherein the CH3 domain of the first polypeptide comprises an amino acid mutation of 349C, and the CH3 domain of the second polypeptide comprises an amino acid mutation of 354C, wherein the CH3 domain of the second polypeptide further comprises an amino acid mutation of 364Y or 364F.

In some embodiments, provided is the method for preparing a heteromultimer according to any one of the foregoing, wherein the CH3 domain of the first polypeptide comprises an amino acid mutation of 349C, and the CH3 domain of the second polypeptide comprises amino acid mutations of 354C and 364Y.

The present disclosure provides a method for preparing a heteromultimer, comprising the following steps:
a) a step of providing a molecule comprising a first polypeptide homomer, wherein the first polypeptide comprises one CH3 domain, and the CH3 domain of the first polypeptide comprises an amino acid mutation of 349C;
b) a step of providing a molecule comprising a second polypeptide homomer, wherein the second polypeptide comprises one CH3 domain, and the CH3 domain of the second polypeptide comprises amino acid mutations of 354C and 364Y; and
c) a step of co-incubating the molecule comprising the first polypeptide homomer and the molecule comprising the second polypeptide homomer under reducing conditions,
wherein mutation sites in the CH3 domains are indicated by EU numbering.

In some embodiments, provided is the method for preparing a heteromultimer according to any one of the foregoing, wherein the CH3 domains of the first polypeptide and the second polypeptide each comprise one cysteine mutation, so that a disulfide bond can be formed; further, the CH3 domain of the first polypeptide or the second polypeptide comprises one amino acid mutation, wherein the mutation is located at a CH3 interaction interface and can weaken the interaction between the CH3 domains of the first polypeptide homomer and/or the second polypeptide homomer, such that the first polypeptide homomer and/or the second polypeptide homomer more easily dissociate(s) into first polypeptide monomers and second polypeptide monomers under reducing conditions, thereby promoting the production of a heteromultimer comprising one first polypeptide and one second polypeptide.

### Preparation Method for Heteromultimer Comprising Fifth-Class Mutation Combination

In some embodiments, provided is the method for preparing a heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to vii):
i) 349;
ii) 354;
iii) 357;
iv) 366;
v) 394;
vi) 405; and
vii) 407.

In some embodiments, provided is the method for preparing a heteromultimer according to any one of the foregoing, wherein:
the CH3 domain of the first polypeptide comprises amino acid mutations at positions 356 and 349, and the CH3 domain of the second polypeptide comprises an amino acid mutation at position 439, wherein the CH3 domain of the second polypeptide further comprises an amino acid mutation at position 354 or 357; or
the CH3 domain of the first polypeptide comprises amino acid mutations at positions 356 and 405, and the CH3 domain of the second polypeptide comprises amino acid mutations at positions 439 and 394; or
the CH3 domain of the first polypeptide comprises amino acid mutations at positions 356 and 366, and the CH3 domain of the second polypeptide comprises an amino acid mutation at position 439, wherein the CH3 domain of the second polypeptide further comprises an amino acid mutation at position 405 or 407; or
the CH3 domain of the first polypeptide comprises amino acid mutations at positions 356 and 407, and the CH3 domain of the second polypeptide comprises amino acid mutations at positions 439 and 366.

In some embodiments, provided is the method for preparing a heteromultimer according to any one of the foregoing, wherein the CH3 domain of the first polypeptide comprises amino acid mutations at positions 356 and 349, and the CH3 domain of the second polypeptide comprises amino acid mutations at positions 439 and 354.

In some embodiments, provided is the method for preparing a heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to vii):
i) 349S or 349C;
ii) 354Y or 354C;
iii) 357C;
iv) 366H;
v) 394F;
vi) 405T or 405L; and
vii) 407L or 407H.

In some embodiments, provided is the method for preparing a heteromultimer according to any one of the foregoing, wherein:
the CH3 domain of the first polypeptide comprises amino acid mutations of 356K and 349S, and the CH3 domain of the second polypeptide comprises amino acid mutations of 439E and 354Y; or
the CH3 domain of the first polypeptide comprises amino acid mutations of 356K and 349C, and the CH3 domain of the second polypeptide comprises amino acid mutations of 439E and 354C; or
the CH3 domain of the first polypeptide comprises amino acid mutations of 356K and 349C, and the CH3 domain of the second polypeptide comprises amino acid mutations of 439E and 357C; or
the CH3 domain of the first polypeptide comprises amino acid mutations of 356K and 405T, and the CH3 domain of the second polypeptide comprises amino acid mutations of 439E and 394F; or
the CH3 domain of the first polypeptide comprises amino acid mutations of 356K and 366H, and the CH3 domain of the second polypeptide comprises an amino acid mutation of 439E, wherein the CH3 domain of the second polypeptide further comprises one amino acid mutation selected from the group consisting of 405L, 407L, and 407H; or
the CH3 domain of the first polypeptide comprises amino acid mutations of 356K and 407L, and the CH3 domain of the second polypeptide comprises amino acid mutations of 439E and 366H; or
the CH3 domain of the first polypeptide comprises amino acid mutations of 356K and 407H, and the CH3 domain of the second polypeptide comprises amino acid mutations of 439E and 366H.

In some embodiments, provided is the method for preparing a heteromultimer according to any one of the foregoing, wherein the CH3 domain of the first polypeptide comprises amino acid mutations of 356K and 349S, and the CH3 domain of the second polypeptide comprises amino acid mutations of 439E and 354Y.

In some embodiments, provided is the method for preparing a heteromultimer according to any one of the foregoing, wherein:
the CH3 domain of the first polypeptide comprises amino acid mutations of D/E356K and Y349S, and the CH3 domain of the second polypeptide comprises amino acid mutations of K439E and S354Y; or
the CH3 domain of the first polypeptide comprises amino acid mutations of D/E356K and Y349C, and the CH3 domain of the second polypeptide comprises amino acid mutations of K439E and S354C; or
the CH3 domain of the first polypeptide comprises amino acid mutations of D/E356K and Y349C, and the CH3 domain of the second polypeptide comprises amino acid mutations of K439E and E357C; or
the CH3 domain of the first polypeptide comprises amino acid mutations of D/E356K and F405T, and the CH3 domain of the second polypeptide comprises amino acid mutations of K439E and T394F; or
the CH3 domain of the first polypeptide comprises amino acid mutations of D/E356K and T366H, and the CH3 domain of the second polypeptide comprises amino acid mutations of K439E and F405L; or
the CH3 domain of the first polypeptide comprises amino acid mutations of D/E356K and T366H, and the CH3 domain of the second polypeptide comprises amino acid mutations of K439E and Y407L; or
the CH3 domain of the first polypeptide comprises amino acid mutations of D/E356K and T366H, and the CH3 domain of the second polypeptide comprises amino acid mutations of K439E and Y407H; or
the CH3 domain of the first polypeptide comprises amino acid mutations of D/E356K and Y407L, and the CH3 domain of the second polypeptide comprises amino acid mutations of K439E and T366H; or
the CH3 domain of the first polypeptide comprises amino acid mutations of D/E356K and Y407H, and the CH3 domain of the second polypeptide comprises amino acid mutations of K439E and T366H.

In some embodiments, provided is the method for preparing a heteromultimer according to any one of the foregoing, wherein the CH3 domains of the first polypeptide and the second polypeptide each comprise one charged amino acid mutation (e.g., a positively charged amino acid or a negatively charged amino acid), and the amino acid mutation in the CH3 domain of the first polypeptide and the amino acid mutation in the CH3 domain of the second polypeptide are oppositely charged. Further, the CH3 domains of the first polypeptide and the second polypeptide comprise amino acid mutations selected from the group consisting of any one of the following groups:
the CH3 domains of the first polypeptide and the second polypeptide each comprise one cysteine mutation, so that a disulfide bond can be formed; or
the CH3 domains of the first polypeptide and the second polypeptide each further comprise one different amino acid mutation, wherein the amino acid mutation in the CH3 domain of the first polypeptide is a mutation of a small-volume amino acid residue into a large-volume amino acid residue, which can produce a protuberance in the CH3 interface, and the amino acid mutation in the CH3 domain of the second polypeptide is a mutation of a large-volume amino acid residue into a small-volume amino acid residue, which can produce a cavity in the CH3 interface; or
the CH3 domains of the first polypeptide and the second polypeptide each comprise one cysteine mutation, so that a disulfide bond can be formed; further, the CH3 domains of the first polypeptide and the second polypeptide each comprise one different amino acid mutation, wherein the amino acid mutation in the CH3 domain of the first polypeptide is a mutation of a large-volume amino acid residue into a small-volume amino acid residue, which can produce a cavity in the CH3 interface, and the amino acid mutation in the CH3 domain of the second polypeptide is a mutation of a small-volume amino acid residue into a large-volume amino acid residue, which can produce a protuberance in the CH3 interface;
consequently, the cavity and the protuberance promote the production of a heteromultimer comprising one first polypeptide and one second polypeptide.

### Preparation Method for Heteromultimer Comprising Sixth-Class Mutation Combination

In some embodiments, provided is the method for preparing a heteromultimer according to any one of the foregoing, wherein the CH3 domain of the first polypeptide comprises an amino acid mutation at position 349, and the CH3 domain of the second polypeptide comprises an amino acid mutation at position 357.

In some embodiments, provided is the method for preparing a heteromultimer according to any one of the foregoing, wherein the CH3 domain of the first polypeptide comprises an amino acid mutation of 349C, and the CH3 domain of the second polypeptide comprises an amino acid mutation of 357C.

In some embodiments, provided is the method for preparing a heteromultimer according to any one of the foregoing, wherein the CH3 domains of the first polypeptide and the second polypeptide each comprise one cysteine mutation, so that a disulfide bond can be formed.

### Preparation Method for Seventh-Class Mutation Combination

In some embodiments, provided is the method for preparing a heteromultimer according to any one of the foregoing, wherein the CH3 domain of the first polypeptide comprises an amino acid mutation at position 354, and the CH3 domain of the second polypeptide comprises an amino acid mutation at position 349, wherein the CH3 domain(s) of the first polypeptide and/or the second polypeptide further comprise(s) at least one amino acid mutation selected from the group consisting of i) and ii):
i) 405; and
ii) 394.

In some embodiments, provided is the method for preparing a heteromultimer according to any one of the foregoing, wherein the CH3 domain of the first polypeptide comprises amino acid mutations at positions 354 and 405, and the CH3 domain of the second polypeptide comprises amino acid mutations at positions 349 and 394.

In some embodiments, provided is the method for preparing a heteromultimer according to any one of the foregoing, wherein the CH3 domain of the first polypeptide comprises an amino acid mutation of 354C, and the CH3 domain of the second polypeptide comprises an amino acid mutation of 349C, wherein the CH3 domain(s) of the first polypeptide and/or the second polypeptide further comprise(s) at least one amino acid mutation selected from the group consisting of i) and ii):
i) 405T; and
ii) 394F.

In some embodiments, provided is the method for preparing a heteromultimer according to any one of the foregoing, wherein the CH3 domain of the first polypeptide comprises amino acid mutations of 354C and 405T, and the CH3 domain of the second polypeptide comprises amino acid mutations of 349C and 394F.

In some embodiments, provided is the method for preparing a heteromultimer according to any one of the foregoing, wherein:
the CH3 domains of the first polypeptide and the second polypeptide each comprise one cysteine mutation, so that a disulfide bond can be formed; further, the CH3 domains of the first polypeptide and the second polypeptide each comprise one different amino acid mutation, wherein the amino acid mutation in the CH3 domain of the first polypeptide is a mutation of a small-volume amino acid residue into a large-volume amino acid residue, which can produce a protuberance in the CH3 interface, and the amino acid mutation in the CH3 domain of the second polypeptide is a mutation of a large-volume amino acid residue into a small-volume amino acid residue, which can produce a cavity in the CH3 interface; or
the CH3 domains of the first polypeptide and the second polypeptide each comprise one cysteine mutation, so that a disulfide bond can be formed; further, the CH3 domains of the first polypeptide and the second polypeptide each comprise one different amino acid mutation, wherein the amino acid mutation in the CH3 domain of the first polypeptide is a mutation of a large-volume amino acid residue into a small-volume amino acid residue, which can produce a cavity in the CH3 interface, and the amino acid mutation in the CH3 domain of the second polypeptide is a mutation of a small-volume amino acid residue into a large-volume amino acid residue, which can produce a protuberance in the CH3 interface;
consequently, the cavity and the protuberance promote the production of a heteromultimer comprising one first polypeptide and one second polypeptide.

In some embodiments, provided is the method for preparing a heteromultimer according to any one of the foregoing, wherein the heterodimeric interaction between the CH3 domains of the first polypeptide and the second polypeptide is stronger than the respective CH3-CH3 homodimeric interactions of the first polypeptide and the second polypeptide. In some embodiments, provided is the method for preparing a heteromultimer according to any one of the foregoing, wherein the first polypeptide and the second polypeptide have different isoelectric points. In some embodiments, provided is the heteromultimer according to any one of the foregoing, wherein the amino acid mutations described in the present disclosure impart or increase the difference between the isoelectric points of the first polypeptide and the second polypeptide. In some embodiments, provided is the method for preparing a heteromultimer according to any one of the foregoing, wherein the first polypeptide and the second polypeptide further comprise additional amino acid mutations that impart or increase the difference between the isoelectric points of the first polypeptide and the second polypeptide. In some embodiments, provided is the heteromultimer according to any one of the foregoing, wherein the first polypeptide and the second polypeptide further comprise additional amino acid mutations that result in a difference between the isoelectric points of the first polypeptide and the second polypeptide.

In some embodiments, the method for preparing a heteromultimer according to any one of the foregoing comprises the following steps:
a) a step of providing a molecule comprising a first polypeptide homomer,
b) a step of providing a molecule comprising a second polypeptide homomer,
c) a step of co-incubating the molecule comprising the first polypeptide homomer and the molecule comprising the second polypeptide homomer under reducing conditions, and
d) a step of obtaining a heteromultimer comprising the first polypeptide and the second polypeptide,

wherein the CH3 domains of the first polypeptide and the second polypeptide differ by at least one mutation site, and mutation sites in the CH3 domains are indicated by EU numbering;
the first polypeptide and the second polypeptide have different isoelectric points, and additional amino acid mutations are further introduced into the first polypeptide and the second polypeptide to impart or increase the difference between the isoelectric points of the first polypeptide and the second polypeptide. In some embodiments, provided is the method for preparing a heteromultimer according to any one of the foregoing, wherein the first polypeptide and the second polypeptide further comprise variable regions. In some embodiments, provided is the method for preparing a heteromultimer according to any one of the foregoing, wherein the first polypeptide and the second polypeptide further comprise a VH and a VL. In some embodiments, provided is the method for preparing a heteromultimer according to any one of the foregoing, wherein the VH(s) of the first polypeptide and/or the second polypeptide further comprise(s) an amino acid mutation selected from the group consisting of Q105E, Q105R, and Q105K, and the mutation site is indicated by KABAT numbering. In some embodiments, provided is the method for preparing a heteromultimer according to any one of the foregoing, wherein the VL(s) of the first polypeptide and/or the second polypeptide further comprise(s) an amino acid mutation of K42E, and the mutation site is indicated by KABAT numbering.

In some embodiments, provided is the method for preparing a heteromultimer according to any one of the foregoing, wherein the reducing conditions enable cysteines in the hinge region to cause disulfide bond isomerization. In some embodiments, provided is the method for preparing a heteromultimer according to any one of the foregoing, wherein the reducing conditions are sufficient to allow reduction of interchain disulfide bonds in the hinge region. In some embodiments, provided is the method for preparing a heteromultimer according to any one of the foregoing, wherein the reducing conditions include, but are not limited to, adding one or more reductants selected from the group consisting of 2-mercaptoethylamine (2-MEA), dithiothreitol (DTT), dithioerythritol (DTE), glutathione (GSH), tris(2-carboxyethyl)phosphine (TCEP), L-cysteine, D-cysteine, and β-mercapto-ethanol, as well as chemical derivatives thereof. In some embodiments, provided is the method for preparing a heteromultimer according to any one of the foregoing, wherein the reductant is one or more selected from the group consisting of 2-MEA, glutathione, L-cysteine, dithiothreitol, β-mercaptoethanol, and TCEP. In some embodiments, provided is the method for preparing a heteromultimer according to any one of the foregoing, wherein the reductant is 2-MEA.

In some embodiments, provided is the method for preparing a heteromultimer according to any one of the foregoing, wherein the reductant has a final concentration of 0.1 mM to 1 M. In some embodiments, provided is the method for preparing a heteromultimer according to any one of the foregoing, wherein the reductant has a final concentration of 1 mM to 1 M. In some embodiments, provided is the method for preparing a heteromultimer according to any one of the foregoing, wherein the reductant has a final concentration of 5 mM to 500 mM. In some embodiments, provided is the method for preparing a heteromultimer according to any one of the foregoing, wherein the reductant has a final concentration of 5 mM to 200 mM. In some embodiments, provided is the method for preparing a heteromultimer according to any one of the foregoing, wherein the reductant has a final concentration of 25 mM to 100 mM. In some embodiments, provided is the method for preparing a heteromultimer according to any one of the foregoing, wherein the reductant has a final concentration of 60 mM to 90 mM. In some embodiments, provided is the method for preparing a heteromultimer according to any one of the foregoing, wherein the reductant has a final concentration of 70 mM to 80 mM. In some embodiments, provided is the method for preparing a heteromultimer according to any one of the foregoing, wherein the reductant has a final concentration of 75 mM.

In some embodiments, provided is the method for preparing a heteromultimer according to any one of the foregoing, wherein the reductant has a final concentration of about 0.1 mM, about 1 mM, about 5 mM, about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 110 mM, about 120 mM, about 130 mM, about 140 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, about 310 mM, about 320 mM, about 330 mM, about 340 mM, about 350 mM, about 360 mM, about 370 mM, about 380 mM, about 390 mM, about 400 mM, about 410 mM, about 420 mM, about 430 mM, about 440 mM, about 450 mM, about 460 mM, about 470 mM, about 480 mM, about 490 mM, about 500 mM, about 550 mM, about 600 mM, about 650 mM, about 700 mM, about 750 mM, about 800 mM, about 850 mM, about 900 mM, about 950 mM, or about 1 M, or any range between these point values.

In some embodiments, provided is the method for preparing a heteromultimer according to any one of the foregoing, wherein the heteromultimer is a multispecific antibody or an Fc fusion protein.

In some embodiments, provided is the method for preparing a heteromultimer according to any one of the foregoing, wherein the heteromultimer is a heterodimer. In some embodiments, provided is the method for preparing a heteromultimer according to any one of the foregoing, wherein the heterodimer comprises one first polypeptide and one second polypeptide. In some embodiments, the first polypeptide and/or the second polypeptide are/is polypeptide structures/a polypeptide structure constituting the heterodimer and comprise(s) at least a CH3 domain. In some embodiments, the first polypeptide is an Fc region (a first Fc region), the second polypeptide is an Fc region (a second Fc region), and the first Fc region and/or the second Fc region comprise(s) CH2 and CH3 domains. In some embodiments, the first polypeptide and/or the second polypeptide comprise(s) CH1, CH2, and CH3 domains.

In some embodiments, provided is the method for preparing a heteromultimer according to any one of the foregoing, wherein the heteromultimer is a bispecific antibody. In some embodiments, provided is the method for preparing a heteromultimer according to any one of the foregoing, wherein the bispecific antibody comprises a first half antibody and a second half antibody. In some embodiments, the first half antibody comprises a first polypeptide, and the second half antibody comprises a second polypeptide. The first polypeptide and/or the second polypeptide are/is polypeptide structures/a polypeptide structure constituting the bispecific antibody and comprise(s) at least a CH3 domain. In some embodiments, the first polypeptide is an Fc region (a first Fc region), and the second polypeptide is an Fc region (a second Fc region). In some embodiments, the first Fc region and/or the second Fc region comprise(s) CH2 and CH3 domains. In some embodiments, the first polypeptide and/or the second polypeptide comprise(s) CH1, CH2, and CH3 domains. In some embodiments, the first half antibody binds to a first antigen, and the second half antibody binds to a second antigen. In some embodiments, the first half antibody comprises a first antigen-binding domain, and the second half antibody comprises a second antigen-binding domain. In some embodiments, the first antigen-binding domain and the second antigen-binding domain have structures selected from the group consisting of Fab, Fab', F(ab')2, Fd, Fv, scFv, dsFv, dAb, and VHH.

In some embodiments, provided is the method for preparing a heteromultimer according to any one of the foregoing, wherein the heteromultimer is a trispecific antibody. In some embodiments, provided is the method for preparing a heteromultimer according to any one of the foregoing, wherein the trispecific antibody comprises a first half antibody and a second half antibody. In some embodiments, the first half antibody binds to a first antigen, and the second half antibody binds to a second antigen and a third antigen. In some embodiments, the first half antibody comprises a first antigen-binding domain, and the second half antibody comprises a second antigen-binding domain and a third antigen-binding domain. In some embodiments, the first antigen-binding domain, the second antigen-binding domain, and the third antigen-binding domain have structures selected from the group consisting of Fab, Fab', F(ab')2, Fd, Fv, scFv, dsFv, dAb, and VHH.

In some embodiments, provided is the method for preparing a heteromultimer according to any one of the foregoing, wherein the molecule comprising the first polypeptide homomer and the molecule comprising the second polypeptide homomer are selected from the group consisting of an Fc region, an antibody, a fusion protein comprising an Fc region (e.g., a receptor-, cytokine-, or hormone-fused Fc region), and an Fc region conjugated with a drug (a peptide or toxin). In some embodiments, provided is the method for preparing a heteromultimer according to any one of the foregoing, wherein the molecule comprising the first polypeptide homomer is a first parental antibody, and the molecule comprising the second polypeptide homomer is a second parental antibody. In some embodiments, the first parental antibody is a first parental monoclonal antibody, and the second parental antibody is a second parental monoclonal antibody.

In some embodiments, provided is the method for preparing a heteromultimer according to any one of the foregoing, wherein the first polypeptide homomer is a homomer consisting of two first polypeptides, and the second polypeptide homomer is a homomer consisting of two second polypeptides.

In some embodiments, provided is the method for preparing a heteromultimer according to any one of the foregoing, wherein the first polypeptide homomer and the second polypeptide homomer each comprise a Cys-Pro-Pro-Cys sequence in the hinge region.

When described in the present disclosure, "first polypeptide" may also refer to each first polypeptide in the first polypeptide homomer. When described in the present disclosure, "second polypeptide" may also refer to each second polypeptide in the second polypeptide homomer. In some embodiments, the first polypeptide and/or the second polypeptide are/is polypeptide structures/a polypeptide structure constituting the heterodimer and comprise(s) at least a CH3 domain. In some embodiments, the first polypeptide is an Fc region (a first Fc region), the second polypeptide is an Fc region (a second Fc region), and the first Fc region and/or the second Fc region comprise(s) CH2 and CH3 domains. In some embodiments, the first polypeptide and/or the second polypeptide comprise(s) CH1, CH2, and CH3 domains.

In some embodiments, provided is the method for preparing a heteromultimer according to any one of the foregoing, wherein the first polypeptide is an antibody heavy chain, and/or the second polypeptide is an antibody heavy chain. In some embodiments, the heteromultimer further comprises one or more antibody light chains.

In some embodiments, the sequences of the first polypeptide and the second polypeptide are different. For example, a molecule comprising two first polypeptides is a first parental antibody, and a molecule comprising two second polypeptides is a second parental antibody. In the present disclosure, the first parental antibody further comprises one or more polypeptides (e.g., each forming a light chain of a half antibody with the two first polypeptides). In the present disclosure, the second parental antibody further comprises one or more polypeptides (e.g., each forming a light chain of a half antibody with the two second polypeptides).

The first polypeptide, the second polypeptide, the first parental antibody, or the second parental antibody is only used to distinguish amino acid sequences, and does not limit the positional relationships between polypeptides and proteins. For example, in a heterodimer consisting of one first polypeptide and one second polypeptide, the first polypeptide and the second polypeptide are two polypeptides whose amino acid sequences are different. When any one of the polypeptides is the first polypeptide, the other is the second polypeptide.

In some embodiments, provided is the method for preparing a heteromultimer according to any one of the foregoing, wherein amino acids in a core hinge region of the heteromultimer form a disulfide bond.

In some embodiments, provided is the method for preparing a heteromultimer according to any one of the foregoing, wherein the CH3 domains are derived from an IgG. In some embodiments, provided is the method for preparing a heteromultimer according to any one of the foregoing, wherein the CH3 domains are derived from IgG1, IgG2, IgG3, or IgG4. In some embodiments, provided is the method for preparing a heteromultimer according to any one of the foregoing, wherein the CH3 domains are derived from IgG1, IgG2, or IgG3. In some embodiments, provided is the method for preparing a heteromultimer according to any one of the foregoing, wherein the CH3 domains are derived from IgG1. In some embodiments, the CH3 domains are derived from human IgG1. In some embodiments, the human IgG1 comprises the amino acid sequence set forth in SEQ ID NO: 39, 40, or 41.

In some embodiments, provided is the method for preparing a heteromultimer according to any one of the foregoing, wherein the heteromultimer comprises at least one replaced Fab comprising a Titin chain and an Obscurin chain that are capable of forming a dimer. In some embodiments, provided is the method for preparing a heteromultimer according to any one of the foregoing, wherein the replaced Fab is obtained by replacing the original CH1 and CL of a Fab with the Obscurin chain and the Titin chain, respectively, or the replaced Fab is obtained by replacing the original CH1 and CL of a Fab with the Titin chain and the Obscurin chain, respectively. In some embodiments, the Titin chain comprises the amino acid sequence set forth in SEQ ID NO: 6, and the Obscurin chain comprises the amino acid sequence set forth in SEQ ID NO: 5.

In some embodiments, provided is the method for preparing a heteromultimer according to any one of the foregoing, wherein more than 70% (e.g., more than 75%, more than 80%, more than 85%, more than 88%, more than 90%, more than 91%, more than 92%, more than 93%, more than 94%, more than 95%, more than 96%, more than 97%, more than 98%, or more than 99%) of the total product is the desired heteromultimer (compared to the other products in the total product, such as half antibodies or homomers).

In some embodiments, the heteromultimer has good thermal stability.

In some embodiments, the heteromultimer has higher thermal stability than a heteromultimer comprising an Fc region mutation from the prior art. In some embodiments, the thermal stability comprises a thermodynamic index.

In some embodiments, the parental antibodies of the heteromultimer have good thermal stability.

In some embodiments, the thermal stability of the heteromultimer is higher than that of the parental antibodies (e.g., the first parental antibody and the second parental antibody). In some embodiments, provided is the method for preparing a heteromultimer according to any one of the foregoing, wherein: steps a) and b) further comprise a step of purifying the molecule comprising the first polypeptide homomer and the molecule comprising the second polypeptide homomer. In some embodiments, the purification method includes, but is not limited to, protein A or protein G chromatography, antigen binding-based affinity chromatography, anti-idiotypic antibody-based affinity chromatography, ion exchange, hydrophobic interaction chromatography, mixed chromatography (such as hydroxyapatite), immobilized metal affinity chromatography, thiophilic adsorption chromatography, size exclusion chromatography (SEC), etc. In some embodiments, the purification method is affinity chromatography. In some embodiments, the purification method is protein A chromatography.

In some embodiments, the method for preparing a heteromultimer according to any one of the foregoing further comprises a step of removing the reductant after step c). In some embodiments, provided is the method for preparing a heteromultimer according to any one of the foregoing, wherein the reductant is removed by, but not limited to, dialysis, precipitation, chromatography, or filtration. In some embodiments, provided is the method for preparing a heteromultimer according to any one of the foregoing, wherein the reductant is removed by dialysis.

In some embodiments, the step for removing the reductant may be, in principle, any method that results in or can achieve separation of the two without damaging the heteromultimer. Such methods include, but are not limited to, dialysis, precipitation, chromatography, or filtration. The step of removing the reductant may be carried out as a continuous process or a batch process.

In some embodiments, a product obtained from step c) has a heteromultimer content of more than 70% (e.g., more than 75%, more than 80%, more than 85%, more than 88%, more than 90%, more than 91%, more than 92%, more than 93%, more than 94%, more than 95%, more than 96%, more than 97%, more than 98%, or more than 99%).

In some embodiments, provided is the method for preparing a heteromultimer according to any one of the foregoing, wherein step d) further comprises a method for purifying the product obtained from step c). In some embodiments, the purification method includes, but is not limited to, protein A or protein G chromatography, antigen binding-based affinity chromatography, anti-idiotypic antibody-based affinity chromatography, ion exchange, hydrophobic interaction chromatography, mixed chromatography (such as hydroxyapatite), immobilized metal affinity chromatography, thiophilic adsorption chromatography, size exclusion chromatography (SEC), etc. In some embodiments, the purification method is affinity chromatography. In some embodiments, the purification method is size exclusion chromatography (SEC).

In some embodiments, the following methods may be provided: a method in which a cell strain that produces the molecule comprising the first polypeptide homomer and a cell strain that produces the molecule comprising the second polypeptide homomer are separately cultured, and after the culture supernatants are purified, the purified antibodies are used to induce an FAE (Fab-arm exchange) reaction; a method in which a cell strain that produces the molecule comprising the first polypeptide homomer and a cell strain that produces the molecule comprising the second polypeptide homomer are separately cultured, the culture supernatants are mixed without purification, an FAE reaction is induced in the mixture of the culture supernatants, and purification is then performed; a method in which a cell strain that produces the molecule comprising the first polypeptide homomer and a cell strain that produces the molecule comprising the second polypeptide homomer are mixed and cultured, and after the culture supernatant is purified, the purified antibodies are used to induce an FAE reaction; and a method in which a cell strain that produces the molecule comprising the first polypeptide homomer and a cell strain that produces the molecule comprising the second polypeptide homomer are mixed and cultured, an FAE reaction is induced in the culture supernatant, and purification is then performed.

In some embodiments, the present disclosure provides a method for preparing a heteromultimer, comprising the following steps a) to c):
a) a step of separately culturing a cell strain that produces a molecule comprising a first polypeptide homomer and a cell strain that produces a molecule comprising a second polypeptide homomer,
b) a step of separately purifying the culture supernatants of the cell strains to obtain the molecule comprising the first polypeptide homomer and the molecule comprising the second polypeptide homomer, and co-incubating the molecule comprising the first polypeptide homomer and the molecule comprising the second polypeptide homomer in the presence of a reductant, and
c) obtaining a heteromultimer comprising the first polypeptide and the second polypeptide.

In some embodiments, the present disclosure provides a method for preparing a heteromultimer, comprising the following steps a) to c):
a) a step of mixing a cell strain that produces a molecule comprising a first polypeptide homomer with a cell strain that produces a molecule comprising a second polypeptide homomer,
b) a step of co-incubating in the culture supernatant the molecule comprising the first polypeptide homomer and the molecule comprising the second polypeptide homomer in the presence of a reductant, and
c) obtaining a heteromultimer comprising the first polypeptide and the second polypeptide.

In some embodiments, the present disclosure provides a method for preparing a heteromultimer, comprising the following steps a) to c):
a) a step of separately culturing a cell strain that produces a molecule comprising a first polypeptide homomer and a cell strain that produces a molecule comprising a second polypeptide homomer,
b) a step of mixing the culture supernatants of the cell strains, and co-incubating the molecule comprising the first polypeptide homomer and the molecule comprising the second polypeptide homomer in the presence of a reductant, and
c) obtaining a heteromultimer comprising the first polypeptide and the second polypeptide.

In some embodiments, the present disclosure provides a method for preparing a heteromultimer, comprising the following steps:
a) providing a first nucleic acid construct encoding a molecule comprising a first polypeptide homomer,
b) providing a second nucleic acid construct encoding a molecule comprising a second polypeptide homomer,
   wherein the sequences of the first polypeptide and the second polypeptide are different and make the heterodimeric interaction between the first polypeptide and the second polypeptide stronger than the respective homodimeric interactions of the first polypeptide and the second polypeptide;
c) co-expressing the first and second nucleic acid constructs in a host cell, and
d) obtaining the heteromultimer from a cell culture.

In another aspect, the present disclosure provides a heteromultimer prepared by the method according to any one of the foregoing.

In another aspect, the present disclosure provides a heteromultimer comprising a first polypeptide and a second polypeptide, wherein the first polypeptide and the second polypeptide each comprise one CH3 domain, wherein the CH3 domain of the first polypeptide and the CH3 domain of the second polypeptide comprise amino acid mutations, wherein the amino acid mutations include at least one amino acid mutation selected from the group consisting of positions 347, 349, 351, 354, 356, 357, 364, 366, 368, 394, 405, 407, 409, 411, and 439; the CH3 domains of the first polypeptide and the second polypeptide differ by at least one mutation site, and mutation sites in the CH3 domains are indicated by EU numbering.

In some embodiments, provided is the heteromultimer according to any one of the foregoing, wherein the amino acid mutations include at least one amino acid mutation selected from the group consisting of positions 349, 351, 354, 356, 364, 366, 368, 394, 405, 409, 411, and 439.

In some embodiments, provided is the heteromultimer according to any one of the foregoing, wherein the CH3 domains comprise one or more mutations that promote heterologous formation. The mutations that promote heterologous formation refer in the present disclosure to a class of amino acid mutations that can promote the dissociation of a homomer (e.g., a homodimer) into monomers and/or promote the formation of a heteromultimer (e.g., a heterodimer) from two heteromonomers. In some embodiments, the mutations that promote heterologous formation are amino acid mutations at a CH3 interaction interface. In some embodiments, the mutations that promote heterologous formation are charged amino acid mutations at a CH3 interaction interface. In some embodiments, the mutations that promote heterologous formation are oppositely charged amino acid mutations at a CH3 interaction interface.

In some embodiments, the mutations that promote heterologous formation are any one of the mutations described in the present disclosure.

In some embodiments, provided is the heteromultimer according to any one of the foregoing, wherein the CH3 domain(s) of the first polypeptide and/or the second polypeptide comprise(s) at least one amino acid mutation selected from the group consisting of the following combinations:
the amino acid at position 356 is mutated into Lys (K), Arg (R), or His (H); and/or
the amino acid at position 439 is mutated into Glu (E) or Asp (D); and/or
the amino acid at position 349 is mutated into Leu (L), Phe (F), Ser (S), Cys (C), Ala (A), Val (V), Thr (T), or Gly (G); and/or
the amino acid at position 351 is mutated into Cys (C), Val (V), Thr (T), Ile (I), Phe (F), or Met (M); and/or
the amino acid at position 364 is mutated into Ala (A), Val (V), Thr (T), Leu (L), Tyr (Y), or Phe (F); and/or
the amino acid at position 366 is mutated into Gly (G), Ser (S), Ala (A), Val (V), Leu (L), His (H), or Ile (I); and/or
the amino acid at position 368 is mutated into Val (V), Ile (I), Met (M), or Ala (A); and/or
the amino acid at position 394 is mutated into Phe (F), Ala (A), Ser (S), Cys (C), Val (V), or Asn (N); and/or
the amino acid at position 405 is mutated into Thr (T), Leu (L), or Tyr (Y); and/or
the amino acid at position 407 is mutated into Cys (C), Val (V), Leu (L), His (H), or Phe (F); and/or
the amino acid at position 409 is mutated into Gln (Q), Arg (R), or Asp (D); and/or
the amino acid at position 411 is mutated into Asn (N), Tyr (Y), or Leu (L); and/or
the amino acid at position 354 is mutated into Tyr (Y), Cys (C), Phe (F), or Trp (W);
   and/or
the amino acid at position 357 is mutated into Cys (C); and/or
the amino acid at position 347 is mutated into Glu (E).

In some embodiments, provided is the heteromultimer according to any one of the foregoing, wherein the CH3 domain of the first polypeptide comprises an amino acid mutation selected from the group consisting of the following (a1) to (c2):
(a1) amino acid residues at positions 356 and 351 (EU numbering),
(a2) amino acid residues at positions 356 and 364 (EU numbering),
(a3) amino acid residues at positions 356 and 366 (EU numbering),
(a4) amino acid residues at positions 356 and 368 (EU numbering),
(a5) amino acid residues at positions 356 and 394 (EU numbering),
(a6) amino acid residues at positions 356 and 405 (EU numbering),
(a7) amino acid residues at positions 356 and 409 (EU numbering),
(a8) amino acid residues at positions 356 and 411 (EU numbering),
(b1) amino acid residues at positions 356 and 349 (EU numbering),
(c1) an amino acid residue at position 405 (EU numbering), or
(c2) an amino acid residue at position 349 (EU numbering);
the CH3 domain of the second polypeptide comprises an amino acid mutation selected from the group consisting of the following (d1) to (i2):
   (d1) amino acid residues at positions 439 and 351 (EU numbering),
   (d2) amino acid residues at positions 439 and 364 (EU numbering),
   (d3) amino acid residues at positions 439 and 366 (EU numbering),
   (d4) amino acid residues at positions 439 and 368 (EU numbering),
   (d5) amino acid residues at positions 439 and 394 (EU numbering),
   (d6) amino acid residues at positions 439 and 405 (EU numbering),
   (d7) amino acid residues at positions 439 and 409 (EU numbering),
   (d8) amino acid residues at positions 439 and 411 (EU numbering),
   (e1) amino acid residues at positions 439 and 354 (EU numbering),
   (f1) amino acid residues at positions 439, 354, and 351 (EU numbering),
   (f2) amino acid residues at positions 439, 354, and 366 (EU numbering),
   (f3) amino acid residues at positions 439, 354, and 368 (EU numbering),
   (g1) an amino acid residue at position 394 (EU numbering),
   (h) amino acid residues at positions 354 and 364 (EU numbering),
   (i1) an amino acid residue at position 364 (EU numbering), or
   (i2) an amino acid residue at position 354 (EU numbering).

In some embodiments, provided is the heteromultimer according to any one of the foregoing, wherein the CH3 domain of the first polypeptide comprises an amino acid mutation selected from the group consisting of the following (a1) to (c2):
(a1) amino acid residues at positions 356 and 351 (EU numbering),
(a5) amino acid residues at positions 356 and 394 (EU numbering),
(a8) amino acid residues at positions 356 and 411 (EU numbering),
(b1) amino acid residues at positions 356 and 349 (EU numbering), or
(c2) an amino acid residue at position 349 (EU numbering);
the CH3 domain of the second polypeptide comprises an amino acid mutation selected from the group consisting of the following (d1) to (i1):
   (d1) amino acid residues at positions 439 and 351 (EU numbering),
   (d5) amino acid residues at positions 439 and 394 (EU numbering),
   (d8) amino acid residues at positions 439 and 411 (EU numbering),
   (f1) amino acid residues at positions 439, 354, and 351 (EU numbering),
   (h) amino acid residues at positions 354 and 364 (EU numbering), or
   (i1) an amino acid residue at position 364 (EU numbering).

In some embodiments, provided is the heteromultimer according to any one of the foregoing, wherein the CH3 domain of the first polypeptide comprises an amino acid mutation selected from the group consisting of the following (a1) to (c2):
(a1) amino acid residues at positions 356 and 351 (EU numbering),
(b1) amino acid residues at positions 356 and 349 (EU numbering), or
(c2) an amino acid residue at position 349 (EU numbering);
the CH3 domain of the second polypeptide comprises an amino acid mutation selected from the group consisting of the following (d1) to (i1):
   (d1) amino acid residues at positions 439 and 351 (EU numbering),
   (f1) amino acid residues at positions 439, 354, and 351 (EU numbering),
   (h) amino acid residues at positions 354 and 364 (EU numbering), or
   (i1) an amino acid residue at position 364 (EU numbering).

In some embodiments, provided is the heteromultimer according to any one of the foregoing, wherein the CH3 domain of the first polypeptide comprises an amino acid mutation selected from the group consisting of the following (a1) to (c2):
(a1) amino acid residues of 356K and 351I (EU numbering), or
amino acid residues of 356K and 351T (EU numbering),
(a2) amino acid residues of 356K and 364A (EU numbering),
(a3) amino acid residues of 356K and 366A (EU numbering),
(a4) amino acid residues of 356K and 368I (EU numbering),
(a5) amino acid residues of 356K and 394A (EU numbering), or
amino acid residues of 356K and 394S (EU numbering),
(a6) amino acid residues of 356K and 405Y (EU numbering),
(a7) amino acid residues of 356K and 409Q (EU numbering),
(a8) amino acid residues of 356K and 411Y (EU numbering),
(b1) amino acid residues of 356K and 349C (EU numbering), or
amino acid residues of 356K and 349S (EU numbering),
(c1) an amino acid residue of 405T (EU numbering), or
(c2) an amino acid residue of 349C, 349S, or 349G (EU numbering);
the CH3 domain of the second polypeptide comprises an amino acid mutation selected from the group consisting of the following (d1) to (i2):
   (d1) amino acid residues of 439E and 351I (EU numbering), or
   amino acid residues of 439E and 351T (EU numbering),
   (d2) amino acid residues of 439E and 364A (EU numbering),
   (d3) amino acid residues of 439E and 366A (EU numbering),
   (d4) amino acid residues of 439E and 368I (EU numbering),
   (d5) amino acid residues of 439E and 394A (EU numbering), or
   amino acid residues of 439E and 394S (EU numbering),
   (d6) amino acid residues of 439E and 405Y (EU numbering),
   (d7) amino acid residues of 439E and 409Q (EU numbering),
   (d8) amino acid residues of 439E and 411Y (EU numbering),
   (e1) amino acid residues of 439E and 354Y (EU numbering),
   (f1) amino acid residues of 439E, 354C, and 351I (EU numbering),
   (f2) amino acid residues of 439E, 354C, and 366A (EU numbering),
   (f3) amino acid residues of 439E, 354C, and 368I (EU numbering),
   (g1) an amino acid residue of 394F (EU numbering),
   (h) amino acid residues of 354C and 364Y (EU numbering), or
   amino acid residues of 354C and 364F (EU numbering),
   (i1) an amino acid residue of 364Y (EU numbering), or
   (i2) an amino acid residue of 354W (EU numbering).

In some embodiments, provided is the heteromultimer according to any one of the foregoing, wherein the CH3 domain of the first polypeptide comprises an amino acid mutation selected from the group consisting of the following (a1) to (c2):
(a1) amino acid residues of 356K and 351I (EU numbering),
(a5) amino acid residues of 356K and 394S (EU numbering),
(a8) amino acid residues of 356K and 411Y (EU numbering),
(b1) amino acid residues of 356K and 349C (EU numbering), or
(c2) an amino acid residue of 349S or 349C (EU numbering);
the CH3 domain of the second polypeptide comprises an amino acid mutation selected from the group consisting of the following (d1) to (i1):
   (d1) amino acid residues of 439E and 351I (EU numbering),
   (d5) amino acid residues of 439E and 394S (EU numbering),
   (d8) amino acid residues of 439E and 411Y (EU numbering),
   (f1) amino acid residues of 439E, 354C, and 351I (EU numbering),
   (h) amino acid residues of 354C and 364Y (EU numbering), or
   amino acid residues of 354C and 364F (EU numbering),
   (i1) an amino acid residue of 364Y (EU numbering).

In some embodiments, provided is the heteromultimer according to any one of the foregoing, wherein the CH3 domain of the first polypeptide comprises an amino acid mutation selected from the group consisting of the following (a1) to (c2):
(a1) amino acid residues of 356K and 351I (EU numbering),
(a5) amino acid residues of 356K and 349C (EU numbering), or
(c2) an amino acid residue of 349S or 349C (EU numbering);
the CH3 domain of the second polypeptide comprises an amino acid mutation selected from the group consisting of the following (d1) to (i1):
   (d1) amino acid residues of 439E and 351I (EU numbering),
   (f1) amino acid residues of 439E, 354C, and 351I (EU numbering),
   (h) amino acid residues of 354C and 364Y (EU numbering), or
   (i1) an amino acid residue of 364Y (EU numbering).

### First-Class Mutation Combinations

In some embodiments, provided is the heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 349, 351, 364, 366, 368, 394, 405, 407, 409, and 411.

In some embodiments, provided is the heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical amino acid mutation selected from the group consisting of positions 351, 364, 366, 368, 394, 405, 409, and 411.

In some embodiments, provided is the heteromultimer according to any one of the foregoing, wherein the CH3 domain of the first polypeptide comprises amino acid mutations at positions 356 and 351, and the CH3 domain of the second polypeptide comprises amino acid mutations at positions 439 and 351.

In some embodiments, provided is the heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of 349L, 349F, 351C, 351V, 351T, 351I, 351F, 351M, 364A, 364V, 364T, 364L, 366G, 366S, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411N, 411L, and 411Y.

In some embodiments, provided is the heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of 349L, 351C, 351V, 351T, 351I, 351F, 351M, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y.

In some embodiments, provided is the heteromultimer according to any one of the foregoing, wherein the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of 349L, 351C, 351V, 351T, 351I, 351F, 351M, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y.

In some embodiments, provided is the heteromultimer according to any one of the foregoing, wherein:
the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of positions 351, 364, 366, 368, 394, 405, 409, and 411, wherein:
the amino acid at position 351 (EU numbering) is mutated into Cys (C), Val (V), Thr (T), Ile (I), Phe (F), or Met (M); or
the amino acid at position 364 (EU numbering) is mutated into Ala (A), Val (V), Thr (T), or Leu (L); or
the amino acid at position 366 (EU numbering) is mutated into Ser (S), Ala (A), Val (V), Leu (L), His (H), or Ile (I); or
the amino acid at position 368 (EU numbering) is mutated into Val (V), Ile (I), Met (M), or Ala (A); or
the amino acid at position 394 (EU numbering) is mutated into Ala (A), Ser (S), Cys (C), Val (V), or Asn (N); or
the amino acid at position 405 (EU numbering) is mutated into Leu (L) or Tyr (Y); or
the amino acid at position 409 (EU numbering) is mutated into Gln (Q); or
the amino acid at position 411 (EU numbering) is mutated into Asn (N), Tyr (Y), or Leu (L).

In some embodiments, provided is the heteromultimer according to any one of the foregoing, wherein:
the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of positions 351, 364, 366, 368, 394, 405, 409, and 411, wherein:
position 351 (EU numbering) is mutated into Cys (C), Val (V), Thr (T), Ile (I), or Phe (F); or
position 364 (EU numbering) is mutated into Ala (A), Val (V), Thr (T), or Leu (L); or
position 366 (EU numbering) is mutated into Ser (S), Ala (A), Val (V), Leu (L), or His (H); or
position 368 (EU numbering) is mutated into Val (V), Ile (I), or Met (M); or
position 394 (EU numbering) is mutated into Ala (A), Ser (S), Cys (C), Val (V), or Asn (N); or
position 405 (EU numbering) is mutated into Leu (L) or Tyr (Y); or
position 409 (EU numbering) is mutated into Gln (Q) or Arg (R); or
position 411 (EU numbering) is mutated into Asn (N) or Tyr (Y).

In some embodiments, provided is the heteromultimer according to any one of the foregoing, wherein the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351T, 351I, 364A, 366A, 368I, 394A, 394S, 405Y, 409Q, and 411Y. In some embodiments, provided is the heteromultimer according to any one of the foregoing, wherein the CH3 domain of the first polypeptide comprises amino acid mutations of 356K and 351I, and the CH3 domain of the second polypeptide comprises amino acid mutations of 439E and 351I.

In some embodiments, provided is the heteromultimer according to any one of the foregoing, wherein:
the CH3 domain of the first polypeptide comprises amino acid mutations of D/E356K and Y349L, and the CH3 domain of the second polypeptide comprises amino acid mutations of K439E and Y349L; or
the CH3 domain of the first polypeptide comprises amino acid mutations of D/E356K and L351C, and the CH3 domain of the second polypeptide comprises amino acid mutations of K439E and L351C; or
the CH3 domain of the first polypeptide comprises amino acid mutations of D/E356K and L351V, and the CH3 domain of the second polypeptide comprises amino acid mutations of K439E and L351V; or
the CH3 domain of the first polypeptide comprises amino acid mutations of D/E356K and L351T, and the CH3 domain of the second polypeptide comprises amino acid mutations of K439E and L351T; or
the CH3 domain of the first polypeptide comprises amino acid mutations of D/E356K and L351I, and the CH3 domain of the second polypeptide comprises amino acid mutations of K439E and L351I; or
the CH3 domain of the first polypeptide comprises amino acid mutations of D/E356K and L351F, and the CH3 domain of the second polypeptide comprises amino acid mutations of K439E and L351F; or
the CH3 domain of the first polypeptide comprises amino acid mutations of D/E356K and L351M, and the CH3 domain of the second polypeptide comprises amino acid mutations of K439E and L351M; or
the CH3 domain of the first polypeptide comprises amino acid mutations of D/E356K and S364A, and the CH3 domain of the second polypeptide comprises amino acid mutations of K439E and S364A; or
the CH3 domain of the first polypeptide comprises amino acid mutations of D/E356K and S364T, and the CH3 domain of the second polypeptide comprises amino acid mutations of K439E and S364T; or
the CH3 domain of the first polypeptide comprises amino acid mutations of D/E356K and S364L, and the CH3 domain of the second polypeptide comprises amino acid mutations of K439E and S364L; or
the CH3 domain of the first polypeptide comprises amino acid mutations of D/E356K and T366G, and the CH3 domain of the second polypeptide comprises amino acid mutations of K439E and T366G; or
the CH3 domain of the first polypeptide comprises amino acid mutations of D/E356K and T366A, and the CH3 domain of the second polypeptide comprises amino acid mutations of K439E and T366A; or
the CH3 domain of the first polypeptide comprises amino acid mutations of D/E356K and T366V, and the CH3 domain of the second polypeptide comprises amino acid mutations of K439E and T366V; or
the CH3 domain of the first polypeptide comprises amino acid mutations of D/E356K and T366L, and the CH3 domain of the second polypeptide comprises amino acid mutations of K439E and T366L; or
the CH3 domain of the first polypeptide comprises amino acid mutations of D/E356K and T366H, and the CH3 domain of the second polypeptide comprises amino acid mutations of K439E and T366H; or
the CH3 domain of the first polypeptide comprises amino acid mutations of D/E356K and T366I, and the CH3 domain of the second polypeptide comprises amino acid mutations of K439E and T366I; or
the CH3 domain of the first polypeptide comprises amino acid mutations of D/E356K and L368V, and the CH3 domain of the second polypeptide comprises amino acid mutations of K439E and L368V; or
the CH3 domain of the first polypeptide comprises amino acid mutations of D/E356K and L368I, and the CH3 domain of the second polypeptide comprises amino acid mutations of K439E and L368I; or
the CH3 domain of the first polypeptide comprises amino acid mutations of D/E356K and L368A, and the CH3 domain of the second polypeptide comprises amino acid mutations of K439E and L368A; or
the CH3 domain of the first polypeptide comprises amino acid mutations of D/E356K and T394A, and the CH3 domain of the second polypeptide comprises amino acid mutations of K439E and T394A; or
the CH3 domain of the first polypeptide comprises amino acid mutations of D/E356K and T394S, and the CH3 domain of the second polypeptide comprises amino acid mutations of K439E and T394S; or
the CH3 domain of the first polypeptide comprises amino acid mutations of D/E356K and T394C, and the CH3 domain of the second polypeptide comprises amino acid mutations of K439E and T394C; or
the CH3 domain of the first polypeptide comprises amino acid mutations of D/E356K and T394V, and the CH3 domain of the second polypeptide comprises amino acid mutations of K439E and T394V; or
the CH3 domain of the first polypeptide comprises amino acid mutations of D/E356K and T394N, and the CH3 domain of the second polypeptide comprises amino acid mutations of K439E and T394N; or
the CH3 domain of the first polypeptide comprises amino acid mutations of D/E356K and F405L, and the CH3 domain of the second polypeptide comprises amino acid mutations of K439E and F405L; or
the CH3 domain of the first polypeptide comprises amino acid mutations of D/E356K and F405Y, and the CH3 domain of the second polypeptide comprises amino acid mutations of K439E and F405Y; or
the CH3 domain of the first polypeptide comprises amino acid mutations of D/E356K and Y407C, and the CH3 domain of the second polypeptide comprises amino acid mutations of K439E and Y407C; or
the CH3 domain of the first polypeptide comprises amino acid mutations of D/E356K and Y407V, and the CH3 domain of the second polypeptide comprises amino acid mutations of K439E and Y407V; or
the CH3 domain of the first polypeptide comprises amino acid mutations of D/E356K and Y407L, and the CH3 domain of the second polypeptide comprises amino acid mutations of K439E and Y407L; or
the CH3 domain of the first polypeptide comprises amino acid mutations of D/E356K and Y407H, and the CH3 domain of the second polypeptide comprises amino acid mutations of K439E and Y407H; or
the CH3 domain of the first polypeptide comprises amino acid mutations of D/E356K and Y407F, and the CH3 domain of the second polypeptide comprises amino acid mutations of K439E and Y407F; or
the CH3 domain of the first polypeptide comprises amino acid mutations of D/E356K and K409Q, and the CH3 domain of the second polypeptide comprises amino acid mutations of K439E and K409Q; or
the CH3 domain of the first polypeptide comprises amino acid mutations of D/E356K and K409R, and the CH3 domain of the second polypeptide comprises amino acid mutations of K439E and K409R; or
the CH3 domain of the first polypeptide comprises amino acid mutations of D/E356K and T411L, and the CH3 domain of the second polypeptide comprises amino acid mutations of K439E and T411L; or
the CH3 domain of the first polypeptide comprises amino acid mutations of D/E356K and T411Y, and the CH3 domain of the second polypeptide comprises amino acid mutations of K439E and T411Y.

In some embodiments, provided is the method for preparing a heteromultimer according to any one of the foregoing, wherein the CH3 domains of the first polypeptide and the second polypeptide each comprise one charged amino acid mutation (e.g., a positively charged amino acid or a negatively charged amino acid), and the amino acid mutation in the CH3 domain of the first polypeptide and the amino acid mutation in the CH3 domain of the second polypeptide are oppositely charged. Further, the CH3 domains of the first polypeptide and the second polypeptide each comprise one identical amino acid mutation, and the mutations are located at a CH3 interaction interface and can weaken the interaction between the CH3 domains of the first polypeptide homomer and/or the second polypeptide homomer, such that the first polypeptide homomer and/or the second polypeptide homomer more easily dissociate(s) into first polypeptide monomers and second polypeptide monomers under reducing conditions, thereby promoting the production of a heteromultimer comprising one first polypeptide and one second polypeptide.

### Second-Class Mutation Combinations

In some embodiments, provided is the heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to iv):
i) 349;
ii) 354 and 351;
iii) 354 and 366; and
iv) 354 and 368.

In some embodiments, provided is the heteromultimer according to any one of the foregoing, wherein the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368.

In some embodiments, provided is the heteromultimer according to any one of the foregoing, wherein the CH3 domain of the first polypeptide comprises amino acid mutations at positions 356 and 349, and the CH3 domain of the second polypeptide comprises amino acid mutations at positions 439, 354, and 351.

In some embodiments, provided is the heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to iv):
i) 349C;
ii) 354C and 351I;
iii) 354C and 366A; and
iv) 354C and 368I.

In some embodiments, provided is the heteromultimer according to any one of the foregoing, wherein the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I.

In some embodiments, provided is the heteromultimer according to any one of the foregoing, wherein the CH3 domain of the first polypeptide comprises amino acid mutations of 356K and 349C, and the CH3 domain of the second polypeptide comprises amino acid mutations of 439E, 354C, and 351I.

In some embodiments, provided is the heteromultimer according to any one of the foregoing, wherein:
the CH3 domain of the first polypeptide comprises amino acid mutations of D/E356K and 349C, and the CH3 domain of the second polypeptide comprises amino acid mutations of K439E, S354C, and L351I; or
the CH3 domain of the first polypeptide comprises amino acid mutations of D/E356K and Y349C, and the CH3 domain of the second polypeptide comprises amino acid mutations of K439E, S354C, and T366A; or
the CH3 domain of the first polypeptide comprises amino acid mutations of D/E356K and Y349C, and the CH3 domain of the second polypeptide comprises amino acid mutations of K439E, S354C, and L368I.

In some embodiments, provided is the heteromultimer according to any one of the foregoing, wherein the CH3 domains of the first polypeptide and the second polypeptide each comprise one charged amino acid mutation (e.g., a positively charged amino acid or a negatively charged amino acid), and the amino acid mutation in the CH3 domain of the first polypeptide and the amino acid mutation in the CH3 domain of the second polypeptide are oppositely charged. Further, the CH3 domains of the first polypeptide and the second polypeptide each further comprise one cysteine mutation, so that a disulfide bond can be formed. Furthermore, the CH3 domain of the first polypeptide or the second polypeptide comprises one amino acid mutation, and the mutation is located at a CH3 interaction interface and can weaken the interaction between the CH3 domains of the first polypeptide homomer or the second polypeptide homomer, such that the first polypeptide homomer or the second polypeptide homomer more easily dissociates into first polypeptide monomers and second polypeptide monomers under reducing conditions, thereby promoting the production of a heteromultimer comprising one first polypeptide and one second polypeptide.

### Third-Class Mutation Combinations

In some embodiments, provided is the heteromultimer according to any one of the foregoing, wherein:
the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394; or
the CH3 domain of the first polypeptide comprises an amino acid mutation at position 366, and the CH3 domain of the second polypeptide comprises an amino acid mutation at position 405 or 407.

In some embodiments, provided is the heteromultimer according to any one of the foregoing, wherein:
the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394.

In some embodiments, provided is the heteromultimer according to any one of the foregoing, wherein the CH3 domain of the first polypeptide comprises an amino acid mutation at position 349, and the CH3 domain of the second polypeptide comprises an amino acid mutation at position 364.

In some embodiments, provided is the heteromultimer according to any one of the foregoing, wherein:
the CH3 domain of the first polypeptide comprises an amino acid mutation of 349S, and the CH3 domain of the second polypeptide comprises one amino acid mutation selected from the group consisting of 364Y, 354F, 354W, and 354Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 349A, and the CH3 domain of the second polypeptide comprises one amino acid mutation selected from the group consisting of 354F, 354Y, and 354W; or
the CH3 domain of the first polypeptide comprises an amino acid mutation of 349V, and the CH3 domain of the second polypeptide comprises an amino acid mutation of 354F; or
the CH3 domain of the first polypeptide comprises an amino acid mutation of 349T, and the CH3 domain of the second polypeptide comprises an amino acid mutation of 354F; or
the CH3 domain of the first polypeptide comprises an amino acid mutation of 405T, and the CH3 domain of the second polypeptide comprises an amino acid mutation of 394F; or
the CH3 domain of the first polypeptide comprises an amino acid mutation of 366H, and the CH3 domain of the second polypeptide comprises an amino acid mutation of 405L or 407L.

In some embodiments, provided is the heteromultimer according to any one of the foregoing, wherein:
the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 405T, and the CH3 domain of the second polypeptide comprises an amino acid mutation of 394F.

In some embodiments, provided is the heteromultimer according to any one of the foregoing, wherein the CH3 domain of the first polypeptide comprises an amino acid mutation of 349S, and the CH3 domain of the second polypeptide comprises an amino acid mutation of 364Y.

In some embodiments, provided is the heteromultimer according to any one of the foregoing, wherein:
the CH3 domain of the first polypeptide comprises one amino acid mutation that is a mutation of a small-volume amino acid residue into a large-volume amino acid residue, which can produce a protuberance in the CH3 interface, and the CH3 domain of the second polypeptide comprises one amino acid mutation that is a mutation of a large-volume amino acid residue into a small-volume amino acid residue, which can produce a cavity in the CH3 interface; or
the CH3 domain of the first polypeptide comprises one amino acid mutation that is a mutation of a large-volume amino acid residue into a small-volume amino acid residue, which can produce a cavity in the CH3 interface, and the CH3 domain of the second polypeptide comprises one amino acid mutation that is a mutation of a small-volume amino acid residue into a large-volume amino acid residue, which can produce a protuberance in the CH3 interface;
consequently, the cavity and the protuberance promote the production of a heteromultimer comprising one first polypeptide and one second polypeptide.

### Fourth-Class Mutation Combinations

In some embodiments, provided is the heteromultimer according to any one of the foregoing, wherein:
the CH3 domain of the first polypeptide comprises an amino acid mutation at position 349, and the CH3 domain of the second polypeptide comprises an amino acid mutation at position 354, wherein the CH3 domain of the second polypeptide further comprises one amino acid mutation selected from the group consisting of positions 351, 364, 366, 368, and 405; or
the CH3 domain of the first polypeptide comprises an amino acid mutation at position 349, and the CH3 domain of the second polypeptide comprises amino acid mutations at positions 347 and 357.

In some embodiments, provided is the heteromultimer according to any one of the foregoing, wherein the CH3 domain of the first polypeptide comprises an amino acid mutation at position 349, and the CH3 domain of the second polypeptide comprises amino acid mutations at positions 354 and 364.

In some embodiments, provided is the heteromultimer according to any one of the foregoing, wherein:
the CH3 domain of the first polypeptide comprises an amino acid mutation of 349C, and the CH3 domain of the second polypeptide comprises an amino acid mutation of 354C, wherein the CH3 domain of the second polypeptide further comprises one amino acid mutation selected from the group consisting of 351I, 364Y, 364F, 366A, 368I, and 405Y; or
the CH3 domain of the first polypeptide comprises an amino acid mutation of 349C, and the CH3 domain of the second polypeptide comprises amino acid mutations of 347E and 357C.

In some embodiments, provided is the heteromultimer according to any one of the foregoing, wherein the CH3 domain of the first polypeptide comprises an amino acid mutation of 349C, and the CH3 domain of the second polypeptide comprises an amino acid mutation of 354C, wherein the CH3 domain of the second polypeptide further comprises an amino acid mutation of 364Y or 364F.

In some embodiments, provided is the heteromultimer according to any one of the foregoing, wherein the CH3 domain of the first polypeptide comprises an amino acid mutation of 349C, and the CH3 domain of the second polypeptide comprises amino acid mutations of 354C and 364Y.

In some embodiments, provided is the heteromultimer according to any one of the foregoing, wherein the CH3 domains of the first polypeptide and the second polypeptide each comprise one cysteine mutation, so that a disulfide bond can be formed; further, the CH3 domain of the first polypeptide or the second polypeptide comprises one amino acid mutation, wherein the mutation is located at a CH3 interaction interface and can weaken the interaction between the CH3 domains of the first polypeptide homomer and/or the second polypeptide homomer, such that the first polypeptide homomer and/or the second polypeptide homomer more easily dissociate(s) into first polypeptide monomers and second polypeptide monomers under reducing conditions, thereby promoting the production of a heteromultimer comprising one first polypeptide and one second polypeptide.

### Fifth-Class Mutation Combinations

In some embodiments, provided is the heteromultimer according to any one of the foregoing, wherein the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to vii):
i) 349;
ii) 354;
iii) 357;
iv) 366;
v) 394;
vi) 405; and
vii) 407.

In some embodiments, provided is the heteromultimer according to any one of the foregoing, wherein:
the CH3 domain of the first polypeptide comprises amino acid mutations at positions 356 and 349, and the CH3 domain of the second polypeptide comprises an amino acid mutation at position 439, wherein the CH3 domain of the second polypeptide further comprises an amino acid mutation at position 354 or 357; or
the CH3 domain of the first polypeptide comprises amino acid mutations at positions 356 and 405, and the CH3 domain of the second polypeptide comprises amino acid mutations at positions 439 and 394; or
the CH3 domain of the first polypeptide comprises amino acid mutations at positions 356 and 366, and the CH3 domain of the second polypeptide comprises an amino acid mutation at position 439, wherein the CH3 domain of the second polypeptide further comprises an amino acid mutation at position 405 or 407; or
the CH3 domain of the first polypeptide comprises amino acid mutations at positions 356 and 407, and the CH3 domain of the second polypeptide comprises amino acid mutations at positions 439 and 366.

In some embodiments, provided is the heteromultimer according to any one of the foregoing, wherein the CH3 domain of the first polypeptide comprises amino acid mutations at positions 356 and 349, and the CH3 domain of the second polypeptide comprises amino acid mutations at positions 439 and 354.

In some embodiments, provided is the heteromultimer according to any one of the foregoing, wherein the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to vii):
i) 349S or 349C;
ii) 354Y or 354C;
iii) 357C;
iv) 366H;
v) 394F;
vi) 405T or 405L; and
vii) 407L or 407H.

In some embodiments, provided is the heteromultimer according to any one of the foregoing, wherein:
the CH3 domain of the first polypeptide comprises amino acid mutations of 356K and 349S, and the CH3 domain of the second polypeptide comprises amino acid mutations of 439E and 354Y; or
the CH3 domain of the first polypeptide comprises amino acid mutations of 356K and 349C, and the CH3 domain of the second polypeptide comprises amino acid mutations of 439E and 354C; or
the CH3 domain of the first polypeptide comprises amino acid mutations of 356K and 349C, and the CH3 domain of the second polypeptide comprises amino acid mutations of 439E and 357C; or
the CH3 domain of the first polypeptide comprises amino acid mutations of 356K and 405T, and the CH3 domain of the second polypeptide comprises amino acid mutations of 439E and 394F; or
the CH3 domain of the first polypeptide comprises amino acid mutations of 356K and 366H, and the CH3 domain of the second polypeptide comprises an amino acid mutation of 439E, wherein the CH3 domain of the second polypeptide further comprises one amino acid mutation selected from the group consisting of 405L, 407L, and 407H; or
the CH3 domain of the first polypeptide comprises amino acid mutations of 356K and 407L, and the CH3 domain of the second polypeptide comprises amino acid mutations of 439E and 366H; or
the CH3 domain of the first polypeptide comprises amino acid mutations of 356K and 407H, and the CH3 domain of the second polypeptide comprises amino acid mutations of 439E and 366H.

In some embodiments, provided is the heteromultimer according to any one of the foregoing, wherein the CH3 domain of the first polypeptide comprises amino acid mutations of 356K and 349S, and the CH3 domain of the second polypeptide comprises amino acid mutations of 439E and 354Y.

In some embodiments, provided is the heteromultimer according to any one of the foregoing, wherein:
the CH3 domain of the first polypeptide comprises amino acid mutations of D/E356K and Y349S, and the CH3 domain of the second polypeptide comprises amino acid mutations of K439E and S354Y; or
the CH3 domain of the first polypeptide comprises amino acid mutations of D/E356K and Y349C, and the CH3 domain of the second polypeptide comprises amino acid mutations of K439E and S354C; or
the CH3 domain of the first polypeptide comprises amino acid mutations of D/E356K and Y349C, and the CH3 domain of the second polypeptide comprises amino acid mutations of K439E and E357C; or
the CH3 domain of the first polypeptide comprises amino acid mutations of D/E356K and F405T, and the CH3 domain of the second polypeptide comprises amino acid mutations of K439E and T394F; or
the CH3 domain of the first polypeptide comprises amino acid mutations of D/E356K and T366H, and the CH3 domain of the second polypeptide comprises amino acid mutations of K439E and F405L; or
the CH3 domain of the first polypeptide comprises amino acid mutations of D/E356K and T366H, and the CH3 domain of the second polypeptide comprises amino acid mutations of K439E and Y407L; or
the CH3 domain of the first polypeptide comprises amino acid mutations of D/E356K and T366H, and the CH3 domain of the second polypeptide comprises amino acid mutations of K439E and Y407H; or
the CH3 domain of the first polypeptide comprises amino acid mutations of D/E356K and Y407L, and the CH3 domain of the second polypeptide comprises amino acid mutations of K439E and T366H; or
the CH3 domain of the first polypeptide comprises amino acid mutations of D/E356K and Y407H, and the CH3 domain of the second polypeptide comprises amino acid mutations of K439E and T366H.

In some embodiments, provided is the heteromultimer according to any one of the foregoing, wherein the CH3 domains of the first polypeptide and the second polypeptide each comprise one charged amino acid mutation (e.g., a positively charged amino acid or a negatively charged amino acid), and the amino acid mutation in the CH3 domain of the first polypeptide and the amino acid mutation in the CH3 domain of the second polypeptide are oppositely charged. Further, the CH3 domains of the first polypeptide and the second polypeptide comprise amino acid mutations selected from the group consisting of any one of the following groups:
the CH3 domains of the first polypeptide and the second polypeptide each comprise one cysteine mutation, so that a disulfide bond can be formed; or
the CH3 domains of the first polypeptide and the second polypeptide each further comprise one different amino acid mutation, wherein the amino acid mutation in the CH3 domain of the first polypeptide is a mutation of a small-volume amino acid residue into a large-volume amino acid residue, which can produce a protuberance in the CH3 interface, and the amino acid mutation in the CH3 domain of the second polypeptide is a mutation of a large-volume amino acid residue into a small-volume amino acid residue, which can produce a cavity in the CH3 interface; or
the CH3 domains of the first polypeptide and the second polypeptide each comprise one cysteine mutation, so that a disulfide bond can be formed; further, the CH3 domains of the first polypeptide and the second polypeptide each comprise one different amino acid mutation, wherein the amino acid mutation in the CH3 domain of the first polypeptide is a mutation of a large-volume amino acid residue into a small-volume amino acid residue, which can produce a cavity in the CH3 interface, and the amino acid mutation in the CH3 domain of the second polypeptide is a mutation of a small-volume amino acid residue into a large-volume amino acid residue, which can produce a protuberance in the CH3 interface;
consequently, the cavity and the protuberance promote the production of a heteromultimer comprising one first polypeptide and one second polypeptide.

### Sixth-Class Mutation Combinations

In some embodiments, provided is the heteromultimer according to any one of the foregoing, wherein the CH3 domain of the first polypeptide comprises an amino acid mutation at position 349, and the CH3 domain of the second polypeptide comprises an amino acid mutation at position 357.

In some embodiments, provided is the heteromultimer according to any one of the foregoing, wherein the CH3 domain of the first polypeptide comprises an amino acid mutation of 349C, and the CH3 domain of the second polypeptide comprises an amino acid mutation of 357C.

In some embodiments, provided is the heteromultimer according to any one of the foregoing, wherein the CH3 domains of the first polypeptide and the second polypeptide each comprise one cysteine mutation, so that a disulfide bond can be formed.

### Seventh-Class Mutation Combinations

In some embodiments, provided is the heteromultimer according to any one of the foregoing, wherein the CH3 domain of the first polypeptide comprises an amino acid mutation at position 354, and the CH3 domain of the second polypeptide comprises an amino acid mutation at position 349, wherein the CH3 domain(s) of the first polypeptide and/or the second polypeptide further comprise(s) at least one amino acid mutation selected from the group consisting of i) and ii):
i) 405; and
ii) 394.

In some embodiments, provided is the heteromultimer according to any one of the foregoing, wherein the CH3 domain of the first polypeptide comprises amino acid mutations at positions 354 and 405, and the CH3 domain of the second polypeptide comprises amino acid mutations at positions 349 and 394.

In some embodiments, provided is the heteromultimer according to any one of the foregoing, wherein the CH3 domain of the first polypeptide comprises an amino acid mutation of 354C, and the CH3 domain of the second polypeptide comprises an amino acid mutation of 349C, wherein the CH3 domain(s) of the first polypeptide and/or the second polypeptide further comprise(s) at least one amino acid mutation selected from the group consisting of i) and ii):
i) 405T; and
ii) 394F.

In some embodiments, provided is the heteromultimer according to any one of the foregoing, wherein the CH3 domain of the first polypeptide comprises amino acid mutations of 354C and 405T, and the CH3 domain of the second polypeptide comprises amino acid mutations of 349C and 394F.

In some embodiments, provided is the heteromultimer according to any one of the foregoing, wherein:
the CH3 domains of the first polypeptide and the second polypeptide each comprise one cysteine mutation, so that a disulfide bond can be formed; further, the CH3 domains of the first polypeptide and the second polypeptide each comprise one different amino acid mutation, wherein the amino acid mutation in the CH3 domain of the first polypeptide is a mutation of a small-volume amino acid residue into a large-volume amino acid residue, which can produce a protuberance in the CH3 interface, and the amino acid mutation in the CH3 domain of the second polypeptide is a mutation of a large-volume amino acid residue into a small-volume amino acid residue, which can produce a cavity in the CH3 interface; or
the CH3 domains of the first polypeptide and the second polypeptide each comprise one cysteine mutation, so that a disulfide bond can be formed; further, the CH3 domains of the first polypeptide and the second polypeptide each comprise one different amino acid mutation, wherein the amino acid mutation in the CH3 domain of the first polypeptide is a mutation of a large-volume amino acid residue into a small-volume amino acid residue, which can produce a cavity in the CH3 interface, and the amino acid mutation in the CH3 domain of the second polypeptide is a mutation of a small-volume amino acid residue into a large-volume amino acid residue, which can produce a protuberance in the CH3 interface;
consequently, the cavity and the protuberance promote the production of a heteromultimer comprising one first polypeptide and one second polypeptide.

In some embodiments, provided is the heteromultimer according to any one of the foregoing, wherein the first polypeptide and the second polypeptide have different isoelectric points. In some embodiments, provided is the heteromultimer according to any one of the foregoing, wherein the amino acid mutations described in the present disclosure impart or increase the difference between the isoelectric points of the first polypeptide and the second polypeptide.

In some embodiments, provided is the heteromultimer according to any one of the foregoing, wherein the first polypeptide and the second polypeptide further comprise additional amino acid mutations that impart or increase the difference between the isoelectric points of the first polypeptide and the second polypeptide. In some embodiments, provided is the heteromultimer according to any one of the foregoing, wherein the first polypeptide and the second polypeptide further comprise additional amino acid mutations that result in a difference between the isoelectric points of the first polypeptide and the second polypeptide. In some embodiments, provided is the heteromultimer according to any one of the foregoing, wherein the first polypeptide and the second polypeptide further comprise variable regions. In some embodiments, provided is the heteromultimer according to any one of the foregoing, wherein the first polypeptide and the second polypeptide further comprise a VH and a VL. In some embodiments, provided is the heteromultimer according to any one of the foregoing, wherein the VH(s) of the first polypeptide and/or the second polypeptide further comprise(s) an amino acid mutation selected from the group consisting of Q105E, Q105R, and Q105K, and the mutation site is indicated by KABAT numbering. In some embodiments, provided is the heteromultimer according to any one of the foregoing, wherein the VL(s) of the first polypeptide and/or the second polypeptide further comprise(s) an amino acid mutation of K42E, and the mutation site is indicated by KABAT numbering.

In some embodiments, provided is the heteromultimer according to any one of the foregoing, wherein the heteromultimer is a multispecific antibody or an Fc fusion protein. In some embodiments, provided is the heteromultimer according to any one of the foregoing, wherein the heteromultimer is a heterodimer. In some embodiments, provided is the heteromultimer according to any one of the foregoing, wherein the heterodimer comprises one first polypeptide and one second polypeptide. In some embodiments, the first polypeptide and/or the second polypeptide are/is polypeptide structures/a polypeptide structure constituting the heterodimer and comprise(s) at least a CH3 domain. In some embodiments, the first polypeptide is an Fc region (a first Fc region), the second polypeptide is an Fc region (a second Fc region), and the first Fc region and/or the second Fc region comprise(s) CH2 and CH3 domains. In some embodiments, the first polypeptide and/or the second polypeptide comprise(s) CH1, CH2, and CH3 domains. In some embodiments, provided is the heteromultimer according to any one of the foregoing, wherein the heteromultimer is a bispecific antibody. In some embodiments, provided is the heteromultimer according to any one of the foregoing, wherein the bispecific antibody comprises a first half antibody and a second half antibody. In some embodiments, the first half antibody comprises a first polypeptide, and the second half antibody comprises a second polypeptide. The first polypeptide and/or the second polypeptide are/is polypeptide structures/a polypeptide structure constituting the bispecific antibody and comprise(s) at least a CH3 domain. In some embodiments, the first polypeptide is an Fc region (a first Fc region), and the second polypeptide is an Fc region (a second Fc region). In some embodiments, the first Fc region and/or the second Fc region comprise(s) CH2 and CH3 domains. In some embodiments, the first polypeptide and/or the second polypeptide comprise(s) CH1, CH2, and CH3 domains. In some embodiments, the first half antibody binds to a first antigen, and the second half antibody binds to a second antigen. In some embodiments, the first half antibody comprises a first antigen-binding domain, and the second half antibody comprises a second antigen-binding domain. In some embodiments, the first antigen-binding domain and the second antigen-binding domain have structures selected from the group consisting of Fab, Fab', F(ab')2, Fd, Fv, scFv, dsFv, dAb, and VHH.

In some embodiments, provided is the heteromultimer according to any one of the foregoing, wherein the heteromultimer is a trispecific antibody. In some embodiments, provided is the heteromultimer according to any one of the foregoing, wherein the trispecific antibody comprises a first half antibody and a second half antibody. In some embodiments, the first half antibody binds to a first antigen, and the second half antibody binds to a second antigen and a third antigen. In some embodiments, the first half antibody comprises a first antigen-binding domain, and the second half antibody comprises a second antigen-binding domain and a third antigen-binding domain. In some embodiments, the first antigen-binding domain, the second antigen-binding domain, and the third antigen-binding domain have structures selected from the group consisting of Fab, Fab', F(ab')2, Fd, Fv, scFv, dsFv, dAb, and VHH.

In some embodiments, provided is the heteromultimer according to any one of the foregoing, wherein the first polypeptide is an antibody heavy chain, and/or the second polypeptide is an antibody heavy chain. In some embodiments, the heteromultimer further comprises one or more antibody light chains.

In some embodiments, the sequences of the first polypeptide and the second polypeptide are different. The first polypeptide or the second polypeptide is only used to distinguish amino acid sequences, and does not limit the positional relationships between polypeptides and proteins. For example, in a heterodimer consisting of one first polypeptide and one second polypeptide, the first polypeptide and the second polypeptide are two polypeptides whose amino acid sequences are different. When any one of the polypeptides is the first polypeptide, the other is the second polypeptide.

In some embodiments, provided is the heteromultimer according to any one of the foregoing, wherein amino acids in a core hinge region of the heteromultimer form a disulfide bond.

In some embodiments, provided is the heteromultimer according to any one of the foregoing, wherein the CH3 domains of the first polypeptide and the second polypeptide are derived from an IgG. In some embodiments, provided is the heteromultimer according to any one of the foregoing, wherein the CH3 domains of the first polypeptide and the second polypeptide are derived from IgG1, IgG2, IgG3, or IgG4. In some embodiments, provided is the heteromultimer according to any one of the foregoing, wherein the CH3 domains are derived from IgG1, IgG2, or IgG3. In some embodiments, provided is the heteromultimer according to any one of the foregoing, wherein the CH3 domains of the first polypeptide and the second polypeptide are derived from IgG1. In some embodiments, the CH3 domains of the first polypeptide and the second polypeptide are derived from human IgG1. In some embodiments, the human IgG1 comprises the amino acid sequence set forth in SEQ ID NO: 39, 40, or 41.

In some embodiments, provided is the heteromultimer according to any one of the foregoing, wherein the heteromultimer comprises at least one replaced Fab comprising a Titin chain and an Obscurin chain that are capable of forming a dimer. In some embodiments, provided is the heteromultimer according to any one of the foregoing, wherein the replaced Fab is obtained by replacing the original CH1 and CL of a Fab with the Obscurin chain and the Titin chain, respectively, or the replaced Fab is obtained by replacing the original CH1 and CL of a Fab with the Titin chain and the Obscurin chain, respectively. In some embodiments, the Titin chain comprises the amino acid sequence set forth in SEQ ID NO: 6, and the Obscurin chain comprises the amino acid sequence set forth in SEQ ID NO: 5.

In another aspect, the present disclosure provides a heteromultimer comprising a first polypeptide and a second polypeptide, wherein:
the first polypeptide comprises one CH3 domain, wherein the CH3 domain of the first polypeptide comprises amino acid mutations of 356K and 351I, and
the second polypeptide comprises one CH3 domain, wherein the CH3 domain of the second polypeptide comprises amino acid mutations of 439E and 351I; the mutation sites are indicated by EU numbering.

In some embodiments, provided is the aforementioned heteromultimer, wherein the first polypeptide comprises one CH3 domain, wherein the CH3 domain of the first polypeptide comprises amino acid mutations of D356K and L351I, and
the second polypeptide comprises one CH3 domain, wherein the CH3 domain of the second polypeptide comprises amino acid mutations of K439E and L351I; the mutation sites are indicated by EU numbering; or
the first polypeptide comprises one CH3 domain, wherein the CH3 domain of the first polypeptide comprises amino acid mutations of E356K and L351I, and
the second polypeptide comprises one CH3 domain, wherein the CH3 domain of the second polypeptide comprises amino acid mutations of K439E and L351I; the mutation sites are indicated by EU numbering.

In another aspect, the present disclosure provides a heteromultimer comprising a first polypeptide and a second polypeptide, wherein:
the first polypeptide comprises one CH3 domain, wherein the CH3 domain of the first polypeptide comprises amino acid mutations of 356K and 349C, and
the second polypeptide comprises one CH3 domain, wherein the CH3 domain of the second polypeptide comprises amino acid mutations of 439E, 354C, and 351I; the mutation sites are indicated by EU numbering.

In some embodiments, provided is the aforementioned heteromultimer, wherein the first polypeptide comprises one CH3 domain, wherein the CH3 domain of the first polypeptide comprises amino acid mutations of D356K and Y349C, and
the second polypeptide comprises one CH3 domain, wherein the CH3 domain of the second polypeptide comprises amino acid mutations of K439E, S354C, and L351I; the mutation sites are indicated by EU numbering; or
the first polypeptide comprises one CH3 domain, wherein the CH3 domain of the first polypeptide comprises amino acid mutations of E356K and Y349C, and
the second polypeptide comprises one CH3 domain, wherein the CH3 domain of the second polypeptide comprises amino acid mutations of K439E, S354C, and L351I; the mutation sites are indicated by EU numbering.

In another aspect, the present disclosure provides a heteromultimer comprising a first polypeptide and a second polypeptide, wherein:
the first polypeptide comprises one CH3 domain, wherein the CH3 domain of the first polypeptide comprises an amino acid mutation of 349S, and
the second polypeptide comprises one CH3 domain, wherein the CH3 domain of the second polypeptide comprises an amino acid mutation of 364Y; the mutation sites are indicated by EU numbering.

In some embodiments, provided is the aforementioned heteromultimer, wherein the first polypeptide comprises one CH3 domain, wherein the CH3 domain of the first polypeptide comprises an amino acid mutation of Y349S, and
the second polypeptide comprises one CH3 domain, wherein the CH3 domain of the second polypeptide comprises an amino acid mutation of S364Y; the mutation sites are indicated by EU numbering.

In another aspect, the present disclosure provides a heteromultimer prepared by an extracellular Fab-arm exchange reaction (extracellular half-antibody recombination reaction), comprising a first polypeptide and a second polypeptide, wherein:
the first polypeptide comprises one CH3 domain, wherein the CH3 domain of the first polypeptide comprises an amino acid mutation of 349S, and
the second polypeptide comprises one CH3 domain, wherein the CH3 domain of the second polypeptide comprises an amino acid mutation of 364Y; the mutation sites are indicated by EU numbering.

In some embodiments, provided is the aforementioned heteromultimer prepared by an extracellular Fab-arm exchange reaction (extracellular half-antibody recombination reaction), wherein the first polypeptide comprises one CH3 domain, wherein the CH3 domain of the first polypeptide comprises an amino acid mutation of Y349S, and
the second polypeptide comprises one CH3 domain, wherein the CH3 domain of the second polypeptide comprises an amino acid mutation of S364Y; the mutation sites are indicated by EU numbering.

In another aspect, the present disclosure provides a heteromultimer comprising a first polypeptide and a second polypeptide, wherein:
the first polypeptide comprises one CH3 domain, wherein the CH3 domain of the first polypeptide comprises an amino acid mutation of 349C, and
the second polypeptide comprises one CH3 domain, wherein the CH3 domain of the second polypeptide comprises amino acid mutations of 354C and 364Y; the mutation sites are indicated by EU numbering.

In some embodiments, provided is the aforementioned heteromultimer, wherein the first polypeptide comprises one CH3 domain, wherein the CH3 domain of the first polypeptide comprises an amino acid mutation of Y349C, and
the second polypeptide comprises one CH3 domain, wherein the CH3 domain of the second polypeptide comprises amino acid mutations of S354C and S364Y; the mutation sites are indicated by EU numbering.

In another aspect, the present disclosure provides a pharmaceutical composition comprising the heteromultimer according to any one of the foregoing and one or more pharmaceutically acceptable carriers, diluents, or excipients.

In another aspect, the present disclosure provides an immunoconjugate comprising the heteromultimer according to any one of the foregoing and an effector, wherein the effector is conjugated to the heteromultimer. In some embodiments, the effector is selected from the group consisting of an anti-tumor agent, an immunomodulator, a biological response modifier, a lectin, a cytotoxic drug, a chromophore, a fluorophore, a chemiluminescent compound, an enzyme, and a metal ion, and any combination thereof.

In another aspect, the present disclosure provides a method for preparing an immunoconjugate, comprising the following steps:
a) a step of providing a molecule comprising a first polypeptide homomer and a molecule comprising a second polypeptide homomer,
b) mixing the molecule comprising the first polypeptide homomer and the molecule comprising the second polypeptide homomer well to form a mixture,
c) adding a reductant to the mixture and incubating the resulting mixture, and
d) obtaining an immunoconjugate comprising the first polypeptide and the second polypeptide,

wherein the first polypeptide and the second polypeptide each comprise one CH3 domain, and the CH3 domains each comprise one or more mutations that promote heterologous formation;
the molecule comprising the first polypeptide homomer and the molecule comprising the second polypeptide homomer comprise an Fc region conjugated with a prodrug, peptide, drug, or toxin.

In some embodiments, the molecule comprising the first polypeptide homomer and the molecule comprising the second polypeptide homomer comprise an Fc region conjugated with a toxin.

In some embodiments, the molecule comprising the first polypeptide homomer and the molecule comprising the second polypeptide homomer are antibody-drug conjugates.

In another aspect, the present disclosure provides a method for preparing an immunoconjugate, comprising:
conjugating a heteromultimer obtained by any one of the methods for preparing a heteromultimer described in the present disclosure with a toxin.

In another aspect, the present disclosure provides one or more isolated nucleic acids encoding the first polypeptide and/or the second polypeptide of the heteromultimer according to any one of the foregoing.

In another aspect, the present disclosure provides one or more vectors comprising the one or more isolated nucleic acids according to any one of the foregoing.

In another aspect, the present disclosure provides one or more host cells comprising the one or more isolated nucleic acids according to any one of the foregoing.

In another aspect, the present disclosure provides a method for preparing the heteromultimer according to any one of the foregoing, comprising expressing the one or more isolated nucleic acids according to any one of the foregoing or culturing the one or more host cells according to any one of the foregoing to produce the heteromultimer.

In another aspect, the present disclosure provides a method for preparing the heteromultimer according to any one of the foregoing, comprising: (a) a step of altering a nucleic acid encoding amino acid residues that form an interface between the polypeptides, (b) a step of culturing a host cell comprising the nucleic acid to express the polypeptides, (c) a step of recovering the polypeptides from the culture of the host cell, and (d) a step of incubating the polypeptides in the presence of a reductant and recovering the desired heteromultimer.

In another aspect, the present disclosure provides a method for preparing a multispecific antibody by a recombination reaction, comprising the following steps:
a) a step of providing a first parental antibody,
b) a step of providing a second parental antibody,
c) a step of co-incubating the first parental antibody and the second parental antibody under reducing conditions sufficient to allow reduction of interchain disulfide bonds in the hinge region, and
d) a step of obtaining the multispecific antibody, wherein:
   the CH3 domain(s) of the first parental antibody and/or the second parental antibody comprise(s) at least one amino acid mutation selected from the group consisting of positions 347, 349, 351, 354, 356, 357, 364, 366, 368, 394, 397, 399, 405, 407, 409, 411, and 439;
   the first parental antibody and the second parental antibody bind to different antigens or epitopes, and mutation sites in the CH3 domains are indicated by EU numbering.

The present disclosure provides a method for preparing a multispecific antibody by a recombination reaction, comprising the following steps:
a) a step of providing a first parental antibody,
b) a step of providing a second parental antibody,
c) a step of co-incubating the first parental antibody and the second parental antibody under reducing conditions sufficient to allow reduction of interchain disulfide bonds in the hinge region, and
d) a step of obtaining the multispecific antibody, wherein:
   the CH3 domain(s) of the first parental antibody and/or the second parental antibody comprise(s) at least one amino acid mutation selected from the group consisting of positions 349, 351, 354, 356, 357, 364, 366, 368, 394, 397, 405, 407, 409, 411, and 439;
   the first parental antibody and the second parental antibody bind to different antigens or epitopes, and mutation sites in the CH3 domains are indicated by EU numbering.

In another aspect, the present disclosure provides a method for preparing a multispecific antibody by a recombination reaction, comprising the following steps:
a) a step of providing a first parental antibody,
b) a step of providing a second parental antibody,
c) a step of co-incubating the first parental antibody and the second parental antibody under reducing conditions sufficient to allow reduction of interchain disulfide bonds in the hinge region, and
d) a step of obtaining the multispecific antibody, wherein:
   the CH3 domain(s) of the first parental antibody and/or the second parental antibody comprise(s) at least one amino acid mutation selected from the group consisting of positions 347, 349, 351, 354, 356, 357, 364, 366, 368, 394, 399, 405, 407, 411, and 439;
   the first parental antibody and the second parental antibody bind to different antigens or epitopes, and mutation sites in the CH3 domains are indicated by EU numbering;
   with the proviso that the following two instances are not included:
      i) the CH3 domain of the first parental antibody comprises only one amino acid mutation at position 356, and the CH3 domain of the second parental antibody comprises only one amino acid mutation at position 439; and
      ii) the CH3 domain of the first parental antibody comprises only one amino acid mutation at position 405, and the CH3 domain of the second parental antibody comprises only one amino acid mutation at position 409.

In another aspect, the present disclosure provides a method for preparing a multispecific antibody by a recombination reaction, comprising the following steps:
a) a step of providing a first parental antibody,
b) a step of providing a second parental antibody,
c) a step of co-incubating the first parental antibody and the second parental antibody under reducing conditions sufficient to allow reduction of interchain disulfide bonds in the hinge region, and
d) a step of obtaining the multispecific antibody, wherein:
   the CH3 domain(s) of the first parental antibody and/or the second parental antibody comprise(s) at least one amino acid mutation selected from the group consisting of positions 349, 351, 354, 356, 357, 364, 366, 368, 394, 397, 405, 407, 409, 411, and 439;
   the first parental antibody and the second parental antibody bind to different antigens or epitopes, and mutation sites in the CH3 domains are indicated by EU numbering;
   with the proviso that the following two instances are not included:
      i) the CH3 domain of the first parental antibody comprises only one amino acid mutation at position 356, and the CH3 domain of the second parental antibody comprises only one amino acid mutation at position 439; and
      ii) the CH3 domain of the first parental antibody comprises only one amino acid mutation at position 405, and the CH3 domain of the second parental antibody comprises only one amino acid mutation at position 409.

In some embodiments, provided is the aforementioned method for preparing a multispecific antibody, wherein the CH3 domain(s) of the first parental antibody and/or the second parental antibody comprise(s) at least one amino acid mutation selected from the group consisting of positions 349, 351, 354, 356, 364, 366, 368, 394, 405, 409, 411, and 439.

In some embodiments, provided is the aforementioned method for preparing a multispecific antibody, wherein the CH3 domain(s) of the first parental antibody and/or the second parental antibody comprise(s) at least one amino acid mutation selected from the group consisting of the following combinations:
the amino acid at position 356 (EU numbering) is mutated into Lys (K); or
the amino acid at position 439 (EU numbering) is mutated into Glu (E); or
the amino acid at position 349 (EU numbering) is mutated into Leu (L), Phe (F), Ser (S), Cys (C), Ala (A), Val (V), Thr (T), or Gly (G); or
the amino acid at position 351 (EU numbering) is mutated into Cys (C), Val (V), Thr (T), Ile (I), Phe (F), or Met (M); or
the amino acid at position 364 (EU numbering) is mutated into Ala (A), Val (V), Thr (T), Leu (L), Tyr (Y), or Phe (F); or
the amino acid at position 366 (EU numbering) is mutated into Gly (G), Ser (S), Ala (A), Val (V), Leu (L), His (H), or Ile (I); or
the amino acid at position 368 (EU numbering) is mutated into Val (V), Ile (I), Met (M), or Ala (A); or
the amino acid at position 394 (EU numbering) is mutated into Phe (F), Ala (A), Ser (S), Cys (C), Val (V), or Asn (N); or
the amino acid at position 397 (EU numbering) is mutated into Thr (T), Ile (I), or Leu (L); or the amino acid at position 405 (EU numbering) is mutated into Thr (T), Leu (L), or Tyr (Y); or
the amino acid at position 407 (EU numbering) is mutated into Cys (C), Val (V), Leu (L), His (H), or Phe (F); or
the amino acid at position 409 (EU numbering) is mutated into Gln (Q), Arg (R), or Asp (D); or
the amino acid at position 411 (EU numbering) is mutated into Asn (N), Tyr (Y), or Leu (L); or
the amino acid at position 354 (EU numbering) is mutated into Tyr (Y), Cys (C), Phe (F), or Trp (W); or
the amino acid at position 357 (EU numbering) is mutated into Cys (C); or
the amino acid at position 347 (EU numbering) is mutated into Glu (E); or
the amino acid at position 399 (EU numbering) is mutated into Lys (K).

In some embodiments, provided is the aforementioned method for preparing a multispecific antibody, wherein the CH3 domain(s) of the first parental antibody and/or the second parental antibody comprise(s) at least one amino acid mutation selected from the group consisting of the following combinations:
position 356 (EU numbering) is mutated into Lys (K); or
position 439 (EU numbering) is mutated into Glu (E); or
position 349 (EU numbering) is mutated into Leu (L), Phe (F), Ser (S), or Cys (C); or
position 351 (EU numbering) is mutated into Cys (C), Val (V), Thr (T), Ile (I), or Phe (F); or
position 364 (EU numbering) is mutated into Ala (A), Val (V), Thr (T), or Leu (L); or
position 366 (EU numbering) is mutated into Gly (G), Ser (S), Ala (A), Val (V), Leu (L), or His (H); or
position 368 (EU numbering) is mutated into Val (V), Ile (I), or Met (M); or
position 394 (EU numbering) is mutated into Phe (F), Ala (A), Ser (S), Cys (C), Val (V), or Asn (N); or
position 397 (EU numbering) is mutated into Thr (T), Ile (I), or Leu (L); or
position 405 (EU numbering) is mutated into Thr (T), Leu (L), or Tyr (Y); or
position 407 (EU numbering) is mutated into Cys (C), Val (V), Leu (L), or His (H); or
position 409 (EU numbering) is mutated into Gln (Q) or Arg (R); or
position 411 (EU numbering) is mutated into Asn (N) or Tyr (Y); or
position 354 (EU numbering) is mutated into Tyr (Y) or Cys (C); or
position 357 (EU numbering) is mutated into Cys (C). In some embodiments, provided is the aforementioned method for preparing a multispecific antibody, wherein:
   1) the CH3 domain of the first parental antibody comprises amino acid mutations at positions 356 and 349, and the CH3 domain of the second parental antibody comprises an amino acid mutation at position 439, wherein the CH3 domain of the second parental antibody further comprises an amino acid mutation at position 354 or 357; or
   2) the CH3 domain of the first parental antibody comprises an amino acid mutation at position 405, and the CH3 domain of the second parental antibody comprises an amino acid mutation at position 394; or
   3) the CH3 domain of the first parental antibody comprises an amino acid mutation at position 356, and the CH3 domain of the second parental antibody comprises an amino acid mutation at position 439, wherein the CH3 domains of the first and second parental antibodies each further comprise one identical or different amino acid mutation selected from the group consisting of positions 349, 351, 364, 366, 368, 394, 397, 405, 407, 409, and 411; or
   4) the CH3 domain of the first parental antibody comprises amino acid mutations at positions 356 and 349, and the CH3 domain of the second parental antibody comprises amino acid mutations at positions 439 and 354, wherein the CH3 domain of the second parental antibody further comprises one amino acid mutation selected from the group consisting of positions 351, 366, and 368; or
   5) the CH3 domain of the first parental antibody comprises amino acid mutations at positions 356 and 405, and the CH3 domain of the second parental antibody comprises amino acid mutations at positions 439 and 394; or
   6) the CH3 domain of the first parental antibody comprises an amino acid mutation at position 349, and the CH3 domain of the second parental antibody comprises an amino acid mutation at position 357; or
   7) the CH3 domain of the first parental antibody comprises an amino acid mutation at position 349, and the CH3 domain of the second parental antibody comprises an amino acid mutation at position 354, wherein the CH3 domain of the second parental antibody further comprises one amino acid mutation selected from the group consisting of positions 351, 364, 366, 368, and 405; or
   8) the CH3 domain of the first parental antibody comprises an amino acid mutation at position 349, and the CH3 domain of the second parental antibody comprises an amino acid mutation at position 364 or 354; or
   9) the CH3 domain of the first parental antibody comprises an amino acid mutation at position 366, and the CH3 domain of the second parental antibody comprises an amino acid mutation at position 405 or 407; or
   10) the CH3 domain of the first parental antibody comprises an amino acid mutation at position 349, and the CH3 domain of the second parental antibody comprises amino acid mutations at positions 347 and 357; or
   11) the CH3 domain of the first parental antibody comprises amino acid mutations at positions 356 and 366, and the CH3 domain of the second parental antibody comprises an amino acid mutation at position 439, wherein the CH3 domain of the second parental antibody further comprises an amino acid mutation at position 405 or 407; or
   12) the CH3 domain of the first parental antibody comprises amino acid mutations at positions 356 and 407, and the CH3 domain of the second parental antibody comprises amino acid mutations at positions 439 and 366; or
   13) the CH3 domain of the first parental antibody comprises amino acid mutations at positions 354 and 405, and the CH3 domain of the second parental antibody comprises amino acid mutations at positions 349 and 394; or
   14) the CH3 domain of the first parental antibody comprises amino acid mutations at positions 356 and 409, and the CH3 domain of the second parental antibody comprises amino acid mutations at positions 439 and 399.

In some embodiments, provided is the aforementioned method for preparing a multispecific antibody, wherein:
i) the CH3 domain of the first parental antibody comprises amino acid mutations at positions 356 and 349, and the CH3 domain of the second parental antibody comprises an amino acid mutation at position 439, wherein the CH3 domain of the second parental antibody further comprises one amino acid mutation selected from the group consisting of positions 354 and 357; or
ii) the CH3 domain of the first parental antibody comprises an amino acid mutation at position 405, and the CH3 domain of the second parental antibody comprises an amino acid mutation at position 394; or
iii) the CH3 domain of the first parental antibody comprises an amino acid mutation at position 356, and the CH3 domain of the second parental antibody comprises an amino acid mutation at position 439, wherein the CH3 domains of the first and second parental antibodies each further comprise one identical or different amino acid mutation selected from the group consisting of positions 349, 351, 364, 366, 368, 394, 397, 405, 407, 409, and 411; or
iv) the CH3 domain of the first parental antibody comprises amino acid mutations at positions 356 and 349, and the CH3 domain of the second parental antibody comprises amino acid mutations at positions 439 and 354, wherein the CH3 domain of the second parental antibody further comprises one amino acid mutation selected from the group consisting of positions 351, 366, and 368; or
v) the CH3 domain of the first parental antibody comprises amino acid mutations at positions 356 and 405, and the CH3 domain of the second parental antibody comprises amino acid mutations at positions 439 and 394; or
vi) the CH3 domain of the first parental antibody comprises an amino acid mutation at position 349, and the CH3 domain of the second parental antibody comprises an amino acid mutation at position 357; or
vii) the CH3 domain of the first parental antibody comprises an amino acid mutation at position 349, and the CH3 domain of the second parental antibody comprises an amino acid mutation at position 354, wherein the CH3 domain of the second parental antibody further comprises one amino acid mutation selected from the group consisting of positions 366, 368, and 405.

In some embodiments, provided is the aforementioned method for preparing a multispecific antibody, wherein:
1) the CH3 domain of the first parental antibody comprises amino acid mutations at positions 356 and 349, and the CH3 domain of the second parental antibody comprises amino acid mutations at positions 439 and 354; or
2) the CH3 domain of the first parental antibody comprises an amino acid mutation at position 405, and the CH3 domain of the second parental antibody comprises an amino acid mutation at position 394; or
3) the CH3 domain of the first parental antibody comprises an amino acid mutation at position 356, and the CH3 domain of the second parental antibody comprises an amino acid mutation at position 439, wherein the CH3 domains of the first and second parental antibodies each further comprise one identical amino acid mutation selected from the group consisting of positions 351, 364, 366, 368, 394, 405, 409, and 411; or
4) the CH3 domain of the first parental antibody comprises amino acid mutations at positions 356 and 349, and the CH3 domain of the second parental antibody comprises amino acid mutations at positions 439, 354, and 351; or
5) the CH3 domain of the first parental antibody comprises an amino acid mutation at position 349, and the CH3 domain of the second parental antibody comprises amino acid mutations at positions 354 and 364; or
6) the CH3 domain of the first parental antibody comprises an amino acid mutation at position 349, and the CH3 domain of the second parental antibody comprises an amino acid mutation at position 364 or 354.

In some embodiments, provided is the aforementioned method for preparing a multispecific antibody, wherein:
i) the CH3 domain of the first parental antibody comprises amino acid mutations at positions 356 and 349, and the CH3 domain of the second parental antibody comprises amino acid mutations at positions 439 and 354; or
ii) the CH3 domain of the first parental antibody comprises an amino acid mutation at position 405, and the CH3 domain of the second parental antibody comprises an amino acid mutation at position 394; or
iii) the CH3 domain of the first parental antibody comprises an amino acid mutation at position 356, and the CH3 domain of the second parental antibody comprises an amino acid mutation at position 439, wherein the CH3 domains of the first and second parental antibodies each further comprise one identical amino acid mutation selected from the group consisting of positions 351, 364, 366, 368, 394, 405, 409, and 411; or
iv) the CH3 domain of the first parental antibody comprises amino acid mutations at positions 356 and 349, and the CH3 domain of the second parental antibody comprises amino acid mutations at positions 439 and 354, wherein the CH3 domain of the second parental antibody further comprises an amino acid mutation at position 351. In some embodiments, provided is the aforementioned method for preparing a multispecific antibody, wherein:
   the CH3 domain of the first parental antibody comprises an amino acid mutation of 356K, and the CH3 domain of the second parental antibody comprises an amino acid mutation of 439E, wherein the CH3 domains of the first and second parental antibodies each further comprise one identical amino acid mutation selected from the group consisting of positions 351, 364, 366, 368, 394, 405, 409, and 411, wherein:
   the amino acid at position 351 (EU numbering) is mutated into Cys (C), Val (V), Thr (T), Ile (I), Phe (F), or Met (M); or
   the amino acid at position 364 (EU numbering) is mutated into Ala (A), Val (V), Thr (T), or Leu (L); or
   the amino acid at position 366 (EU numbering) is mutated into Ser (S), Ala (A), Val (V), Leu (L), His (H), or Ile (I); or
   the amino acid at position 368 (EU numbering) is mutated into Val (V), Ile (I), Met (M), or Ala (A); or
   the amino acid at position 394 (EU numbering) is mutated into Ala (A), Ser (S), Cys (C), Val (V), or Asn (N); or
   the amino acid at position 405 (EU numbering) is mutated into Leu (L) or Tyr (Y); or
   the amino acid at position 407 (EU numbering) is mutated into Phe (F); or
   the amino acid at position 409 (EU numbering) is mutated into Gln (Q); or
   the amino acid at position 411 (EU numbering) is mutated into Asn (N), Tyr (Y), or Leu (L).

In some embodiments, provided is the aforementioned method for preparing a multispecific antibody, wherein:
the CH3 domain of the first parental antibody comprises an amino acid mutation of 356K, and the CH3 domain of the second parental antibody comprises an amino acid mutation of 439E, wherein the CH3 domains of the first and second parental antibodies each further comprise one identical amino acid mutation selected from the group consisting of positions 351, 364, 366, 368, 394, 405, 409, and 411, wherein:
position 351 (EU numbering) is mutated into Cys (C), Val (V), Thr (T), Ile (I), or Phe (F);
   or
position 364 (EU numbering) is mutated into Ala (A), Val (V), Thr (T), or Leu (L); or
position 366 (EU numbering) is mutated into Ser (S), Ala (A), Val (V), Leu (L), or His (H); or
position 368 (EU numbering) is mutated into Val (V), Ile (I), or Met (M); or
position 394 (EU numbering) is mutated into Ala (A), Ser (S), Cys (C), Val (V), or Asn (N); or
position 405 (EU numbering) is mutated into Leu (L) or Tyr (Y); or
position 409 (EU numbering) is mutated into Gln (Q) or Arg (R); or
position 411 (EU numbering) is mutated into Asn (N) or Tyr (Y). In some embodiments, provided is the aforementioned method for preparing a multispecific antibody, wherein:
   1) the CH3 domain of the first parental antibody comprises amino acid mutations of 356K and 349S, and the CH3 domain of the second parental antibody comprises amino acid mutations of 439E and 354Y; or
      the CH3 domain of the first parental antibody comprises amino acid mutations of 356K and 349C, and the CH3 domain of the second parental antibody comprises amino acid mutations of 439E and 357C; or
   2) the CH3 domain of the first parental antibody comprises an amino acid mutation of 405T, and the CH3 domain of the second parental antibody comprises an amino acid mutation of 394F; or
   3) the CH3 domain of the first parental antibody comprises an amino acid mutation of 356K, and the CH3 domain of the second parental antibody comprises an amino acid mutation of 439E, wherein the CH3 domains of the first and second parental antibodies each further comprise one identical or different amino acid mutation selected from the group consisting of 349L, 351C, 351V, 351T, 351I, 351F, 351M, 364A, 364T, 364L, 366G, 366A, 366V, 366L, 366H, 366I, 368V, 368I, 368A, 394A, 394S, 394C, 394V, 394N, 397I, 397L, 405L, 405Y, 407C, 407V, 407L, 407H, 407F, 409Q, 409R, 411L, and 411Y; or
   4) the CH3 domain of the first parental antibody comprises amino acid mutations of 356K and 349C, and the CH3 domain of the second parental antibody comprises amino acid mutations of 439E and 354C, wherein the CH3 domain of the second parental antibody further comprises one amino acid mutation selected from the group consisting of 351I, 366A, and 368I; or
   5) the CH3 domain of the first parental antibody comprises amino acid mutations of 356K and 405T, and the CH3 domain of the second parental antibody comprises amino acid mutations of 439E and 394F; or
   6) the CH3 domain of the first parental antibody comprises an amino acid mutation of 349C, and the CH3 domain of the second parental antibody comprises an amino acid mutation of 357C; or
   7) the CH3 domain of the first parental antibody comprises an amino acid mutation of 349C, and the CH3 domain of the second parental antibody comprises an amino acid mutation of 354C, wherein the CH3 domain of the second parental antibody further comprises one amino acid mutation selected from the group consisting of 351I, 364Y, 364F, 366A, 368I, and 405Y; or
   8) the CH3 domain of the first parental antibody comprises an amino acid mutation of 349S, and the CH3 domain of the second parental antibody comprises one amino acid mutation selected from the group consisting of 364Y, 354F, 354W, and 354Y; or
the CH3 domain of the first parental antibody comprises an amino acid mutation of 349G, and the CH3 domain of the second parental antibody comprises an amino acid mutation of 354W; or
the CH3 domain of the first parental antibody comprises an amino acid mutation of 349A, and the CH3 domain of the second parental antibody comprises one amino acid mutation selected from the group consisting of 354F, 354Y, and 354W; or
the CH3 domain of the first parental antibody comprises an amino acid mutation of 349V, and the CH3 domain of the second parental antibody comprises an amino acid mutation of 354F; or
the CH3 domain of the first parental antibody comprises an amino acid mutation of 349T, and the CH3 domain of the second parental antibody comprises an amino acid mutation of 354F; or
9) the CH3 domain of the first parental antibody comprises an amino acid mutation of 366H, and the CH3 domain of the second parental antibody comprises an amino acid mutation of 405L or 407L; or
10) the CH3 domain of the first parental antibody comprises an amino acid mutation of 349C, and the CH3 domain of the second parental antibody comprises amino acid mutations of 347E and 357C; or
11) the CH3 domain of the first parental antibody comprises amino acid mutations of 356K and 366H, and the CH3 domain of the second parental antibody comprises an amino acid mutation of 439E, wherein the CH3 domain of the second parental antibody further comprises one amino acid mutation selected from the group consisting of 405L, 407L, and 407H; or
12) the CH3 domain of the first parental antibody comprises amino acid mutations of 356K and 407L, and the CH3 domain of the second parental antibody comprises amino acid mutations of 439E and 366H; or
the CH3 domain of the first parental antibody comprises amino acid mutations of 356K and 407H, and the CH3 domain of the second parental antibody comprises amino acid mutations of 439E and 366H; or
13) the CH3 domain of the first parental antibody comprises amino acid mutations of 354C and 405T, and the CH3 domain of the second parental antibody comprises amino acid mutations of 349C and 394F; or
14) the CH3 domain of the first parental antibody comprises amino acid mutations of 356K and 409D, and the CH3 domain of the second parental antibody comprises amino acid mutations of 439E and 399K.

In some embodiments, provided is the aforementioned method for preparing a multispecific antibody, wherein:
i) the CH3 domain of the first parental antibody comprises amino acid mutations of 356K and 349S, and the CH3 domain of the second parental antibody comprises amino acid mutations of 439E and 354Y; or
   the CH3 domain of the first parental antibody comprises amino acid mutations of 356K and 349C, and the CH3 domain of the second parental antibody comprises amino acid mutations of 439E and 357C; or
ii) the CH3 domain of the first parental antibody comprises an amino acid mutation of 405T, and the CH3 domain of the second parental antibody comprises an amino acid mutation of 394F; or
iii) the CH3 domain of the first parental antibody comprises an amino acid mutation of 356K, and the CH3 domain of the second parental antibody comprises an amino acid mutation of 439E, wherein the CH3 domains of the first and second parental antibodies each further comprise one identical or different amino acid mutation selected from the group consisting of 349L, 351C, 351V, 351T, 351I, 351F, 364A, 364T, 364L, 366G, 366A, 366V, 366L, 366H, 368V, 368I, 394A, 394S, 394C, 394V, 394N, 397I, 397L, 405L, 405Y, 407C, 407V, 407L, 407H, 409Q, 409R, and 411Y; or
iv) the CH3 domain of the first parental antibody comprises amino acid mutations of 356K and 349C, and the CH3 domain of the second parental antibody comprises amino acid mutations of 439E and 354C, wherein the CH3 domain of the second parental antibody further comprises one amino acid mutation selected from the group consisting of 351I, 366A, and 368I; or
v) the CH3 domain of the first parental antibody comprises amino acid mutations of 356K and 405T, and the CH3 domain of the second parental antibody comprises amino acid mutations of 439E and 394F; or
vi) the CH3 domain of the first parental antibody comprises an amino acid mutation of 349C, and the CH3 domain of the second parental antibody comprises an amino acid mutation of 357C; or
vii) the CH3 domain of the first parental antibody comprises an amino acid mutation of 349C, and the CH3 domain of the second parental antibody comprises an amino acid mutation of 354C, wherein the CH3 domain of the second parental antibody further comprises one amino acid mutation selected from the group consisting of 366A, 368I, and 405Y.

In some embodiments, provided is the aforementioned method for preparing a multispecific antibody, wherein:
1) the CH3 domain of the first parental antibody comprises amino acid mutations of 356K and 349S, and the CH3 domain of the second parental antibody comprises amino acid mutations of 439E and 354Y; or
2) the CH3 domain of the first parental antibody comprises an amino acid mutation of 405T, and the CH3 domain of the second parental antibody comprises an amino acid mutation of 394F; or
3) the CH3 domain of the first parental antibody comprises an amino acid mutation of 356K, and the CH3 domain of the second parental antibody comprises an amino acid mutation of 439E, wherein the CH3 domains of the first and second parental antibodies each further comprise one identical amino acid mutation selected from the group consisting of 351T, 351I, 364A, 366A, 368I, 394A, 394S, 405Y, 409Q, and 411Y; or
4) the CH3 domain of the first parental antibody comprises amino acid mutations of 356K and 349C, and the CH3 domain of the second parental antibody comprises amino acid mutations of 439E, 354C, and 351I; or
5) the CH3 domain of the first parental antibody comprises an amino acid mutation of 349C, and the CH3 domain of the second parental antibody comprises an amino acid mutation of 354C, wherein the CH3 domain of the second parental antibody further comprises an amino acid mutation of 364Y or 364F; or
6) the CH3 domain of the first parental antibody comprises an amino acid mutation of 349S, and the CH3 domain of the second parental antibody comprises an amino acid mutation of 364Y; or
the CH3 domain of the first parental antibody comprises an amino acid mutation of 349G, and the CH3 domain of the second parental antibody comprises an amino acid mutation of 354W.

In some embodiments, provided is the aforementioned method for preparing a multispecific antibody, wherein:
i) the CH3 domain of the first parental antibody comprises amino acid mutations of 356K and 349S, and the CH3 domain of the second parental antibody comprises amino acid mutations of 439E and 354Y; or
ii) the CH3 domain of the first parental antibody comprises an amino acid mutation of 405T, and the CH3 domain of the second parental antibody comprises an amino acid mutation of 394F; or
iii) the CH3 domain of the first parental antibody comprises an amino acid mutation of 356K, and the CH3 domain of the second parental antibody comprises an amino acid mutation of 439E, wherein the CH3 domains of the first and second parental antibodies each further comprise one identical amino acid mutation selected from the group consisting of 351T, 351I, 364A, 366A, 368I, 394A, 394S, 405Y, 409Q, and 411Y; or
iv) the CH3 domain of the first parental antibody comprises amino acid mutations of 356K and 349C, and the CH3 domain of the second parental antibody comprises amino acid mutations of 439E, 354C, and 351I.

In some embodiments, provided is the aforementioned method for preparing a multispecific antibody, wherein:
1) the CH3 domain of the first parental antibody comprises amino acid mutations of 356K and 349S, and the CH3 domain of the second parental antibody comprises amino acid mutations of 439E and 354Y; or
   the CH3 domain of the first parental antibody comprises amino acid mutations of 356K and 349C, and the CH3 domain of the second parental antibody comprises amino acid mutations of 439E and 357C; or
2) the CH3 domain of the first parental antibody comprises an amino acid mutation of 405T, and the CH3 domain of the second parental antibody comprises an amino acid mutation of 394F; or
3) the CH3 domain of the first parental antibody comprises amino acid mutations of 356K and 349L, and the CH3 domain of the second parental antibody comprises amino acid mutations of 439E and 349L; or
   the CH3 domain of the first parental antibody comprises amino acid mutations of 356K and 351C, and the CH3 domain of the second parental antibody comprises amino acid mutations of 439E and 351C; or
   the CH3 domain of the first parental antibody comprises amino acid mutations of 356K and 351V, and the CH3 domain of the second parental antibody comprises amino acid mutations of 439E and 351V; or
   the CH3 domain of the first parental antibody comprises amino acid mutations of 356K and 351T, and the CH3 domain of the second parental antibody comprises amino acid mutations of 439E and 351T; or
   the CH3 domain of the first parental antibody comprises amino acid mutations of 356K and 351I, and the CH3 domain of the second parental antibody comprises amino acid mutations of 439E and 351I; or
   the CH3 domain of the first parental antibody comprises amino acid mutations of 356K and 351F, and the CH3 domain of the second parental antibody comprises amino acid mutations of 439E and 351F; or
   the CH3 domain of the first parental antibody comprises amino acid mutations of 356K and 351M, and the CH3 domain of the second parental antibody comprises amino acid mutations of 439E and 351M; or
   the CH3 domain of the first parental antibody comprises amino acid mutations of 356K and 364A, and the CH3 domain of the second parental antibody comprises amino acid mutations of 439E and 364A; or
   the CH3 domain of the first parental antibody comprises amino acid mutations of 356K and 364T, and the CH3 domain of the second parental antibody comprises amino acid mutations of 439E and 364T; or
   the CH3 domain of the first parental antibody comprises amino acid mutations of 356K and 364L, and the CH3 domain of the second parental antibody comprises amino acid mutations of 439E and 364L; or
   the CH3 domain of the first parental antibody comprises amino acid mutations of 356K and 366G, and the CH3 domain of the second parental antibody comprises amino acid mutations of 439E and 366G; or
   the CH3 domain of the first parental antibody comprises amino acid mutations of 356K and 366A, and the CH3 domain of the second parental antibody comprises amino acid mutations of 439E and 366A; or
   the CH3 domain of the first parental antibody comprises amino acid mutations of 356K and 366V, and the CH3 domain of the second parental antibody comprises amino acid mutations of 439E and 366V; or
   the CH3 domain of the first parental antibody comprises amino acid mutations of 356K and 366L, and the CH3 domain of the second parental antibody comprises amino acid mutations of 439E and 366L; or
   the CH3 domain of the first parental antibody comprises amino acid mutations of 356K and 366H, and the CH3 domain of the second parental antibody comprises amino acid mutations of 439E and 366H; or
   the CH3 domain of the first parental antibody comprises amino acid mutations of 356K and 366I, and the CH3 domain of the second parental antibody comprises amino acid mutations of 439E and 366I; or
   the CH3 domain of the first parental antibody comprises amino acid mutations of 356K and 368V, and the CH3 domain of the second parental antibody comprises amino acid mutations of 439E and 368V; or
   the CH3 domain of the first parental antibody comprises amino acid mutations of 356K and 368I, and the CH3 domain of the second parental antibody comprises amino acid mutations of 439E and 368I; or
   the CH3 domain of the first parental antibody comprises amino acid mutations of 356K and 368A, and the CH3 domain of the second parental antibody comprises amino acid mutations of 439E and 368A; or
   the CH3 domain of the first parental antibody comprises amino acid mutations of 356K and 394A, and the CH3 domain of the second parental antibody comprises amino acid mutations of 439E and 394A; or
   the CH3 domain of the first parental antibody comprises amino acid mutations of 356K and 394S, and the CH3 domain of the second parental antibody comprises amino acid mutations of 439E and 394S; or
   the CH3 domain of the first parental antibody comprises amino acid mutations of 356K and 394C, and the CH3 domain of the second parental antibody comprises amino acid mutations of 439E and 394C; or
   the CH3 domain of the first parental antibody comprises amino acid mutations of 356K and 394V, and the CH3 domain of the second parental antibody comprises amino acid mutations of 439E and 394V; or
   the CH3 domain of the first parental antibody comprises amino acid mutations of 356K and 394N, and the CH3 domain of the second parental antibody comprises amino acid mutations of 439E and 394N; or
   the CH3 domain of the first parental antibody comprises amino acid mutations of 356K and 397I, and the CH3 domain of the second parental antibody comprises amino acid mutations of 439E and 397I; or
   the CH3 domain of the first parental antibody comprises amino acid mutations of 356K and 397L, and the CH3 domain of the second parental antibody comprises amino acid mutations of 439E and 397L; or
   the CH3 domain of the first parental antibody comprises amino acid mutations of 356K and 405L, and the CH3 domain of the second parental antibody comprises amino acid mutations of 439E and 405L; or
   the CH3 domain of the first parental antibody comprises amino acid mutations of 356K and 405Y, and the CH3 domain of the second parental antibody comprises amino acid mutations of 439E and 405Y; or
   the CH3 domain of the first parental antibody comprises amino acid mutations of 356K and 407C, and the CH3 domain of the second parental antibody comprises amino acid mutations of 439E and 407C; or
   the CH3 domain of the first parental antibody comprises amino acid mutations of 356K and 407V, and the CH3 domain of the second parental antibody comprises amino acid mutations of 439E and 407V; or
   the CH3 domain of the first parental antibody comprises amino acid mutations of 356K and 407L, and the CH3 domain of the second parental antibody comprises amino acid mutations of 439E and 407L; or
   the CH3 domain of the first parental antibody comprises amino acid mutations of 356K and 407H, and the CH3 domain of the second parental antibody comprises amino acid mutations of 439E and 407H; or
   the CH3 domain of the first parental antibody comprises amino acid mutations of 356K and 407F, and the CH3 domain of the second parental antibody comprises amino acid mutations of 439E and 407F; or
   the CH3 domain of the first parental antibody comprises amino acid mutations of 356K and 409Q, and the CH3 domain of the second parental antibody comprises amino acid mutations of 439E and 409Q; or
   the CH3 domain of the first parental antibody comprises amino acid mutations of 356K and 409R, and the CH3 domain of the second parental antibody comprises amino acid mutations of 439E and 409R; or
   the CH3 domain of the first parental antibody comprises amino acid mutations of 356K and 411L, and the CH3 domain of the second parental antibody comprises amino acid mutations of 439E and 411L; or
   the CH3 domain of the first parental antibody comprises amino acid mutations of 356K and 411Y, and the CH3 domain of the second parental antibody comprises amino acid mutations of 439E and 411Y; or
4) the CH3 domain of the first parental antibody comprises amino acid mutations of 356K and 349C, and the CH3 domain of the second parental antibody comprises amino acid mutations of 439E, 354C, and 351I; or
   the CH3 domain of the first parental antibody comprises amino acid mutations of 356K and 349C, and the CH3 domain of the second parental antibody comprises amino acid mutations of 439E, 354C, and 366A; or
   the CH3 domain of the first parental antibody comprises amino acid mutations of 356K and 349C, and the CH3 domain of the second parental antibody comprises amino acid mutations of 439E, 354C, and 368I; or
5) the CH3 domain of the first parental antibody comprises amino acid mutations of 356K and 405T, and the CH3 domain of the second parental antibody comprises amino acid mutations of 439E and 394F; or
6) the CH3 domain of the first parental antibody comprises an amino acid mutation of 349C, and the CH3 domain of the second parental antibody comprises an amino acid mutation of 357C; or
7) the CH3 domain of the first parental antibody comprises an amino acid mutation of 349C, and the CH3 domain of the second parental antibody comprises amino acid mutations of 354C and 351I; or
   the CH3 domain of the first parental antibody comprises an amino acid mutation of 349C, and the CH3 domain of the second parental antibody comprises amino acid mutations of 354C and 364Y; or
   the CH3 domain of the first parental antibody comprises an amino acid mutation of 349C, and the CH3 domain of the second parental antibody comprises amino acid mutations of 354C and 364F; or
   the CH3 domain of the first parental antibody comprises an amino acid mutation of 349C, and the CH3 domain of the second parental antibody comprises amino acid mutations of 354C and 366A; or
   the CH3 domain of the first parental antibody comprises an amino acid mutation of 349C, and the CH3 domain of the second parental antibody comprises amino acid mutations of 354C and 368I; or
   the CH3 domain of the first parental antibody comprises an amino acid mutation of 349C, and the CH3 domain of the second parental antibody comprises amino acid mutations of 354C and 405Y; or
8) the CH3 domain of the first parental antibody comprises an amino acid mutation of 349S, and the CH3 domain of the second parental antibody comprises an amino acid mutation of 364Y; or
   the CH3 domain of the first parental antibody comprises an amino acid mutation of 349S, and the CH3 domain of the second parental antibody comprises an amino acid mutation of 354F; or
   the CH3 domain of the first parental antibody comprises an amino acid mutation of 349S, and the CH3 domain of the second parental antibody comprises an amino acid mutation of 354W; or
   the CH3 domain of the first parental antibody comprises an amino acid mutation of 349S, and the CH3 domain of the second parental antibody comprises an amino acid mutation of 354Y; or
   the CH3 domain of the first parental antibody comprises an amino acid mutation of 349G, and the CH3 domain of the second parental antibody comprises an amino acid mutation of 354W; or
   the CH3 domain of the first parental antibody comprises an amino acid mutation of 349A, and the CH3 domain of the second parental antibody comprises an amino acid mutation of 354F; or
   the CH3 domain of the first parental antibody comprises an amino acid mutation of 349A, and the CH3 domain of the second parental antibody comprises an amino acid mutation of 354Y; or
   the CH3 domain of the first parental antibody comprises an amino acid mutation of 349A, and the CH3 domain of the second parental antibody comprises an amino acid mutation of 354W; or
   the CH3 domain of the first parental antibody comprises an amino acid mutation of 349V, and the CH3 domain of the second parental antibody comprises an amino acid mutation of 354F; or
   the CH3 domain of the first parental antibody comprises an amino acid mutation of 349T, and the CH3 domain of the second parental antibody comprises an amino acid mutation of 354F; or
9) the CH3 domain of the first parental antibody comprises an amino acid mutation of 366H, and the CH3 domain of the second parental antibody comprises an amino acid mutation of 405L; or
   the CH3 domain of the first parental antibody comprises an amino acid mutation of 366H, and the CH3 domain of the second parental antibody comprises an amino acid mutation of 407L; or
10) the CH3 domain of the first parental antibody comprises an amino acid mutation of 349C, and the CH3 domain of the second parental antibody comprises amino acid mutations of 347E and 357C; or
11) the CH3 domain of the first parental antibody comprises amino acid mutations of 356K and 366H, and the CH3 domain of the second parental antibody comprises amino acid mutations of 439E and 405L; or
   the CH3 domain of the first parental antibody comprises amino acid mutations of 356K and 366H, and the CH3 domain of the second parental antibody comprises amino acid mutations of 439E and 407L; or
   the CH3 domain of the first parental antibody comprises amino acid mutations of 356K and 366H, and the CH3 domain of the second parental antibody comprises amino acid mutations of 439E and 407H; or
12) the CH3 domain of the first parental antibody comprises amino acid mutations of 356K and 407L, and the CH3 domain of the second parental antibody comprises amino acid mutations of 439E and 366H; or
   the CH3 domain of the first parental antibody comprises amino acid mutations of 356K and 407H, and the CH3 domain of the second parental antibody comprises amino acid mutations of 439E and 366H; or
13) the CH3 domain of the first parental antibody comprises amino acid mutations of 354C and 405T, and the CH3 domain of the second parental antibody comprises amino acid mutations of 349C and 394F; or
14) the CH3 domain of the first parental antibody comprises amino acid mutations of 356K and 409D, and the CH3 domain of the second parental antibody comprises amino acid mutations of 439E and 399K.

In some embodiments, provided is the aforementioned method for preparing a multispecific antibody, wherein:
i) the CH3 domain of the first parental antibody comprises amino acid mutations of 356K and 349S, and the CH3 domain of the second parental antibody comprises amino acid mutations of 439E and 354Y; or
   the CH3 domain of the first parental antibody comprises amino acid mutations of 356K and 349C, and the CH3 domain of the second parental antibody comprises amino acid mutations of 439E and 357C; or
ii) the CH3 domain of the first parental antibody comprises an amino acid mutation of 405T, and the CH3 domain of the second parental antibody comprises an amino acid mutation of 394F; or
iii) the CH3 domain of the first parental antibody comprises amino acid mutations of 356K and 349L, and the CH3 domain of the second parental antibody comprises amino acid mutations of 439E and 349L; or
   the CH3 domain of the first parental antibody comprises amino acid mutations of 356K and 351C, and the CH3 domain of the second parental antibody comprises amino acid mutations of 439E and 351C; or
   the CH3 domain of the first parental antibody comprises amino acid mutations of 356K and 351V, and the CH3 domain of the second parental antibody comprises amino acid mutations of 439E and 351V; or
   the CH3 domain of the first parental antibody comprises amino acid mutations of 356K and 351T, and the CH3 domain of the second parental antibody comprises amino acid mutations of 439E and 351T; or
   the CH3 domain of the first parental antibody comprises amino acid mutations of 356K and 351I, and the CH3 domain of the second parental antibody comprises amino acid mutations of 439E and 351I; or
   the CH3 domain of the first parental antibody comprises amino acid mutations of 356K and 351F, and the CH3 domain of the second parental antibody comprises amino acid mutations of 439E and 351F; or
   the CH3 domain of the first parental antibody comprises amino acid mutations of 356K and 364A, and the CH3 domain of the second parental antibody comprises amino acid mutations of 439E and 364A; or
   the CH3 domain of the first parental antibody comprises amino acid mutations of 356K and 364T, and the CH3 domain of the second parental antibody comprises amino acid mutations of 439E and 364T; or
   the CH3 domain of the first parental antibody comprises amino acid mutations of 356K and 364L, and the CH3 domain of the second parental antibody comprises amino acid mutations of 439E and 364L; or
   the CH3 domain of the first parental antibody comprises amino acid mutations of 356K and 366G, and the CH3 domain of the second parental antibody comprises amino acid mutations of 439E and 366G; or
   the CH3 domain of the first parental antibody comprises amino acid mutations of 356K and 366A, and the CH3 domain of the second parental antibody comprises amino acid mutations of 439E and 366A; or
   the CH3 domain of the first parental antibody comprises amino acid mutations of 356K and 366V, and the CH3 domain of the second parental antibody comprises amino acid mutations of 439E and 366V; or
   the CH3 domain of the first parental antibody comprises amino acid mutations of 356K and 366L, and the CH3 domain of the second parental antibody comprises amino acid mutations of 439E and 366L; or
   the CH3 domain of the first parental antibody comprises amino acid mutations of 356K and 366H, and the CH3 domain of the second parental antibody comprises amino acid mutations of 439E and 366H; or
   the CH3 domain of the first parental antibody comprises amino acid mutations of 356K and 368V, and the CH3 domain of the second parental antibody comprises amino acid mutations of 439E and 368V; or
   the CH3 domain of the first parental antibody comprises amino acid mutations of 356K and 368I, and the CH3 domain of the second parental antibody comprises amino acid mutations of 439E and 368I; or
   the CH3 domain of the first parental antibody comprises amino acid mutations of 356K and 394A, and the CH3 domain of the second parental antibody comprises amino acid mutations of 439E and 394A; or
   the CH3 domain of the first parental antibody comprises amino acid mutations of 356K and 394S, and the CH3 domain of the second parental antibody comprises amino acid mutations of 439E and 394S; or
   the CH3 domain of the first parental antibody comprises amino acid mutations of 356K and 394C, and the CH3 domain of the second parental antibody comprises amino acid mutations of 439E and 394C; or
   the CH3 domain of the first parental antibody comprises amino acid mutations of 356K and 394V, and the CH3 domain of the second parental antibody comprises amino acid mutations of 439E and 394V; or
   the CH3 domain of the first parental antibody comprises amino acid mutations of 356K and 394N, and the CH3 domain of the second parental antibody comprises amino acid mutations of 439E and 394N; or
   the CH3 domain of the first parental antibody comprises amino acid mutations of 356K and 397I, and the CH3 domain of the second parental antibody comprises amino acid mutations of 439E and 397I; or
   the CH3 domain of the first parental antibody comprises amino acid mutations of 356K and 397L, and the CH3 domain of the second parental antibody comprises amino acid mutations of 439E and 397L; or
   the CH3 domain of the first parental antibody comprises amino acid mutations of 356K and 405L, and the CH3 domain of the second parental antibody comprises amino acid mutations of 439E and 405L; or
   the CH3 domain of the first parental antibody comprises amino acid mutations of 356K and 405Y, and the CH3 domain of the second parental antibody comprises amino acid mutations of 439E and 405Y; or
   the CH3 domain of the first parental antibody comprises amino acid mutations of 356K and 407C, and the CH3 domain of the second parental antibody comprises amino acid mutations of 439E and 407C; or
   the CH3 domain of the first parental antibody comprises amino acid mutations of 356K and 407V, and the CH3 domain of the second parental antibody comprises amino acid mutations of 439E and 407V; or
   the CH3 domain of the first parental antibody comprises amino acid mutations of 356K and 407L, and the CH3 domain of the second parental antibody comprises amino acid mutations of 439E and 407L; or
   the CH3 domain of the first parental antibody comprises amino acid mutations of 356K and 407H, and the CH3 domain of the second parental antibody comprises amino acid mutations of 439E and 407H; or
   the CH3 domain of the first parental antibody comprises amino acid mutations of 356K and 409Q, and the CH3 domain of the second parental antibody comprises amino acid mutations of 439E and 409Q; or
   the CH3 domain of the first parental antibody comprises amino acid mutations of 356K and 409R, and the CH3 domain of the second parental antibody comprises amino acid mutations of 439E and 409R; or
   the CH3 domain of the first parental antibody comprises amino acid mutations of 356K and 411Y, and the CH3 domain of the second parental antibody comprises amino acid mutations of 439E and 411Y; or
iv) the CH3 domain of the first parental antibody comprises amino acid mutations of 356K and 349C, and the CH3 domain of the second parental antibody comprises amino acid mutations of 439E, 354C, and 351I; or
   the CH3 domain of the first parental antibody comprises amino acid mutations of 356K and 349C, and the CH3 domain of the second parental antibody comprises amino acid mutations of 439E, 354C, and 366A; or
   the CH3 domain of the first parental antibody comprises amino acid mutations of 356K and 349C, and the CH3 domain of the second parental antibody comprises amino acid mutations of 439E, 354C, and 368I; or
v) the CH3 domain of the first parental antibody comprises amino acid mutations of 356K and 405T, and the CH3 domain of the second parental antibody comprises amino acid mutations of 439E and 394F; or
vi) the CH3 domain of the first parental antibody comprises an amino acid mutation of 349C, and the CH3 domain of the second parental antibody comprises an amino acid mutation of 357C; or
vii) the CH3 domain of the first parental antibody comprises an amino acid mutation of 349C, and the CH3 domain of the second parental antibody comprises amino acid mutations of 354C and 366A; or
   the CH3 domain of the first parental antibody comprises an amino acid mutation of 349C, and the CH3 domain of the second parental antibody comprises amino acid mutations of 354C and 368I; or
   the CH3 domain of the first parental antibody comprises an amino acid mutation of 349C, and the CH3 domain of the second parental antibody comprises amino acid mutations of 354C and 405Y.

In some embodiments, provided is the aforementioned method for preparing a multispecific antibody, wherein:
1) the CH3 domain of the first parental antibody comprises amino acid mutations of 356K and 349S, and the CH3 domain of the second parental antibody comprises amino acid mutations of 439E and 354Y; or
2) the CH3 domain of the first parental antibody comprises an amino acid mutation of 405T, and the CH3 domain of the second parental antibody comprises an amino acid mutation of 394F; or
3) the CH3 domain of the first parental antibody comprises amino acid mutations of 356K and 351T, and the CH3 domain of the second parental antibody comprises amino acid mutations of 439E and 351T; or
   the CH3 domain of the first parental antibody comprises amino acid mutations of 356K and 351I, and the CH3 domain of the second parental antibody comprises amino acid mutations of 439E and 351I; or
   the CH3 domain of the first parental antibody comprises amino acid mutations of 356K and 364A, and the CH3 domain of the second parental antibody comprises amino acid mutations of 439E and 364A; or
   the CH3 domain of the first parental antibody comprises amino acid mutations of 356K and 366A, and the CH3 domain of the second parental antibody comprises amino acid mutations of 439E and 366A; or
   the CH3 domain of the first parental antibody comprises amino acid mutations of 356K and 368I, and the CH3 domain of the second parental antibody comprises amino acid mutations of 439E and 368I; or
   the CH3 domain of the first parental antibody comprises amino acid mutations of 356K and 394A, and the CH3 domain of the second parental antibody comprises amino acid mutations of 439E and 394A; or
   the CH3 domain of the first parental antibody comprises amino acid mutations of 356K and 394S, and the CH3 domain of the second parental antibody comprises amino acid mutations of 439E and 394S; or
   the CH3 domain of the first parental antibody comprises amino acid mutations of 356K and 405Y, and the CH3 domain of the second parental antibody comprises amino acid mutations of 439E and 405Y; or
   the CH3 domain of the first parental antibody comprises amino acid mutations of 356K and 409Q, and the CH3 domain of the second parental antibody comprises amino acid mutations of 439E and 409Q; or
   the CH3 domain of the first parental antibody comprises amino acid mutations of 356K and 411Y, and the CH3 domain of the second parental antibody comprises amino acid mutations of 439E and 411Y; or
4) the CH3 domain of the first parental antibody comprises amino acid mutations of 356K and 349C, and the CH3 domain of the second parental antibody comprises amino acid mutations of 439E, 354C, and 351I; or
5) the CH3 domain of the first parental antibody comprises an amino acid mutation of 349C, and the CH3 domain of the second parental antibody comprises amino acid mutations of 354C and 364Y; or
   the CH3 domain of the first parental antibody comprises an amino acid mutation of 349C, and the CH3 domain of the second parental antibody comprises amino acid mutations of 354C and 364F; or
6) the CH3 domain of the first parental antibody comprises an amino acid mutation of 349S, and the CH3 domain of the second parental antibody comprises an amino acid mutation of 364Y; or
the CH3 domain of the first parental antibody comprises an amino acid mutation of 349G, and the CH3 domain of the second parental antibody comprises an amino acid mutation of 354W.

In some embodiments, provided is the aforementioned method for preparing a multispecific antibody, wherein:
i) the CH3 domain of the first parental antibody comprises amino acid mutations of 356K and 349S, and the CH3 domain of the second parental antibody comprises amino acid mutations of 439E and 354Y; or
ii) the CH3 domain of the first parental antibody comprises an amino acid mutation of 405T, and the CH3 domain of the second parental antibody comprises an amino acid mutation of 394F; or
iii) the CH3 domain of the first parental antibody comprises amino acid mutations of 356K and 351T, and the CH3 domain of the second parental antibody comprises amino acid mutations of 439E and 351T; or
   the CH3 domain of the first parental antibody comprises amino acid mutations of 356K and 351I, and the CH3 domain of the second parental antibody comprises amino acid mutations of 439E and 351I; or
   the CH3 domain of the first parental antibody comprises amino acid mutations of 356K and 364A, and the CH3 domain of the second parental antibody comprises amino acid mutations of 439E and 364A; or
   the CH3 domain of the first parental antibody comprises amino acid mutations of 356K and 366A, and the CH3 domain of the second parental antibody comprises amino acid mutations of 439E and 366A; or
   the CH3 domain of the first parental antibody comprises amino acid mutations of 356K and 368I, and the CH3 domain of the second parental antibody comprises amino acid mutations of 439E and 368I; or
   the CH3 domain of the first parental antibody comprises amino acid mutations of 356K and 394A, and the CH3 domain of the second parental antibody comprises amino acid mutations of 439E and 394A; or
   the CH3 domain of the first parental antibody comprises amino acid mutations of 356K and 394S, and the CH3 domain of the second parental antibody comprises amino acid mutations of 439E and 394S; or
   the CH3 domain of the first parental antibody comprises amino acid mutations of 356K and 405Y, and the CH3 domain of the second parental antibody comprises amino acid mutations of 439E and 405Y; or
   the CH3 domain of the first parental antibody comprises amino acid mutations of 356K and 409Q, and the CH3 domain of the second parental antibody comprises amino acid mutations of 439E and 409Q; or
   the CH3 domain of the first parental antibody comprises amino acid mutations of 356K and 411Y, and the CH3 domain of the second parental antibody comprises amino acid mutations of 439E and 411Y; or
iv) the CH3 domain of the first parental antibody comprises amino acid mutations of 356K and 349C, and the CH3 domain of the second parental antibody comprises amino acid mutations of 439E, 354C, and 351I.

In some embodiments, provided is the aforementioned method for preparing a multispecific antibody, wherein the CH3 domain of the first parental antibody comprises amino acid mutations of 356K and 349S, and the CH3 domain of the second parental antibody comprises amino acid mutations of 439E and 354Y.

In some embodiments, provided is the aforementioned method for preparing a multispecific antibody, wherein the CH3 domain of the first parental antibody comprises an amino acid mutation of 405T, and the CH3 domain of the second parental antibody comprises an amino acid mutation of 394F.

In some embodiments, provided is the aforementioned method for preparing a multispecific antibody, wherein the CH3 domain of the first parental antibody comprises amino acid mutations of 356K and 351I, and the CH3 domain of the second parental antibody comprises amino acid mutations of 439E and 351I.

In some embodiments, provided is the aforementioned method for preparing a multispecific antibody, wherein the CH3 domain of the first parental antibody comprises amino acid mutations of 356K and 394S, and the CH3 domain of the second parental antibody comprises amino acid mutations of 439E and 394S.

In some embodiments, provided is the aforementioned method for preparing a multispecific antibody, wherein the CH3 domain of the first parental antibody comprises amino acid mutations of 356K and 411Y, and the CH3 domain of the second parental antibody comprises amino acid mutations of 439E and 411Y.

In some embodiments, provided is the aforementioned method for preparing a multispecific antibody, wherein the CH3 domain of the first parental antibody comprises an amino acid mutation of 349S, and the CH3 domain of the second parental antibody comprises an amino acid mutation of 364Y.

In some embodiments, provided is the aforementioned method for preparing a multispecific antibody, wherein the CH3 domain of the first parental antibody comprises amino acid mutations of 356K and 349C, and the CH3 domain of the second parental antibody comprises amino acid mutations of 439E, 354C, and 351I.

In some embodiments, provided is the aforementioned method for preparing a multispecific antibody, wherein the CH3 domain of the first parental antibody comprises an amino acid mutation of 349C, and the CH3 domain of the second parental antibody comprises amino acid mutations of 354C and 364Y.

In some embodiments, provided is the aforementioned method for preparing a multispecific antibody, wherein the CH3 domain of the first parental antibody comprises an amino acid mutation of 349C, and the CH3 domain of the second parental antibody comprises amino acid mutations of 354C and 364F.

In some embodiments, provided is the aforementioned method for preparing a multispecific antibody, wherein the CH3 domain of the first parental antibody comprises only amino acid mutations of 356K and 349S, and the CH3 domain of the second parental antibody comprises only amino acid mutations of 439E and 354Y.

In some embodiments, provided is the aforementioned method for preparing a multispecific antibody, wherein the CH3 domain of the first parental antibody comprises only an amino acid mutation of 405T, and the CH3 domain of the second parental antibody comprises only an amino acid mutation of 394F.

In some embodiments, provided is the aforementioned method for preparing a multispecific antibody, wherein the CH3 domain of the first parental antibody comprises only amino acid mutations of 356K and 351I, and the CH3 domain of the second parental antibody comprises only amino acid mutations of 439E and 351I.

In some embodiments, provided is the aforementioned method for preparing a multispecific antibody, wherein the CH3 domain of the first parental antibody comprises only amino acid mutations of 356K and 394S, and the CH3 domain of the second parental antibody comprises only amino acid mutations of 439E and 394S.

In some embodiments, provided is the aforementioned method for preparing a multispecific antibody, wherein the CH3 domain of the first parental antibody comprises only amino acid mutations of 356K and 411Y, and the CH3 domain of the second parental antibody comprises only amino acid mutations of 439E and 411Y.

In some embodiments, provided is the aforementioned method for preparing a multispecific antibody, wherein the CH3 domain of the first parental antibody comprises only an amino acid mutation of 349S, and the CH3 domain of the second parental antibody comprises only an amino acid mutation of 364Y.

In some embodiments, provided is the aforementioned method for preparing a multispecific antibody, wherein the CH3 domain of the first parental antibody comprises only amino acid mutations of 356K and 349C, and the CH3 domain of the second parental antibody comprises only amino acid mutations of 439E, 354C, and 351I.

In some embodiments, provided is the aforementioned method for preparing a multispecific antibody, wherein the CH3 domain of the first parental antibody comprises only an amino acid mutation of 349C, and the CH3 domain of the second parental antibody comprises only amino acid mutations of 354C and 364Y.

In some embodiments, provided is the aforementioned method for preparing a multispecific antibody, wherein the CH3 domain of the first parental antibody comprises only an amino acid mutation of 349C, and the CH3 domain of the second parental antibody comprises only amino acid mutations of 354C and 364F.

In some embodiments, provided is the aforementioned method for preparing a multispecific antibody, wherein the first parental antibody is a first parental monoclonal antibody, and the second parental antibody is a second parental monoclonal antibody.

In some embodiments, provided is the aforementioned method for preparing a multispecific antibody, wherein the CH3 domains are derived from IgG1. In some embodiments, the CH3 domains are derived from human IgG1.

In some embodiments, provided is the aforementioned method for preparing a multispecific antibody, wherein the multispecific antibody comprises at least one replaced Fab comprising a Titin chain and an Obscurin chain that are capable of forming a dimer. In some embodiments, provided is the aforementioned method for preparing a multispecific antibody, wherein the replaced Fab is obtained by replacing the original CH1 and CL of a Fab with the Obscurin chain and the Titin chain, respectively, or the replaced Fab is obtained by replacing the original CH1 and CL of a Fab with the Titin chain and the Obscurin chain, respectively. In some embodiments, the Titin chain comprises the amino acid sequence set forth in SEQ ID NO: 6, and the Obscurin chain comprises the amino acid sequence set forth in SEQ ID NO: 5.

In some embodiments, provided is the aforementioned method for preparing a multispecific antibody, wherein the reducing conditions include, but are not limited to, adding a reductant selected from the group consisting of 2-mercaptoethylamine (2-MEA), dithiothreitol (DTT), dithioerythritol (DTE), glutathione (GSH), tris(2-carboxyethyl)phosphine (TCEP), L-cysteine, D-cysteine, and β-mercapto-ethanol, as well as chemical derivatives thereof. In some embodiments, provided is the aforementioned method for preparing a multispecific antibody, wherein the reductant is selected from the group consisting of 2-MEA, glutathione, L-cysteine, dithiothreitol, β-mercaptoethanol, and TCEP. In some embodiments, provided is the aforementioned method for preparing a multispecific antibody, wherein the reductant is 2-MEA.

In some embodiments, provided is the aforementioned method for preparing a multispecific antibody, wherein the multispecific antibody is a bispecific antibody. In some embodiments, provided is the aforementioned method for preparing a multispecific antibody, wherein more than 80% (e.g., more than 85%, more than 88%, more than 90%, more than 91%, more than 92%, more than 93%, more than 94%, more than 95%, more than 96%, more than 97%, more than 98%, or more than 99%) of the total product is the desired multispecific antibody. In some embodiments, provided is the aforementioned method for preparing a multispecific antibody, wherein more than 92% (e.g., more than 93%, more than 94%, more than 95%, more than 96%, more than 97%, more than 98%, or more than 99%) of the total product is the desired multispecific antibody. In some embodiments, provided is the aforementioned method for preparing a multispecific antibody, wherein more than 94% (e.g., more than 95%, more than 96%, more than 97%, more than 98%, or more than 99%) of the total product is the desired multispecific antibody. In some embodiments, provided is the aforementioned method for preparing a multispecific antibody, wherein more than 95% (e.g., more than 96%, more than 97%, more than 98%, or more than 99%) of the total product is the desired multispecific antibody.

In some embodiments, provided is the aforementioned method for preparing a multispecific antibody, wherein steps a) and b) further comprise a step of purifying the first parental antibody and the second parental antibody. In some embodiments, the purification method includes, but is not limited to, protein A or protein G chromatography, antigen binding-based affinity chromatography, anti-idiotypic antibody-based affinity chromatography, ion exchange, hydrophobic interaction chromatography, mixed chromatography (such as hydroxyapatite), immobilized metal affinity chromatography, thiophilic adsorption chromatography, size exclusion chromatography (SEC), etc.

In some embodiments, the aforementioned method for preparing a multispecific antibody further comprises a step of separating the multispecific antibody from the reductant after step c). In some embodiments, methods for separating the multispecific antibody from the reductant include, but are not limited to, any of the methods described below, e.g., dialysis, precipitation, chromatography, or filtration.

A method for separating the multispecific antibody from the reductant may be, in principle, any method that results in or can achieve separation of the two without damaging the multispecific antibody. Such methods include, but are not limited to, dialysis, precipitation, chromatography, or filtration. Separating the multispecific antibody from the reductant may be carried out as a continuous process or a batch process. In some embodiments, provided is the aforementioned method for preparing a multispecific antibody, wherein step d) further comprises a method for purifying a composition obtained from step c). In some embodiments, the purification method includes, but is not limited to, protein A or protein G chromatography, antigen binding-based affinity chromatography, anti-idiotypic antibody-based affinity chromatography, ion exchange, hydrophobic interaction chromatography, mixed chromatography (such as hydroxyapatite), immobilized metal affinity chromatography, thiophilic adsorption chromatography, size exclusion chromatography (SEC), etc.

In some embodiments, the following methods may be provided: a method in which a cell strain that produces the first parental antibody and a cell strain that produces the second parental antibody are separately cultured, and after the culture supernatants are purified, the purified antibodies are used to induce an FAE (Fab-arm exchange) reaction; a method in which a cell strain that produces the first parental antibody and a cell strain that produces the second parental antibody are separately cultured, the culture supernatants are mixed without purification, an FAE reaction is induced in the mixture of the culture supernatants, and purification is then performed; a method in which a cell strain that produces the first parental antibody and a cell strain that produces the second parental antibody are mixed and cultured, and after the culture supernatant is purified, the purified antibodies are used to induce an FAE reaction; and a method in which a cell strain that produces the first parental antibody and a cell strain that produces the second parental antibody are mixed and cultured, an FAE reaction is induced in the culture supernatant, and purification is then performed.

In some embodiments, the present disclosure provides a method for preparing a multispecific antibody, comprising the following steps a) to c):
a) a step of separately culturing a cell strain that produces a first parental antibody and a cell strain that produces a second parental antibody,
b) a step of separately purifying the culture supernatants of the cell strains to obtain the first and second parental antibodies, and co-incubating the first parental antibody and the second parental antibody to enable cysteines in the hinge region to cause disulfide bond isomerization, and
c) a step of obtaining a multispecific antibody comprising the first and second parental antibodies.

In some embodiments, the present disclosure provides a method for preparing a multispecific antibody, comprising the following steps a) to c):
a) a step of mixing a cell strain that produces a first parental antibody with a cell strain that produces a second parental antibody,
b) a step of co-incubating the first parental antibody and the second parental antibody in the culture supernatant to enable cysteines in the hinge region to cause disulfide bond isomerization, and
c) a step of obtaining a multispecific antibody comprising the first and second parental antibodies.

In some embodiments, the present disclosure provides a method for preparing a multispecific antibody, comprising the following steps a) to c):
a) a step of separately culturing a cell strain that produces a first parental antibody and a cell strain that produces a second parental antibody,
b) a step of mixing the culture supernatants of the cell strains, and co-incubating the first parental antibody and the second parental antibody to enable cysteines in the hinge region to cause disulfide bond isomerization, and
c) a step of obtaining a multispecific antibody comprising the first and second parental antibodies.

In another aspect, the present disclosure provides a multispecific antibody prepared by the aforementioned method. In some embodiments, the present disclosure provides a multispecific antibody comprising polypeptides in which two CH3 domains bind to each other, wherein amino acid mutations are introduced into a CH3 domain of a first polypeptide and a CH3 domain of a second polypeptide to promote the formation of a heterologous multispecific antibody, wherein the amino acid mutations include at least one amino acid mutation selected from the group consisting of positions 347, 349, 351, 354, 356, 357, 364, 366, 368, 394, 397, 399, 405, 407, 409, 411, and 439; the first polypeptide and the second polypeptide bind to different antigens or epitopes, and mutation sites in the CH3 domains are indicated by EU numbering.

In some embodiments, the present disclosure provides a multispecific antibody comprising polypeptides in which two CH3 domains bind to each other, wherein amino acid mutations are introduced into a CH3 domain of a first polypeptide and a CH3 domain of a second polypeptide to promote the formation of a heterologous multispecific antibody, wherein the amino acid mutations include at least one amino acid mutation selected from the group consisting of positions 349, 351, 354, 356, 357, 364, 366, 368, 394, 397, 405, 407, 409, 411, and 439; the first polypeptide and the second polypeptide bind to different antigens or epitopes, and mutation sites in the CH3 domains are indicated by EU numbering.

In some embodiments, provided is the aforementioned multispecific antibody, wherein the amino acid mutations in the CH3 domains include at least one amino acid mutation selected from the group consisting of positions 349, 351, 354, 356, 364, 366, 368, 394, 405, 409, 411, and 439; the first polypeptide and the second polypeptide bind to different antigens or epitopes, and mutation sites in the CH3 domains are indicated by EU numbering.

In some embodiments, provided is the aforementioned multispecific antibody, wherein the amino acid mutations in the CH3 domains include at least one amino acid mutation selected from the group consisting of the following combinations:
the amino acid at position 356 (EU numbering) is mutated into Lys (K); or
the amino acid at position 439 (EU numbering) is mutated into Glu (E); or
the amino acid at position 349 (EU numbering) is mutated into Leu (L), Phe (F), Ser (S), Cys (C), Ala (A), Val (V), Thr (T), or Gly (G); or
the amino acid at position 351 (EU numbering) is mutated into Cys (C), Val (V), Thr (T), Ile (I), Phe (F), or Met (M); or
the amino acid at position 364 (EU numbering) is mutated into Ala (A), Val (V), Thr (T), Leu (L), Tyr (Y), or Phe (F); or
the amino acid at position 366 (EU numbering) is mutated into Gly (G), Ser (S), Ala (A), Val (V), Leu (L), His (H), or Ile (I); or
the amino acid at position 368 (EU numbering) is mutated into Val (V), Ile (I), Met (M), or Ala (A); or
the amino acid at position 394 (EU numbering) is mutated into Phe (F), Ala (A), Ser (S), Cys (C), Val (V), or Asn (N); or
the amino acid at position 397 (EU numbering) is mutated into Thr (T), Ile (I), or Leu (L); or the amino acid at position 405 (EU numbering) is mutated into Thr (T), Leu (L), or Tyr (Y); or
the amino acid at position 407 (EU numbering) is mutated into Cys (C), Val (V), Leu (L), His (H), or Phe (F); or
the amino acid at position 409 (EU numbering) is mutated into Gln (Q) or Asp (D); or
the amino acid at position 411 (EU numbering) is mutated into Asn (N), Tyr (Y), or Leu (L); or
the amino acid at position 354 (EU numbering) is mutated into Tyr (Y), Cys (C), Phe (F), or Trp (W); or
the amino acid at position 357 (EU numbering) is mutated into Cys (C); or
the amino acid at position 347 (EU numbering) is mutated into Glu (E); or
the amino acid at position 399 (EU numbering) is mutated into Lys (K).

In some embodiments, provided is the aforementioned multispecific antibody, wherein the amino acid mutations in the CH3 domains include at least one amino acid mutation selected from the group consisting of the following combinations:
position 356 (EU numbering) is mutated into Lys (K); or
position 439 (EU numbering) is mutated into Glu (E); or
position 349 (EU numbering) is mutated into Leu (L), Phe (F), Ser (S), or Cys (C); or
position 351 (EU numbering) is mutated into Cys (C), Val (V), Thr (T), Ile (I), or Phe (F); or
position 364 (EU numbering) is mutated into Ala (A), Val (V), Thr (T), or Leu (L); or
position 366 (EU numbering) is mutated into Gly (G), Ser (S), Ala (A), Val (V), Leu (L), or His (H); or
position 368 (EU numbering) is mutated into Val (V), Ile (I), or Met (M); or
position 394 (EU numbering) is mutated into Phe (F), Ala (A), Ser (S), Cys (C), Val (V), or Asn (N); or
position 397 (EU numbering) is mutated into Thr (T), Ile (I), or Leu (L); or
position 405 (EU numbering) is mutated into Thr (T), Leu (L), or Tyr (Y); or
position 407 (EU numbering) is mutated into Cys (C), Val (V), Leu (L), or His (H); or
position 409 (EU numbering) is mutated into Gln (Q) or Arg (R); or
position 411 (EU numbering) is mutated into Asn (N) or Tyr (Y); or
position 354 (EU numbering) is mutated into Tyr (Y) or Cys (C); or
position 357 (EU numbering) is mutated into Cys (C).

In some embodiments, provided is the aforementioned multispecific antibody, wherein:
1) the CH3 domain of the first polypeptide comprises amino acid mutations at positions 356 and 349, and the CH3 domain of the second polypeptide comprises an amino acid mutation at position 439, wherein the CH3 domain of the second polypeptide further comprises an amino acid mutation at position 354 or 357; or
2) the CH3 domain of the first polypeptide comprises an amino acid mutation at position 405, and the CH3 domain of the second polypeptide comprises an amino acid mutation at position 394; or
3) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 349, 351, 364, 366, 368, 394, 397, 405, 407, 409, and 411; or
4) the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368; or
5) the CH3 domain of the first polypeptide comprises amino acid mutations at positions 356 and 405, and the CH3 domain of the second polypeptide comprises amino acid mutations at positions 439 and 394; or
6) the CH3 domain of the first polypeptide comprises an amino acid mutation at position 349, and the CH3 domain of the second polypeptide comprises an amino acid mutation at position 357; or
7) the CH3 domain of the first polypeptide comprises an amino acid mutation at position 349, and the CH3 domain of the second polypeptide comprises an amino acid mutation at position 354, wherein the CH3 domain of the second polypeptide further comprises one amino acid mutation selected from the group consisting of positions 351, 364, 366, 368, and 405; or
8) the CH3 domain of the first polypeptide comprises an amino acid mutation at position 349, and the CH3 domain of the second polypeptide comprises an amino acid mutation at position 364 or 354; or
9) the CH3 domain of the first polypeptide comprises an amino acid mutation at position 366, and the CH3 domain of the second polypeptide comprises an amino acid mutation at position 405 or 407; or
10) the CH3 domain of the first polypeptide comprises an amino acid mutation at position 349, and the CH3 domain of the second polypeptide comprises amino acid mutations at positions 347 and 357; or
11) the CH3 domain of the first polypeptide comprises amino acid mutations at positions 356 and 366, and the CH3 domain of the second polypeptide comprises an amino acid mutation at position 439, wherein the CH3 domain of the second polypeptide further comprises an amino acid mutation at position 405 or 407; or
12) the CH3 domain of the first polypeptide comprises amino acid mutations at positions 356 and 407, and the CH3 domain of the second polypeptide comprises amino acid mutations at positions 439 and 366; or
13) the CH3 domain of the first polypeptide comprises amino acid mutations at positions 354 and 405, and the CH3 domain of the second polypeptide comprises amino acid mutations at positions 349 and 394; or
14) the CH3 domain of the first polypeptide comprises amino acid mutations at positions 356 and 409, and the CH3 domain of the second polypeptide comprises amino acid mutations at positions 439 and 399.

In some embodiments, provided is the aforementioned multispecific antibody, wherein:
i) the CH3 domain of the first polypeptide comprises amino acid mutations at positions 356 and 349, and the CH3 domain of the second polypeptide comprises an amino acid mutation at position 439, wherein the CH3 domain of the second polypeptide further comprises one amino acid mutation selected from the group consisting of positions 354 and 357; or
ii) the CH3 domain of the first polypeptide comprises an amino acid mutation at position 405, and the CH3 domain of the second polypeptide comprises an amino acid mutation at position 394; or
iii) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 349, 351, 364, 366, 368, 394, 397, 405, 407, 409, and 411; or
iv) the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368; or
v) the CH3 domain of the first polypeptide comprises amino acid mutations at positions 356 and 405, and the CH3 domain of the second polypeptide comprises amino acid mutations at positions 439 and 394; or
vi) the CH3 domain of the first polypeptide comprises an amino acid mutation at position 349, and the CH3 domain of the second polypeptide comprises an amino acid mutation at position 357; or
vii) the CH3 domain of the first polypeptide comprises an amino acid mutation at position 349, and the CH3 domain of the second polypeptide comprises an amino acid mutation at position 354, wherein the CH3 domain of the second polypeptide further comprises one amino acid mutation selected from the group consisting of positions 366, 368, and 405.

In some embodiments, provided is the aforementioned multispecific antibody, wherein:
1) the CH3 domain of the first polypeptide comprises amino acid mutations at positions 356 and 349, and the CH3 domain of the second polypeptide comprises amino acid mutations at positions 439 and 354; or
2) the CH3 domain of the first polypeptide comprises an amino acid mutation at position 405, and the CH3 domain of the second polypeptide comprises an amino acid mutation at position 394; or
3) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical amino acid mutation selected from the group consisting of positions 351, 364, 366, 368, 394, 405, 409, and 411; or
4) the CH3 domain of the first polypeptide comprises amino acid mutations at positions 356 and 349, and the CH3 domain of the second polypeptide comprises amino acid mutations at positions 439, 354, and 351; or
5) the CH3 domain of the first polypeptide comprises an amino acid mutation at position 349, and the CH3 domain of the second polypeptide comprises amino acid mutations at positions 354 and 364; or
6) the CH3 domain of the first polypeptide comprises an amino acid mutation at position 349, and the CH3 domain of the second polypeptide comprises an amino acid mutation at position 364 or 354.

In some embodiments, provided is the aforementioned multispecific antibody, wherein:
i) the CH3 domain of the first polypeptide comprises amino acid mutations at positions 356 and 349, and the CH3 domain of the second polypeptide comprises amino acid mutations at positions 439 and 354; or
ii) the CH3 domain of the first polypeptide comprises an amino acid mutation at position 405, and the CH3 domain of the second polypeptide comprises an amino acid mutation at position 394; or
iii) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical amino acid mutation selected from the group consisting of positions 351, 364, 366, 368, 394, 405, 409, and 411; or
iv) the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one identical amino acid mutation selected from the group consisting of position 351.

In some embodiments, provided is the aforementioned multispecific antibody, wherein: the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of positions 351, 364, 366, 368, 394, 405, 409, and 411, wherein:
the amino acid at position 351 (EU numbering) is mutated into Cys (C), Val (V), Thr (T), Ile (I), Phe (F), or Met (M); or
the amino acid at position 364 (EU numbering) is mutated into Ala (A), Val (V), Thr (T), or Leu (L); or
the amino acid at position 366 (EU numbering) is mutated into Ser (S), Ala (A), Val (V), Leu (L), His (H), or Ile (I); or
the amino acid at position 368 (EU numbering) is mutated into Val (V), Ile (I), Met (M), or Ala (A); or
the amino acid at position 394 (EU numbering) is mutated into Ala (A), Ser (S), Cys (C), Val (V), or Asn (N); or
the amino acid at position 405 (EU numbering) is mutated into Leu (L) or Tyr (Y); or
the amino acid at position 407 (EU numbering) is mutated into Phe (F); or
the amino acid at position 409 (EU numbering) is mutated into Gln (Q); or
the amino acid at position 411 (EU numbering) is mutated into Asn (N), Tyr (Y), or Leu (L).

In some embodiments, provided is the aforementioned multispecific antibody, wherein: the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of positions 351, 364, 366, 368, 394, 405, 409, and 411, wherein:
position 351 (EU numbering) is mutated into Cys (C), Val (V), Thr (T), Ile (I), or Phe (F);
   or
position 364 (EU numbering) is mutated into Ala (A), Val (V), Thr (T), or Leu (L); or
position 366 (EU numbering) is mutated into Ser (S), Ala (A), Val (V), Leu (L), or His (H); or
position 368 (EU numbering) is mutated into Val (V), Ile (I), or Met (M); or
position 394 (EU numbering) is mutated into Ala (A), Ser (S), Cys (C), Val (V), or Asn (N); or
position 405 (EU numbering) is mutated into Leu (L) or Tyr (Y); or
position 409 (EU numbering) is mutated into Gln (Q) or Arg (R); or
position 411 (EU numbering) is mutated into Asn (N) or Tyr (Y).

In some embodiments, provided is the aforementioned multispecific antibody, wherein:
1) the CH3 domain of the first polypeptide comprises amino acid mutations of 356K and 349S, and the CH3 domain of the second polypeptide comprises amino acid mutations of 439E and 354Y; or
   the CH3 domain of the first polypeptide comprises amino acid mutations of 356K and 349C, and the CH3 domain of the second polypeptide comprises amino acid mutations of 439E and 357C; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of 349L, 351C, 351V, 351T, 351I, 351F, 351M, 364A, 364T, 364L, 366G, 366A, 366V, 366L, 366H, 366I, 368V, 368I, 368A, 394A, 394S, 394C, 394V, 394N, 397I, 397L, 405L, 405Y, 407C, 407V, 407L, 407H, 407F, 409Q, 409R, 411L, and 411Y; or
4) the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I; or
5) the CH3 domain of the first polypeptide comprises amino acid mutations of 356K and 405T, and the CH3 domain of the second polypeptide comprises amino acid mutations of 439E and 394F; or
6) the CH3 domain of the first polypeptide comprises an amino acid mutation of 349C, and the CH3 domain of the second polypeptide comprises an amino acid mutation of 357C; or
7) the CH3 domain of the first polypeptide comprises an amino acid mutation of 349C, and the CH3 domain of the second polypeptide comprises an amino acid mutation of 354C, wherein the CH3 domain of the second polypeptide further comprises one amino acid mutation selected from the group consisting of 351I, 364Y, 364F, 366A, 368I, and 405Y; or
8) the CH3 domain of the first polypeptide comprises an amino acid mutation of 349S, and the CH3 domain of the second polypeptide comprises one amino acid mutation selected from the group consisting of 364Y, 354F, 354W, and 354Y; or
   the CH3 domain of the first polypeptide comprises an amino acid mutation of 349G, and the CH3 domain of the second polypeptide comprises an amino acid mutation of 354W; or
   the CH3 domain of the first polypeptide comprises an amino acid mutation of 349A, and the CH3 domain of the second polypeptide comprises one amino acid mutation selected from the group consisting of 354F, 354Y, and 354W; or
   the CH3 domain of the first polypeptide comprises an amino acid mutation of 349V, and the CH3 domain of the second polypeptide comprises an amino acid mutation of 354F; or
   the CH3 domain of the first polypeptide comprises an amino acid mutation of 349T, and the CH3 domain of the second polypeptide comprises an amino acid mutation of 354F; or
9) the CH3 domain of the first polypeptide comprises an amino acid mutation of 366H, and the CH3 domain of the second polypeptide comprises an amino acid mutation of 405L or 407L; or
10) the CH3 domain of the first polypeptide comprises an amino acid mutation of 349C, and the CH3 domain of the second polypeptide comprises amino acid mutations of 347E and 357C; or
11) the CH3 domain of the first polypeptide comprises amino acid mutations of 356K and 366H, and the CH3 domain of the second polypeptide comprises an amino acid mutation of 439E, wherein the CH3 domain of the second polypeptide further comprises one amino acid mutation selected from the group consisting of 405L, 407L, and 407H; or
12) the CH3 domain of the first polypeptide comprises amino acid mutations of 356K and 407L, and the CH3 domain of the second polypeptide comprises amino acid mutations of 439E and 366H; or
   the CH3 domain of the first polypeptide comprises amino acid mutations of 356K and 407H, and the CH3 domain of the second polypeptide comprises amino acid mutations of 439E and 366H; or
13) the CH3 domain of the first polypeptide comprises amino acid mutations of 354C and 405T, and the CH3 domain of the second polypeptide comprises amino acid mutations of 349C and 394F; or
14) the CH3 domain of the first polypeptide comprises amino acid mutations of 356K and 409D, and the CH3 domain of the second polypeptide comprises amino acid mutations of 439E and 399K.

In some embodiments, provided is the aforementioned multispecific antibody, wherein:
i) the CH3 domain of the first polypeptide comprises amino acid mutations of 356K and 349S, and the CH3 domain of the second polypeptide comprises amino acid mutations of 439E and 354Y; or
   the CH3 domain of the first polypeptide comprises amino acid mutations of 356K and 349C, and the CH3 domain of the second polypeptide comprises amino acid mutations of 439E and 357C; or
ii) the CH3 domain of the first polypeptide comprises an amino acid mutation of 405T, and the CH3 domain of the second polypeptide comprises an amino acid mutation of 394F; or
iii) the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of 349L, 351C, 351V, 351T, 351I, 351F, 364A, 364T, 364L, 366G, 366A, 366V, 366L, 366H, 368V, 368I, 394A, 394S, 394C, 394V, 394N, 397I, 397L, 405L, 405Y, 407C, 407V, 407L, 407H, 409Q, 409R, and 411Y; or
iv) the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I; or
v) the CH3 domain of the first polypeptide comprises amino acid mutations of 356K and 405T, and the CH3 domain of the second polypeptide comprises amino acid mutations of 439E and 394F; or
vi) the CH3 domain of the first polypeptide comprises an amino acid mutation of 349C, and the CH3 domain of the second polypeptide comprises an amino acid mutation of 357C; or
vii) the CH3 domain of the first polypeptide comprises an amino acid mutation of 349C, and the CH3 domain of the second polypeptide comprises an amino acid mutation of 354C, wherein the CH3 domain of the second polypeptide further comprises one amino acid mutation selected from the group consisting of 366A, 368I, and 405Y.

In some embodiments, provided is the aforementioned multispecific antibody, wherein:
1) the CH3 domain of the first polypeptide comprises amino acid mutations of 356K and 349S, and the CH3 domain of the second polypeptide comprises amino acid mutations of 439E and 354Y; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351T, 351I, 364A, 366A, 368I, 394A, 394S, 405Y, 409Q, and 411Y; or
4) the CH3 domain of the first polypeptide comprises amino acid mutations of 356K and 349C, and the CH3 domain of the second polypeptide comprises amino acid mutations of 439E, 354C, and 351I; or
5) the CH3 domain of the first polypeptide comprises an amino acid mutation of 349C, and the CH3 domain of the second polypeptide comprises an amino acid mutation of 354C, wherein the CH3 domain of the second polypeptide further comprises an amino acid mutation of 364Y or 364F; or
6) the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W.

In some embodiments, provided is the aforementioned multispecific antibody, wherein:
i) the CH3 domain of the first polypeptide comprises amino acid mutations of 356K and 349S, and the CH3 domain of the second polypeptide comprises amino acid mutations of 439E and 354Y; or
ii) the CH3 domain of the first polypeptide comprises an amino acid mutation of 405T, and the CH3 domain of the second polypeptide comprises an amino acid mutation of 394F; or
iii) the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351T, 351I, 364A, 366A, 368I, 394A, 394S, 405Y, 409Q, and 411Y; or
iv) the CH3 domain of the first polypeptide comprises amino acid mutations of 356K and 349C, and the CH3 domain of the second polypeptide comprises amino acid mutations of 439E, 354C, and 351I.

In some embodiments, provided is the aforementioned multispecific antibody, wherein:
1) the CH3 domain of the first polypeptide comprises amino acid mutations of 356K and 349S, and the CH3 domain of the second polypeptide comprises amino acid mutations of 439E and 354Y; or
   the CH3 domain of the first polypeptide comprises amino acid mutations of 356K and 349C, and the CH3 domain of the second polypeptide comprises amino acid mutations of 439E and 357C; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises amino acid mutations of 356K and 349L, and the CH3 domain of the second polypeptide comprises amino acid mutations of 439E and 349L; or
   the CH3 domain of the first polypeptide comprises amino acid mutations of 356K and 351C, and the CH3 domain of the second polypeptide comprises amino acid mutations of 439E and 351C; or
   the CH3 domain of the first polypeptide comprises amino acid mutations of 356K and 351V, and the CH3 domain of the second polypeptide comprises amino acid mutations of 439E and 351V; or
   the CH3 domain of the first polypeptide comprises amino acid mutations of 356K and 351T, and the CH3 domain of the second polypeptide comprises amino acid mutations of 439E and 351T; or
   the CH3 domain of the first polypeptide comprises amino acid mutations of 356K and 351I, and the CH3 domain of the second polypeptide comprises amino acid mutations of 439E and 351I; or
   the CH3 domain of the first polypeptide comprises amino acid mutations of 356K and 351F, and the CH3 domain of the second polypeptide comprises amino acid mutations of 439E and 351F; or
   the CH3 domain of the first polypeptide comprises amino acid mutations of 356K and 351M, and the CH3 domain of the second polypeptide comprises amino acid mutations of 439E and 351M; or
   the CH3 domain of the first polypeptide comprises amino acid mutations of 356K and 364A, and the CH3 domain of the second polypeptide comprises amino acid mutations of 439E and 364A; or
   the CH3 domain of the first polypeptide comprises amino acid mutations of 356K and 364T, and the CH3 domain of the second polypeptide comprises amino acid mutations of 439E and 364T; or
   the CH3 domain of the first polypeptide comprises amino acid mutations of 356K and 364L, and the CH3 domain of the second polypeptide comprises amino acid mutations of 439E and 364L; or
   the CH3 domain of the first polypeptide comprises amino acid mutations of 356K and 366G, and the CH3 domain of the second polypeptide comprises amino acid mutations of 439E and 366G; or
   the CH3 domain of the first polypeptide comprises amino acid mutations of 356K and 366A, and the CH3 domain of the second polypeptide comprises amino acid mutations of 439E and 366A; or
   the CH3 domain of the first polypeptide comprises amino acid mutations of 356K and 366V, and the CH3 domain of the second polypeptide comprises amino acid mutations of 439E and 366V; or
   the CH3 domain of the first polypeptide comprises amino acid mutations of 356K and 366L, and the CH3 domain of the second polypeptide comprises amino acid mutations of 439E and 366L; or
   the CH3 domain of the first polypeptide comprises amino acid mutations of 356K and 366H, and the CH3 domain of the second polypeptide comprises amino acid mutations of 439E and 366H; or
   the CH3 domain of the first polypeptide comprises amino acid mutations of 356K and 366I, and the CH3 domain of the second polypeptide comprises amino acid mutations of 439E and 366I; or
   the CH3 domain of the first polypeptide comprises amino acid mutations of 356K and 368V, and the CH3 domain of the second polypeptide comprises amino acid mutations of 439E and 368V; or
   the CH3 domain of the first polypeptide comprises amino acid mutations of 356K and 368I, and the CH3 domain of the second polypeptide comprises amino acid mutations of 439E and 368I; or
   the CH3 domain of the first polypeptide comprises amino acid mutations of 356K and 368A, and the CH3 domain of the second polypeptide comprises amino acid mutations of 439E and 368A; or
   the CH3 domain of the first polypeptide comprises amino acid mutations of 356K and 394A, and the CH3 domain of the second polypeptide comprises amino acid mutations of 439E and 394A; or
   the CH3 domain of the first polypeptide comprises amino acid mutations of 356K and 394S, and the CH3 domain of the second polypeptide comprises amino acid mutations of 439E and 394S; or
   the CH3 domain of the first polypeptide comprises amino acid mutations of 356K and 394C, and the CH3 domain of the second polypeptide comprises amino acid mutations of 439E and 394C; or
   the CH3 domain of the first polypeptide comprises amino acid mutations of 356K and 394V, and the CH3 domain of the second polypeptide comprises amino acid mutations of 439E and 394V; or
   the CH3 domain of the first polypeptide comprises amino acid mutations of 356K and 394N, and the CH3 domain of the second polypeptide comprises amino acid mutations of 439E and 394N; or
   the CH3 domain of the first polypeptide comprises amino acid mutations of 356K and 397I, and the CH3 domain of the second polypeptide comprises amino acid mutations of 439E and 397I; or
   the CH3 domain of the first polypeptide comprises amino acid mutations of 356K and 397L, and the CH3 domain of the second polypeptide comprises amino acid mutations of 439E and 397L; or the CH3 domain of the first polypeptide comprises amino acid mutations of 356K and 405L, and the CH3 domain of the second polypeptide comprises amino acid mutations of 439E and 405L; or
   the CH3 domain of the first polypeptide comprises amino acid mutations of 356K and 405Y, and the CH3 domain of the second polypeptide comprises amino acid mutations of 439E and 405Y; or
   the CH3 domain of the first polypeptide comprises amino acid mutations of 356K and 407C, and the CH3 domain of the second polypeptide comprises amino acid mutations of 439E and 407C; or
   the CH3 domain of the first polypeptide comprises amino acid mutations of 356K and 407V, and the CH3 domain of the second polypeptide comprises amino acid mutations of 439E and 407V; or
   the CH3 domain of the first polypeptide comprises amino acid mutations of 356K and 407L, and the CH3 domain of the second polypeptide comprises amino acid mutations of 439E and 407L; or
   the CH3 domain of the first polypeptide comprises amino acid mutations of 356K and 407H, and the CH3 domain of the second polypeptide comprises amino acid mutations of 439E and 407H; or
   the CH3 domain of the first polypeptide comprises amino acid mutations of 356K and 407F, and the CH3 domain of the second polypeptide comprises amino acid mutations of 439E and 407F; or
   the CH3 domain of the first polypeptide comprises amino acid mutations of 356K and 409Q, and the CH3 domain of the second polypeptide comprises amino acid mutations of 439E and 409Q; or
   the CH3 domain of the first polypeptide comprises amino acid mutations of 356K and 409R, and the CH3 domain of the second polypeptide comprises amino acid mutations of 439E and 409R; or
   the CH3 domain of the first polypeptide comprises amino acid mutations of 356K and 411L, and the CH3 domain of the second polypeptide comprises amino acid mutations of 439E and 411L; or
   the CH3 domain of the first polypeptide comprises amino acid mutations of 356K and 411Y, and the CH3 domain of the second polypeptide comprises amino acid mutations of 439E and 411Y; or
4) the CH3 domain of the first polypeptide comprises amino acid mutations of 356K and 349C, and the CH3 domain of the second polypeptide comprises amino acid mutations of 439E, 354C, and 351I; or
   the CH3 domain of the first polypeptide comprises amino acid mutations of 356K and 349C, and the CH3 domain of the second polypeptide comprises amino acid mutations of 439E, 354C, and 366A; or
   the CH3 domain of the first polypeptide comprises amino acid mutations of 356K and 349C, and the CH3 domain of the second polypeptide comprises amino acid mutations of 439E, 354C, and 368I; or
5) the CH3 domain of the first polypeptide comprises amino acid mutations of 356K and 405T, and the CH3 domain of the second polypeptide comprises amino acid mutations of 439E and 394F; or
6) the CH3 domain of the first polypeptide comprises an amino acid mutation of 349C, and the CH3 domain of the second polypeptide comprises an amino acid mutation of 357C; or
7) the CH3 domain of the first polypeptide comprises an amino acid mutation of 349C, and the CH3 domain of the second polypeptide comprises amino acid mutations of 354C and 351I; or
   the CH3 domain of the first polypeptide comprises an amino acid mutation of 349C, and the CH3 domain of the second polypeptide comprises amino acid mutations of 354C and 364Y; or
   the CH3 domain of the first polypeptide comprises an amino acid mutation of 349C, and the CH3 domain of the second polypeptide comprises amino acid mutations of 354C and 364F; or
   the CH3 domain of the first polypeptide comprises an amino acid mutation of 349C, and the CH3 domain of the second polypeptide comprises amino acid mutations of 354C and 366A; or
   the CH3 domain of the first polypeptide comprises an amino acid mutation of 349C, and the CH3 domain of the second polypeptide comprises amino acid mutations of 354C and 368I; or
   the CH3 domain of the first polypeptide comprises an amino acid mutation of 349C, and the CH3 domain of the second polypeptide comprises amino acid mutations of 354C and 405Y; or
8) the CH3 domain of the first polypeptide comprises an amino acid mutation of 349S, and the CH3 domain of the second polypeptide comprises an amino acid mutation of 364Y; or
   the CH3 domain of the first polypeptide comprises an amino acid mutation of 349S, and the CH3 domain of the second polypeptide comprises an amino acid mutation of 354F; or
   the CH3 domain of the first polypeptide comprises an amino acid mutation of 349S, and the CH3 domain of the second polypeptide comprises an amino acid mutation of 354W; or
   the CH3 domain of the first polypeptide comprises an amino acid mutation of 349S, and the CH3 domain of the second polypeptide comprises an amino acid mutation of 354Y; or
   the CH3 domain of the first polypeptide comprises an amino acid mutation of 349G, and the CH3 domain of the second polypeptide comprises an amino acid mutation of 354W; or
   the CH3 domain of the first polypeptide comprises an amino acid mutation of 349A, and the CH3 domain of the second polypeptide comprises an amino acid mutation of 354F; or
   the CH3 domain of the first polypeptide comprises an amino acid mutation of 349A, and the CH3 domain of the second polypeptide comprises an amino acid mutation of 354Y; or
   the CH3 domain of the first polypeptide comprises an amino acid mutation of 349A, and the CH3 domain of the second polypeptide comprises an amino acid mutation of 354W; or
   the CH3 domain of the first polypeptide comprises an amino acid mutation of 349V, and the CH3 domain of the second polypeptide comprises an amino acid mutation of 354F; or
   the CH3 domain of the first polypeptide comprises an amino acid mutation of 349T, and the CH3 domain of the second polypeptide comprises an amino acid mutation of 354F; or
9) the CH3 domain of the first polypeptide comprises an amino acid mutation of 366H, and the CH3 domain of the second polypeptide comprises an amino acid mutation of 405L; or
   the CH3 domain of the first polypeptide comprises an amino acid mutation of 366H, and the CH3 domain of the second polypeptide comprises an amino acid mutation of 407L; or
10) the CH3 domain of the first polypeptide comprises an amino acid mutation of 349C, and the CH3 domain of the second polypeptide comprises amino acid mutations of 347E and 357C; or
11) the CH3 domain of the first polypeptide comprises amino acid mutations of 356K and 366H, and the CH3 domain of the second polypeptide comprises amino acid mutations of 439E and 405L; or
   the CH3 domain of the first polypeptide comprises amino acid mutations of 356K and 366H, and the CH3 domain of the second polypeptide comprises amino acid mutations of 439E and 407L; or
   the CH3 domain of the first polypeptide comprises amino acid mutations of 356K and 366H, and the CH3 domain of the second polypeptide comprises amino acid mutations of 439E and 407H; or
12) the CH3 domain of the first polypeptide comprises amino acid mutations of 356K and 407L, and the CH3 domain of the second polypeptide comprises amino acid mutations of 439E and 366H; or
   the CH3 domain of the first polypeptide comprises amino acid mutations of 356K and 407H, and the CH3 domain of the second polypeptide comprises amino acid mutations of 439E and 366H; or
13) the CH3 domain of the first polypeptide comprises amino acid mutations of 354C and 405T, and the CH3 domain of the second polypeptide comprises amino acid mutations of 349C and 394F; or
14) the CH3 domain of the first polypeptide comprises amino acid mutations of 356K and 409D, and the CH3 domain of the second polypeptide comprises amino acid mutations of 439E and 399K.

In some embodiments, provided is the aforementioned multispecific antibody, wherein:
i) the CH3 domain of the first polypeptide comprises amino acid mutations of 356K and 349S, and the CH3 domain of the second polypeptide comprises amino acid mutations of 439E and 354Y; or
   the CH3 domain of the first polypeptide comprises amino acid mutations of 356K and 349C, and the CH3 domain of the second polypeptide comprises amino acid mutations of 439E and 357C; or
ii) the CH3 domain of the first polypeptide comprises an amino acid mutation of 405T, and the CH3 domain of the second polypeptide comprises an amino acid mutation of 394F; or
iii) the CH3 domain of the first polypeptide comprises amino acid mutations of 356K and 349L, and the CH3 domain of the second polypeptide comprises amino acid mutations of 439E and 349L; or
   the CH3 domain of the first polypeptide comprises amino acid mutations of 356K and 351C, and the CH3 domain of the second polypeptide comprises amino acid mutations of 439E and 351C; or
   the CH3 domain of the first polypeptide comprises amino acid mutations of 356K and 351V, and the CH3 domain of the second polypeptide comprises amino acid mutations of 439E and 351V; or
   the CH3 domain of the first polypeptide comprises amino acid mutations of 356K and 351T, and the CH3 domain of the second polypeptide comprises amino acid mutations of 439E and 351T; or
   the CH3 domain of the first polypeptide comprises amino acid mutations of 356K and 351I, and the CH3 domain of the second polypeptide comprises amino acid mutations of 439E and 351I; or
   the CH3 domain of the first polypeptide comprises amino acid mutations of 356K and 351F, and the CH3 domain of the second polypeptide comprises amino acid mutations of 439E and 351F; or
   the CH3 domain of the first polypeptide comprises amino acid mutations of 356K and 364A, and the CH3 domain of the second polypeptide comprises amino acid mutations of 439E and 364A; or
   the CH3 domain of the first polypeptide comprises amino acid mutations of 356K and 364T, and the CH3 domain of the second polypeptide comprises amino acid mutations of 439E and 364T; or
   the CH3 domain of the first polypeptide comprises amino acid mutations of 356K and 364L, and the CH3 domain of the second polypeptide comprises amino acid mutations of 439E and 364L; or
   the CH3 domain of the first polypeptide comprises amino acid mutations of 356K and 366G, and the CH3 domain of the second polypeptide comprises amino acid mutations of 439E and 366G; or
   the CH3 domain of the first polypeptide comprises amino acid mutations of 356K and 366A, and the CH3 domain of the second polypeptide comprises amino acid mutations of 439E and 366A; or
   the CH3 domain of the first polypeptide comprises amino acid mutations of 356K and 366V, and the CH3 domain of the second polypeptide comprises amino acid mutations of 439E and 366V; or
   the CH3 domain of the first polypeptide comprises amino acid mutations of 356K and 366L, and the CH3 domain of the second polypeptide comprises amino acid mutations of 439E and 366L; or
   the CH3 domain of the first polypeptide comprises amino acid mutations of 356K and 366H, and the CH3 domain of the second polypeptide comprises amino acid mutations of 439E and 366H; or
   the CH3 domain of the first polypeptide comprises amino acid mutations of 356K and 368V, and the CH3 domain of the second polypeptide comprises amino acid mutations of 439E and 368V; or
   the CH3 domain of the first polypeptide comprises amino acid mutations of 356K and 368I, and the CH3 domain of the second polypeptide comprises amino acid mutations of 439E and 368I; or
   the CH3 domain of the first polypeptide comprises amino acid mutations of 356K and 394A, and the CH3 domain of the second polypeptide comprises amino acid mutations of 439E and 394A; or
   the CH3 domain of the first polypeptide comprises amino acid mutations of 356K and 394S, and the CH3 domain of the second polypeptide comprises amino acid mutations of 439E and 394S; or
   the CH3 domain of the first polypeptide comprises amino acid mutations of 356K and 394C, and the CH3 domain of the second polypeptide comprises amino acid mutations of 439E and 394C; or
   the CH3 domain of the first polypeptide comprises amino acid mutations of 356K and 394V, and the CH3 domain of the second polypeptide comprises amino acid mutations of 439E and 394V; or
   the CH3 domain of the first polypeptide comprises amino acid mutations of 356K and 394N, and the CH3 domain of the second polypeptide comprises amino acid mutations of 439E and 394N; or
   the CH3 domain of the first polypeptide comprises amino acid mutations of 356K and 397I, and the CH3 domain of the second polypeptide comprises amino acid mutations of 439E and 397I; or
   the CH3 domain of the first polypeptide comprises amino acid mutations of 356K and 397L, and the CH3 domain of the second polypeptide comprises amino acid mutations of 439E and 397L; or
   the CH3 domain of the first polypeptide comprises amino acid mutations of 356K and 405L, and the CH3 domain of the second polypeptide comprises amino acid mutations of 439E and 405L; or
   the CH3 domain of the first polypeptide comprises amino acid mutations of 356K and 405Y, and the CH3 domain of the second polypeptide comprises amino acid mutations of 439E and 405Y; or
   the CH3 domain of the first polypeptide comprises amino acid mutations of 356K and 407C, and the CH3 domain of the second polypeptide comprises amino acid mutations of 439E and 407C; or
   the CH3 domain of the first polypeptide comprises amino acid mutations of 356K and 407V, and the CH3 domain of the second polypeptide comprises amino acid mutations of 439E and 407V; or
   the CH3 domain of the first polypeptide comprises amino acid mutations of 356K and 407L, and the CH3 domain of the second polypeptide comprises amino acid mutations of 439E and 407L; or
   the CH3 domain of the first polypeptide comprises amino acid mutations of 356K and 407H, and the CH3 domain of the second polypeptide comprises amino acid mutations of 439E and 407H; or
   the CH3 domain of the first polypeptide comprises amino acid mutations of 356K and 409Q, and the CH3 domain of the second polypeptide comprises amino acid mutations of 439E and 409Q; or
   the CH3 domain of the first polypeptide comprises amino acid mutations of 356K and 409R, and the CH3 domain of the second polypeptide comprises amino acid mutations of 439E and 409R; or
   the CH3 domain of the first polypeptide comprises amino acid mutations of 356K and 411Y, and the CH3 domain of the second polypeptide comprises amino acid mutations of 439E and 411Y; or
iv) the CH3 domain of the first polypeptide comprises amino acid mutations of 356K and 349C, and the CH3 domain of the second polypeptide comprises amino acid mutations of 439E, 354C, and 351I; or
   the CH3 domain of the first polypeptide comprises amino acid mutations of 356K and 349C, and the CH3 domain of the second polypeptide comprises amino acid mutations of 439E, 354C, and 366A; or
   the CH3 domain of the first polypeptide comprises amino acid mutations of 356K and 349C, and the CH3 domain of the second polypeptide comprises amino acid mutations of 439E, 354C, and 368I; or
v) the CH3 domain of the first polypeptide comprises amino acid mutations of 356K and 405T, and the CH3 domain of the second polypeptide comprises amino acid mutations of 439E and 394F; or
vi) the CH3 domain of the first polypeptide comprises an amino acid mutation of 349C, and the CH3 domain of the second polypeptide comprises an amino acid mutation of 357C; or
vii) the CH3 domain of the first polypeptide comprises an amino acid mutation of 349C, and the CH3 domain of the second polypeptide comprises amino acid mutations of 354C and 366A; or
   the CH3 domain of the first polypeptide comprises an amino acid mutation of 349C, and the CH3 domain of the second polypeptide comprises amino acid mutations of 354C and 368I; or
   the CH3 domain of the first polypeptide comprises an amino acid mutation of 349C, and the CH3 domain of the second polypeptide comprises amino acid mutations of 354C and 405Y.

In some embodiments, provided is the aforementioned multispecific antibody, wherein:
1) the CH3 domain of the first polypeptide comprises amino acid mutations of 356K and 349S, and the CH3 domain of the second polypeptide comprises amino acid mutations of 439E and 354Y; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises amino acid mutations of 356K and 351T, and the CH3 domain of the second polypeptide comprises amino acid mutations of 439E and 351T; or
   the CH3 domain of the first polypeptide comprises amino acid mutations of 356K and 351I, and the CH3 domain of the second polypeptide comprises amino acid mutations of 439E and 351I; or
   the CH3 domain of the first polypeptide comprises amino acid mutations of 356K and 364A, and the CH3 domain of the second polypeptide comprises amino acid mutations of 439E and 364A; or
   the CH3 domain of the first polypeptide comprises amino acid mutations of 356K and 366A, and the CH3 domain of the second polypeptide comprises amino acid mutations of 439E and 366A; or
   the CH3 domain of the first polypeptide comprises amino acid mutations of 356K and 368I, and the CH3 domain of the second polypeptide comprises amino acid mutations of 439E and 368I; or
   the CH3 domain of the first polypeptide comprises amino acid mutations of 356K and 394A, and the CH3 domain of the second polypeptide comprises amino acid mutations of 439E and 394A; or
   the CH3 domain of the first polypeptide comprises amino acid mutations of 356K and 394S, and the CH3 domain of the second polypeptide comprises amino acid mutations of 439E and 394S; or
   the CH3 domain of the first polypeptide comprises amino acid mutations of 356K and 405Y, and the CH3 domain of the second polypeptide comprises amino acid mutations of 439E and 405Y; or
   the CH3 domain of the first polypeptide comprises amino acid mutations of 356K and 409Q, and the CH3 domain of the second polypeptide comprises amino acid mutations of 439E and 409Q; or
   the CH3 domain of the first polypeptide comprises amino acid mutations of 356K and 411Y, and the CH3 domain of the second polypeptide comprises amino acid mutations of 439E and 411Y; or
4) the CH3 domain of the first polypeptide comprises amino acid mutations of 356K and 349C, and the CH3 domain of the second polypeptide comprises amino acid mutations of 439E, 354C, and 351I; or
5) the CH3 domain of the first polypeptide comprises an amino acid mutation of 349C, and the CH3 domain of the second polypeptide comprises amino acid mutations of 354C and 364Y; or
   the CH3 domain of the first polypeptide comprises an amino acid mutation of 349C, and the CH3 domain of the second polypeptide comprises amino acid mutations of 354C and 364F; or
6) the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W.

In some embodiments, provided is the aforementioned multispecific antibody, wherein:
i) the CH3 domain of the first polypeptide comprises amino acid mutations of 356K and 349S, and the CH3 domain of the second polypeptide comprises amino acid mutations of 439E and 354Y; or
ii) the CH3 domain of the first polypeptide comprises an amino acid mutation of 405T, and the CH3 domain of the second polypeptide comprises an amino acid mutation of 394F; or
iii) the CH3 domain of the first polypeptide comprises amino acid mutations of 356K and 351T, and the CH3 domain of the second polypeptide comprises amino acid mutations of 439E and 351T; or
   the CH3 domain of the first polypeptide comprises amino acid mutations of 356K and 351I, and the CH3 domain of the second polypeptide comprises amino acid mutations of 439E and 351I; or
   the CH3 domain of the first polypeptide comprises amino acid mutations of 356K and 364A, and the CH3 domain of the second polypeptide comprises amino acid mutations of 439E and 364A; or
   the CH3 domain of the first polypeptide comprises amino acid mutations of 356K and 366A, and the CH3 domain of the second polypeptide comprises amino acid mutations of 439E and 366A; or
   the CH3 domain of the first polypeptide comprises amino acid mutations of 356K and 368I, and the CH3 domain of the second polypeptide comprises amino acid mutations of 439E and 368I; or
   the CH3 domain of the first polypeptide comprises amino acid mutations of 356K and 394A, and the CH3 domain of the second polypeptide comprises amino acid mutations of 439E and 394A; or
   the CH3 domain of the first polypeptide comprises amino acid mutations of 356K and 394S, and the CH3 domain of the second polypeptide comprises amino acid mutations of 439E and 394S; or
   the CH3 domain of the first polypeptide comprises amino acid mutations of 356K and 405Y, and the CH3 domain of the second polypeptide comprises amino acid mutations of 439E and 405Y; or
   the CH3 domain of the first polypeptide comprises amino acid mutations of 356K and 409Q, and the CH3 domain of the second polypeptide comprises amino acid mutations of 439E and 409Q; or
   the CH3 domain of the first polypeptide comprises amino acid mutations of 356K and 411Y, and the CH3 domain of the second polypeptide comprises amino acid mutations of 439E and 411Y; or
iv) the CH3 domain of the first polypeptide comprises amino acid mutations of 356K and 349C, and the CH3 domain of the second polypeptide comprises amino acid mutations of 439E, 354C, and 351I.

In some embodiments, provided is the aforementioned multispecific antibody, wherein the CH3 domain of the first polypeptide comprises amino acid mutations of 356K and 349S, and the CH3 domain of the second polypeptide comprises amino acid mutations of 439E and 354Y.

In some embodiments, provided is the aforementioned multispecific antibody, wherein the CH3 domain of the first polypeptide comprises an amino acid mutation of 405T, and the CH3 domain of the second polypeptide comprises an amino acid mutation of 394F.

In some embodiments, provided is the aforementioned multispecific antibody, wherein the CH3 domain of the first polypeptide comprises amino acid mutations of 356K and 351I, and the CH3 domain of the second polypeptide comprises amino acid mutations of 439E and 351I.

In some embodiments, provided is the aforementioned multispecific antibody, wherein the CH3 domain of the first polypeptide comprises amino acid mutations of 356K and 394S, and the CH3 domain of the second polypeptide comprises amino acid mutations of 439E and 394S.

In some embodiments, provided is the aforementioned multispecific antibody, wherein the CH3 domain of the first polypeptide comprises amino acid mutations of 356K and 411Y, and the CH3 domain of the second polypeptide comprises amino acid mutations of 439E and 411Y.

In some embodiments, provided is the aforementioned multispecific antibody, wherein the CH3 domain of the first polypeptide comprises an amino acid mutation of 349S, and the CH3 domain of the second polypeptide comprises an amino acid mutation of 364Y.

In some embodiments, provided is the aforementioned multispecific antibody, wherein the CH3 domain of the first polypeptide comprises amino acid mutations of 356K and 349C, and the CH3 domain of the second polypeptide comprises amino acid mutations of 439E, 354C, and 351I.

In some embodiments, provided is the aforementioned multispecific antibody, wherein the CH3 domain of the first polypeptide comprises an amino acid mutation of 349C, and the CH3 domain of the second polypeptide comprises amino acid mutations of 354C and 364Y.

In some embodiments, provided is the aforementioned multispecific antibody, wherein the CH3 domain of the first polypeptide comprises an amino acid mutation of 349C, and the CH3 domain of the second polypeptide comprises amino acid mutations of 354C and 364F.

In some embodiments, provided is the aforementioned multispecific antibody, wherein the CH3 domain of the first polypeptide comprises only amino acid mutations of 356K and 349S, and the CH3 domain of the second polypeptide comprises only amino acid mutations of 439E and 354Y.

In some embodiments, provided is the aforementioned multispecific antibody, wherein the CH3 domain of the first polypeptide comprises only an amino acid mutation of 405T, and the CH3 domain of the second polypeptide comprises only an amino acid mutation of 394F.

In some embodiments, provided is the aforementioned multispecific antibody, wherein the CH3 domain of the first polypeptide comprises only amino acid mutations of 356K and 351I, and the CH3 domain of the second polypeptide comprises only amino acid mutations of 439E and 351I.

In some embodiments, provided is the aforementioned multispecific antibody, wherein the CH3 domain of the first polypeptide comprises only amino acid mutations of 356K and 394S, and the CH3 domain of the second polypeptide comprises only amino acid mutations of 439E and 394S.

In some embodiments, provided is the aforementioned multispecific antibody, wherein the CH3 domain of the first polypeptide comprises only amino acid mutations of 356K and 411Y, and the CH3 domain of the second polypeptide comprises only amino acid mutations of 439E and 411Y.

In some embodiments, provided is the aforementioned multispecific antibody, wherein the CH3 domain of the first polypeptide comprises only an amino acid mutation of 349S, and the CH3 domain of the second polypeptide comprises only an amino acid mutation of 364Y.

In some embodiments, provided is the aforementioned multispecific antibody, wherein the CH3 domain of the first polypeptide comprises only amino acid mutations of 356K and 349C, and the CH3 domain of the second polypeptide comprises only amino acid mutations of 439E, 354C, and 351I.

In some embodiments, provided is the aforementioned multispecific antibody, wherein the CH3 domain of the first polypeptide comprises only an amino acid mutation of 349C, and the CH3 domain of the second polypeptide comprises only amino acid mutations of 354C and 364Y.

In some embodiments, provided is the aforementioned multispecific antibody, wherein the CH3 domain of the first polypeptide comprises only an amino acid mutation of 349C, and the CH3 domain of the second polypeptide comprises only amino acid mutations of 354C and 364F.

In some embodiments, provided is the aforementioned multispecific antibody, wherein the CH3 domains of the first polypeptide and the second polypeptide are derived from IgG1. In some embodiments, the CH3 domains of the first polypeptide and the second polypeptide are derived from human IgG1.

In some embodiments, provided is the aforementioned multispecific antibody, wherein the multispecific antibody comprises at least one replaced Fab comprising a Titin chain and an Obscurin chain that are capable of forming a dimer. In some embodiments, provided is the aforementioned multispecific antibody, wherein the replaced Fab is obtained by replacing the original CH1 and CL of a Fab with the Obscurin chain and the Titin chain, respectively, or the replaced Fab is obtained by replacing the original CH1 and CL of a Fab with the Titin chain and the Obscurin chain, respectively. In some embodiments, the Titin chain comprises the amino acid sequence set forth in SEQ ID NO: 6, and the Obscurin chain comprises the amino acid sequence set forth in SEQ ID NO: 5.

In another aspect, the present disclosure provides a pharmaceutical composition comprising the aforementioned multispecific antibody and one or more pharmaceutically acceptable carriers, diluents, or excipients.

In another aspect, the present disclosure provides an immunoconjugate comprising the aforementioned multispecific antibody and an effector, wherein the effector is conjugated to the multispecific antibody. In some embodiments, the effector is selected from the group consisting of an anti-tumor agent, an immunomodulator, a biological response modifier, a lectin, a cytotoxic drug, a chromophore, a fluorophore, a chemiluminescent compound, an enzyme, and a metal ion, and any combination thereof.

In another aspect, the present disclosure provides an isolated nucleic acid encoding the aforementioned multispecific antibody.

In another aspect, the present disclosure provides a vector comprising the aforementioned isolated nucleic acid.

In another aspect, the present disclosure provides a host cell comprising the aforementioned isolated nucleic acid.

In another aspect, the present disclosure provides a method for preparing the aforementioned multispecific antibody, comprising culturing the aforementioned host cell and recovering the multispecific antibody from the host cell culture.

In another aspect, the present disclosure provides a method for preparing the aforementioned multispecific antibody, comprising: (a) a step of altering a nucleic acid encoding amino acid residues that form an interface between the polypeptides, etc., (b) a step of culturing a host cell comprising the nucleic acid to express the polypeptides, (c) a step of recovering the polypeptides from the culture of the host cell, and (d) a step of incubating the polypeptides under reducing conditions and recovering the desired multispecific antibody.

In some embodiments, provided is the aforementioned multispecific antibody, wherein the first polypeptide is an antibody heavy chain, and/or the second polypeptide is an antibody heavy chain.

In some embodiments, the aforementioned multispecific antibody further comprises one or more antibody light chains.

In some embodiments, the aforementioned multispecific antibody is a bispecific antibody. In some embodiments, the aforementioned multispecific antibody is a bispecific antibody that specifically binds to CCR8 and CTLA4.

In some embodiments, the present disclosure provides a bispecific antibody that specifically binds to CCR8 and CTLA4, comprising: one first chain comprising the amino acid sequence set forth in SEQ ID NO: 7, one second chain comprising the amino acid sequence set forth in SEQ ID NO: 8, one third chain comprising the amino acid sequence set forth in SEQ ID NO: 9, and one fourth chain comprising the amino acid sequence set forth in SEQ ID NO: 10.

In some embodiments, the aforementioned multispecific antibody is a bispecific antibody that specifically binds to two epitopes of HER2.

In some embodiments, the present disclosure provides a bispecific antibody that specifically binds to two epitopes of HER2, comprising: one first chain comprising the amino acid sequence set forth in SEQ ID NO: 15, one second chain comprising the amino acid sequence set forth in SEQ ID NO: 16, one third chain comprising the amino acid sequence set forth in SEQ ID NO: 17, and one fourth chain comprising the amino acid sequence set forth in SEQ ID NO: 18.

In another aspect, the present disclosure provides a trispecific antibody that specifically binds to CD3, CEA, and MUC1, comprising: one first chain comprising the amino acid sequence set forth in SEQ ID NO: 32, one second chain comprising the amino acid sequence set forth in SEQ ID NO: 31, one third chain comprising the amino acid sequence set forth in SEQ ID NO: 37, and one fourth chain comprising the amino acid sequence set forth in SEQ ID NO: 38.

In another aspect, the present disclosure provides an anti-CD3 antibody comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein the HCDR1, the HCDR2, and the HCDR3 of the heavy chain variable region comprise the amino acid sequences of a HCDR1, a HCDR2, and a HCDR3, respectively, in SEQ ID NO: 27, and the LCDR1, the LCDR2, and the LCDR3 of the light chain variable region comprise the amino acid sequences of a LCDR1, a LCDR2, and a LCDR3, respectively, in SEQ ID NO: 28.

In some embodiments, provided is the anti-CD3 antibody according to any one of the foregoing, wherein in the heavy chain variable region, the HCDR1 comprises the amino acid sequence of SEQ ID NO: 21, the HCDR2 comprises the amino acid sequence of SEQ ID NO: 22, and the HCDR3 comprises the amino acid sequence of SEQ ID NO: 23; and in the light chain variable region, the LCDR1 comprises the amino acid sequence of SEQ ID NO: 24, the LCDR2 comprises the amino acid sequence of SEQ ID NO: 25, and the LCDR3 comprises the amino acid sequence of SEQ ID NO: 26.

In some embodiments, the anti-CD3 antibody according to any one of the foregoing is a murine-derived antibody, a chimeric antibody, a humanized antibody, or a fully human-derived antibody. In some embodiments, the anti-CD3 antibody according to any one of the foregoing is a humanized antibody.

In some embodiments, provided is the anti-CD3 antibody according to any one of the foregoing, wherein the heavy chain variable region comprises an amino acid sequence having at least 80% (e.g., at least 85%, 90%, 91%, 92%, 93%, 94%, 95%, 96%, 97%, 98%, or 99%) sequence identity to SEQ ID NO: 27, and the light chain variable region comprises an amino acid sequence having at least 80% (e.g., at least 85%, 90%, 91%, 92%, 93%, 94%, 95%, 96%, 97%, 98%, or 99%) sequence identity to SEQ ID NO: 28. In some embodiments, provided is the anti-CD3 antibody according to any one of the foregoing, wherein the heavy chain variable region comprises the amino acid sequence of SEQ ID NO: 27, and the light chain variable region comprises the amino acid sequence of SEQ ID NO: 28.

In some embodiments, provided is the anti-CD3 antibody according to any one of the foregoing, wherein the anti-CD3 antibody is an antibody fragment. In some embodiments, provided is the anti-CD3 antibody according to any one of the foregoing, wherein the antibody fragment is a Fab, Fab', F(ab')2, Fd, Fv, scFv, dsFv, or dAb.

In some embodiments, provided is the anti-CD3 antibody according to any one of the foregoing, wherein the anti-CD3 antibody comprises a heavy chain constant region and a light chain constant region.

In some embodiments, provided is the anti-CD3 antibody according to any one of the foregoing, wherein the heavy chain constant region is a human IgG1 heavy chain constant region or a variant thereof, and the light chain constant region is a human lambda light chain constant region.

In some embodiments, provided is the anti-CD3 antibody according to any one of the foregoing, wherein the heavy chain constant region comprises the amino acid sequence of SEQ ID NO: 29, and the light chain constant region comprises the amino acid sequence of SEQ ID NO: 30.

In some embodiments, provided is the anti-CD3 antibody according to any one of the foregoing, wherein the anti-CD3 antibody comprises a heavy chain and a light chain, wherein the heavy chain comprises an amino acid sequence having at least 80% (e.g., at least 85%, 90%, 91%, 92%, 93%, 94%, 95%, 96%, 97%, 98%, or 99%) sequence identity to SEQ ID NO: 31, and the light chain comprises an amino acid sequence having at least 80% (e.g., at least 85%, 90%, 91%, 92%, 93%, 94%, 95%, 96%, 97%, 98%, or 99%) sequence identity to SEQ ID NO: 32.

In some embodiments, provided is the anti-CD3 antibody according to any one of the foregoing, wherein the heavy chain comprises the amino acid sequence of SEQ ID NO: 31, and the light chain comprises the amino acid sequence of SEQ ID NO: 32.

In another aspect, the present disclosure provides a multispecific antibody that specifically binds to human CD3 and further binds to a tumor-associated antigen (TAA) or a cell surface antigen, receptor, or receptor ligand, wherein the multispecific antibody comprises the anti-CD3 antibody according to any one of the foregoing.

In some embodiments, provided is the multispecific antibody according to any one of the foregoing, wherein the tumor antigen is an antigen on a tumor cell selected from the group consisting of cells of breast cancer, prostate cancer, gastric cancer, lung cancer, colorectal cancer (including colon cancer and rectal cancer), pancreatic cancer, liver cancer, ovarian cancer, oral cancer, nasopharyngeal carcinoma, esophageal cancer, laryngeal cancer, bone cancer, skin cancer, melanoma, uterine cancer, testicular cancer, bladder cancer, renal cancer, brain cancer, glioblastoma, thyroid cancer, lymphoma, myeloma, and leukemia. In some embodiments, the multispecific antibody according to any one of the foregoing is a bispecific antibody.

In another aspect, the present disclosure provides a pharmaceutical composition comprising a therapeutically effective amount of an anti-CD3 antibody, or the multispecific antibody according to any one of the foregoing, and one or more pharmaceutically acceptable carriers, diluents, or excipients. In some embodiments, the pharmaceutical composition according to any one of the foregoing is used for activating T cells. In some embodiments, the pharmaceutical composition according to any one of the foregoing is used for treating a tumor, an autoimmune disease, or an inflammatory disease. In some embodiments, the pharmaceutical composition according to any one of the foregoing is used for treating cancer.

In another aspect, the present disclosure provides an isolated nucleic acid encoding the anti-CD3 antibody according to any one of the foregoing.

In another aspect, the present disclosure provides a vector comprising the aforementioned nucleic acid molecule.

In another aspect, the present disclosure provides a host cell comprising the isolated nucleic acid according to any one of the foregoing.

In another aspect, the present disclosure provides a method for producing the anti-CD3 antibody according to any one of the foregoing or the multispecific antibody according to any one of the foregoing, comprising a step of expressing the isolated nucleic acid according to any one of the foregoing or culturing the host cell according to any one of the foregoing to produce the anti-CD3 antibody or the multispecific antibody.

In another aspect, the present disclosure provides the anti-CD3 antibody according to any one of the foregoing, or the multispecific antibody according to any one of the foregoing, or the pharmaceutical composition according to any one of the foregoing, or the isolated nucleic acid molecule according to any one of the foregoing, for use as a medicament. In some embodiments, the medicament is used for activating T cells. In some embodiments, the medicament is used for treating, preventing, or ameliorating a disease or disorder.

In another aspect, the present disclosure provides use of the anti-CD3 antibody according to any one of the foregoing, or the multispecific antibody according to any one of the foregoing, or the pharmaceutical composition according to any one of the foregoing, or the isolated nucleic acid molecule according to any one of the foregoing, in the manufacture of a medicament for activating T cells.

In another aspect, the present disclosure provides use of the anti-CD3 antibody according to any one of the foregoing, or the multispecific antibody according to any one of the foregoing, or the pharmaceutical composition according to any one of the foregoing, or the isolated nucleic acid molecule according to any one of the foregoing, in the manufacture of a medicament for treating, preventing, or ameliorating a disease or disorder.

In another aspect, the present disclosure provides a method for activating T cells, comprising administering to a subject a therapeutically effective amount of the anti-CD3 antibody according to any one of the foregoing, or the multispecific antibody according to any one of the foregoing, or the pharmaceutical composition according to any one of the foregoing, or the isolated nucleic acid molecule according to any one of the foregoing. In some embodiments, the method comprises administering to a subject a unit dose of a composition comprising 0.1-3000 mg (more preferably 1-1000 mg) of the multispecific antibody according to any one of the foregoing, or the pharmaceutical composition according to any one of the foregoing, or the isolated nucleic acid molecule according to any one of the foregoing.

In another aspect, the present disclosure provides a method for treating, preventing, or ameliorating a disease or disorder. In some embodiments, the disease or disorder is a disease or disorder associated with CD3. In some embodiments, the disease or disorder is selected from the group consisting of a tumor, an autoimmune disease, and an inflammatory disease. In some embodiments, the disease or disorder is cancer. In some embodiments, the method comprises administering to a subject a therapeutically effective amount of the anti-CD3 antibody according to any one of the foregoing, or the multispecific antibody according to any one of the foregoing, or the pharmaceutical composition according to any one of the foregoing, or the isolated nucleic acid molecule according to any one of the foregoing. In some embodiments, the method comprises administering to a subject a unit dose of a composition comprising 0.1-3000 mg (more preferably 1-1000 mg) of the multispecific antibody according to any one of the foregoing, or the pharmaceutical composition according to any one of the foregoing, or the isolated nucleic acid molecule according to any one of the foregoing.

### BRIEF DESCRIPTION OF THE DRAWINGS

FIG. 1A shows the binding levels of the recombinant reaction products Ipi-F405L*CP11-K409R, Ipi-K409R*CP11-F405L, Ipi-D356K*CP11-K439E, and Ipi-K439E*CP11-D356K to the two targets CCR8 and CTLA4, as determined by flow cytometry.
FIG. 1B shows the binding levels of the recombinant reaction products Ipi-D356K+T366A*CP11-K439E+T366A, Ipi-D356K+Y407V*CP11-K439E+Y407V, and Ipi-D356K+Y407L*CP11-K439E+Y407L to the two targets CCR8 and CTLA4, as determined by flow cytometry.
FIG. 1C shows the binding levels of the recombinant reaction products Ipi-D356K+T366L*CP11-K439E+T366L, Ipi-D356K+L368V*CP11-K439E+L368V, Ipi-D356K+K409R*CP11-K439E+K409R, and Ipi-D356K+K409Q*CP11-K439E+K409Q to the two targets CCR8 and CTLA4, as determined by flow cytometry.
FIG. 1D shows the binding levels of the recombinant reaction products Ipi-D356K+Y349F*CP11-K439E+Y349F, Ipi-D356K+Y349L*CP11-K439E+Y349L, Ipi-D356K+L351V*CP11-K439E+L351V, Ipi-D356K+L351T*CP11-K439E+L351T, Ipi-D356K+L351I*CP11-K439E+L351I, Ipi-D356K+S364A*CP11-K439E+S364A, Ipi-D356K+T366V*CP11-K439E+T366V, and Ipi-D356K+T411N*CP11-K439E+T411N to the two targets CCR8 and CTLA4, as determined by flow cytometry.
FIG. 1E shows the binding levels of the recombinant reaction products Ipi-D356K+T394S*CP11-K439E+T394S, Ipi-D356K+V397M*CP11-K439E+V397M, Ipi-D356K+V397T*CP11-K439E+V397T, Ipi-D356K+F405L*CP11-K439E+F405L, and Ipi-D356K+F405Y*CP11-K439E+F405Y to the two targets CCR8 and CTLA4, as determined by flow cytometry.
FIG. 1F shows the binding levels of the recombinant reaction products Ipi-D356K+L351F*CP11-K439E+L351F, Ipi-D356K+S364T*CP11-K439E+S364T, Ipi-D356K+S364V*CP11-K439E+S364V, Ipi-D356K+S364L*CP11-K439E+S364L, Ipi-D356K+T394A*CP11-K439E+T394A, and Ipi-D356K+T411Y*CP11-K439E+T411Y to the two targets CCR8 and CTLA4, as determined by flow cytometry.
FIG. 1G shows the binding levels of the recombinant reaction products Ipi-D356K+L351C*CP11-K439E+L351C, Ipi-D356K+T366G*CP11-K439E+T366G, Ipi-D356K+T366S*CP11-K439E+T366S, Ipi-D356K+T394N*CP11-K439E+T394N, Ipi-D356K+T394C*CP11-K439E+T394C, Ipi-D356K+V397I*CP11-K439E+V397I, Ipi-D356K+Y407C*CP11-K439E+Y407C, Ipi-D356K+Y349C*CP11-K439E+E357C, and Ipi-D356K+Y349C*CP11-K439E+S354C to the two targets CCR8 and CTLA4, as determined by flow cytometry.
FIG. 1H shows the binding levels of the recombinant reaction products Ipi-F405T*CP11-T394F, Ipi-D356K+Y349C*CP11-K439E+S354C+T366A, Ipi-Y349C*CP11-S354C+T366A, Ipi-Y349C*CP11-S354C+F405Y, and Ipi-Y349C*CP11-E357C to the two targets CCR8 and CTLA4, as determined by flow cytometry.
FIG. 1I shows the binding levels of the recombinant reaction products Ipi-D356K+T366H*CP11-K439E+T366H, Ipi-D356K+L368M*CP11-K439E+L368M, Ipi-D356K+L368I*CP11-K439E+L368I, Ipi-D356K+T394V*CP11-K439E+T394V, Ipi-D356K+V397L*CP11-K439E+V397L, Ipi-D356K+Y407H*CP11-K439E+Y407H, Ipi-D356K+Y349S*CP11-K439E+S354Y, Ipi-D356K+F405T*CP11-K439E+T394F, Ipi-D356K+Y349C*CP11-K439E+S354C+L368I, Ipi-D356K+Y349C*CP11-K439E+S354C+L351I, and Ipi-Y349C*CP11-S354C+L368I to the two targets CCR8 and CTLA4, as determined by flow cytometry.
FIG. 2A shows superimposed HPLC-IEC chromatograms of the parental monoclonal antibodies of Ipi-D356K*CP11-K439E and the corresponding bispecific antibody.
FIG. 2B shows superimposed HPLC-IEC chromatograms of the parental monoclonal antibodies of Ipi-F405L*CP11-K409R and the corresponding bispecific antibody.
FIG. 2C shows superimposed HPLC-IEC chromatograms of the parental monoclonal antibodies of Ipi-D356K+L351T*CP11-K439E+L351T and the corresponding bispecific antibody.
FIG. 2D shows superimposed HPLC-IEC chromatograms of the parental monoclonal antibodies of Ipi-D356K+L351I*CP11-K439E+L351I and the corresponding bispecific antibody.
FIG. 2E shows superimposed HPLC-IEC chromatograms of the parental monoclonal antibodies of Ipi-D356K+S364A*CP11-K439E+S364A and the corresponding bispecific antibody.
FIG. 2F shows superimposed HPLC-IEC chromatograms of the parental monoclonal antibodies of Ipi-D356K+T366A*CP11-K439E+T366A and the corresponding bispecific antibody.
FIG. 2G shows superimposed HPLC-IEC chromatograms of the parental monoclonal antibodies of Ipi-D356K+L368I*CP11-K439E+L368I and the corresponding bispecific antibody.
FIG. 2H shows superimposed HPLC-IEC chromatograms of the parental monoclonal antibodies of Ipi-D356K+T394A*CP11-K439E+T394A and the corresponding bispecific antibody.
FIG. 2I shows superimposed HPLC-IEC chromatograms of the parental monoclonal antibodies of Ipi-D356K+T394S*CP11-K439E+T394S and the corresponding bispecific antibody.
FIG. 2J shows superimposed HPLC-IEC chromatograms of the parental monoclonal antibodies of Ipi-D356K+F405Y*CP11-K439E+F405Y and the corresponding bispecific antibody.
FIG. 2K shows superimposed HPLC-IEC chromatograms of the parental monoclonal antibodies of Ipi-D356K+K409Q*CP11-K439E+K409Q and the corresponding bispecific antibody.
FIG. 2L shows superimposed HPLC-IEC chromatograms of the parental monoclonal antibodies of Ipi-D356K+T411Y*CP11-K439E+T411Y and the corresponding bispecific antibody.
FIG. 2M shows superimposed HPLC-IEC chromatograms of the parental monoclonal antibodies of Ipi-D356K+Y349S*CP11-K439E+S354Y and the corresponding bispecific antibody.
FIG. 2N shows superimposed HPLC-IEC chromatograms of the parental monoclonal antibodies of Ipi-F405T*CP11-T394F and the corresponding bispecific antibody.
FIG. 2O shows superimposed HPLC-IEC chromatograms of the parental monoclonal antibodies of Ipi-D356K+Y349C*CP11-K439E+S354C+L351I and the corresponding bispecific antibody.
FIG. 2P shows superimposed Biomix chromatograms of the parental monoclonal antibodies of Ipi-D356K+Y349C*CP11-K439E+S354C+L351I and the corresponding bispecific antibody.
FIG. 2Q shows superimposed HPLC-IEC chromatograms of the parental monoclonal antibodies of Ipi-Y349S*CP11-S364Y and the corresponding bispecific antibody.
FIG. 2R shows superimposed HPLC-IEC chromatograms of the parental monoclonal antibodies of Ipi-Y349C*CP11-S354C+S364Y and the corresponding bispecific antibody.
FIG. 2S shows superimposed HPLC-IEC chromatograms of the parental monoclonal antibodies of Ipi-Y349C*CP11-S354C+S364F and the corresponding bispecific antibody.
FIG. 3A shows the ability of the mixture (Tra+Per) Mix and the recombinant reaction products Tra-F405L*Per-K409R, Tra-K409R*Per-F405L, Tra-E356K+V397L*PerK439E+V397L, Tra-E356K+V397M*Per-K439E+V397M, Tra-E356K+V397T*PerK439E+V397T, Tra-E356K+V397I*Per-K439E+V397I, Tra-E356K+F405Y*PerK439E+F405Y, Tra-E356K+F405L*Per-K439E+F405L, Tra-E356K+T411L*PerK439E+T411L, Tra-E356K+T411Y*Per-K439E+T411Y, and Tra-E356K+L351C*PerK439E+L351C to bind to two epitopes of HER2, as measured by Bridging ELISA.
FIG. 3B shows the ability of the mixture (Tra+Per) Mix and the recombinant re action products Tra-F405L*Per-K409R, Tra-K409R*Per-F405L, Tra-E356K+T394C *Per-K439E+T394C, Tra-E356K+Y349C*Per-K439E+E357C, Tra-E356K+T366G*P er-K439E+T366G, Tra-E356K+T366S*Per-K439E+T366S, Tra-E356K+Y349L*PerK439E+Y349L, Tra-E356K+L351I*Per-K439E+L351I, Tra-E356K+L351T*Per-K439 E+L351T, and Tra-E356K+L351F*Per-K439E+L351F to bind to two epitopes of HER2, as measured by Bridging ELISA.
FIG. 3C shows the ability of the mixture (Tra+Per) Mix and the recombinant re action products Tra-F405L*Per-K409R, Tra-K409R*Per-F405L, Tra-E356K+T394A *Per-K439E+T394A, Tra-E356K+T394N*Per-K439E+T394N, Tra-E356K+T366V*P er-K439E+T366V, Tra-E356K+T366A*Per-K439E+T366A, Tra-E356K+L368A*PerK439E+L368A, Tra-E356K+Y407L*Per-K439E+Y407L, Tra-E356K+Y407V*Per-K4 39E+Y407V, and Tra-Y407L*Per-T366H to bind to two epitopes of HER2, as m easured by Bridging ELISA.
FIG. 3D shows the ability of the mixture (Tra+Per) Mix and the recombinant re action products Tra-F405L*Per-K409R, Tra-K409R*Per-F405L, Tra-K439E*Per-E3 56K, Tra-E356K*Per-K439E, Tra-E356K+Y407C*Per-K439E+Y407C, Tra-E356K+ S364T*Per-K439E+S364T, Tra-E356K+T394V*Per-K439E+T394V, Tra-E356K+K40 9R*Per-K439E+K409R, and Tra-E356K+K409Q*Per-K439E+K409Q to bind to tw o epitopes of HER2, as measured by Bridging ELISA.
FIG. 3E shows the ability of the mixture (Tra+Per) Mix and the recombinant rea ction products Tra-F405L*Per-K409R, Tra-K409R*Per-F405L, Tra-E356K+T366L* Per-K439E+T366L, Tra-E356K+T366I*Per-K439E+T366I, Tra-E356K+L368V*PerK439E+L368V, Tra-E356K+Y407F*Per-K439E+Y407F, Tra-E356K+Y407H*Per-K4 39E+Y407H, Tra-E356K+T366H*Per-K439E+Y407H, Tra-E356K+T366H*Per-K439 E+Y407L, and Tra-F405L*Per-T366H to bind to two epitopes of HER2, as meas ured by Bridging ELISA.
FIG. 3F shows the ability of the mixture (Tra+Per) Mix and the recombinant rea ction products Tra-F405L*Per-K409R, Tra-K409R*Per-F405L, Tra-E356K+L351V* Per-K439E+L351V, Tra-E356K+S364A*Per-K439E+S364A, Tra-E356K+S364L*PerK439E+S364L, Tra-E356K+T394S*Per-K439E+T394S, Tra-E356K+L368I*Per-K439 E+L368I, Tra-E356K+L368M*Per-K439E+L368M, Tra-E356K+T366H*Per-K439E+ T366H, Tra-E356K+Y407H*Per-K439E+T366H, and Tra-E356K+Y349S*Per-K439 E+S354Y to bind to two epitopes of HER2, as measured by Bridging ELISA.
FIG. 3G shows the ability of the mixture (Tra+Per) Mix and the recombinant re action products Tra-F405L*Per-K409R, Tra-K409R*Per-F405L, Tra-Y349S*Per-S35 4Y, Tra-F405T*Per-T394F, Tra-E356K+Y349C*Per-K439E+S354C+L368I, Tra-E35 6K+Y349C*Per-K439E+S354C+L351I, Tra-E356K+Y349C*Per-K439E+S354C+T36 6A, Tra-Y349C*Per-S354C+T366A, Tra-Y349C*Per-E357C, Tra-Y349C*Per-E357C +Q347E, and Tra-E356K+Y407L*Per-K439E+T366H to bind to two epitopes of HER2, as measured by Bridging ELISA.
FIG. 3H shows the ability of the mixture (Tra+Per) Mix and the recombinant re action products Tra-F405L*Per-K409R, Tra-K409R*Per-F405L, Tra-T366H*Per-Y4 07L, Tra-E356K+T366H*Per-K439E+F405L, Tra-T366H*Per-F405L, Tra-E356K+D 399K*Per-K439E+K409D, Tra-E356K+K409D*Per-K439E+D399K, Tra-Y349V*Per -S354F, Tra-Y349T*Per-S354F, and Tra-Y349S*Per-S354F to bind to two epitopes of HER2, as measured by Bridging ELISA.
FIG. 3I shows the ability of the mixture (Tra+Per) Mix and the recombinant rea ction products Tra-F405L*Per-K409R, Tra-K409R*Per-F405L, Tra-E356K+F405T* Per-K439E+T394F, Tra-Y349C*Per-S354C+L351I, Tra-Y349C*Per-S354C+F405Y, a nd Tra-E356K+L351M*Per-K439E+L351M to bind to two epitopes of HER2, as measured by Bridging ELISA.
FIG. 3J shows the ability of the mixture (Tra+Per) Mix and the recombinant rea ction products Tra-F405L*Per-K409R, Tra-K409R*Per-F405L, Tra-Y349C*Per-S35 4C+L368I, Tra-Y349A*Per-S354F, Tra-Y349S*Per-S354W, Tra-Y349A*Per-S354W, and Tra-Y349G*Per-S354W to bind to two epitopes of HER2, as measured by Bridging ELISA.
FIG. 3K shows the ability of the mixture (Tra+Per) Mix and the recombinant re action products Tra-F405L*Per-K409R, Tra-K409R*Per-F405L, Tra-Y349S*Per-S36 4Y, Tra-Y349C*Per-S354C+S364Y, Tra-Y349C*Per-S354C+S364F, and Tra-F405T+ S354C*Per-T394F+Y349C to bind to two epitopes of HER2, as measured by Bri dging ELISA.
FIG. 4A shows superimposed HPLC-IEC chromatograms of the parental monoclo nal antibodies of Tra-E356K*Per-K439E and the corresponding bispecific antibod y.
FIG. 4B shows superimposed HPLC-RP chromatograms of the parental monoclon al antibodies of Tra-F405L*Per-K409R and the corresponding bispecific antibody.
FIG. 4C shows superimposed HPLC-RP chromatograms of the parental monoclon al antibodies of Tra-K409R*Per-F405L and the corresponding bispecific antibody.
FIG. 4D shows superimposed HPLC-IEC chromatograms of the parental monoclo nal antibodies of Tra-E356K+L351I*Per-K439E+L351I and the corresponding bisp ecific antibody.
FIG. 4E shows superimposed HPLC-IEC chromatograms of the parental monoclo nal antibodies of Tra-E356K+S364A*Per-K439E+S364A and the corresponding bis pecific antibody.
FIG. 4F shows superimposed HPLC-IEC chromatograms of the parental monoclo nal antibodies of Tra-E356K+T366A*Per-K439E+T366A and the corresponding bi specific antibody.
FIG. 4G shows superimposed HPLC-IEC chromatograms of the parental monoclo nal antibodies of Tra-E356K+T394A*Per-K439E+T394A and the corresponding bi specific antibody.
FIG. 4H shows superimposed HPLC-IEC chromatograms of the parental monoclo nal antibodies of Tra-E356K+T394S*Per-K439E+T394S and the corresponding bis pecific antibody.
FIG. 4I shows superimposed HPLC-IEC chromatograms of the parental monoclon al antibodies of Tra-E356K+F405Y*Per-K439E+F405Y and the corresponding bisp ecific antibody.
FIG. 4J shows superimposed HPLC-IEC chromatograms of the parental monoclon al antibodies of Tra-E356K+T411Y*Per-K439E+T411Y and the corresponding bisp ecific antibody.
FIG. 4K shows superimposed HPLC-IEC chromatograms of the parental monoclo nal antibodies of Tra-E356K+Y349S *Per-K439E+S354Y and the corresponding b ispecific antibody.
FIG. 4L shows superimposed HPLC-IEC chromatograms of the parental monoclo nal antibodies of Tra-E356K+Y349C*Per-K439E+S354C+L351I and the correspon ding bispecific antibody.
FIG. 4M shows superimposed HPLC-RP chromatograms of the parental monoclon al antibodies of Tra-Y349G*Per-S354W and the corresponding bispecific antibody.
FIG. 4N shows superimposed HPLC-RP chromatograms of the parental monoclon al antibodies of Tra-Y349S*Per-S364Y and the corresponding bispecific antibody.
FIG. 4O shows superimposed HPLC-RP chromatograms of the parental monoclon al antibodies of Tra-Y349C*Per-S354C+S364Y and the corresponding bispecific a ntibody.
FIG. 4P shows superimposed HPLC-RP chromatograms of the parental monoclon al antibodies of Tra-Y349C*Per-S354C+S364F and the corresponding bispecific a ntibody.
FIG. 5A shows superimposed HPLC-IEC chromatograms of the parental mutants of C-E356K+L351I*CM-K439E+L351I and the corresponding recombinant reactio n product.
FIG. 5B shows superimposed HPLC-IEC chromatograms of the parental mutants of C-E356K+S364A*CM-K439E+S364A and the corresponding recombinant reacti on product.
FIG. 5C shows superimposed HPLC-IEC chromatograms of the parental mutants of C-E356K+T366A*CM-K439E+T366A and the corresponding recombinant reacti on product.
FIG. 5D shows superimposed HPLC-IEC chromatograms of the parental mutants of C-E356K+T394A*CM-K439E+T394A and the corresponding recombinant reacti on product.
FIG. 5E shows superimposed HPLC-IEC chromatograms of the parental mutants of C-E356K+T394S*CM-K439E+T394S and the corresponding recombinant reacti on product.
FIG. 5F shows superimposed HPLC-IEC chromatograms of the parental mutants of C-E356K+F405Y*CM-K439E+F405Y and the corresponding recombinant reacti on product.
FIG. 5G shows superimposed HPLC-IEC chromatograms of the parental mutants of C-E356K+T411Y*CM-K439E+T411Y and the corresponding recombinant reacti on product.
FIG. 5H shows superimposed HPLC-IEC chromatograms of the parental mutants of C-E356K+Y349S*CM-K439E+S354Y and the corresponding recombinant reacti on product.
FIG. 5I shows superimposed HPLC-IEC chromatograms of the parental mutants o f C-E356K+Y349C*CM-S354C+L351I and the corresponding recombinant reaction product.
FIG. 5J shows superimposed HPLC-IEC chromatograms of the parental mutants of C-F405T*CM-T394F and the corresponding recombinant reaction product.
FIG. 5K shows superimposed HPLC-IEC chromatograms of the parental mutants of C-Y349G*CM-S354W and the corresponding recombinant reaction product.
FIG. 5L shows superimposed HPLC-IEC chromatograms of the parental mutants of C-Y349S*CM-S364Y and the corresponding recombinant reaction product.
FIG. 5M shows superimposed HPLC-IEC chromatograms of the parental mutants of C-Y349C*CM-S354C+S364Y and the corresponding recombinant reaction product.
FIG. 5N shows superimposed HPLC-IEC chromatograms of the parental mutants of C-Y349C*CM-S354C+S364F and the corresponding recombinant reaction product.

### DETAILED DESCRIPTION

### Terminology

To facilitate the understanding of the present disclosure, certain technical and scientific terms are described below. Unless otherwise specifically defined herein, all technical and scientific terms used herein have the same meanings as commonly understood by those of ordinary skill in the art.

The singular forms "a", "an", and "the" used in the description and claims include plural references, unless the context clearly dictates otherwise.

Unless otherwise clearly stated in the context, throughout the description and the claims, the words "comprise", "have", "include", etc., should be construed in an inclusive sense as opposed to an exclusive or exhaustive sense.

The term "and/or" is meant to include the two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used in the present disclosure are as described in J. Biol. Chem., 243, p3558 (1968).

The term "amino acid" refers to naturally occurring and synthetic amino acids, as well as amino acid analogs and amino acid mimics that function similarly to naturally occurring amino acids. Naturally occurring amino acids are those that are encoded by the genetic code and those that are later modified, e.g., hydroxyproline, γ-carboxyglutamic acid, and O-phosphoserine. Amino acid analogs refer to compounds that have the same basic chemical structure (i.e., α carbons that bind to hydrogen, carboxyl, amino, and R groups) as naturally occurring amino acids, e.g., homoserine, norleucine, methionine sulfoxide, and methioninemethyl sulfonium. Such an analog has a modified R group (e.g., norleucine) or a modified peptide backbone, but retains a basic chemical structure that is the same as that of naturally occurring amino acids. Amino acid mimics refer to chemical compounds that have a structure different from the general chemical structure of amino acids, but function similarly to naturally occurring amino acids.

The term "amino acid mutation" includes amino acid substitutions (also known as amino acid replacements), deletions, insertions, and modifications. The final construct can be achieved through any combination of substitutions, deletions, insertions, and modifications, so long as the final construct possesses the desired properties, such as reduced binding to Fc receptors. Amino acid sequence deletions and insertions include deletions and insertions at the amino-terminus and/or the carboxyl-terminus of a polypeptide chain. Specific amino acid mutations may be amino acid substitutions. In one embodiment, an amino acid mutation is a non-conservative amino acid substitution, i.e., a replacement of one amino acid with another amino acid having different structural and/or chemical properties. Amino acid substitutions include replacements with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, etc. It is contemplated that methods for altering amino acid side chain groups other than genetic engineering, such as chemical modifications, may also be used. Various names may be used herein to refer to the same amino acid mutation. Herein, the amino acid residue at a particular position may be indicated by the position + the amino acid residue; for example, 356K indicates that the amino acid residue at position 356 is K. D356K indicates that the amino acid residue at position 356, D, is mutated into K. D/E356K indicates that the amino acid residue at position 356, D, is mutated into K, or that the amino acid residue at position 356, E, is mutated into K. It will be appreciated that when an amino acid sequence is defined in the claims using a position + a residue, the amino acid at that position before mutation does not limit the technical solutions. Herein, "the Fc region comprises amino acid mutations of 356K and 349S" means that the amino acid mutations in the Fc region include a mutation of position 356 into lysine (K) and a mutation of position 349 into serine (S).

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "heteromultimer" refers to a multimer of proteins composed of multiple polypeptides that can associate with one another. In more detail, a "heteromultimer" comprises at least a first polypeptide and a second polypeptide, wherein the second polypeptide is a molecule whose amino acid sequence differs from the amino acid sequence of the first polypeptide by at least 1 amino acid residue. Moreover, although not particularly limited, the heteromultimer may have antigen-binding activity for at least two different ligands, antigens (or epitopes), receptors, substrates, or the like. In addition to the "heterodimer" formed from the first polypeptide and the second polypeptide, the heteromultimer may further comprise other kinds of polypeptides. That is, the "heteromultimer" of the present disclosure is not limited to a heterodimer, and also includes, for example, a heterotrimer, a heterotetramer, etc.

The term "homomer" refers to a state in which polypeptide partners having the same amino acid sequence associate.

The term "the molecule comprising the first polypeptide homomer" or "the molecule comprising the second polypeptide homomer" is selected from the group consisting of (including but not limited to) a CH3 domain, an Fc region, an antibody, a fusion protein comprising a CH3 domain, an Fc region conjugated with a drug (peptide or toxin), etc. In some embodiments, the molecule comprising the first polypeptide homomer and the molecule comprising the second polypeptide homomer comprise, in addition to the Fc region, one or more or all other regions of an antibody, i.e., the CH1 region, the VH region, the CL region, and/or the VL region. When described in the present disclosure, "first polypeptide" may also refer to each first polypeptide in the first polypeptide homomer. When described in the present disclosure, "second polypeptide" may also refer to each second polypeptide in the second polypeptide homomer.

In the present disclosure, the terms "multispecific antibody" and "polyspecific antibody" have the same meaning and refer to an antibody that is capable of specifically binding to multiple different epitopes. That is, multispecific antibodies are antibodies that have specificity for at least two different epitopes and include, in addition to antibodies that recognize different antigens, antibodies that recognize different epitopes on the same antigen. (For example, when the antigens are heterologous receptors, multispecific antibodies recognize different domains constituting the heterologous receptors; or when the antigens are monomers, multispecific antibodies recognize multiple sites on the monomer antigens). Generally, such molecules bind to two antigens (bispecific antibodies, having the same meaning as "dual-specific antibodies" in the present specification), but may have specificity for two or more (e.g., three) antigens.

The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties), so long as they exhibit the desired antigen-binding activity. In the antibodies of the present disclosure, as human IgG1, human IgG2, human IgG3, and human IgG4 constant regions, several allotype sequences resulting from genetic polymorphism are described in Sequences of Proteins of Immunological Interest, NIH Publication No. 91-3242, and any one of the sequences may be used in the present disclosure. As a sequence of human IgG1, the amino acid sequence at positions 356-358 (EU numbering) may be DEL or EEM, and particularly, the amino acid at position 356 (EU numbering) may be D or E (Jefferis R, Lefranc M P. Human immunoglobulin allotypes: possible implications for immunogenicity//MAbs. Taylor & Francis, 2009, 1(4): 332-338).

"Natural antibody" refers to a naturally occurring immunoglobulin molecule. For example, a natural IgG antibody is a heterotetrameric glycoprotein of about 150,000 Daltons composed of two light chains and two heavy chains linked by disulfide bonds. From the N-terminus to the C-terminus, each heavy chain comprises one variable region (VH), also known as variable heavy domain or heavy chain variable region, followed by heavy chain constant regions. Generally, a natural IgG heavy chain constant region comprises three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL).

The terms "full-length antibody", "intact antibody", and "whole antibody" are used herein interchangeably and refer to an antibody having a structure substantially similar to a natural antibody structure or comprising a heavy chain with an Fc region as defined herein. In a natural intact antibody, the light chain comprises a light chain variable region VL and a light chain constant region CL. The VL is positioned at the amino-terminus of the light chain, and the light chain constant region comprises a κ chain and a λ chain. The heavy chain comprises a variable region VH and constant regions (CH1, CH2, and CH3). The VH is positioned at the amino-terminus of the heavy chain, and the constant regions are positioned at the carboxyl-terminus, wherein the CH3 is the closest to the carboxyl-terminus of the polypeptide. The heavy chain can be of any isotype, including IgG (including subtypes IgG1, IgG2, IgG3, and IgG4), IgA (including subtypes IgA1 and IgA2), IgM, and IgE.

The antibodies used in the methods of the present disclosure may be of any allotype. Allotypes are not expected to influence Fab-arm exchange. Antibody allotypes are associated with variations in amino acid sequences at particular positions in the constant region sequences of antibodies.

Common wild-type human IgG1 heavy chain constant region sequences are shown below:
> hIgG1-1 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKRVEPKSCDKTHTCP
PCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEK
 
TISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLS
LSPGK
SEQ ID NO: 39
> hIgG1-2 







SEQ ID NO: 40
> hIgG1-3

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
SEQ ID NO: 41

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules, can be classified into bivalent, trivalent, tetravalent, or higher-valency bispecific antibodies according to the number of antigen-binding regions, and can be classified into symmetric bispecific antibodies and asymmetric bispecific antibodies based on whether their structures are left-right symmetrical. Bispecific antibodies based on antibody fragments, such as Fab fragments lacking Fc fragments, are formed by combining 2 or more Fab fragments in one molecule; they have low immunogenicity, a small molecular weight, and relatively high tumor tissue permeability, and typical antibody structures of this type include such bispecific antibodies as F(ab)2, scFv-Fab, and (scFv)2-Fab. IgG-like bispecific antibodies (e.g., having Fc fragments) have a relatively large molecular weight; the Fc fragments facilitate later purification of the antibodies and increase their solubility and stability, and the Fc fragments may further bind to the receptor FcRn, increasing the serum half-life of the antibodies. Typical structural models of bispecific antibodies include such bispecific antibodies as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "parental antibody" refers to an antibody from which one or more antibody fragments are derived. A parental antibody may comprise a natural or wild-type sequence. A parental antibody may comprise an amino acid sequence in which one or more amino acid residues are replaced with one or more cysteine residues. A parental antibody may have pre-existing amino acid sequence modifications (e.g., additions, deletions, and/or substitutions), compared to other natural, wild-type, or modified forms of the antibody. A parental antibody may be against the target antigen of interest, e.g., a biologically important polypeptide. A parental antibody may be against a non-polypeptide antigen (e.g., the human CCR8 antigen; e.g., Patent No. WO2023208182A1). Exemplary parental antibodies include, but are not limited to, antibodies with affinity and selectivity for cell surface receptors, transmembrane receptors, and tumor-associated antigens (TAAs).

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl-terminus. Suitable Fc regions used for the antibodies described herein include Fc regions of human IgG1, IgG2 (IgG2A and IgG2B), IgG3, and IgG4. In some embodiments, the boundaries of an Fc region may also vary; for example, the C-terminal lysine of the Fc region (residue 447 according to the EU numbering scheme) is deleted, or the C-terminal glycine and lysine of the Fc region (residues 446 and 447 according to the EU numbering scheme) are deleted. Unless otherwise stated, the numbering scheme for the Fc region is the EU numbering scheme, also known as the EU index (KabatEA et al.,1991, Sequences of Proteins of Immunological Interest. NIH). Although a numbering scheme (e.g., EU) is employed to define amino acid residues in specific embodiments, technical solutions corresponding to other numbering schemes are to be considered equivalent technical solutions.

The Fc region can be appropriately obtained by the partial digestion of an IgG monoclonal antibody or the like with a proteolytic enzyme such as pepsin followed by the elution of a fraction adsorbed on a protein A or protein G column. The proteolytic enzyme is not particularly limited as long as the enzyme is capable of restrictively digesting a full-length antibody to produce Fab or F(ab')2 under appropriately set reaction conditions (e.g., pH) of the enzyme; examples can include pepsin and papain.

In the present disclosure, the term "Fc region" or "Fc domain" refers to an antibody region comprising at least a CH2 domain and a CH3 domain (see, e.g., Kabat EA, US Department of Health and Human Services, NIH publication n91-3242, Edn. 5th Edition, 662,680,689 (1991)). In the present disclosure, the term "CH2 region" or "CH2 domain" is intended to refer to the CH2 region of an immunoglobulin. Thus, for example, the CH2 region of the human IgG1 antibody corresponds to amino acids 228-340 according to the EU numbering system. However, the CH2 region may also be any other antibody isotype as described in the present disclosure.

In the present disclosure, the term "CH3 region" or "CH3 domain" is intended to refer to the CH3 region of an immunoglobulin. Thus, for example, the CH3 region of the human IgG1 antibody corresponds to amino acids 341-447 according to the EU numbering system. However, the CH3 region may also be any other antibody isotype as described in the present disclosure.

In the present disclosure, the association of polypeptides refers to, for example, a state in which multiple polypeptide regions interact.

In the present disclosure, "modulating association" refers to modulating to obtain a desired association state, and more specifically to preventing the formation of undesired association within polypeptides.

In the present disclosure, "interface" generally refers to an association surface during association (interaction). The amino acid residues that form an interface generally refer to one or more amino acid residues contained in polypeptide regions undergoing the association, more preferably amino acid residues that come close and are involved in the interaction when associated. The interaction specifically includes an instance in which a hydrogen bond, an electrostatic interaction, or a salt bridge is formed between amino acid residues that come close during association, etc.

In the present disclosure, "amino acid residues that form an interface" refers specifically to amino acid residues contained in polypeptide regions that constitute interfaces. Examples of polypeptide regions that constitute interfaces include polypeptide regions that are responsible for selective bonding within or between molecules of antibodies, ligands, receptors, substrates, etc. In the present disclosure, the term may refer to amino acid residues at an interface where two CH3 regions interact.

The term "Fab-arm" refers to one heavy chain-light chain pair of an antibody, which may also be referred to as a half antibody.

The term "variable region" or "variable domain" of an antibody refers to the domain in an antibody heavy or light chain that is involved in the binding of the antibody to an antigen. Herein, the heavy chain variable region (VH) and light chain variable region (VL) of an antibody each comprise four conserved framework regions (FRs) and three complementarity determining regions (CDRs). The term "complementarity determining region" or "CDR" refers to the region in a variable domain that primarily contributes to antigen binding; "framework" or "FR" refers to variable domain residues other than CDR residues. A VH comprises 3 CDRs: HCDR1, HCDR2, and HCDR3, and a VL comprises 3 CDRs: LCDR1, LCDR2, and LCDR3. Each VH and VL is composed of three CDRs and four FRs arranged from the amino-terminus (also known as N-terminus) to the carboxyl-terminus (also known as C-terminus) in the following order: FR1, CDR1, FR2, CDR2, FR3, CDR3, and FR4.

The boundaries of the amino acid sequences of CDRs can be determined by a variety of well-known schemes, for example, the "Kabat" numbering scheme (see Kabat et al., (1991), Sequences of Proteins of Immunological Interest, 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), etc. The corresponding relationships between the various numbering schemes are well known to those skilled in the art and, illustratively, are shown in Table 1 below.

**Table 1. The relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure. Although the Kabat numbering scheme is employed to define amino acid residues in specific embodiments, technical solutions corresponding to other numbering schemes are to be considered equivalent technical solutions.

The term "antibody fragment" refers to a molecule different from an intact antibody, wherein the molecule comprises a moiety of an intact antibody, and the moiety binds to an antigen to which the intact antibody binds. Examples of antibody fragments include, but are not limited to, Fv, dsFv, Fab, Fab', Fab'-SH, F(ab')2, Fd, single-domain antibodies (sdAbs, e.g., VH, VL, VHH, or V_{HH}), single-chain Fab (scFab), diabodies, linear antibodies, single-chain antibodies (e.g., scFv and sc(Fv)2), and multispecific antibodies formed from antibody fragments.

The term "chimeric" antibody refers to an antibody in which a portion of the heavy and/or the light chain is derived from a particular source or species, while the rest of the heavy and/or the light chain is derived from another different source or species.

The term "humanized" antibody refers to an antibody that retains the reactivity of a non-human antibody while having relatively low immunogenicity in humans. For example, it can be achieved by keeping the non-human CDRs and replacing the rest of the antibody with its human counterparts (i.e., the constant regions and the framework region portions of the variable regions).

The terms "human antibody", "human-derived antibody", "fully human antibody", and "completely human antibody" are used interchangeably and mean antibodies whose variable regions and constant regions are human sequences. The term encompasses antibodies that are derived from human genes but have, for example, sequences that have been altered to reduce possible immunogenicity, increase affinity, and eliminate cysteines or glycosylation sites that may cause undesired folding. The term encompasses antibodies recombinantly produced in non-human cells (that may impart glycosylation without the characteristics of human cells). The term also encompasses antibodies that have been produced in transgenic mice comprising some or all of the human immunoglobulin heavy and light chain loci. The meaning of human antibodies explicitly excludes humanized antibodies.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to the internal binding affinity that reflects the 1:1 interaction between members of a binding pair (e.g., an antibody and an antigen). The affinity of molecule X for its ligand Y can be generally denoted by the dissociation constant (KD). Affinity can be measured by conventional methods known in the art, including those described herein.

As used herein, the term "kassoc" or "ka" refers to the association rate of a particular antibody-antigen interaction, and the term "kdis" or "kd" refers to the dissociation rate of a particular antibody-antigen interaction. The term "KD" refers to the dissociation constant, which is obtained from the ratio of kd to ka (i.e., kd/ka) and expressed as a molar concentration (M). The KD value of an antibody can be determined using a method well known in the art. For example, surface plasmon resonance is measured using a biosensing system such as a system (e.g., Biacore), or the affinity in a solution is measured by solution equilibrium titration (SET).

The term "surface plasmon resonance" refers to an optical phenomenon that enables analysis of real-time interactions by detecting changes in protein concentration within a biosensor matrix, for example, using a BIAcoreTM system (Biacore Life Sciences division of GE Healthcare, Piscataway, NJ).

The term "effector function" refers to biological activities that can be attributed to the Fc region of an antibody (either a natural sequence Fc region or an amino acid sequence variant Fc region) and vary with the antibody isotype. Examples of antibody effector functions include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a substantially homogeneous population of antibodies; that is, the amino acid sequences of the antibody molecules in the population are identical, except for natural mutations that may be present in small quantities. In contrast, a polyclonal antibody generally comprises multiple different antibodies having different amino acid sequences in their variable domains, which are generally specific for different epitopes. "Monoclonal" should not be construed as requiring the production of the antibody by any particular method. In some embodiments, an antibody provided by the present disclosure is a monoclonal antibody.

The term "antigen" refers to a molecule or a portion of a molecule that can be selectively bound, for example, by an antigen-binding protein (including, for example, an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen. The difference between the conformational epitope and the linear epitope is that in the presence of a denaturing solvent, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (see Prot. Sci. 9 (2000) 487-496), and cross-blocking.

The term "capable of specifically binding", "specifically binding", or "binding" means that an antibody is capable of binding to a certain antigen or an epitope thereof with higher affinity than to other antigens or epitopes. Generally, an antibody binds to an antigen or an epitope thereof with an equilibrium dissociation constant (KD) of about 1 × 10⁻⁷ M or less (e.g., about 1 × 10⁻⁸ M, 1 × 10⁻⁹ M, 1 × 10⁻¹⁰ M, 1 × 10⁻¹¹ M, or less). In some embodiments, the KD of the binding of an antibody to an antigen is 10% or less (e.g., 1%) of the KD of the binding of the antibody to a non-specific antigen (e.g., BSA or casein). KD may be measured using known methods, for example, by a BIACORE^{®} surface plasmon resonance assay. However, an antibody that specifically binds to an antigen or an epitope thereof may have cross-reactivity to other related antigens, e.g., to corresponding antigens from other species (homologous), such as humans or monkeys, e.g., Macaca fascicularis (cynomolgus, cyno), Pan troglodytes (chimpanzee, chimp), or Callithrix jacchus (common marmoset, marmoset).

The term "Titin chain" refers to a peptide fragment that is 78-118 amino acids in length and comprises a Titin Ig-like 152 domain in the Titin protein, or a functional variant thereof. The Titin chain is capable of binding to an Obscurin Ig-like 1 domain or an Obscurin-like Ig-like 1 domain to form a dimeric complex.

The term "Obscurin chain" refers to a peptide fragment that is 87-117 amino acids in length and comprises an Obscurin Ig-like 1 domain in the Obscurin protein, or a functional variant thereof, or to a peptide fragment that is 78-118 amino acids in length and comprises an Obscurin-like Ig-like 1 domain in the Obscurin-like 1 protein, or a functional variant thereof.

The Obscurin chain is capable of binding to the Titin chain to form a dimeric complex. The Titin chain and the Obscurin chain of the present disclosure can be used to replace the CH1 and CL in a Fab, respectively, to form a replaced Fab (Fab-S), and the replacement does not affect the binding of the antibody to the antigen (WO2021139758A1 and WO2022237882A1).

The term "antibody-dependent cellular cytotoxicity", "antibody-dependent cell-mediated cytotoxicity", or "ADCC" is a mechanism for inducing cell death. The mechanism relies on the interaction of antibody-coated target cells with effector cells with lytic activity, such as natural killer cells (NK), monocytes, macrophages, and neutrophils, via an Fcγ receptor (FcγR) expressed on the effector cells. For example, NK cells express FcγRIIIa, while monocytes express FcγRI, FcγRII, and FcγRIIIa. The ADCC activity of the antibodies provided herein can be assessed by *in vitro* assays, using cells expressing the antigen as target cells and NK cells as effector cells. Cell lysis is detected based on a label (e.g., radioactive substrate, fluorescent dye, or natural intracellular protein) released from lysed cells.

The term "antibody-dependent cellular phagocytosis (ADCP)" refers to a mechanism in which antibody-coated target cells are eliminated by the internalization effect of phagocytic cells (such as macrophages or dendritic cells).

The term "complement-dependent cytotoxicity" or "CDC" refers to a mechanism for inducing cell death in which the Fc effector domain of a target-binding antibody binds to and activates the complement component C1q, and C1q then activates the complement cascade, resulting in the death of the target cell. The activation of the complement may also result in the deposition of complement components on the surface of the target cell, and these complement components promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof, in either single-stranded or double-stranded form. The term encompasses nucleic acids comprising known nucleotide analogs or modified backbone residues or linkages, and the nucleic acids are synthetic, naturally occurring, and non-naturally occurring, have similar binding properties to the reference nucleic acid, and are metabolized similarly to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA).

"Isolated" nucleic acid refers to a nucleic acid molecule that has been separated from the components of its natural environment. An isolated nucleic acid encoding a polypeptide refers to one or more nucleic acid molecules encoding the polypeptide, including one or more such nucleic acid molecules in a single vector or separate vectors, and one or more such nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The term sequence "identity" refers to the degree (percentage) to which the amino acids/nucleic acids of two sequences are identical at equivalent positions when the two sequences are optimally aligned; gaps are introduced, when necessary, to achieve the maximum percent sequence identity, and any conservative substitution is not seen as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring an alignment, including any algorithm required to achieve the maximum alignment on the full length of the aligned sequences.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomously replicating in a host cell into which they are introduced (e.g., bacterial vectors having a bacterial origin of replication and episomal mammalian vectors). Other vectors (e.g., non-episomal mammalian vectors) can be integrated into the genome of a host cell after introduction into the host cell, so that they are replicated along with the host genome. The term "expression vector" or "expression construct" refers to a vector with which a host cell can be transformed, and the vector comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including the progeny of such cells. Host cells include "transformants" and "transformed cells", which include primary transformed cells and the progeny derived therefrom, regardless of the number of passages. The progeny may not be completely identical to the parental cells in terms of nucleic acid contents and may contain mutations. Mutant progeny that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia, Saccharomyces cerevisiae, Saccharomyces, Hansenula polymorpha, Kluyveromyces, Kluyveromyces lactis, Candida albicans, Aspergillus, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens, and Neurospora crassa.

"Optional" or "optionally" means that the event or circumstance subsequently described may, but does not necessarily, occur. This description includes the instance where the event or circumstance occurs or does not occur.

The term "pharmaceutical composition" refers to a mixture comprising one or more of the multispecific antibodies described herein and other chemical components; the other components are, for example, physiological/pharmaceutically acceptable carriers and excipients.

The term "pharmaceutically acceptable carriers, diluents, or excipients" refers to ingredients of a pharmaceutical formulation that are different from the active ingredient and are not toxic to subjects. Pharmaceutically acceptable carriers, diluents, or excipients include, but are not limited to, buffers, excipients, stabilizers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless otherwise indicated, the terms "patient" and "subject" are used interchangeably herein. In certain embodiments, an individual or subject is a human.

"Administer" or "give", when applied to animals, humans, experimental subjects, cells, tissues, organs, or biological fluids, refers to contact of an exogenous drug, a therapeutic agent, a diagnostic agent, or a composition with the animals, humans, subjects, cells, tissues, organs, or biological fluids.

The term "sample" refers to a collected material isolated from a subject (such as fluids, cells, or tissues), as well as fluids, cells, or tissues present in a subject. Exemplary samples are biological fluids, such as blood, serum and serosal fluids, plasma, lymph, urine, saliva, cystic fluids, tears, excretions, sputum, mucosal secretions of secretory tissues or organs, vaginal secretions, ascites, fluids in the pleura, pericardium, peritoneum, abdominal cavity, and other body cavities, fluids collected from bronchial lavage fluids, synovial fluids, liquid solutions in contact with a subject or biological source, e.g., culture media (including conditioned culture media) and lavage fluids, tissue biopsy samples, fine-needle punctures, surgically excised tissues, organ cultures, or cell cultures.

"Treatment" or "treat" (and grammatical variations thereof) refers to clinical intervention applied to the treated individual, and the clinical intervention may be performed either for prophylaxis or during the course of clinical pathology. Desirable effects of treatment include, but are not limited to, preventing the occurrence or recurrence of a disease, palliating symptoms, palliating/reducing any direct or indirect pathological consequences of the disease, preventing metastasis, decreasing the rate of disease progression, ameliorating or palliating the disease state, and regression or improved prognosis. In some embodiments, an antibody of the present disclosure is used to delay the development of, or slow the progression of, a disease.

"Effective amount" is generally an amount sufficient to reduce the severity and/or frequency of symptoms, eliminate these symptoms and/or underlying causes, prevent the appearance of symptoms and/or their underlying causes, and/or alleviate or ameliorate damage caused by or associated with a disease state (e.g., a lung disease). In some examples, an effective amount is a therapeutically effective amount or a prophylactically effective amount.

"Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and body weight of the individual, and the ability of a therapeutic agent or a combination of therapeutic agents to induce the desired response in the individual. Exemplary indicators of an effective therapeutic agent or combination of therapeutic agents include, for example, improved patient health.

### Exemplary Heteromultimers (Bispecific Antibodies)

### Bispecific Antibodies of Present Disclosure and Preparation Method Therefor

In one aspect, the present disclosure provides a method for preparing a bispecific antibody by a recombination reaction, comprising the following steps:
a) a step of providing a first parental monoclonal antibody that binds to a first antigen,
b) a step of providing a second parental monoclonal antibody that binds to a second antigen,
c) a step of co-incubating the first parental monoclonal antibody and the second parental monoclonal antibody under reducing conditions sufficient to allow reduction of interchain disulfide bonds in the hinge region, and
d) a step of obtaining the bispecific antibody, wherein:
   the CH3 domain(s) of the first parental monoclonal antibody and/or the second parental monoclonal antibody comprise(s) at least one amino acid mutation selected from the group consisting of positions 349, 351, 354, 356, 364, 366, 368, 394, 405, 409, 411, and 439;
   the first parental monoclonal antibody and the second parental monoclonal antibody bind to different antigens or epitopes, and mutation sites in the CH3 domains are indicated by EU numbering.

In some embodiments, provided is the aforementioned method for preparing a bispecific antibody, wherein:
1) the CH3 domain of the first parental monoclonal antibody comprises amino acid mutations at positions 356 and 349, and the CH3 domain of the second parental monoclonal antibody comprises amino acid mutations at positions 439 and 354; or
2) the CH3 domain of the first parental monoclonal antibody comprises an amino acid mutation at position 405, and the CH3 domain of the second parental monoclonal antibody comprises an amino acid mutation at position 394; or
3) the CH3 domain of the first parental monoclonal antibody comprises an amino acid mutation at position 356, and the CH3 domain of the second parental monoclonal antibody comprises an amino acid mutation at position 439, wherein the CH3 domains of the first and second parental monoclonal antibodies each further comprise one identical or different amino acid mutation selected from the group consisting of positions 351, 364, 366, 368, 394, 405, 409, and 411; or
4) the CH3 domain of the first parental monoclonal antibody comprises amino acid mutations at positions 356 and 349, and the CH3 domain of the second parental monoclonal antibody comprises amino acid mutations at positions 439 and 354, wherein the CH3 domain of the second parental monoclonal antibody further comprises one amino acid mutation selected from the group consisting of positions 351, 366, and 368; or
5) the CH3 domain of the first parental monoclonal antibody comprises an amino acid mutation at position 349, and the CH3 domain of the second parental monoclonal antibody comprises amino acid mutations at positions 354 and 364; or
6) the CH3 domain of the first parental monoclonal antibody comprises an amino acid mutation at position 349, and the CH3 domain of the second parental monoclonal antibody comprises an amino acid mutation at position 364 or 354.

In some embodiments, provided is the aforementioned method for preparing a bispecific antibody, wherein:
i) the CH3 domain of the first parental monoclonal antibody comprises amino acid mutations at positions 356 and 349, and the CH3 domain of the second parental monoclonal antibody comprises amino acid mutations at positions 439 and 354; or
ii) the CH3 domain of the first parental monoclonal antibody comprises an amino acid mutation at position 405, and the CH3 domain of the second parental monoclonal antibody comprises an amino acid mutation at position 394; or
iii) the CH3 domain of the first parental monoclonal antibody comprises an amino acid mutation at position 356, and the CH3 domain of the second parental monoclonal antibody comprises an amino acid mutation at position 439, wherein the CH3 domains of the first and second parental monoclonal antibodies each further comprise one identical or different amino acid mutation selected from the group consisting of positions 351, 364, 366, 368, 394, 405, 409, and 411; or
iv) the CH3 domain of the first parental monoclonal antibody comprises amino acid mutations at positions 356 and 349, and the CH3 domain of the second parental monoclonal antibody comprises amino acid mutations at positions 439 and 354, wherein the CH3 domain of the second parental monoclonal antibody further comprises one amino acid mutation selected from the group consisting of positions 351, 366, and 368.

In some embodiments, provided is the aforementioned method for preparing a bispecific antibody, wherein:
1) the CH3 domain of the first parental monoclonal antibody comprises amino acid mutations of 356K and 349S, and the CH3 domain of the second parental monoclonal antibody comprises amino acid mutations of 439E and 354Y; or
2) the CH3 domain of the first parental monoclonal antibody comprises an amino acid mutation of 405T, and the CH3 domain of the second parental monoclonal antibody comprises an amino acid mutation of 394F; or
3) the CH3 domain of the first parental monoclonal antibody comprises an amino acid mutation of 356K, and the CH3 domain of the second parental monoclonal antibody comprises an amino acid mutation of 439E, wherein the CH3 domains of the first and second parental monoclonal antibodies each further comprise one identical or different amino acid mutation selected from the group consisting of 351T, 351I, 364A, 366A, 368I, 394A, 394S, 405Y, 409Q, and 411Y; or
4) the CH3 domain of the first parental monoclonal antibody comprises amino acid mutations of 356K and 349C, and the CH3 domain of the second parental monoclonal antibody comprises amino acid mutations of 439E and 354C, wherein the CH3 domain of the second parental monoclonal antibody further comprises one amino acid mutation selected from the group consisting of 351I, 366A, and 368I; or
5) the CH3 domain of the first parental monoclonal antibody comprises an amino acid mutation of 349C, and the CH3 domain of the second parental monoclonal antibody comprises an amino acid mutation of 354C, wherein the CH3 domain of the second parental monoclonal antibody further comprises an amino acid mutation of 364Y or 364F; or
6) the CH3 domain of the first parental monoclonal antibody comprises an amino acid mutation of 349S, and the CH3 domain of the second parental monoclonal antibody comprises an amino acid mutation of 364Y; or
the CH3 domain of the first parental monoclonal antibody comprises an amino acid mutation of 349G, and the CH3 domain of the second parental monoclonal antibody comprises an amino acid mutation of 354W.

In some embodiments, provided is the aforementioned method for preparing a bispecific antibody, wherein:
i) the CH3 domain of the first parental monoclonal antibody comprises amino acid mutations of 356K and 349S, and the CH3 domain of the second parental monoclonal antibody comprises amino acid mutations of 439E and 354Y; or
ii) the CH3 domain of the first parental monoclonal antibody comprises an amino acid mutation of 405T, and the CH3 domain of the second parental monoclonal antibody comprises an amino acid mutation of 394F; or
iii) the CH3 domain of the first parental monoclonal antibody comprises an amino acid mutation of 356K, and the CH3 domain of the second parental monoclonal antibody comprises an amino acid mutation of 439E, wherein the CH3 domains of the first and second parental monoclonal antibodies each further comprise one identical or different amino acid mutation selected from the group consisting of 351T, 351I, 364A, 366A, 368I, 394A, 394S, 405Y, 409Q, and 411Y; or
iv) the CH3 domain of the first parental monoclonal antibody comprises amino acid mutations of 356K and 349C, and the CH3 domain of the second parental monoclonal antibody comprises amino acid mutations of 439E and 354C, wherein the CH3 domain of the second parental monoclonal antibody further comprises one amino acid mutation selected from the group consisting of 351I, 366A, and 368I.

In some embodiments, provided is the aforementioned method for preparing a bispecific antibody, wherein:
1) the CH3 domain of the first parental monoclonal antibody comprises amino acid mutations of 356K and 349S, and the CH3 domain of the second parental monoclonal antibody comprises amino acid mutations of 439E and 354Y; or
2) the CH3 domain of the first parental monoclonal antibody comprises an amino acid mutation of 405T, and the CH3 domain of the second parental monoclonal antibody comprises an amino acid mutation of 394F; or
3) the CH3 domain of the first parental monoclonal antibody comprises amino acid mutations of 356K and 351T, and the CH3 domain of the second parental monoclonal antibody comprises amino acid mutations of 439E and 351T; or
   the CH3 domain of the first parental monoclonal antibody comprises amino acid mutations of 356K and 351I, and the CH3 domain of the second parental monoclonal antibody comprises amino acid mutations of 439E and 351I; or
   the CH3 domain of the first parental monoclonal antibody comprises amino acid mutations of 356K and 364A, and the CH3 domain of the second parental monoclonal antibody comprises amino acid mutations of 439E and 364A; or
   the CH3 domain of the first parental monoclonal antibody comprises amino acid mutations of 356K and 366A, and the CH3 domain of the second parental monoclonal antibody comprises amino acid mutations of 439E and 366A; or
   the CH3 domain of the first parental monoclonal antibody comprises amino acid mutations of 356K and 368I, and the CH3 domain of the second parental monoclonal antibody comprises amino acid mutations of 439E and 368I; or
   the CH3 domain of the first parental monoclonal antibody comprises amino acid mutations of 356K and 394A, and the CH3 domain of the second parental monoclonal antibody comprises amino acid mutations of 439E and 394A; or
   the CH3 domain of the first parental monoclonal antibody comprises amino acid mutations of 356K and 394S, and the CH3 domain of the second parental monoclonal antibody comprises amino acid mutations of 439E and 394S; or
   the CH3 domain of the first parental monoclonal antibody comprises amino acid mutations of 356K and 405Y, and the CH3 domain of the second parental monoclonal antibody comprises amino acid mutations of 439E and 405Y; or
   the CH3 domain of the first parental monoclonal antibody comprises amino acid mutations of 356K and 409Q, and the CH3 domain of the second parental monoclonal antibody comprises amino acid mutations of 439E and 409Q; or
   the CH3 domain of the first parental monoclonal antibody comprises amino acid mutations of 356K and 411Y, and the CH3 domain of the second parental monoclonal antibody comprises amino acid mutations of 439E and 411Y; or
4) the CH3 domain of the first parental monoclonal antibody comprises amino acid mutations of 356K and 349C, and the CH3 domain of the second parental monoclonal antibody comprises amino acid mutations of 439E, 354C, and 351I; or
   the CH3 domain of the first parental monoclonal antibody comprises amino acid mutations of 356K and 349C, and the CH3 domain of the second parental monoclonal antibody comprises amino acid mutations of 439E, 354C, and 366A; or
   the CH3 domain of the first parental monoclonal antibody comprises amino acid mutations of 356K and 349C, and the CH3 domain of the second parental monoclonal antibody comprises amino acid mutations of 439E, 354C, and 368I; or
5) the CH3 domain of the first parental monoclonal antibody comprises an amino acid mutation of 349C, and the CH3 domain of the second parental monoclonal antibody comprises amino acid mutations of 354C and 364Y; or
   the CH3 domain of the first parental monoclonal antibody comprises an amino acid mutation of 349C, and the CH3 domain of the second parental monoclonal antibody comprises amino acid mutations of 354C and 364F; or
6) the CH3 domain of the first parental monoclonal antibody comprises an amino acid mutation of 349S, and the CH3 domain of the second parental monoclonal antibody comprises an amino acid mutation of 364Y; or
the CH3 domain of the first parental monoclonal antibody comprises an amino acid mutation of 349G, and the CH3 domain of the second parental monoclonal antibody comprises an amino acid mutation of 354W.

In some embodiments, provided is the aforementioned method for preparing a bispecific antibody, wherein:
i) the CH3 domain of the first parental monoclonal antibody comprises amino acid mutations of 356K and 349S, and the CH3 domain of the second parental monoclonal antibody comprises amino acid mutations of 439E and 354Y; or
ii) the CH3 domain of the first parental monoclonal antibody comprises an amino acid mutation of 405T, and the CH3 domain of the second parental monoclonal antibody comprises an amino acid mutation of 394F; or
iii) the CH3 domain of the first parental monoclonal antibody comprises amino acid mutations of 356K and 351T, and the CH3 domain of the second parental monoclonal antibody comprises amino acid mutations of 439E and 351T; or
   the CH3 domain of the first parental monoclonal antibody comprises amino acid mutations of 356K and 351I, and the CH3 domain of the second parental monoclonal antibody comprises amino acid mutations of 439E and 351I; or
   the CH3 domain of the first parental monoclonal antibody comprises amino acid mutations of 356K and 364A, and the CH3 domain of the second parental monoclonal antibody comprises amino acid mutations of 439E and 364A; or
   the CH3 domain of the first parental monoclonal antibody comprises amino acid mutations of 356K and 366A, and the CH3 domain of the second parental monoclonal antibody comprises amino acid mutations of 439E and 366A; or
   the CH3 domain of the first parental monoclonal antibody comprises amino acid mutations of 356K and 368I, and the CH3 domain of the second parental monoclonal antibody comprises amino acid mutations of 439E and 368I; or
   the CH3 domain of the first parental monoclonal antibody comprises amino acid mutations of 356K and 394A, and the CH3 domain of the second parental monoclonal antibody comprises amino acid mutations of 439E and 394A; or
   the CH3 domain of the first parental monoclonal antibody comprises amino acid mutations of 356K and 394S, and the CH3 domain of the second parental monoclonal antibody comprises amino acid mutations of 439E and 394S; or
   the CH3 domain of the first parental monoclonal antibody comprises amino acid mutations of 356K and 405Y, and the CH3 domain of the second parental monoclonal antibody comprises amino acid mutations of 439E and 405Y; or
   the CH3 domain of the first parental monoclonal antibody comprises amino acid mutations of 356K and 409Q, and the CH3 domain of the second parental monoclonal antibody comprises amino acid mutations of 439E and 409Q; or
   the CH3 domain of the first parental monoclonal antibody comprises amino acid mutations of 356K and 411Y, and the CH3 domain of the second parental monoclonal antibody comprises amino acid mutations of 439E and 411Y; or
iv) the CH3 domain of the first parental monoclonal antibody comprises amino acid mutations of 356K and 349C, and the CH3 domain of the second parental monoclonal antibody comprises amino acid mutations of 439E, 354C, and 351I; or

the CH3 domain of the first parental monoclonal antibody comprises amino acid mutations of 356K and 349C, and the CH3 domain of the second parental monoclonal antibody comprises amino acid mutations of 439E, 354C, and 366A; or
the CH3 domain of the first parental monoclonal antibody comprises amino acid mutations of 356K and 349C, and the CH3 domain of the second parental monoclonal antibody comprises amino acid mutations of 439E, 354C, and 368I.

In some embodiments, provided is the aforementioned method for preparing a bispecific antibody, wherein:
1) the CH3 domain of the first parental monoclonal antibody comprises amino acid mutations as represented by 356K and 349S, and the CH3 domain of the second parental monoclonal antibody comprises amino acid mutations as represented by 439E and 354Y; or
2) the CH3 domain of the first parental monoclonal antibody comprises an amino acid mutation as represented by 405T, and the CH3 domain of the second parental monoclonal antibody comprises an amino acid mutation as represented by 394F; or
3) the CH3 domain of the first parental monoclonal antibody comprises amino acid mutations as represented by 356K and 351T, and the CH3 domain of the second parental monoclonal antibody comprises amino acid mutations as represented by 439E and 351T; or
   the CH3 domain of the first parental monoclonal antibody comprises amino acid mutations as represented by 356K and 351I, and the CH3 domain of the second parental monoclonal antibody comprises amino acid mutations as represented by 439E and 351I; or
   the CH3 domain of the first parental monoclonal antibody comprises amino acid mutations as represented by 356K and 364A, and the CH3 domain of the second parental monoclonal antibody comprises amino acid mutations as represented by 439E and 364A; or
   the CH3 domain of the first parental monoclonal antibody comprises amino acid mutations as represented by 356K and 366A, and the CH3 domain of the second parental monoclonal antibody comprises amino acid mutations as represented by 439E and 366A; or
   the Fc region of the first parental monoclonal antibody comprises amino acid mutations as represented by 356K and 368I, and the CH3 domain of the second parental monoclonal antibody comprises amino acid mutations as represented by 439E and 368I; or
   the CH3 domain of the first parental monoclonal antibody comprises amino acid mutations as represented by 356K and 394A, and the CH3 domain of the second parental monoclonal antibody comprises amino acid mutations as represented by 439E and 394A; or
   the CH3 domain of the first parental monoclonal antibody comprises amino acid mutations as represented by 356K and 394S, and the CH3 domain of the second parental monoclonal antibody comprises amino acid mutations as represented by 439E and 394S; or
   the CH3 domain of the first parental monoclonal antibody comprises amino acid mutations as represented by 356K and 405Y, and the CH3 domain of the second parental monoclonal antibody comprises amino acid mutations as represented by 439E and 405Y; or
   the CH3 domain of the first parental monoclonal antibody comprises amino acid mutations as represented by 356K and 409Q, and the CH3 domain of the second parental monoclonal antibody comprises amino acid mutations as represented by 439E and 409Q; or
   the CH3 domain of the first parental monoclonal antibody comprises amino acid mutations as represented by 356K and 411Y, and the CH3 domain of the second parental monoclonal antibody comprises amino acid mutations as represented by 439E and 411Y; or
4) the CH3 domain of the first parental monoclonal antibody comprises amino acid mutations as represented by 356K and 349C, and the CH3 domain of the second parental monoclonal antibody comprises amino acid mutations as represented by 439E, 354C, and 351I; or
   the CH3 domain of the first parental monoclonal antibody comprises amino acid mutations as represented by 356K and 349C, and the CH3 domain of the second parental monoclonal antibody comprises amino acid mutations as represented by 439E, 354C, and 366A; or
   the CH3 domain of the first parental monoclonal antibody comprises amino acid mutations as represented by 356K and 349C, and the CH3 domain of the second parental monoclonal antibody comprises amino acid mutations as represented by 439E, 354C, and 368I; or
5) the CH3 domain of the first parental monoclonal antibody comprises an amino acid mutation as represented by 349C, and the CH3 domain of the second parental monoclonal antibody comprises amino acid mutations as represented by 354C and 364Y; or
   the CH3 domain of the first parental monoclonal antibody comprises an amino acid mutation as represented by 349C, and the CH3 domain of the second parental monoclonal antibody comprises amino acid mutations as represented by 354C and 364F; or
6) the CH3 domain of the first parental monoclonal antibody comprises an amino acid mutation as represented by 349S, and the CH3 domain of the second parental monoclonal antibody comprises an amino acid mutation as represented by 364Y; or
the CH3 domain of the first parental monoclonal antibody comprises an amino acid mutation as represented by 349G, and the CH3 domain of the second parental monoclonal antibody comprises an amino acid mutation as represented by 354W.

In some embodiments, provided is the aforementioned method for preparing a bispecific antibody, wherein:
i) the CH3 domain of the first parental monoclonal antibody comprises amino acid mutations as represented by 356K and 349S, and the CH3 domain of the second parental monoclonal antibody comprises amino acid mutations as represented by 439E and 354Y; or
ii) the CH3 domain of the first parental monoclonal antibody comprises an amino acid mutation as represented by 405T, and the CH3 domain of the second parental monoclonal antibody comprises an amino acid mutation as represented by 394F; or
iii) the CH3 domain of the first parental monoclonal antibody comprises amino acid mutations as represented by 356K and 351T, and the CH3 domain of the second parental monoclonal antibody comprises amino acid mutations as represented by 439E and 351T; or
   the CH3 domain of the first parental monoclonal antibody comprises amino acid mutations as represented by 356K and 351I, and the CH3 domain of the second parental monoclonal antibody comprises amino acid mutations as represented by 439E and 351I; or
   the CH3 domain of the first parental monoclonal antibody comprises amino acid mutations as represented by 356K and 364A, and the CH3 domain of the second parental monoclonal antibody comprises amino acid mutations as represented by 439E and 364A; or
   the CH3 domain of the first parental monoclonal antibody comprises amino acid mutations as represented by 356K and 366A, and the CH3 domain of the second parental monoclonal antibody comprises amino acid mutations as represented by 439E and 366A; or
   the Fc region of the first parental monoclonal antibody comprises amino acid mutations as represented by 356K and 368I, and the CH3 domain of the second parental monoclonal antibody comprises amino acid mutations as represented by 439E and 368I; or
   the CH3 domain of the first parental monoclonal antibody comprises amino acid mutations as represented by 356K and 394A, and the CH3 domain of the second parental monoclonal antibody comprises amino acid mutations as represented by 439E and 394A; or
   the CH3 domain of the first parental monoclonal antibody comprises amino acid mutations as represented by 356K and 394S, and the CH3 domain of the second parental monoclonal antibody comprises amino acid mutations as represented by 439E and 394S; or
   the CH3 domain of the first parental monoclonal antibody comprises amino acid mutations as represented by 356K and 405Y, and the CH3 domain of the second parental monoclonal antibody comprises amino acid mutations as represented by 439E and 405Y; or
   the CH3 domain of the first parental monoclonal antibody comprises amino acid mutations as represented by 356K and 409Q, and the CH3 domain of the second parental monoclonal antibody comprises amino acid mutations as represented by 439E and 409Q; or
   the CH3 domain of the first parental monoclonal antibody comprises amino acid mutations as represented by 356K and 411Y, and the CH3 domain of the second parental monoclonal antibody comprises amino acid mutations as represented by 439E and 411Y; or
iv) the CH3 domain of the first parental monoclonal antibody comprises amino acid mutations as represented by 356K and 349C, and the CH3 domain of the second parental monoclonal antibody comprises amino acid mutations as represented by 439E, 354C, and 351I; or

the CH3 domain of the first parental monoclonal antibody comprises amino acid mutations as represented by 356K and 349C, and the CH3 domain of the second parental monoclonal antibody comprises amino acid mutations as represented by 439E, 354C, and 366A; or
the CH3 domain of the first parental monoclonal antibody comprises amino acid mutations as represented by 356K and 349C, and the CH3 domain of the second parental monoclonal antibody comprises amino acid mutations as represented by 439E, 354C, and 368I.

In some embodiments, provided is the aforementioned method for preparing a bispecific antibody, wherein:
1) the CH3 domain of the first parental monoclonal antibody comprises amino acid mutations of 356K and 349S, and the CH3 domain of the second parental monoclonal antibody comprises amino acid mutations of 439E and 354Y; or
2) the CH3 domain of the first parental monoclonal antibody comprises an amino acid mutation of 405T, and the CH3 domain of the second parental monoclonal antibody comprises an amino acid mutation of 394F; or
3) the CH3 domain of the first parental monoclonal antibody comprises amino acid mutations of 356K and 351I, and the CH3 domain of the second parental monoclonal antibody comprises amino acid mutations of 439E and 351I; or
4) the CH3 domain of the first parental monoclonal antibody comprises amino acid mutations of 356K and 394S, and the CH3 domain of the second parental monoclonal antibody comprises amino acid mutations of 439E and 394S; or
5) the CH3 domain of the first parental monoclonal antibody comprises amino acid mutations of 356K and 411Y, and the CH3 domain of the second parental monoclonal antibody comprises amino acid mutations of 439E and 411Y; or
6) the CH3 domain of the first parental monoclonal antibody comprises an amino acid mutation of 349S, and the CH3 domain of the second parental monoclonal antibody comprises an amino acid mutation of 364Y; or
7) the CH3 domain of the first parental monoclonal antibody comprises amino acid mutations of 356K and 349C, and the CH3 domain of the second parental monoclonal antibody comprises amino acid mutations of 439E, 354C, and 351I; or
8) the CH3 domain of the first parental monoclonal antibody comprises an amino acid mutation of 349C, and the CH3 domain of the second parental monoclonal antibody comprises amino acid mutations of 354C and 364Y; or
9) the CH3 domain of the first parental monoclonal antibody comprises an amino acid mutation of 349C, and the CH3 domain of the second parental monoclonal antibody comprises amino acid mutations of 354C and 364F.

In some embodiments, provided is the aforementioned method for preparing a bispecific antibody, wherein:
i) the CH3 domain of the first parental monoclonal antibody comprises amino acid mutations of 356K and 349S, and the CH3 domain of the second parental monoclonal antibody comprises amino acid mutations of 439E and 354Y; or
ii) the CH3 domain of the first parental monoclonal antibody comprises an amino acid mutation of 405T, and the CH3 domain of the second parental monoclonal antibody comprises an amino acid mutation of 394F.

In some embodiments, provided is the aforementioned method for preparing a bispecific antibody, wherein the CH3 domain of the first parental monoclonal antibody comprises only amino acid mutations of 356K and 351I, and the CH3 domain of the second parental monoclonal antibody comprises only amino acid mutations of 439E and 351I.

In some embodiments, provided is the aforementioned method for preparing a bispecific antibody, wherein the CH3 domain of the first parental monoclonal antibody comprises only an amino acid mutation of 349S, and the CH3 domain of the second parental monoclonal antibody comprises only an amino acid mutation of 364Y.

In some embodiments, provided is the aforementioned method for preparing a bispecific antibody, wherein the CH3 domain of the first parental monoclonal antibody comprises only amino acid mutations of 356K and 349C, and the CH3 domain of the second parental monoclonal antibody comprises only amino acid mutations of 439E, 354C, and 351I.

In some embodiments, provided is the aforementioned method for preparing a bispecific antibody, wherein the CH3 domain of the first parental monoclonal antibody comprises only an amino acid mutation of 349C, and the CH3 domain of the second parental monoclonal antibody comprises only amino acid mutations of 354C and 364Y.

In some embodiments, provided is the aforementioned method for preparing a bispecific antibody, wherein the CH3 domains are derived from IgG1. In some embodiments, provided is the aforementioned method for preparing a bispecific antibody, wherein the CH3 domains are derived from human IgG1.

In some embodiments, provided is the aforementioned method for preparing a bispecific antibody, wherein the bispecific antibody comprises at least one replaced Fab comprising a Titin chain and an Obscurin chain that are capable of forming a dimer. In some embodiments, provided is the aforementioned method for preparing a bispecific antibody, wherein the replaced Fab is obtained by replacing the original CH1 and CL of a Fab with the Obscurin chain and the Titin chain, respectively, or the replaced Fab is obtained by replacing the original CH1 and CL with the Titin chain and the Obscurin chain, respectively. In some embodiments, the Titin chain comprises the amino acid sequence set forth in SEQ ID NO: 6, and the Obscurin chain comprises the amino acid sequence set forth in SEQ ID NO: 5.

In some embodiments, provided is the aforementioned method for preparing a bispecific antibody, wherein more than 80% (e.g., more than 85%, more than 88%, more than 90%, more than 91%, more than 92%, more than 93%, more than 94%, more than 95%, more than 96%, more than 97%, more than 98%, or more than 99%) of the product is the desired bispecific antibody. In some embodiments, provided is the aforementioned method for preparing a bispecific antibody, wherein more than 92% (e.g., more than 93%, more than 94%, more than 95%, more than 96%, more than 97%, more than 98%, or more than 99%) of the product is the desired bispecific antibody. In some embodiments, provided is the aforementioned method for preparing a bispecific antibody, wherein more than 94% (e.g., more than 95%, more than 96%, more than 97%, more than 98%, or more than 99%) of the product is the desired bispecific antibody. In some embodiments, provided is the aforementioned method for preparing a bispecific antibody, wherein more than 95% (e.g., more than 96%, more than 97%, more than 98%, or more than 99%) of the product is the desired bispecific antibody.

In some embodiments, provided is the aforementioned method for preparing a bispecific antibody, wherein the reducing conditions include, but are not limited to, adding a reductant selected from the group consisting of 2-mercaptoethylamine (2-MEA), dithiothreitol (DTT), dithioerythritol (DTE), glutathione (GSH), tris(2-carboxyethyl)phosphine (TCEP), L-cysteine, D-cysteine, and β-mercapto-ethanol, as well as chemical derivatives thereof. In some embodiments, provided is the aforementioned method for preparing a bispecific antibody, wherein the reductant is selected from the group consisting of 2-MEA, glutathione, L-cysteine, dithiothreitol, β-mercaptoethanol, and TCEP. In some embodiments, provided is the aforementioned method for preparing a bispecific antibody, wherein the reductant is 2-MEA.

In some embodiments, provided is the aforementioned method for preparing a bispecific antibody, wherein steps a) and b) further comprise a step of purifying the first parental monoclonal antibody and the second parental monoclonal antibody. In some embodiments, the purification method includes, but is not limited to, protein A or protein G chromatography, antigen binding-based affinity chromatography, anti-idiotypic antibody-based affinity chromatography, ion exchange, hydrophobic interaction chromatography, mixed chromatography (such as hydroxyapatite), immobilized metal affinity chromatography, thiophilic adsorption chromatography, size exclusion chromatography (SEC), etc.

In some embodiments, the aforementioned method for preparing a bispecific antibody further comprises a step of separating the bispecific antibody from the reductant after step c). In some embodiments, methods for separating the bispecific antibody from the reductant include, but are not limited to, any of the methods described below, e.g., dialysis, precipitation, chromatography, or filtration.

In some embodiments, provided is the aforementioned method for preparing a bispecific antibody, wherein step d) further comprises a method for purifying a composition obtained from step c). In some embodiments, the purification method includes, but is not limited to, protein A or protein G chromatography, antigen binding-based affinity chromatography, anti-idiotypic antibody-based affinity chromatography, ion exchange, hydrophobic interaction chromatography, mixed chromatography (such as hydroxyapatite), immobilized metal affinity chromatography, thiophilic adsorption chromatography, size exclusion chromatography (SEC), etc.

In another aspect, the present disclosure provides a bispecific antibody comprising polypeptides in which two CH3 domains bind to each other, wherein amino acid mutations are introduced into a CH3 domain of a first polypeptide and a CH3 domain of a second polypeptide to promote the formation of a heterologous bispecific antibody, wherein the amino acid mutations include at least one amino acid mutation selected from the group consisting of positions 349, 351, 354, 356, 364, 366, 368, 394, 405, 409, 411, and 439; the first polypeptide and the second polypeptide bind to different antigens or epitopes, and mutation sites in the CH3 domains are indicated by EU numbering.

In some embodiments, provided is the aforementioned bispecific antibody, wherein:
1) the CH3 domain of the first polypeptide comprises amino acid mutations at positions 356 and 349, and the CH3 domain of the second polypeptide comprises amino acid mutations at positions 439 and 354; or
2) the CH3 domain of the first polypeptide comprises an amino acid mutation at position 405, and the CH3 domain of the second polypeptide comprises an amino acid mutation at position 394; or
3) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 351, 364, 366, 368, 394, 405, 409, and 411; or
4) the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368; or
5) the CH3 domain of the first polypeptide comprises an amino acid mutation at position 349, and the CH3 domain of the second polypeptide comprises amino acid mutations at positions 354 and 364; or
6) the CH3 domain of the first polypeptide comprises an amino acid mutation at position 349, and the CH3 domain of the second polypeptide comprises an amino acid mutation at position 364 or 354.

In some embodiments, provided is the aforementioned bispecific antibody, wherein:
i) the CH3 domain of the first polypeptide comprises amino acid mutations at positions 356 and 349, and the CH3 domain of the second polypeptide comprises amino acid mutations at positions 439 and 354; or
ii) the CH3 domain of the first polypeptide comprises an amino acid mutation at position 405, and the CH3 domain of the second polypeptide comprises an amino acid mutation at position 394; or
iii) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one amino acid mutation selected from the group consisting of positions 351, 364, 366, 368, 394, 405, 409, and 411; or
iv) the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368.

In some embodiments, provided is the aforementioned bispecific antibody, wherein:
1) the CH3 domain of the first polypeptide comprises amino acid mutations of 356K and 349S, and the CH3 domain of the second polypeptide comprises amino acid mutations of 439E and 354Y; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises an amino acid mutation of 356K, and the CH3 domain of the second polypeptide comprises an amino acid mutation of 439E, wherein the CH3 domains of the first polypeptide and the second polypeptide further comprise one identical or different amino acid mutation selected from the group consisting of 351T, 351I, 364A, 366A, 368I, 394A, 394S, 405Y, 409Q, and 411Y; or
4) the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I; or
5) the CH3 domain of the first polypeptide comprises an amino acid mutation of 349C, and the CH3 domain of the second polypeptide comprises an amino acid mutation of 354C, wherein the CH3 domain of the second polypeptide further comprises an amino acid mutation of 364Y or 364F; or
6) the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W. In some embodiments, provided is the aforementioned bispecific antibody, wherein:
i) the CH3 domain of the first polypeptide comprises amino acid mutations of 356K and 349S, and the CH3 domain of the second polypeptide comprises amino acid mutations of 439E and 354Y; or
ii) the CH3 domain of the first polypeptide comprises an amino acid mutation of 405T, and the CH3 domain of the second polypeptide comprises an amino acid mutation of 394F; or
iii) the CH3 domain of the first polypeptide comprises an amino acid mutation of 356K, and the CH3 domain of the second polypeptide comprises an amino acid mutation of 439E, wherein the CH3 domains of the first polypeptide and the second polypeptide further comprise one amino acid mutation selected from the group consisting of 351T, 351I, 364A, 366A, 368I, 394A, 394S, 405Y, 409Q, and 411Y; or
iv) the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I.

In some embodiments, provided is the aforementioned bispecific antibody, wherein:
1) the CH3 domain of the first polypeptide comprises amino acid mutations of 356K and 349S, and the Fc region of the second polypeptide comprises amino acid mutations of 439E and 354Y; or
2) the CH3 domain of the first polypeptide comprises an amino acid mutation of 405T, and the Fc region of the second polypeptide comprises an amino acid mutation of 394F; or
3) the CH3 domain of the first polypeptide comprises amino acid mutations of 356K and 351T, and the CH3 domain of the second polypeptide comprises amino acid mutations of 439E and 351T; or
   the CH3 domain of the first polypeptide comprises amino acid mutations of 356K and 351I, and the CH3 domain of the second polypeptide comprises amino acid mutations of 439E and 351I; or
   the CH3 domain of the first polypeptide comprises amino acid mutations of 356K and 364A, and the CH3 domain of the second polypeptide comprises amino acid mutations of 439E and 364A; or
   the CH3 domain of the first polypeptide comprises amino acid mutations of 356K and 366A, and the CH3 domain of the second polypeptide comprises amino acid mutations of 439E and 366A; or
   the CH3 domain of the first polypeptide comprises amino acid mutations of 356K and 368I, and the CH3 domain of the second polypeptide comprises amino acid mutations of 439E and 368I; or
   the CH3 domain of the first polypeptide comprises amino acid mutations of 356K and 394A, and the CH3 domain of the second polypeptide comprises amino acid mutations of 439E and 394A; or
   the CH3 domain of the first polypeptide comprises amino acid mutations of 356K and 394S, and the CH3 domain of the second polypeptide comprises amino acid mutations of 439E and 394S; or
   the CH3 domain of the first polypeptide comprises amino acid mutations of 356K and 405Y, and the CH3 domain of the second polypeptide comprises amino acid mutations of 439E and 405Y; or
   the CH3 domain of the first polypeptide comprises amino acid mutations of 356K and 409Q, and the CH3 domain of the second polypeptide comprises amino acid mutations of 439E and 409Q; or
   the CH3 domain of the first polypeptide comprises amino acid mutations of 356K and 411Y, and the CH3 domain of the second polypeptide comprises amino acid mutations of 439E and 411Y; or
4) the CH3 domain of the first polypeptide comprises amino acid mutations of 356K and 349C, and the CH3 domain of the second polypeptide comprises amino acid mutations of 439E, 354C, and 351I; or
   the CH3 domain of the first polypeptide comprises amino acid mutations of 356K and 349C, and the CH3 domain of the second polypeptide comprises amino acid mutations of 439E, 354C, and 366A; or
   the CH3 domain of the first polypeptide comprises amino acid mutations of 356K and 349C, and the CH3 domain of the second polypeptide comprises amino acid mutations of 439E, 354C, and 368I; or
5) the CH3 domain of the first polypeptide comprises an amino acid mutation of 349C, and the CH3 domain of the second polypeptide comprises amino acid mutations of 354C and 364Y; or
   the CH3 domain of the first polypeptide comprises an amino acid mutation of 349C, and the CH3 domain of the second polypeptide comprises amino acid mutations of 354C and 364F; or
6) the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W; preferably,
1) the CH3 domain of the first polypeptide comprises amino acid mutations of 356K and 349S, and the CH3 domain of the second polypeptide comprises amino acid mutations of 439E and 354Y; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises amino acid mutations of 356K and 351I, and the CH3 domain of the second polypeptide comprises amino acid mutations of 439E and 351I; or
4) the CH3 domain of the first polypeptide comprises amino acid mutations of 356K and 394S, and the CH3 domain of the second polypeptide comprises amino acid mutations of 439E and 394S; or
5) the CH3 domain of the first polypeptide comprises amino acid mutations of 356K and 411Y, and the CH3 domain of the second polypeptide comprises amino acid mutations of 439E and 411Y; or
6) the CH3 domain of the first polypeptide comprises an amino acid mutation of 349S, and the CH3 domain of the second polypeptide comprises an amino acid mutation of 364Y; or
7) the CH3 domain of the first polypeptide comprises amino acid mutations of 356K and 349C, and the CH3 domain of the second polypeptide comprises amino acid mutations of 439E, 354C, and 351I; or
8) the CH3 domain of the first polypeptide comprises an amino acid mutation of 349C, and the CH3 domain of the second polypeptide comprises amino acid mutations of 354C and 364Y; or
9) the CH3 domain of the first polypeptide comprises an amino acid mutation of 349C, and the CH3 domain of the second polypeptide comprises amino acid mutations of 354C and 364F.

In some embodiments, provided is the aforementioned bispecific antibody, wherein:
i) the CH3 domain of the first polypeptide comprises amino acid mutations of 356K and 349S, and the Fc region of the second polypeptide comprises amino acid mutations of 439E and 354Y; or
ii) the CH3 domain of the first polypeptide comprises an amino acid mutation of 405T, and the Fc region of the second polypeptide comprises an amino acid mutation of 394F; or
iii) the CH3 domain of the first polypeptide comprises amino acid mutations of 356K and 351T, and the CH3 domain of the second polypeptide comprises amino acid mutations of 439E and 351T; or
   the CH3 domain of the first polypeptide comprises amino acid mutations of 356K and 351I, and the CH3 domain of the second polypeptide comprises amino acid mutations of 439E and 351I; or
   the CH3 domain of the first polypeptide comprises amino acid mutations of 356K and 364A, and the CH3 domain of the second polypeptide comprises amino acid mutations of 439E and 364A; or
   the CH3 domain of the first polypeptide comprises amino acid mutations of 356K and 366A, and the CH3 domain of the second polypeptide comprises amino acid mutations of 439E and 366A; or
   the CH3 domain of the first polypeptide comprises amino acid mutations of 356K and 368I, and the CH3 domain of the second polypeptide comprises amino acid mutations of 439E and 368I; or
   the CH3 domain of the first polypeptide comprises amino acid mutations of 356K and 394A, and the CH3 domain of the second polypeptide comprises amino acid mutations of 439E and 394A; or
   the CH3 domain of the first polypeptide comprises amino acid mutations of 356K and 394S, and the CH3 domain of the second polypeptide comprises amino acid mutations of 439E and 394S; or
   the CH3 domain of the first polypeptide comprises amino acid mutations of 356K and 405Y, and the CH3 domain of the second polypeptide comprises amino acid mutations of 439E and 405Y; or
   the CH3 domain of the first polypeptide comprises amino acid mutations of 356K and 409Q, and the CH3 domain of the second polypeptide comprises amino acid mutations of 439E and 409Q; or
   the CH3 domain of the first polypeptide comprises amino acid mutations of 356K and 411Y, and the CH3 domain of the second polypeptide comprises amino acid mutations of 439E and 411Y; or
iv) the CH3 domain of the first polypeptide comprises amino acid mutations of 356K and 349C, and the CH3 domain of the second polypeptide comprises amino acid mutations of 439E, 354C, and 351I; or
   the CH3 domain of the first polypeptide comprises amino acid mutations of 356K and 349C, and the CH3 domain of the second polypeptide comprises amino acid mutations of 439E, 354C, and 366A; or
   the CH3 domain of the first polypeptide comprises amino acid mutations of 356K and 349C, and the CH3 domain of the second polypeptide comprises amino acid mutations of 439E, 354C, and 368I;
   preferably,
      i) the CH3 domain of the first polypeptide comprises amino acid mutations of 356K and 349S, and the CH3 domain of the second polypeptide comprises amino acid mutations of 439E and 354Y; or
      ii) the CH3 domain of the first polypeptide comprises an amino acid mutation of 405T, and the CH3 domain of the second polypeptide comprises an amino acid mutation of 394F.

In some embodiments, provided is the aforementioned bispecific antibody, wherein:
1) the CH3 domain of the first polypeptide comprises amino acid mutations of 356K and 349S, and the CH3 domain of the second polypeptide comprises amino acid mutations of 439E and 354Y; or
2) the CH3 domain of the first polypeptide comprises an amino acid mutation of 405T, and the CH3 domain of the second polypeptide comprises an amino acid mutation of 394F; or
3) the CH3 domain of the first polypeptide comprises amino acid mutations of 356K and 351I, and the CH3 domain of the second polypeptide comprises amino acid mutations of 439E and 351I; or
4) the CH3 domain of the first polypeptide comprises amino acid mutations of 356K and 394S, and the CH3 domain of the second polypeptide comprises amino acid mutations of 439E and 394S; or
5) the CH3 domain of the first polypeptide comprises amino acid mutations of 356K and 411Y, and the CH3 domain of the second polypeptide comprises amino acid mutations of 439E and 411Y; or
6) the CH3 domain of the first polypeptide comprises an amino acid mutation of 349S, and the CH3 domain of the second polypeptide comprises an amino acid mutation of 364Y; or
7) the CH3 domain of the first polypeptide comprises amino acid mutations of 356K and 349C, and the CH3 domain of the second polypeptide comprises amino acid mutations of 439E, 354C, and 351I; or
8) the CH3 domain of the first polypeptide comprises an amino acid mutation of 349C, and the CH3 domain of the second polypeptide comprises amino acid mutations of 354C and 364Y; or
9) the CH3 domain of the first polypeptide comprises an amino acid mutation of 349C, and the CH3 domain of the second polypeptide comprises amino acid mutations of 354C and 364F.

In some embodiments, provided is the aforementioned bispecific antibody, wherein:
i) the CH3 domain of the first polypeptide comprises amino acid mutations of 356K and 349S, and the CH3 domain of the second polypeptide comprises amino acid mutations of 439E and 354Y; or
ii) the CH3 domain of the first polypeptide comprises an amino acid mutation of 405T, and the CH3 domain of the second polypeptide comprises an amino acid mutation of 394F.

In some embodiments, provided is the aforementioned bispecific antibody, wherein the CH3 domain of the first polypeptide comprises only amino acid mutations of 356K and 351I, and the CH3 domain of the second polypeptide comprises only amino acid mutations of 439E and 351I.

In some embodiments, provided is the aforementioned bispecific antibody, wherein the CH3 domain of the first polypeptide comprises only an amino acid mutation of 349S, and the CH3 domain of the second polypeptide comprises only an amino acid mutation of 364Y.

In some embodiments, provided is the aforementioned bispecific antibody, wherein the CH3 domain of the first polypeptide comprises only amino acid mutations of 356K and 349C, and the CH3 domain of the second polypeptide comprises only amino acid mutations of 439E, 354C, and 351I.

In some embodiments, provided is the aforementioned bispecific antibody, wherein the CH3 domain of the first polypeptide comprises only an amino acid mutation of 349C, and the CH3 domain of the second polypeptide comprises only amino acid mutations of 354C and 364Y. In some embodiments, provided is the aforementioned bispecific antibody, wherein the CH3 domains of the first polypeptide and the second polypeptide are derived from IgG1. In some embodiments, the CH3 domains of the first polypeptide and the second polypeptide are derived from human IgG1.

In some embodiments, provided is the aforementioned bispecific antibody, wherein the bispecific antibody comprises at least one replaced Fab comprising a Titin chain and an Obscurin chain that are capable of forming a dimer. In some embodiments, provided is the aforementioned bispecific antibody, wherein the replaced Fab is obtained by replacing the original CH1 and CL of a Fab with the Obscurin chain and the Titin chain, respectively, or the replaced Fab is obtained by replacing the original CH1 and CL of a Fab with the Titin chain and the Obscurin chain, respectively. In some embodiments, the Titin chain comprises the amino acid sequence set forth in SEQ ID NO: 6, and the Obscurin chain comprises the amino acid sequence set forth in SEQ ID NO: 5.

In another aspect, the present disclosure provides a pharmaceutical composition comprising the aforementioned bispecific antibody and one or more pharmaceutically acceptable carriers, diluents, or excipients.

In another aspect, the present disclosure provides an immunoconjugate comprising the aforementioned bispecific antibody and an effector, wherein the effector is conjugated to the bispecific antibody. In some embodiments, the effector is selected from the group consisting of an anti-tumor agent, an immunomodulator, a biological response modifier, a lectin, a cytotoxic drug, a chromophore, a fluorophore, a chemiluminescent compound, an enzyme, and a metal ion, and any combination thereof.

In another aspect, the present disclosure provides an isolated nucleic acid encoding the aforementioned bispecific antibody.

In another aspect, the present disclosure provides a vector comprising the aforementioned isolated nucleic acid.

In another aspect, the present disclosure provides a host cell comprising the aforementioned isolated nucleic acid.

In another aspect, the present disclosure provides a method for preparing the aforementioned bispecific antibody, comprising culturing the aforementioned host cell and recovering the bispecific antibody from the host cell culture.

In another aspect, the present disclosure provides a method for preparing the aforementioned bispecific antibody, comprising: (a) a step of altering a nucleic acid encoding amino acid residues that form an interface between the polypeptides, etc., (b) a step of culturing a host cell comprising the nucleic acid to express the polypeptides, (c) a step of recovering the polypeptides from the culture of the host cell, and (d) a step of incubating the polypeptides under reducing conditions and recovering the desired bispecific antibody.

### Combination with Isoelectric Point Alternation Techniques, etc.

In a further preferred embodiment of the present disclosure, an amino acid mutation that alters the isoelectric point (pI value) of a polypeptide can be introduced into a polypeptide of the present disclosure to effectively purify or prepare a polypeptide multimer comprising the first to fourth polypeptides of interest with higher purity (WO2007114325 and US20130171095). As an amino acid mutation that is introduced to promote the association of polypeptides, a method for hetero-associating polypeptides comprising 2 types of heavy chain constant regions by altering the CH3 domains of the heavy chain constant regions (which is described in Protein Eng. 1996 Jul;9(7):617-21, Protein Eng Des Sel. 2010 Apr;23(4):195-202, J Biol Chem. 2010 Jun 18;285(25):19637-46, WO2009080254, US20130195849, etc.), a method for promoting the association of a particular combination of a heavy chain and a light chain (which is described in WO2009080251, WO2009080252, WO2009080253, etc.), etc., may also be used.

### Combination with Other Constant Region and/or Variable Region Alteration Techniques

Non-limiting embodiments of the present disclosure include a combination with the following alteration technique: a technique for altering constant regions with the aim of enhancing binding to FcγR (WO2013047752).

Embodiments of other combinations of the present disclosure with other constant region alteration techniques include a combination with a technique for controlling binding to a complement. Any complement component can be used as a complement as long as the complement is a polypeptide that forms a complement cascade. Examples of preferred complements include a complement component of C1q, C1r, or C1s involved in the binding of opsonin. An Fc region whose binding activity to a complement is higher than that of a naturally occurring Fc region to the complement can be made by altering amino acids of the naturally occurring Fc region. Here, the naturally occurring Fc region refers to a human IgG1, IgG2, IgG3, or IgG4 Fc region. Whether the binding activity of an Fc region to a complement is higher than that of a naturally occurring Fc region to the complement can be appropriately determined using a known immunological method such as FACS or ELISA. The "alteration of amino acid(s)" or "amino acid alteration" of the Fc region includes altering to an amino acid sequence that is different from the amino acid sequence of a starting Fc region. Any Fc region can be used as a starting domain as long as modified and altered forms of the starting Fc region bind to a complement in a neutral pH range. Moreover, an Fc region to which an alteration has been added is used as a starting Fc region, and an Fc region to which a further alteration has been added may also be appropriately used as the Fc region of the present disclosure. The starting Fc region refers to the polypeptide itself, a composition comprising the starting Fc region, or an amino acid sequence encoding the starting Fc region. The starting Fc region may include a known IgG antibody Fc region produced by the recombination summarized in the section on antibodies. The origin of the starting Fc region is not particularly limited, and it can be obtained from an arbitrary organism of a non-human animal or a human. As arbitrary organisms, preferred and appropriate examples include organisms selected from the group consisting of mice, rats, guinea pigs, hamsters, gerbils, cats, rabbits, dogs, goats, sheep, cows, horses, camels, and non-human primates. In another embodiment, the starting Fc region may also be obtained from a cynomolgus monkey, a marmoset, a macaque, a chimpanzee, or a human. Preferably, the starting Fc region can be obtained from human IgG1, but is not limited to a particular IgG class. This means that a human IgG1, IgG2, IgG3, or IgG4 Fc region can be appropriately used as the starting Fc region. This also means that: in the present disclosure, an Fc region of any IgG class or subclass from the arbitrary organisms described above can be preferably used as the starting Fc region. Examples of variants or engineered models of naturally occurring IgGs are described in known literature (Curr.Opin.Biotechnol.(2009)20(6),685-91, Curr.Opin.Immunol.(2008)20(4),460-470, ProteinEng.Des.Sel.(2010)23(4),195-202, WO2009086320, WO2008092117, WO2007041635, and WO2006105338), but are not limited thereto.

The amino acid at any position can be altered as long as the amino acid alteration results in binding activity to a complement or improves binding activity for binding to a complement. When an antibody comprises a human IgG1 Fc region as a human Fc region, it preferably comprises an alteration that produces the effect of the binding to a complement being stronger than the binding activity of the starting Fc region of human IgG1. Examples of amino acids for altering the binding activity to a complement include amino acids of Fc regions with altered binding activity to C1q reported in Duncan et al. (Nature (1988)332,738-740), Tao et al. (J. Exp. Med. (1993)178,661-667), Brekke et al. (Eur. J. Immunol. (1994)24,2542-2547), Xu et al. (Immunol. (1993)150,152A), WO1994029351, WO2000042072, WO2011091078, etc.

Examples of such amino acids that can be altered to enhance the binding activity to C1q include at least one or more amino acids selected from the group consisting of positions 231-238 and positions 318-337 (EU numbering). One non-limiting example of such amino acids includes at least one or more amino acids selected from the group consisting of position 235, position 237, position 318, position 320, position 322, position 324, position 327, position 331, and position 333. These amino acids can be altered to enhance the binding of an IgG-type immunoglobulin Fc region to a complement.

Other combination embodiments of the present disclosure with other constant region alteration techniques include combinations with antibody alteration techniques such as an Fc alteration technique for enhancing binding to FcRn at acidic pH (WO2002060919, WO2004035752, and WO2000042072), an Fc alteration technique for enhancing binding to FcRn at neutral pH (WO2011122011 and WO2012133782), a technique for enhancing selective binding to inhibitory Fcγ receptors (WO2012115241 and WO2013125667), a technique for enhancing selective binding to active Fcγ receptors (ADCC activity enhancement technique) (WO2013002362), and a technique for reducing binding activity to rheumatoid factors (WO2013046704), and Fc mutation combinations for modulating effector functions (Liu R, Oldham R J, Teal E, et al. Fc-engineering for modulated effector functions-improving antibodies for cancer treatment[J]. Antibodies, 2020, 9(4): 64).

The C-terminus of the Fc region may be an intact C-terminus ending with the amino acid residues PGK or a shortened C-terminus; for example, in the shortened C-terminus, one or two C-terminal amino acid residues have been removed. In one preferred aspect, the C-terminus of the heavy chain is a shortened C-terminus ending with PG. Thus, in some embodiments, a composition of intact antibodies may comprise an antibody population with all K447 residues and/or G446 + K447 residues removed. In some embodiments, a composition of intact antibodies may comprise an antibody population with K447 residues and/or G446 + K447 residues not removed. In some embodiments, a composition of intact antibodies comprises an antibody population having a mixture of antibodies with and without a K447 residue and/or G446 + K447 residues.

Non-limiting combination embodiments of the present disclosure with variable region alteration techniques include combinations with alteration techniques such as pH-dependent antibodies (WO2009125825) and calcium-dependent antibodies (WO2012073992).

### Alteration of Nucleic Acid

In another embodiment of the preparation method of the present disclosure, the present disclosure provides a preparation method for a heteromultimer (e.g., a multispecific antibody). The method is a preparation method for a heteromultimer in which amino acid residues that form an interface between polypeptides (e.g., at least one amino acid residue selected from the group consisting of positions 347, 349, 351, 354, 356, 357, 364, 366, 368, 394, 397, 399, 405, 407, 409, 411, and 439 (EU numbering)) are mutated to control the dissociation and/or association between the polypeptides. The preparation method comprises the following steps: (a) a step of altering a nucleic acid encoding the amino acid residues that form the interface between the polypeptides, etc., according to the original nucleic acid to control the dissociation and association between the polypeptides, (b) a step of culturing a host cell comprising the nucleic acid to express the polypeptides, (c) a step of recovering the polypeptides from the culture of the host cell, and (d) a step of incubating the polypeptides under reducing conditions and recovering the desired heteromultimer.

Moreover, a method comprising a step of utilizing the above dissociation and/or association control method of the present disclosure to alter a nucleic acid encoding the amino acid residues that form the interface between the polypeptides according to the original nucleic acid to inhibit the association between the polypeptides is also one of the preferred embodiments of the above preparation method of the present disclosure.

In the above method of the present disclosure, "altering a nucleic acid" refers to altering the nucleic acid to correspond to amino acid residues that are introduced by the "alteration" in the present disclosure. More specifically, it refers to altering the nucleic acid encoding the original (before the alteration) amino acid residues to a nucleic acid encoding amino acid residues that are introduced by the alteration. Generally, it refers to subjecting the original nucleic acid to gene manipulation or mutagenesis treatment for the insertion, deletion, or substitution of at least one base to form a codon encoding the amino acid residues of interest. That is, the codon encoding the original amino acid residues is substituted with a codon encoding the amino acid residues that are introduced by the alteration. Such nucleic acid alteration can be appropriately carried out using a technique known to those skilled in the art, such as site-directed mutagenesis or PCR mutagenesis. Moreover, the nucleic acid of the present disclosure is generally carried by (or inserted into) an appropriate vector and introduced into a host cell. The vector is not particularly limited as long as it stably retains the inserted nucleic acid.

The host cell is not particularly limited, and various host cells may be used according to the purpose.

For the secretion of a polypeptide expressed in a host cell into the lumen of the endoplasmic reticulum, into the periplasmic space of cells, or into the extracellular environment, appropriate secretion signals may be combined with the polypeptide of interest. These signals may be endogenous or heterologous to the polypeptide of interest. As for the recovery of the polypeptides in the preparation method described above, when the polypeptides of the present disclosure are secreted into the culture medium, the culture medium is recovered. When the polypeptides of the present disclosure are produced within cells, first, the cells are lysed, and then, the polypeptides are recovered.

When the polypeptides of the present disclosure are recovered from a recombinant cell culture for purification, known methods, including ammonium sulfate or ethanol precipitation, acid extraction, anion or cation exchange chromatography, phosphocellulose chromatography, hydrophobic interaction chromatography, affinity chromatography, hydroxyapatite chromatography, and lectin chromatography, can be used.

### Recombination Methods

Heteromultimers (e.g., multispecific antibodies) can be produced using recombinant methods. For these methods, one or more isolated nucleic acids encoding the heteromultimers are provided.

In one embodiment, the present disclosure provides an isolated nucleic acid encoding the aforementioned heteromultimer. Such nucleic acids may each independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In one embodiment, provided is a method for preparing a heteromultimer, wherein the method comprises culturing a host cell comprising a nucleic acid encoding the heteromultimer, as provided above, under conditions suitable for expression, and optionally recovering the protein from the host cell (or host cell culture medium).

For recombinant production of the heteromultimer, the nucleic acid encoding the protein is isolated and inserted into one or more vectors for further cloning and/or expression in the host cell. Such nucleic acids can be readily isolated and sequenced using conventional procedures, or produced by recombination methods, or obtained by chemical synthesis.

Suitable host cells for cloning or expressing a vector encoding the heteromultimer include the prokaryotic or eukaryotic cells described herein. For example, it can be produced in bacteria, particularly when glycosylation and Fc effector functions are not needed. After expression, it can be isolated from the bacterial cell paste in a soluble fraction and can be further purified.

In addition to prokaryotes, eukaryotic microorganisms such as filamentous fungi or yeast are suitable cloning or expression hosts for vectors encoding antibodies, including fungal and yeast strains. Suitable host cells for antibody expression may also be derived from multicellular organisms (invertebrates and vertebrates); examples of invertebrate cells include plant and insect cells. Many baculovirus strains have been identified, and they can be used in combination with insect cells, particularly for transfection of Spodoptera frugiperda cells; plant cell cultures may also be used as hosts (see, e.g., US5959177, US 6040498, US6420548, US 7125978, and US6417429); vertebrate cells can also be used as hosts, e.g., mammalian cell lines adapted for growth in a suspension. Other examples of suitable mammalian host cell lines include an SV40-transformed monkey kidney CVl line (COS-7); a human embryonic kidney line (293 or 293T cells); baby hamster kidney cells (BHK); mouse Sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines, such as Y0, NS0, and Sp2/0. For reviews of certain mammalian host cell lines suitable for antibody production, see, e.g., Yazaki, P. and Wu, A.M., Methods in Molecular Biology, Vol. 248, Lo, B.K.C. (eds.), Humana Press, Totowa, NJ (2004), pp. 255-268.

### Assays

The physical/chemical characteristics and/or biological activity of the heteromultimers provided herein can be identified, screened, or characterized through a variety of assays known in the art. In one aspect, the activity of the heteromultimers of the present disclosure is tested, e.g., by known methods such as flow cytometry, ELISA, western blot, SEC, IEC, and mass spectrometry.

### Treatment Methods and Routes of Administration

Any of the heteromultimers provided herein can be used in treatment methods. In yet another aspect, the present disclosure provides use of the heteromultimer in the manufacture or preparation of a medicament. In some embodiments, in one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the heteromultimer, e.g., for use in any of the above pharmaceutical uses or treatment methods. In one embodiment, the pharmaceutical composition comprises any of the heteromultimers provided herein and a pharmaceutically acceptable carrier. In another embodiment, the pharmaceutical composition further comprises at least one additional therapeutic agent.

The heteromultimers of the present disclosure (and any additional therapeutic agent) may be administered by any suitable means, including parenteral, intrapulmonary, and intranasal administration, and if local treatment is needed, they are administered intralesionally. Parenteral infusion includes intramuscular, intravenous, intra-arterial, intraperitoneal, or subcutaneous administration. Administration may be performed via any suitable route, e.g., by injection, such as intravenous or subcutaneous injection, depending in part on whether the administration is short-term or long-term. Various administration schedules are contemplated herein, including but not limited to a single administration or multiple administrations at multiple time points, bolus injection administration, and pulse infusion.

The heteromultimers of the present disclosure will be formulated, administered, and applied in a manner consistent with Good Medical Practice. Factors considered in this context include the specific disorder being treated, the specific mammal being treated, the clinical state of the individual patient, the cause of the disorder, the site to which the agent is delivered, the administration method, the timing of administration, and other factors known to medical practitioners. The heteromultimers can be formulated with or without one or more agents currently used for preventing or treating the disorder. The effective amount of such additional agents depends on the amount present in the pharmaceutical composition, the type of the disorder or treatment, and other factors. These are generally used at the same dosage and via the same route of administration as described herein, or at about 1% to 99% of the dosage described herein, or at other dosages, and via any route empirically/clinically determined to be appropriate.

For the prevention or treatment of a disease, the appropriate dosage of the heteromultimers of the present disclosure (when used alone or in combination with one or more additional therapeutic agents) will depend on the type of the disease to be treated, the type of the therapeutic molecule, the severity and course of the disease, the purpose of administration (prophylactic or therapeutic), the previous treatment, the clinical history and response to the therapeutic molecule of the patient, and the discretion of the attending physician. The therapeutic molecule is suitably administered to a patient in one treatment or a series of treatments.

### Product

In another aspect of the present disclosure, provided is a product comprising materials useful for the treatment, prevention, and/or diagnosis of the disorders described above. The product comprises a container and a label or package insert on or associated with the container. Suitable containers include, for example, bottles, vials, syringes, IV solution bags, etc. The container may be formed of a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is a heteromultimer of the present disclosure. The label or package insert indicates that the composition is used to treat the selected condition. In addition, the product may comprise: (a) a first container containing a composition, wherein the composition comprises a heteromultimer of the present disclosure, and (b) a second container containing a composition, wherein the composition comprises an additional cytotoxic agent or a therapeutic agent of other aspects. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further comprise other required materials, including other buffers, diluents, filters, needles, and syringes. Although the antibodies used in the examples target specific antigens, those skilled in the art, in light of the teachings of the present disclosure, can understand that the technical effects are achieved independently of particular CDR sequences and independently of particular antigen sequences, but with the benefit of mutation of Fc region amino acids (for example, at least one amino acid is selected from the group consisting of positions 347, 349, 351, 354, 356, 357, 364, 366, 368, 394, 397, 399, 405, 407, 409, 411, and 439), to promote the formation of bispecific antibodies.

### Examples

The following examples further describe the present disclosure, and should not be construed as limiting the scope of the present disclosure. The examples of the present disclosure do not include detailed descriptions of conventional methods. Experimental methods without specific conditions indicated are generally performed under conventional conditions, because such methods are well known to those of ordinary skill in the art and are described in many publications, such as Molecular Cloning published by Cold Spring Harbor Laboratory (Green M R, Sambrook J. Molecular cloning. A Laboratory Manual 4th, 2012) or Antibody Engineering: Methods and Protocols of Springer Protocols (Antibody Engineering: Methods and Protocols. Humana Press, 2018); or conditions recommended by the manufacturers of the starting materials or commercial products are used. Reagent materials without specific sources indicated are commercially available.

### Example 1.1: Source and Preparation of Anti-CTLA4 Monoclonal Antibody Ipilimumab

The amino acid sequences of the heavy and light chains of ipilimumab were from the Drugbank database (Accession Number: DB06186).

The amino acid sequence of the heavy chain of ipilimumab is shown below (SEQ ID NO: 1):

QVQLVESGGGVVQPGRSLRLSCAASGFTFSSYTMHWVRQAPGKGLEWVTFISY
DGNNKYYADSVKGRFTISRDNSKNTLYLQMNSLRAEDTAIYYCARTGWLGPFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK

The amino acid sequence of the light chain of ipilimumab is shown below (SEQ ID NO: 2):

EIVLTQSPGTLSLSPGERATLSCRASQSVGSSYLAWYQQKPGQAPRLLIYGAFSR
ATGIPDRFSGSGSGTDFTLTISRLEPEDFAVYYCQQYGSSPWTFGQGTKVEIKRTV

AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

Note: The italicized regions are the constant regions of the antibody.

The DNAs encoding the above variable regions were synthesized by Suzhou Genewiz Biological Technology Co., Ltd. The heavy chain variable region of ipilimumab (ipilimumab-VH) and its light chain variable region (ipilimumab-VL) were ligated to DNAs encoding the human IgG1 heavy chain constant region and the human kappa light chain constant region, respectively, to construct full-length heavy and light chain genes of ipilimumab, designated ipilimumab-HC and ipilimumab-LC, respectively. The above heavy and light chain genes were cloned into two pcDNA3.4 expression vectors, respectively, and the expression vectors were simultaneously transfected into FreeStyle^{™} 293-F cells (Thermo Fisher Scientific, Cat. No. R79007) using PEI (polyethylenimine) to express the antibody. The 293-F cells were cultured in a serum-free culture medium for 5 days. The cell supernatant was collected, and the antibody was purified by protein A affinity chromatography.

The purification procedure is described below: Impurities in the cell culture supernatant were removed by high-speed centrifugation, and the antibody in the supernatant was captured by MabSelect SuRe (Cytiva, Cat. No. 17543801) affinity chromatography. The MabSelect Sure affinity column was washed with 0.2 M NaOH, then rinsed with purified water, and then equilibrated with PBS. The supernatant was allowed to flow through the affinity column, and the affinity column was washed with PBS until A280 dropped to baseline. The target protein was eluted with a 0.1 M acetic acid buffer (pH 3.5), and the antibody solution was neutralized with 1 M Tris-HCl (pH 8.0). The antibody solution was properly concentrated by ultrafiltration. Then, the antibody was further purified using a HiLoad Superdex 200 gel chromatography column (Cytiva, Cat. No. 28989335). The antibody concentration was determined by ultraviolet spectrophotometry, and the antibody solution was sterilized by filtration and stored in a refrigerator (4 °C). This method was used to purify related monoclonal antibodies, and can also be used to purify other antibodies or recombinant proteins in the present disclosure.

### Example 1.2: Source and Preparation of Anti-CCR8 Monoclonal Antibody CP11

CP11 was a humanized anti-human CCR8 monoclonal antibody, and the sequences of its heavy and light chain variable regions were from Patent No. WO2023208182A1. The humanized heavy and light chain variable regions were designated CP11-VH and CP11-VL (SEQ ID NOs: 13 and 14), respectively.
CP11-VH (SEQ ID NO: 13) 
EVTLKESGPVLVKPTETLTLTCTVSGFSLTRYGINWIRQPPGKGLEWLGVIWTGG
DTNYNSALKSRLTISKDKSKSQVVLTMTNMDPVDTATYYCAREGVVNYYGSRY
SFFDVWGQGTTVTVSS
CP11-VL (SEQ ID NO: 14)

DIQMTQSPSSLSASVGDRVTITCRASSSVSYMHWYQQKPGKSPKPWIYATSNLA
SGVPSRFSGSGSGTDYTLTISSLQPEDFATYYCQQWSSNPYTFGGGTKVEIK

The DNAs encoding the above humanized heavy and light chain variable regions were synthesized by Suzhou Genewiz Biological Technology Co., Ltd. The synthesized humanized heavy chain variable region was ligated to the human IgG1 heavy chain constant region to obtain a full-length humanized heavy chain gene, designated CP11-HC (SEQ ID NO: 3). The humanized light chain variable region was ligated to the human kappa chain constant region to obtain a full-length humanized light chain gene, designated CP11-LC (SEQ ID NO: 4). The CP11-HC and CP11-LC genes were constructed into pcDNA3.4 expression vectors, respectively. The monoclonal antibody CP11 was prepared according to the expression and purification methods described in Example 1.

The amino acid sequence of the heavy chain of CP11 is shown below (SEQ ID NO: 3):

EVTLKESGPVLVKPTETLTLTCTVSGFSLTRYGINWIRQPPGKGLEWLGVIWTGG
DTNYNSALKSRLTISKDKSKSQVVLTMTNMDPVDTATYYCAREGVVNYYGSRY
SFFDVWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

The amino acid sequence of the light chain of CP11 is shown below (SEQ ID NO: 4):

DIQMTQSPSSLSASVGDRVTITCRASSSVSYMHWYQQKPGKSPKPWIYATSNLA
SGVPSRFSGSGSGTDYTLTISSLQPEDFATYYCQQWSSNPYTFGGGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

Note: The italicized regions are the constant regions of the antibody.

### Example 1.3: Preparation of Anti-CTLA4 and CCR8 Bispecific Antibody

To examine the recombination efficiency of a bispecific antibody, one anti-CTLA4 and CCR8 bispecific antibody was prepared here. The technique employed for the bispecific antibody was derived from Patent No. WO2022237882A1. The heavy chain CH1 domain of ipilimumab was replaced with a domain from Obscurin, and the light chain CL domain of ipilimumab was replaced with a domain from Titin. This approach was intended to prevent incorrect pairing between heavy and light chains during the preparation of the bispecific antibody. In addition, the classical Knob-into-Hole method was used to facilitate heterodimerization between heavy chains. The bispecific antibody (designated CP11-Ip-F31) contained four different polypeptide chains. Their amino acid sequences are shown below:
The amino acid sequence of the Obscurin domain (SEQ ID NO: 5): 
SGAPRFLTCPKASVVSVGKDATLSSQIVGNPFPQVSWEKDKQPVTAGVRFRLAQ
DGDLYRLKILDLQLSDSGQYVSRARNAHGEAFACLGLQVDAEA
The amino acid sequence of the Titin domain (SEQ ID NO: 6): 
GIPPKIECLPIDISIDEGKVLTVASCFTGEPTPEVTWSTGGRKIHSQEQGRFHIENT
DDSTTLTIKDVQKQDGGLYTLTLRNEFGSDSATVNIHIRSI
The amino acid sequence of the anti-CTLA4 arm heavy chain of CP11-Ip-F31 is shown below (SEQ ID NO: 7): 
QVQLVESGGGVVQPGRSLRLSCAASGFTFSSYTMHWVRQAPGKGLEWVTFISY
DGNNKYYADSVKGRFTISRDNSKNTLYLQMNSLRAEDTAIYYCARTGWLGPFD
YWGQGTLVTVSSGGGGSSGAPRFLTCPKASVVSVGKDATLSSQIVGNPFPQVSW
EKDKQPVTAGVRFRLAQDGDLYRLKILDLQLSDSGQYVSRARNAHGEAFACLG
LQVDAEADKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVCTLPPSRDELTKNQVSLSCAVKGFYP
SDIAVEWESNGQPENNYKTTPPVLDSDGSFFLVSKLTVDKSRWQQGNVFSCSVM
HEALHNHYTQKSLSLSPGK
The amino acid sequence of the anti-CTLA4 arm light chain of CP11-Ip-F31 is shown below (SEQ ID NO: 8): 
EIVLTQSPGTLSLSPGERATLSCRASQSVGSSYLAWYQQKPGQAPRLLIYGAFSR
ATGIPDRFSGSGSGTDFTLTISRLEPEDFAVYYCQQYGSSPWTFGQGTKVEIKGG
GGSGIPPKIECLPIDISIDEGKVLTVASCFTGEPTPEVTWSTGGRKIHSQEQGRFHI
ENTDDSTTLTIKDVQKQDGGLYTLTLRNEFGSDSATVNIHIRSI
The amino acid sequence of the anti-CCR8 arm heavy chain of CP11-Ip-F31 is shown below (SEQ ID NO: 9): 
EVTLKESGPVLVKPTETLTLTCTVSGFSLTRYGINWIRQPPGKGLEWLGVIWTGG
DTNYNSALKSRLTISKDKSKSQVVLTMTNMDPVDTATYYCAREGVVNYYGSRY
SFFDVWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
 
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPCRDELTKNQVSLWCLVK
GFYPSDIAVEWESNGQPENNYKTTPPVLDSDGSFFLYSKLTVDKSRWQQGNVFS
CSVMHEALHNHYTQKSLSLSPGK
The amino acid sequence of the anti-CCR8 arm light chain of CP11-Ip-F31 is shown below (SEQ ID NO: 10):

DIQMTQSPSSLSASVGDRVTITCRASSSVSYMHWYQQKPGKSPKPWIYATSNLA
SGVPSRFSGSGSGTDYTLTISSLQPEDFATYYCQQWSSNPYTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

The bispecific antibody CP11-Ip-F31 was prepared according to the expression and purification methods described in the above examples. CP11-Ip-F31 was subjected to fine purification by HPLC-SEC to remove molecular fragments that were not correctly assembled, and finally, a sample with SEC purity exceeding 95% was obtained. The bispecific antibody sample was sterilized by filtration using a 0.2-µm filter head, and the concentration of the antibody was determined using a NanoDrop microspectrophotometer.

### Example 1.4: Preparation of Ipilimumab and CP11 Mutants

Gene mutations were introduced into the coding regions for the heavy chain CH3 domains of ipilimumab and CP11 by site-directed mutagenesis to mutate the amino acid residues at particular positions into particular amino acids. The positions of amino acid residues were identified using the Eu numbering scheme. Mutants of ipilimumab and CP11 were prepared according to the expression and purification methods described in Example 1. The antibody samples obtained were sterilized by filtration using a 0.2-µm filter head, and the concentrations of the antibodies were determined using a NanoDrop microspectrophotometer. The rule for naming the antibody mutants is described below: Illustratively, ipilimumab-D356K+T366A means that the D at position 356 of ipilimumab was mutated into K, and the T at position 366 was mutated into A. The rule applies to other mutants. See Table 2.

**Table 2. Parental monoclonal antibody mutations and corresponding recombination reactions**

| **No.** | **Ipilimumab CH3 mutation(s)** | **CP11 CH3 mutation(s)** | **Recombination reaction** |
|---|---|---|---|
| 1 | F405L | K409R | Ipi-F405L*CP11-K409R |
| 2 | K409R | F405L | Ipi-K409R*CP11-F405L |
| 3 | D356K | K439E | Ipi-D356K*CP11-K439E |
| 4 | K439E | D356K | Ipi-K439E*CP11-D356K |
| 5 | D356K+Y349L | K439E+Y349L | Ipi-D356K+Y349L*CP11-K439E+Y349L |
| 6 | D356K+Y349F | K439E+Y349F | Ipi-D356K+Y349F*CP 11-K439E+Y349F |
| 7 | D356K+L351C | K439E+L351C | Ipi-D356K+L351C*CP11-K439E+L351C |
| 8 | D356K+L351V | K439E+L351V | Ipi-D356K+L351V*CP11-K439E+L351V |
| 9 | D356K+L351T | K439E+L351T | Ipi-D356K+L351T*CP11-K439E+L351T |
| 10 | D356K+L351I | K439E+L351I | Ipi-D356K+L351I*CP11-K439E+L351I |
| 11 | D356K+L351F | K439E+L351F | Ipi-D356K+L351F*CP11-K439E+L351F |
| 12 | D356K+S364A | K439E+S364A | Ipi-D356K+S364A*CP11-K439E+S364A |
| 13 | D356K+S364V | K439E+S364V | Ipi-D356K+S364V*CP11-K439E+S364V |
| 14 | D356K+S364T | K439E+S364T | Ipi-D356K+S364T*CP11-K439E+S364T |
| 15 | D356K+S364L | K439E+S364L | Ipi-D356K+S364L*CP11-K439E+S364L |
| 16 | D356K+T366G | K439E+T366G | Ipi-D356K+T366G*CP11-K439E+T366G |
| 17 | D356K+T366S | K439E+T366S | Ipi-D356K+T366S*CP11-K439E+T366S |
| 18 | D356K+T366A | K439E+T366A | Ipi-D356K+T366A*CP11-K439E+T366A |
| 19 | D356K+T366V | K439E+T366V | Ipi-D356K+T366V*CP11-K439E+T366V |
| 20 | D356K+T366L | K439E+T366L | Ipi-D356K+T366L*CP11-K439E+T366L |
| 21 | D356K+T366H | K439E+T366H | Ipi-D356K+T366H*CP11-K439E+T366H |
| 22 | D356K+L368V | K439E+L368V | Ipi-D356K+L368V*CP11-K439E+L368V |
| 23 | D356K+L368I | K439E+L368I | Ipi-D356K+L368I*CP11-K439E+L368I |
| 24 | D356K+L368M | K439E+L368M | Ipi-D356K+L368M*CP11-K439E+L368M |
| 25 | D356K+T394A | K439E+T394A | Ipi-D356K+T394A*CP11-K439E+T394A |
| 26 | D356K+T394S | K439E+T394S | Ipi-D356K+T394S*CP11-K439E+T394S |
| 27 | D356K+T394C | K439E+T394C | Ipi-D356K+T394C*CP11-K439E+T394C |
| 28 | D356K+T394V | K439E+T394V | Ipi-D356K+T394V*CP11-K439E+T394V |
| 29 | D356K+T394N | K439E+T394N | Ipi-D356K+T394N*CP 11-K439E+T394N |
| 30 | D356K+V397T | K439E+V397T | Ipi-D356K+V397T*CP11-K439E+V397T |
| 31 | D356K+V397I | K439E+V397I | Ipi-D356K+V397I*CP11-K439E+V397I |
| 32 | D356K+V397L | K439E+V397L | Ipi-D356K+V397L*CP11-K439E+V397L |
| 33 | D356K+V397M | K439E+V397M | Ipi-D356K+V397M*CP11-K439E+V397M |
| 34 | D356K+F405L | K439E+F405L | Ipi-D356K+F405L*CP11-K439E+F405L |
| 35 | D356K+F405Y | K439E+F405Y | Ipi-D356K+F405Y*CP11-K439E+F405Y |
| 36 | D356K+Y407C | K439E+Y407C | Ipi-D356K+Y407C*CP11-K439E+Y407C |
| 37 | D356K+Y407V | K439E+Y407V | Ipi-D356K+Y407V*CP11-K439E+Y407V |
| 38 | D356K+Y407L | K439E+Y407L | Ipi - D356K +Y407L*CP11-K439E+Y407L |
| 39 | D356K+Y407H | K439E+Y407H | Ipi-D356K+Y407H*CP11-K439E+Y407H |
| 40 | D356K+K409Q | K439E+K409Q | Ipi-D356K+K409Q* CP11-K439E+K409Q |
| 41 | D356K+K409R | K439E+K409R | Ipi-D356K+K409R*CP11-K439E+K409R |
| 42 | D356K+T411N | K439E+T411N | Ipi-D356K+T411N*CP11-K439E+T411N |
| 43 | D356K+T411Y | K439E+T411Y | Ipi-D356K+T411Y*CP11-K439E+T411Y |
| 44 | D356K+Y349S | K439E+S354Y | Ipi-D356K+Y349S*CP11-K439E+S354Y |
| 45 | D356K+Y349C | K439E+S354C | Ipi-D356K+Y349C*CP11-K439E+S354C |
| 46 | D356K+Y349C | K439E+E357C | Ipi-D356K+Y349C*CP11-K439E+E357C |
| 47 | D356K+F405T | K439E+T394F | Ipi-D356K+F405T*CP11-K439E+T394F |
| 48 | D356K+Y349C | K439E+S354C+L351I | Ipi-D356K+Y349C*CP11-K439E+S354C+L351I |
| 49 | D356K+Y349C | K439E+S354C+T366A | Ipi-D356K+Y349C*CP11-K439E+S354C+T366A |
| 50 | D356K+Y349C | K439E+S354C+L368I | Ipi-D356K+Y349C*CP11-K439E+S354C+L368I |
| 51 | Y349C | S354C+T366A | Ipi-Y349C*CP11-S354C+T366A |
| 52 | Y349C | S354C+L368I | Ipi-Y349C*CP11-S354C+L368I |
| 53 | Y349C | S354C+F405Y | Ipi-Y349C*CP11-S354C+F405Y |
| 54 | Y349C | E357C | Ipi-Y349C*CP11-E357C |
| 55 | F405T | T394F | Ipi-F405T*CP11-T394F |
| 56 | Y349A | S354F | Ipi-Y349A*CP11-S354F |
| 57 | Y349S | S354F | Ipi-Y349S*CP11-S354F |
| 58 | Y349V | S354F | Ipi-Y349V*CP11-S354F |
| 59 | Y349T | S354F | Ipi-Y349T*CP11-S354F |
| 60 | Y349A | S354Y | Ipi-Y349A*CP11-S354Y |
| 61 | Y349G | S354W | Ipi-Y349G*CP11-S354W |
| 62 | Y349A | S354W | Ipi-Y349A*CP11-S354W |
| 63 | Y349S | S354W | Ipi-Y349S*CP11-S354W |
| 64 | Y349S | S364Y | Ipi-Y349S*CP11-S364Y |
| 65 | Y349C | S354C+S364Y | Ipi-Y349C*CP11-S354C+S364Y |
| 66 | Y349C | S354C+S364F | Ipi-Y349C*CP11-S354C+S364F |
| 67 | D356K+D399K | K439E+K409D | Ipi-D356K+D399K*CP11-K439E+K409D |
| 68 | D356K+K409D | K439E+D399K | Ipi-D356K+K409D*CP11-K439E+D399K |

| | | | |
|---|---|---|---|
| Note: * means that a recombination reaction was performed between the two monoclonal antibodies. | | | |

### Example 1.5: Recombination Method for Anti-CTLA4 and CCR8 Antibodies

1700 mg of 2-MEA (Sigma-Aldrich, Cat. No./specification: 30078/100G) was weighed out and dissolved in 20 mL of PBS, and the pH was adjusted to 7.4 with 1 M NaOH. The amount-of-substance concentration of 2-MEA in this solution was about 750 mM. The concentrations of the ipilimumab and CP11 mutants prepared in the above examples were adjusted to 1.5 mg/mL with PBS. 0.9 mL of an ipilimumab mutant was mixed well with 0.9 mL of a corresponding CP11 mutant, and 0.2 mL of the 2-MEA mother liquor was then added to and mixed well with the above antibody mixture. The mixture (containing the ipilimumab mutant, the CP11 mutant, and 2-MEA) was incubated in a water bath at 37 °C for 2.5 h. The mixture was transferred to Slide-A-Lyzer^{™} G3 Dialysis Cassettes (Thermo Fisher Scientific, Cat. No. A52971) and dialyzed against 5 L of PBS at room temperature. After 5 h, the PBS was refreshed, and the mixture was dialyzed in a cool store (4 °C). Samples were collected the following morning, and the concentrations were re-determined. The physicochemical properties of each sample and the corresponding parental monoclonal antibodies were assessed.

### Example 1.6: Recombination Efficiency of Anti-CTLA4 and CCR8 Bispecific Antibodies

The full-length human CCR8 gene was cloned into a pCDH lentiviral expression vector (System Biosciences, Cat. No. CD510B-1) with a puromycin resistance gene, and HEK293T cells (ATCC, Cat. No. CRL-11268) were co-transfected with pCDH-CCR8 and a lentiviral packaging vector to produce viral particles. CHO-K1 cells (ATCC, CCL-61) were infected with the HEK293T cell supernatant containing viral particles. After 48 h, the culture medium was refreshed, and puromycin was added for pressure screening. After the cells grew to a certain density, the expression of CCR8 on the cell surface was measured by flow cytometry. A monoclonal cell strain overexpressing CCR8 was further obtained by limiting dilution and designated CHOK1-CCR8. CHOK1-CCR8 cells were cultured with DME/F12 (containing 10% FBS + 10 µg/mL puromycin) in a thermostatic incubator at 37 °C with 5% CO₂.

The full-length amino acid sequence of human CCR8 is shown below (SEQ ID NO: 11):

MDYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVV
SGFYYIGFYSSMFFITLMSVDRYLAVVHAVYALKVRTIRMGTTLCLAVWLTAIM
ATIPLLVFYQVASEDGVLQCYSFYNQQTLKWKIFTNFKMNILGLLIPFTIFMFCYI
KILHQLKRCQNHNKTKAIRLVLIVVIASLLFWVPFNVVLFLTSLHSMHILDGCSIS
QQLTYATHVTEIISFTHCCVNPVIYAFVGEKFKKHLSEIFQKSCSQIFNYLGRQMP
RESCEKSSSCQQHSSRSSSVDYIL

The gene encoding the extracellular segment of human CTLA4 (containing the signal peptide) was ligated to the gene encoding the human IgG1 Fc segment, and the fusion protein gene was cloned into an expression vector. An Fc-bearing CTLA4 fusion protein was prepared according to the expression and purification methods described in the above examples and designated CTLA4-Fc. The antibody sample obtained was sterilized by filtration using a 0.2-µm filter head, and the concentration of CTLA4-Fc was determined using a NanoDrop microspectrophotometer. CTLA4-Fc was labeled with an Alexa Fluor^{™} 488 protein labeling kit (Thermo Fisher Scientific, Cat. No. A10235), and the fluorescently labeled CTLA4-Fc protein, designated CTLA4-Fc-A488, was stored in a refrigerator at 4 °C, protected from light.

The amino acid sequence of CTLA4-Fc is shown below (SEQ ID NO: 12):

MACLGFQRHKAQLNLATRTWPCTLLFFLLFIPVFCKAMHVAQPAVVLASSRGIA
SFVCEYASPGKATEVRVTVLRQADSQVTEVCAATYMMGNELTFLDDSICTGTSS
GNQVNLTIQGLRAMDTGLYICKVELMYPPPYYLGIGNGTQIYVIDPEPCPDSDFG
GGGSDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLSLS
PGK

Note: The Fc domain is italicized.

CHOK1-CCR8 cells were digested with 0.25% tryspin-EDTA, so that they stopped adhering to the wall. After 5 min of centrifugation at 300 g, the cells were collected, washed with PBS containing 1% BSA, resuspended, counted, and then transferred to a 96-well round-bottom plate at 100,000 cells/well. Recombination reaction products, the bispecific antibody CP11-Ipi-F31, and monoclonal antibody mixtures were serially diluted 5-fold with PBS containing 1% BSA in a 96-well plate. The serially diluted antibodies were transferred to the above 96-well round-bottom plate containing CHOK1-CCR8 cells, with the final concentrations of the samples being 200 nM, 40 nM, 8 nM, 1.6 nM, 0.32 nM, 0.064 nM, 0.0128 nM, and 0.00256 nM. The cells and the antibody solutions were well mixed using a multi-channel pipette, and the plate was incubated in a refrigerator at 4 °C for 1 h. The 96-well round-bottom plate was centrifuged at 300 g for 5 min. After the supernatant was discarded, the cells were washed twice with PBS, and 100 µL of 3 µg/mL CTLA4-Fc-A488 (prepared in PBS containing 1% BSA) was added to each well. Then, the plate was incubated in a dark place at 4 °C for 30 min. The 96-well round-bottom plate was centrifuged at 300 g for 5 min. After the supernatant was discarded, the cells were washed twice with PBS and fixed with Fix Buffer I (BD Biosciences, Cat. No. 557870) at room temperature for 5 min. The 96-well round-bottom plate was centrifuged at 300 g for 5 min. After the supernatant was discarded, the cells were washed twice with PBS, and the fluorescence intensity of the cells was measured on a FACS Canto II. The flow cytometry data were analyzed using Flowjo 10.9, and the antibody concentration and the fluorescence intensity of the cells were processed and plotted using Graphpad Prism 10.0.

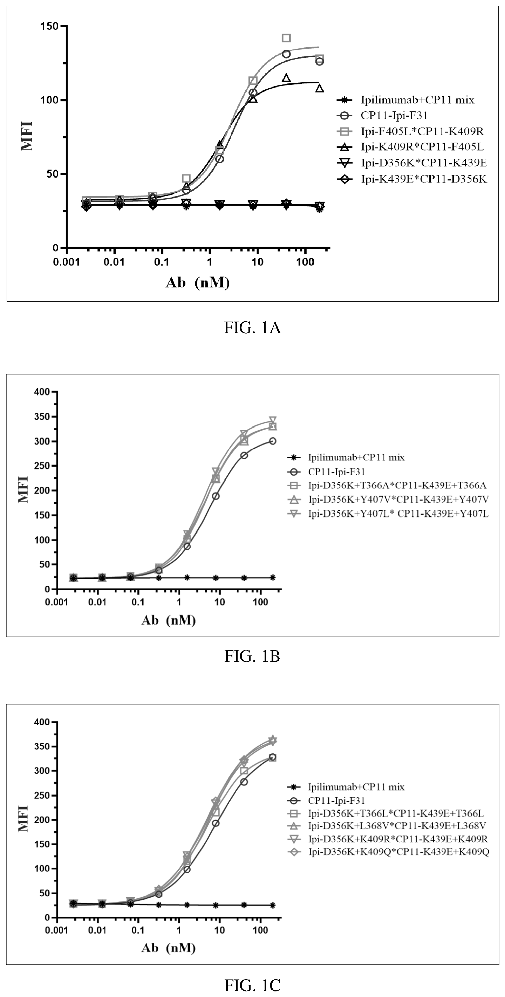

In FIGs. 1A to 1I, the Top values represent the high plateaus of the fitted curves, and a larger Top value indicates stronger binding activity. Ipilimumab+CP11 mix represents a 1:1 mixture of the monoclonal antibodies ipilimumab and CP11. The binding parameters of the recombination reaction products to CCR8 and CTLA4 measured by flow cytometry are shown in Table 3.

**Table 3. The ability of recombination reaction products to bind to CCR8 and CTLA4 simultaneously**

| **Figure No.** | **Control antibody or recombination reaction product** | **EC₅₀ (nM)** | **Top** |
|---|---|---|---|
| **1A** | CP11-Ipi-F31 | 3.26 | 130.4 |
| | Ipi-F405L*CP11-K409R | 2.85 | 136.4 |
| | Ipi-K409R*CP11-F405L | 1.74 | 112.1 |
| | Ipi-D356K*CP11-K439E | NA | NA |
| | Ipi-K439E*CP11-D356K | NA | NA |
| **1B** | CP11-Ipi-F31 | 5.75 | 309.4 |
| | Ipi-D356K+T366A*CP11-K439E+T366A | 4.51 | 334.7 |
| | Ipi-D356K+Y407V* CP11-K439E+Y407V | 4.39 | 336.8 |
| | Ipi -D356K+Y407L*CP11-K439E+Y407L | 4.12 | 347.3 |
| **1C** | CP11-Ipi-F31 | 7.57 | 349.0 |
| | Ipi-D356K+T366L*CP11-K439E+T366L | 4.64 | 339.3 |
| | Ipi-D356K+L368V*CP11-K439E+L368V | 5.75 | 380.6 |
| | Ipi-D356K+K409R*CP11-K439E+K409R | 5.05 | 374.4 |
| | Ipi-D356K+K409Q* CP11-K439E+K409Q | 4.76 | 373.6 |
| **1D** | CP11-Ipi-F31 | 7.87 | 174.5 |
| | Ipi-D356K+Y349F*CP11-K439E+Y349F | NA | NA |
| | Ipi-D356K+Y349L*CP11-K439E+Y349L | 3.37 | 105.3 |
| | Ipi-D356K+L351V*CP11-K439E+L351V | 5.52 | 196.7 |
| | Ipi-D356K+L351T*CP11-K439E+L351T | 5.74 | 201.4 |
| | Ipi-D356K+L351I*CP11-K439E+L351I | 6.27 | 195.9 |
| | Ipi-D356K+S364A*CP11-K439E+S364A | 7.89 | 203.3 |
| | Ipi-D356K+T366V*CP11-K439E+T366V | 4.03 | 188.0 |
| | Ipi-D356K+T411N*CP11-K439E+T411N | NA | NA |
| **1E** | CP11-Ipi-F31 | 7.31 | 198.0 |
| | Ipi-D356K+T394S*CP11-K439E+T394S | 4.64 | 210.7 |
| | Ipi-D356K+V397M*CP11-K439E+V397M | 5.33 | 177.6 |
| | Ipi-D356K+V397T*CP11-K439E+V397T | NA | NA |
| | Ipi-D356K+F405L*CP11-K439E+F405L | 5.59 | 228.3 |
| | Ipi-D356K+F405Y*CP11-K439E+F405Y | 6.10 | 220.0 |
| **1F** | CP11-Ipi-F31 | 6.21 | 184.2 |
| | Ipi-D356K+L351F*CP11-K439E+L351F | 8.00 | 114.9 |
| | Ipi-D356K+S364T*CP11-K439E+S364T | 6.00 | 107.8 |
| | Ipi-D356K+S364V*CP11-K439E+S364V | NA | NA |
| | Ipi-D356K+S364L*CP11-K439E+S364L | 6.49 | 190.0 |
| | Ipi-D356K+T394A*CP11-K439E+T394A | 4.51 | 199.3 |
| | Ipi-D356K+T411Y*CP11-K439E+T411Y | 7.64 | 206.9 |
| **1G** | CP11-Ipi-F31 | 7.12 | 231.8 |
| | Ipi-D356K+L351C*CP11-K439E+L351C | 4.08 | 204.9 |
| | Ipi-D356K+T366G*CP11-K439E+T366G | 5.02 | 243.8 |
| | Ipi-D356K+T366S*CP11-K439E+T366S | NA | NA |
| | Ipi-D356K+T394N*CP11-K439E+T394N | 6.53 | 271.7 |
| | Ipi-D356K+T394C*CP11-K439E+T394C | 2.11 | 93.7 |
| | Ipi-D356K+V397I*CP11-K439E+V397I | 1.98 | 102.3 |
| | Ipi-D356K+Y407C*CP11-K439E+Y407C | 4.60 | 249.4 |
| | Ipi-D356K+Y349C*CP11-K439E+E357C | 5.65 | 260.8 |
| | Ipi-D356K+Y349C*CP11-K439E+S354C | NA | NA |
| **1H** | CP11-Ipi-F31 | 6.89 | 226.2 |
| | Ipi-F405T*CP11-T394F | 6.22 | 265.8 |
| | Ipi-D356K+Y349C*CP11-K439E+S354C+T366A | 6.83 | 286.2 |
| | Ipi-Y349C*CP11-S354C+T366A | 3.85 | 194.1 |
| | Ipi-Y349C*CP11-S354C+F405Y | 3.45 | 150.0 |
| | Ipi-Y349C*CP11-E357C | 3.60 | 214.1 |
| **1I** | CP11-Ipi-F31 | 6.89 | 226.2 |
| | Ipi-D356K+T366H*CP11-K439E+T366H | 33.00 | 273.6 |
| | Ipi-D356K+L368M*CP11-K439E+L368M | NA | NA |
| | Ipi-D356K+L368I*CP11-K439E+L368I | 5.47 | 259.9 |
| | Ipi-D356K+T394V*CP11-K439E+T394V | 4.35 | 228.6 |
| | Ipi-D356K+V397L*CP11-K439E+V397L | 3.35 | 180.0 |
| | Ipi-D356K+Y407H*CP11-K439E+Y407H | 4.27 | 222.0 |
| | Ipi-D356K+Y349S*CP11-K439E+S354Y | 4.42 | 244.9 |
| | Ipi-D356K+F405T*CP11-K439E+T394F | 3.85 | 260.0 |
| | Ipi-D356K+Y349C*CP11-K439E+S354C+L368I | 4.55 | 265.1 |
| | Ipi-D356K+Y349C*CP11-K439E+S354C+L351I | 4.78 | 255.7 |
| | Ipi-Y349C*CP11-S354C+L368I | 3.28 | 175.2 |

| | | | |
|---|---|---|---|
| Note: NA means "not measurable". | | | |

The results in FIG. 1A show that Ipi-F405L*CP11-K409R (Top 136.4), a recombinant product of Ipi-F405L and CP11-K409R, could bind to CHO cell surface CCR8 and fluorescein-labeled CTLA4 simultaneously, and its binding activity was comparable to that of the bispecific antibody CP11-Ipi-F31 (Top 130.4). The simultaneous binding activity of the bispecific antibody Ipi-K409R*CP11-F405L (Top 112.1), a recombinant product of Ipi-K409R and CP11-F405L, to CCR8 and CTLA4 was slightly weaker than that of CP11-Ipi-F31. The signal curves of Ipi-D356K*CP11-K439E and Ipi-K439E*CP11-D356K overlap with that of ipilimumab+CP11 mix, indicating that these two combinations could not produce bispecific antibodies under the conditions of this example.

The results in FIG. 1B show that Ipi-D356K+T366A*CP11-K439E+T366A (Top 334.7), Ipi-D356K+Y407V*CP11-K439E+Y407V (Top 336.8), and Ipi-D356K+Y407L*CP11-K439E+Y407L (Top 347.3) could all bind to CCR8 and CTLA4 effectively and simultaneously, and their binding activity was stronger than that of CP11-Ipi-F31 (Top 309.4).

The results in FIG. 1C show that Ipi-D356K+T366L*CP11-K439E+T366L (Top 339.3), Ipi-D356K+L368V*CP11-K439E+L368V (Top 380.6), Ipi-D356K+K409R*CP11-K439E+K409R (Top 374.4), and Ipi-D356K+K409Q*CP11-K439E+K409Q (Top 373.6) could all bind to CCR8 and CTLA4 effectively and simultaneously; the binding activity of Ipi-D356K+T366L*CP11-K439E+T366L was comparable to that of CP11-Ipi-F31 (Top 349), and the binding activity of the other three was stronger than that of CP11-Ipi-F31.

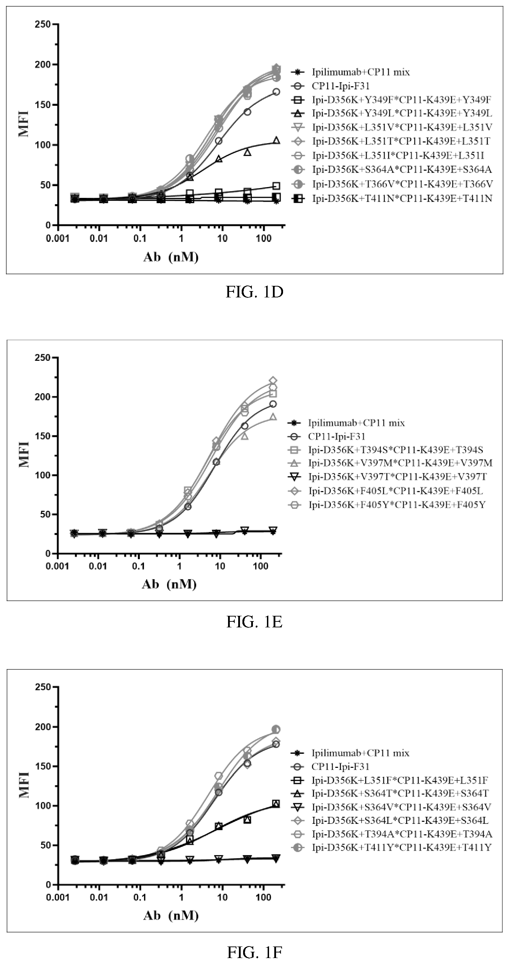

The results in FIG. 1D show that Ipi-D356K+L351V*CP11-K439E+L351V (Top 196.7), Ipi-D356K+L351T*CP11-K439E+L351T (Top 201.4), Ipi-D356K+L351I*CP11-K439E+L351I (Top 195.9), Ipi-D356K+S364A*CP11-K439E+S364A (Top 203.3), and Ipi-D356K+T366V*CP11-K439E+T366V (Top 188.0) could all bind to CCR8 and CTLA4 effectively and simultaneously, and their binding activity was stronger than that of CP11-Ipi-F31 (Top 174.5).

The results in FIG. 1E show that Ipi-D356K+T394S*CP11-K439E+T394S (Top 210.7), Ipi-D356K+V397M*CP11-K439E+V397M (Top 177.6), Ipi-D356K+F405L*CP11-K439E+F405L (Top 228.3), and Ipi-D356K+F405Y*CP11-K439E+F405Y (Top 220.0) could all bind to CCR8 and CTLA4 effectively and simultaneously; the binding activity of D356K+V397M*CP11-K439E+V397M was slightly weaker than that of CP11-Ipi-F31 (Top 198.0), and the binding activity of the other three was stronger than that of CP11-Ipi-F31.

The results in FIG. 1F show that Ipi-D356K+S364L*CP11-K439E+S364L (Top 190.0), Ipi-D356K+T394A*CP11-K439E+T394A (Top 199.3), and Ipi-D356K+T411Y*CP11-K439E+T411Y (Top 206.9) could all bind to CCR8 and CTLA4 effectively and simultaneously, and their binding activity was stronger than that of CP11-Ipi-F31 (Top 184.2).

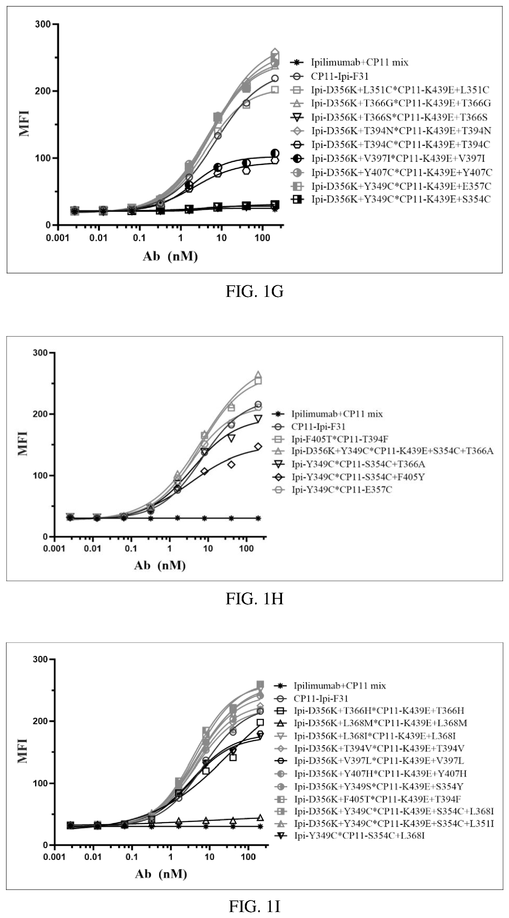

The results in FIG. 1G show that Ipi-D356K+L351C*CP11-K439E+L351C (Top 204.9), Ipi-D356K+T366G*CP11-K439E+T366G (Top 243.8), Ipi-D356K+T394N*CP11-K439E+T394N (Top 271.7), Ipi-D356K+Y407C*CP11-K439E+Y407C (Top 249.4), and Ipi-D356K+Y349C*CP11-K439E+E357C (Top 260.8) could all bind to CCR8 and CTLA4 effectively and simultaneously; the binding activity of Ipi-D356K+L351C*CP11-K439E+L351C was slightly weaker than that of CP11-Ipi-F31 (Top 231.8), and the binding activity of the others was stronger than that of CP11-Ipi-F31. The results in FIG. 1H show that Ipi-F405T*CP11-T394F (Top 265.8), Ipi-D356K+Y349C*CP11-K439E+S354C+T366A (Top 286.2), and Ipi-Y349C*CP11-E357C (Top 214.1) could all bind to CCR8 and CTLA4 effectively and simultaneously; the binding activity of Ipi-Y349C*CP11-E357C was slightly weaker than that of CP11-Ipi-F31 (Top 226.2), and the binding activity of the others was stronger than that of CP11-Ipi-F31.

The results in FIG. 1I show that Ipi-D356K+L368I*CP11-K439E+L368I (Top 259.9), Ipi-D356K+T394V*CP11-K439E+T394V (Top 228.6), Ipi-D356K+Y407H*CP11-K439E+Y407H (Top 222.0), Ipi-D356K+Y349S*CP11-K439E+S354Y (Top 244.9), Ipi-D356K+F405T*CP11-K439E+T394F (Top 260.0), Ipi-D356K+Y349C*CP11-K439E+S354C+L368I (Top 265.1), and Ipi-D356K+Y349C*CP11-K439E+S354C+L351I (Top 255.7) could all bind to CCR8 and CTLA4 effectively and simultaneously; the binding activity of Ipi-D356K+T394V*CP11-K439E+T394V was comparable to that of CP11-Ipi-F31 (Top 226.2), and the binding activity of the others was stronger than that of CP11-Ipi-F31.

### Example 1.7: Recombination Efficiency of Anti-CTLA4 and CCR8 Bispecific Antibodies

The physicochemical property analysis methods used in this example are described below.

### 1. High-performance liquid chromatography-size exclusion chromatography (HPLC-SEC)

Antibodies are high-molecular-weight proteins with highly complex secondary and tertiary structures. Due to changes such as post-translational modifications, aggregation, and degradation, antibodies are heterogeneous in terms of biochemical and biophysical properties. When bispecific antibodies are analyzed using separation techniques, variants, aggregates, and degradation fragments are often observed, and their presence may compromise safety and effectiveness. During the production and storage of antibodies, aggregates, degradation fragments, and incompletely assembled molecules are prone to occur. The content of the above impurities in samples is determined by HPLC-SEC. The molecular weight of an aggregate is greater than that of a monomer, resulting in a relatively short retention time for the corresponding peak. The molecular weight of a degradation fragment or an incompletely assembled molecule is less than that of a monomer, resulting in a relatively long retention time for the corresponding peak.

A Waters e2695-2489 chromatograph was used for HPLC-SEC.

The mobile phase was prepared as follows: 6.28 g of K₂HPO₄•3H₂O, 3.06 g of K₂HPO₄, and 22.37 g of KCl were weighed out and dissolved in a proper amount of purified water, purified water was then added until the total volume was 1 L, and the pH was adjusted to 6.8 with a 2 M KOH solution. A Waters XBridge BEH 200A SEC chromatography column (7.8 × 300 mm, 3.5 µm) was used. The injection amount was set at 30 µg, the flow rate at 0.5 mL/min, the elution time at 30 min, the column temperature at 30 °C, the sample chamber temperature at 15 °C, and the detection wavelength at 280 nm.

### 2. High-performance liquid chromatography-ion exchange chromatography (HPLC-IEC)

Many post-translational modifications of proteins, such as N-glycosylation, modification of C-terminal lysine residues, cyclization of N-terminal glutamine or glutamic acid, deamidation of asparagine, isomerization of aspartic acid, and oxidation of amino acid residues, can directly or indirectly alter the surface charges of antibodies, leading to charge heterogeneity. Charge variants can be separated and analyzed based on the charges that they carry. Common analysis methods include cation exchange chromatography (CEX) and anion exchange chromatography (AEX). In a chromatography-based analysis, acidic species and basic species are defined based on their retention times relative to the main peak. Acidic species are variants that are eluted earlier than the main peak in CEX or later than the main peak in AEX, while basic species are variants that are eluted later than the main peak in CEX or earlier than the main peak in AEX. The peaks corresponding to acidic species and basic species are referred to as acidic peaks and basic peaks, respectively. Here, the proportion of each component in a recombination reaction mixture was analyzed using HPLC-IEC to calculate the recombination reaction efficiency. An Agilent 1260 Infinity II DAD BIO-LC chromatograph was used for HPLC-IEC.

Mobile phase A was 20 mM MES pH 6.0 (3.9048 g of MES (molecular weight: 195.24 g/mol) was weighed out and dissolved in a proper amount of purified water, purified water was then added until the total volume was 1 L, and the pH was adjusted to 6.0 with 10 M NaOH). Mobile phase B was 20 mM MES + 500 mM NaCl pH 6.0 (3.9048 g of MES and 29.22 g of NaCl were weighed out and dissolved in a proper amount of purified water, purified water was then added until the total volume was 1 L, and the pH was adjusted to 6.0 with 10 M NaOH). The mixing ratio of the two mobile phases was adjusted over time according to a preset program, and the flow rate was 0.4 mL/min. A YMC BioPro SP-F chromatography column (4.6 × 100 mm, 5 µm) was used. The injection amount was set at 30 µg, the column temperature at 30 °C, the sample chamber temperature at 15 °C, and the detection wavelength at 280 nm.

### 3. The Biomix analysis method is described below.

An Agilent 1260 DAD/Agilent 1290 UPLC DAD chromatograph was used for Biomix analysis; the mobile phase was prepared as follows: 150 mM PB (pH 6.8)-62.01 g of Na₂HPO₄•12H₂O and 19.795 g of NaH₂PO₄•2H₂O were weighed out and placed in a 2 L beaker, 2000 mL of ultrapure water was added, and the pH was fine-adjusted to 6.8 with phosphoric acid or sodium hydroxide; injection amount: 30 µg; chromatography column: Sepax BioMix SEC 300A, 7.8 × 300 mm, 3 µm; flow rate: 0.5 mL/min; elution time: 30 min; column temperature: 30 °C; sample chamber temperature: 15 °C; detection wavelength: 280 nm.

The parental monoclonal antibodies and corresponding bispecific antibodies produced in the above examples were analyzed by HPLC-SEC and IEC. The obtained data on purity (here, the SEC purity was the proportion of the area of the main peak, and the IEC purity was the sum of the proportions of the areas of primary peaks such as the main peak, the acidic peak, and the basic peak) are summarized in Table 4.

**Table 4. The SEC purity of parental monoclonal antibodies and the SEC and IEC purity of corresponding bispecific antibodies**

| **Parental monoclonal antibody** | | **Recombination reaction product** | |
|---|---|---|---|
| **Name** | **SEC (%)** | **SEC (%)** | **IEC (%)** |
| Ipi-F405L | 86.61 | 83.30 | 89.12 |
| CP11-K409R | 97.67 | | |
| Ipi-K409R | 97.00 | 81.97 | 86.72 |
| CP11-F405L | 88.39 | | |
| Ipi-D356K | 98.24 | NA | NA |
| CP11-K439E | 98.21 | | |
| Ipi-K439E | 98.10 | NA | NA |
| CP11-D356K | 98.21 | | |
| Ipi-D356K+Y349L | 98.18 | 73.45 | 76.99 |
| CP11-K439E+Y349L | 97.72 | | |
| Ipi-D356K+L351C | 96.14 | 90.81 | 94.76 |
| CP11-K439E+L351C | 96.98 | | |
| Ipi-D356K+L351V | 97.56 | 90.49 | 93.70 |
| CP11-K439E+L351V | 97.16 | | |
| Ipi-D356K+L351T | 97.16 | 95.47 | 97.02 |
| CP11-K439E+L351T | 96.94 | | |
| Ipi-D356K+L351I | 98.40 | 93.91 | 96.40 |
| CP11-K439E+L351I | 97.52 | | |
| Ipi-D356K+L351F | 95.92 | 75.67 | 81.16 |
| CP11-K439E+L351F | 97.92 | | |
| Ipi-D356K+S364A | 97.53 | 92.44 | 94.23 |
| CP11-K439E+S364A | 98.23 | | |
| Ipi-D356K+S364T | 96.60 | 75.94 | 79.93 |
| CP11-K439E+S364T | 96.74 | | |
| Ipi-D356K+S364L | 72.29 | 91.40 | 98.12 |
| CP11-K439E+S364L | 93.80 | | |
| Ipi-D356K+T366G | 97.53 | 96.91 | 93.10 |
| CP11-K439E+T366G | 98.46 | | |
| Ipi-D356K+T366A | 98.98 | 97.17 | 97.64 |
| CP11-K439E+T366A | 98.78 | | |
| Ipi-D356K+T366V | 72.27 | 88.71 | 95.76 |
| CP11-K439E+T366V | 87.57 | | |
| Ipi-D356K+T366L | 92.94 | 93.92 | 93.47 |
| CP11-K439E+T366L | 94.06 | | |
| Ipi-D356K+T366H | 87.40 | 85.42 | 89.62 |
| CP11-K439E+T366H | 97.87 | | |
| Ipi-D356K+L368V | 87.52 | 95.54 | 94.37 |
| CP11-K439E+L368V | 96.69 | | |
| Ipi-D356K+L368I | 93.51 | 96.28 | 94.67 |
| CP11-K439E+L368I | 97.24 | | |
| Ipi-D356K+T394A | 97.69 | 97.45 | 96.63 |
| CP11-K439E+T394A | 97.27 | | |
| Ipi-D356K+T394S | 98.05 | 98.53 | 96.32 |
| CP11-K439E+T394S | 98.05 | | |
| Ipi-D356K+T394C | 95.69 | 60.70 | 60.81 |
| CP11-K439E+T394C | 97.29 | | |
| Ipi-D356K+T394V | 98.20 | 93.05 | 92.62 |
| CP11-K439E+T394V | 96.06 | | |
| Ipi-D356K+T394N | 92.14 | 97.26 | 92.84 |
| CP11-K439E+T394N | 95.37 | | |
| Ipi-D356K+V397I | 97.68 | 66.10 | 66.45 |
| CP11-K439E+V397I | 97.93 | | |
| Ipi-D356K+V397L | 97.65 | 89.96 | 92.35 |
| CP11-K439E+V397L | 98.88 | | |
| Ipi-D356K+V397M | 97.73 | 90.27 | 90.34 |
| CP11-K439E+V397M | 97.73 | | |
| Ipi-D356K+F405L | 96.02 | 94.16 | 93.73 |
| CP11-K439E+F405L | 93.42 | | |
| Ipi-D356K+F405Y | 96.16 | 97.37 | 95.17 |
| CP11-K439E+F405Y | 96.02 | | |
| Ipi-D356K+Y407C | 80.45 | 96.07 | 94.41 |
| CP11-K439E+Y407C | 90.78 | | |
| Ipi-D356K+Y407V | 84.39 | 95.85 | 97.70 |
| CP11-K439E+Y407V | 95.00 | | |
| Ipi-D356K+Y407L | 81.26 | 94.47 | 96.69 |
| CP11-K439E+Y407L | 91.64 | | |
| Ipi-D356K+Y407H | 41.85 | 85.17 | 86.04 |
| CP11-K439E+Y407H | 62.48 | | |
| Ipi-D356K+K409Q | 95.91 | 95.14 | 96.06 |
| CP11-K439E+K409Q | 96.17 | | |
| Ipi-D356K+K409R | 94.30 | 94.25 | 93.41 |
| CP11-K439E+K409R | 99.25 | | |
| Ipi-D356K+T411Y | 97.92 | 94.68 | 95.23 |
| CP11-K439E+T411Y | 95.00 | | |
| Ipi-D356K+Y349S | 97.59 | 94.24 | 97.37 |
| CP11-K439E+S354Y | 98.75 | | |
| Ipi-D356K+Y349C | 91.36 | 92.10 | 97.21 |
| CP11-K439E+E357C | 75.69 | | |
| Ipi-D356K+F405T | 59.38 | 92.36 | 96.09 |
| CP11-K439E+T394F | 93.98 | | |
| Ipi-D356K+Y349C | 90.08 | 93.83 | 97.66 |
| CP11-K439E+S354C+L351I | 97.13 | | |
| Ipi-D356K+Y349C | 90.08 | 90.49 | 96.94 |
| CP11-K439E+S354C+T366A | 95.02 | | |
| Ipi-D356K+Y349C | 90.08 | 91.53 | 96.44 |
| CP11-K439E+S354C+L368I | 91.89 | | |
| Ipi-Y349C | 95.11 | 84.23 | 89.00 |
| CP11-S354C+T366A | 97.80 | | |
| Ipi-Y349C | 95.11 | 80.64 | 85.13 |
| CP11-S354C+L368I | 94.65 | | |
| Ipi-Y349C | 95.11 | 76.22 | 76.78 |
| CP11-S354C+F405Y | 97.33 | | |
| Ipi-Y349C | 95.11 | 89.09 | 93.11 |
| CP11-E357C | 86.74 | | |
| Ipi-F405T | 91.16 | 92.82 | 95.90 |
| CP11-T394F | 98.38 | | |
| Ipi-Y349A | 98.44 | 93.45 | 94.43 |
| CP11-S354F | 97.61 | | |
| Ipi-Y349S | 98.86 | 87.42 | 87.60 |
| CP11-S354F | 97.61 | | |
| Ipi-Y349V | 99.05 | 84.71 | 83.44 |
| CP11-S354F | 97.61 | | |
| Ipi-Y349T | 98.17 | 85.60 | 85.69 |
| CP11-S354F | 97.61 | | |
| Ipi-Y349A | 98.44 | 87.96 | 88.60 |
| CP11-S354Y | 97.45 | | |
| Ipi-Y349G | 90.88 | 91.69 | 93.58 |
| CP11-S354W | 97.86 | | |
| Ipi-Y349A | 98.44 | 91.41 | 92.54 |
| CP11-S354W | 97.86 | | |
| Ipi-Y349S | 98.86 | 89.57 | 89.80 |
| CP11-S354W | 97.86 | | |
| Ipi-Y349S | 98.86 | 94.58 | 96.00 |
| CP11-S364Y | 97.28 | | |
| Ipi-Y349C | 96.22 | 92.51 | 94.74 |
| CP11-S354C+S364Y | 96.67 | | |
| Ipi-Y349C | 96.22 | 92.26 | 95.00 |
| CP11-S354C+S364F | 97.88 | | |
| Ipi-D356K+D399K | 33.70 | 78.32 | 76.10 |
| CP11-K439E+K409D | 86.09 | | |
| Ipi-D356K+K409D | 92.56 | 91.18 | 94.61 |
| CP11-K439E+D399K | 86.96 | | |

| | | | |
|---|---|---|---|
| Note: NA means that it could not be determined accurately. | | | |

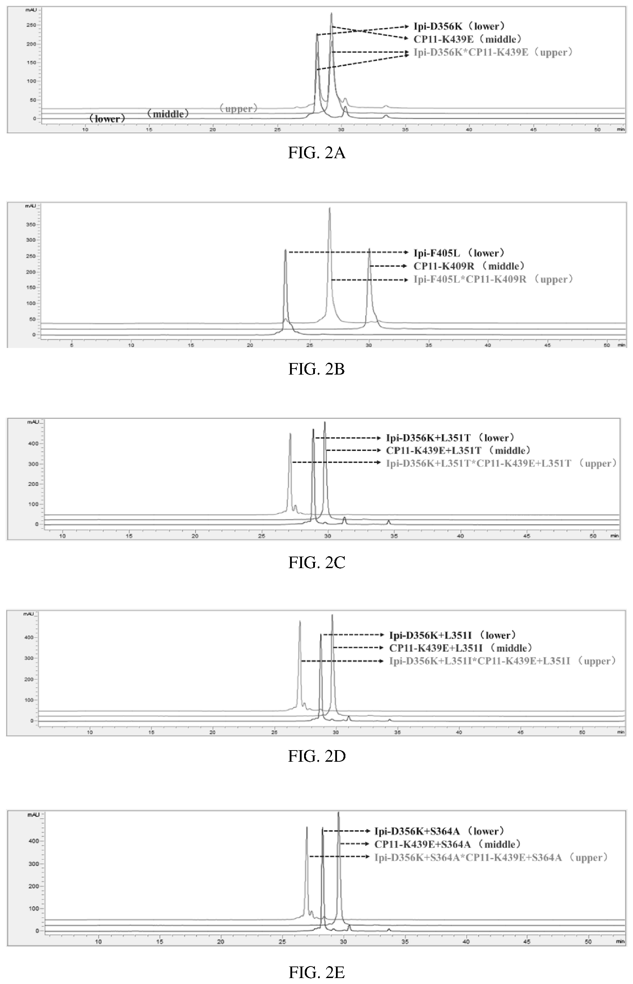

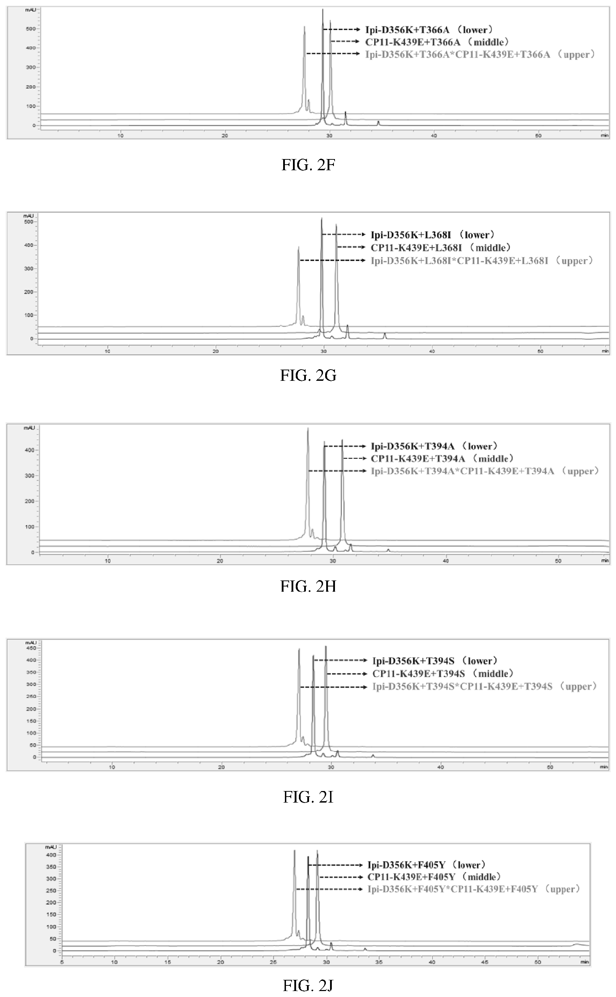

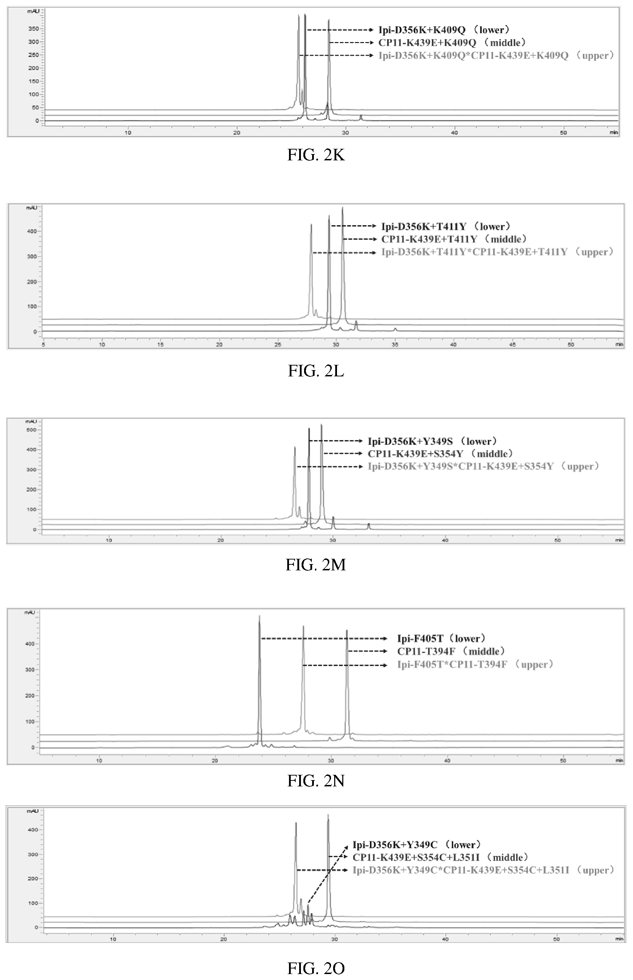

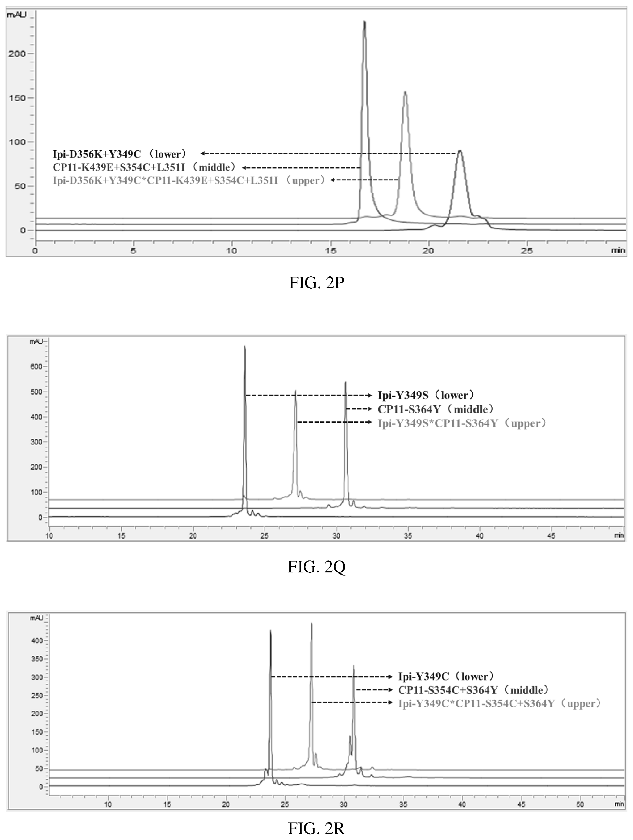

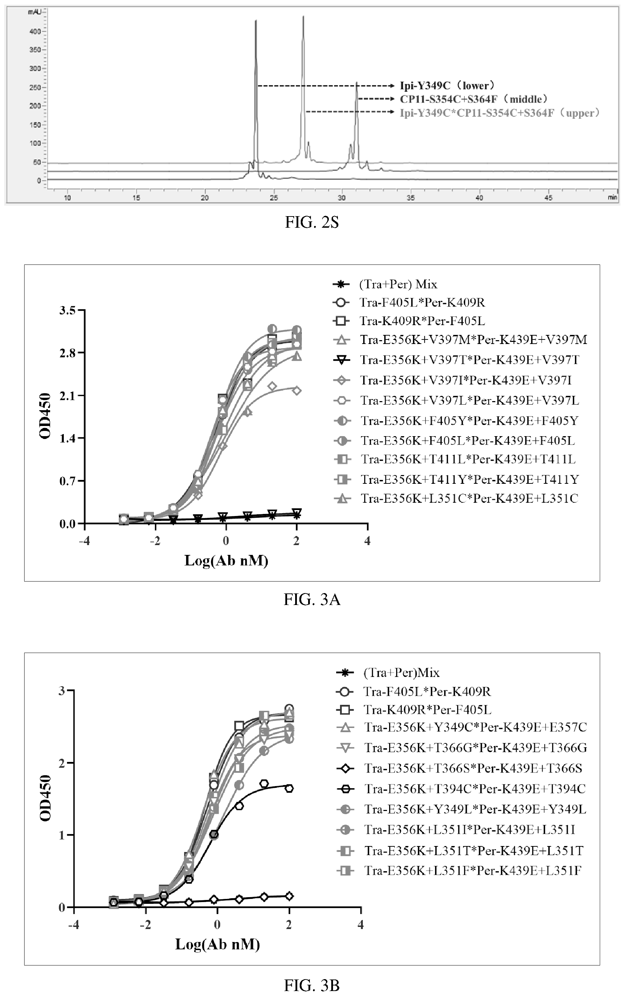

The data in FIGs. 1A to 1I, Table 3, and Table 4 were comprehensively analyzed. Some parental monoclonal antibodies and corresponding bispecific antibodies were selected, and their HPLC-IEC chromatograms were superimposed. The selected combinations included Ipi-D356K*CP11-K439E (FIG. 2A), Ipi-F405L*CP11-K409R (FIG. 2B), Ipi-D356K+L351T*CP11-K439E+L351T (FIG. 2C), Ipi-D356K+L351I*CP11-K439E+L3511 (FIG. 2D), Ipi-D356K+S364A*CP11-K439E+S364A (FIG. 2E), Ipi-D356K+T366A*CP11-K439E+T366A (FIG. 2F), Ipi-D356K+L368I*CP11-K439E+L368I (FIG. 2G), Ipi-D356K+T394A*CP11-K439E+T394A (FIG. 2H), Ipi-D356K+T394S*CP11-K439E+T394S (FIG. 2I), Ipi-D356K+F405Y*CP11-K439E+F405Y (FIG. 2J), Ipi-D356K+K409Q*CP11-K439E+K409Q (FIG. 2K), Ipi-D356K+T411Y*CP11-K439E+T411Y (FIG. 2L), Ipi-D356K+Y349S*CP11-K439E+S354Y (FIG. 2M), Ipi-F405T*CP11-T394F (FIG. 2N), Ipi-D356K+Y349C*CP11-K439E+S354C+L351I (FIGs. 2O and 2P), Ipi-Y349S*CP11-S364Y (FIG. 2Q), Ipi-Y349C*CP11-S354C+S364Y (FIG. 2R), and Ipi-Y349C*CP11-S354C+S364F (FIG. 2S). FIG. 2A shows that Ipi-D356K and CP11-K439E could not be recombined to form a bispecific antibody under the conditions of this example. If a recombination reaction yields a bispecific antibody, new peaks corresponding to the bispecific antibody will be found in the chromatogram, and the peaks at the positions corresponding to the parental monoclonal antibodies will relatively decrease. The percentage of the area of the peaks corresponding to the parental monoclonal antibodies is the amount that remains after the recombination reaction.

The formula for calculating the efficiency of a recombination reaction was: 100% - (percentage of area of peaks corresponding to parental monoclonal antibody A + percentage of area of peaks corresponding to parental monoclonal antibody B). The monoclonal antibodies used for recombination reactions were not subjected to fine purification. As a result, aggregates and a small amount of impurities could be present.

**Table 5. Recombination reaction efficiency calculations based on the amount of parental monoclonal antibody residues**

| **Parental monoclonal antibody** | | **Recombination reaction efficiency (%)** | **Figure No.** |
|---|---|---|---|
| **Name** | **Amount of residues (%)** | | |
| Ipi-D356K | 46.2 | 5.2 | 2A |
| CP11-K439E | 48.6 | | |
| Ipi-F405L | 4.6 | 90.4 | 2B |
| CP11-K409R | 5.0 | | |
| Ipi-D356K+L351T | 2.1 | 97.9 | 2C |
| CP11-K439E+L351T | 0.0 | | |
| Ipi-D356K+L351I | 2.7 | 96.5 | 2D |
| CP11-K439E+L351I | 0.8 | | |
| Ipi-D356K+S364A | 3.7 | 95.1 | 2E |
| CP11-K439E+S364A | 1.2 | | |
| Ipi-D356K+T366A | 1.7 | 97.6 | 2F |
| CP11-K439E+T366A | 0.7 | | |
| Ipi-D356K+L368I | 0.3 | 99.3 | 2G |
| CP11-K439E+L368I | 0.4 | | |
| Ipi-D356K+T394A | 0.7 | 99.3 | 2H |
| CP11-K439E+T394A | 0.0 | | |
| Ipi-D356K+T394S | 2.0 | 97.3 | 2I |
| CP11-K439E+T394S | 0.7 | | |
| Ipi-D356K+F405Y | 2.2 | 97.1 | 2J |
| CP11-K439E+F405Y | 0.7 | | |
| Ipi-D356K+K409Q | 2.8 | 96.5 | 2K |
| CP11-K439E+K409Q | 0.7 | | |
| Ipi-D356K+T411Y | 4.8 | 95.2 | 2L |
| CP11-K439E+T411Y | 0.0 | | |
| Ipi-D356K+Y349S | 1.8 | 97.6 | 2M |
| CP11-K439E+S354Y | 0.6 | | |
| Ipi-F405T | 1.8 | 97.2 | 2N |
| CP11-T394F | 1.0 | | |
| Ipi-D356K+Y349C | 2.1 | 94.1 | 2O+2P |
| CP11-K439E+S354C+L351I | 3.7 | | |
| Ipi-Y349S | 3.0 | 96.0 | 2Q |
| CP11-S364Y | 1.0 | | |
| Ipi-Y349C | 0.8 | 95.0 | 2R |
| CP11-S354C+S364Y | 4.2 | | |
| Ipi-Y349C | 3.0 | 95.0 | 2S |
| CP11-S354C+S364F | 2.0 | | |

It should be noted that the retention time of a recombinantly produced bispecific antibody is not necessarily between those of the two parental monoclonal antibodies, which may be related to an electrostatic change in the antibody resulting from site-directed mutagenesis.

The results of the above recombination reaction efficiency calculations (FIG. 2 and Table 5) show that the recombination reaction efficiency of Ipi-D356K*CP11-K439E was very low (about 5.2%), the recombination reaction efficiency of Ipi-F405L*CP11-K409R was about 90.4%, and the recombination reaction efficiency of all the other combinations in Table 5 was higher than 94%. Due to the relatively significant charge heterogeneity of the parental mAb Ipi-D356K+Y349C, its IEC peaks overlap with the peaks of the recombinant product (FIG. 2O). Therefore, the recombination reaction of Ipi-D356K+Y349C*CP11-K439E+S354C+L351I was analyzed using Biomix (see FIG. 2P for the results), and the recombination efficiency of this reaction was calculated. The recombination efficiency of all the remaining reactions was analyzed by HPLC-IEC and calculated. 4. The method for determining the molecular weights of antibodies by mass spectrometry is described below.

In the determination of the deglycosylated intact molecular weight, the sample treatment method was as follows: 30 µg of an expressed antibody was lyophilized, and 10 µL of 8 M guanidine-HCl was then added. The antibody was denatured in a water bath at 70 °C for 10 min. 90 µL of distilled water was added. 30 µL of the mixture was taken, and 0.8 µL of PNGase F was added. The resulting mixture was incubated in a water bath at 37 °C for 2 h, and a 0.5 µg sample was taken for the determination of deglycosylated intact molecular weight. In the determination of the deglycosylated reduced molecular weight, the sample treatment method was as follows: 30 µg of an expressed antibody was lyophilized, and 10 µL of 8 M guanidine-HCl was then added. The antibody was denatured in a water bath at 70 °C for 10 min. 90 µL of distilled water was added. 30 µL of the mixture was taken, and 0.8 µL of PNGase F was added. The resulting mixture was incubated in a water bath at 37 °C for 2 h. Then, 2 µL of 0.025 M DTT was added. After 10 min of reduction in a water bath at 70 °C, a 0.5 µg sample was taken for the determination of deglycosylated reduced molecular weight.

The chromatography separation conditions are described below.

A Poroshell 300SB-C8 chromatography column (5 µm, 2.1 × 75 mm) was used.

Mobile phase A was 0.1% HCOOH/H₂O, and mobile phase B was 0.1% HCOOH/ACN. The column temperature was 75 °C. The mass spectrometry analysis conditions are described below: An Agilent 6530 Q-TOF LC-MS mass spectrometer was used, and the ionization mode was electrospray ionization (ESI). The acquisition mode was 1 GHz (mass range: 500-5000 m/z). The drying gas temperature was set at 325 °C, the drying gas flow rate at 10 L/min, the nebulizer pressure at 40 psi, the sheath gas temperature at 350 °C, the sheath gas flow rate at 12 L/min, the spray voltage at 500 V, the capillary voltage at 3500 V, the fragmentation voltage at 200 V, the Skimmer voltage at 65 V, and the Rf voltage at 7500 V.

**Table 6. The results of mass spectrometry analysis of bispecific antibodies**

| **Parental monoclonal antibody** | | **Recombination reaction product** | |
|---|---|---|---|
| **Name** | **Calculated heavy chain/light chain molecular weight** | **Calculated intact molecular weight** | **Found heavy chain/light chain/intact molecular weight** |
| Ipi-D356K+L351T | 49113/23455 | 145456 | 49108/49634/23452/23279/145460 |
| CP11-K439E+L351T | 49639/23282 | | |
| Ipi-D356K+L351I | 49125/23455 | 145481 | 49120/49647/23452/23279/145484 |
| CP11-K439E+L351I | 49651/23282 | | |
| Ipi-D356K+S364A | 49109/23455 | 145453 | 49105/49631/23452/23279/145453 |
| CP11-K439E+S364A | 49635/23282 | | |
| Ipi-D356K+T366A | 49094/23455 | 145420 | 49098/49624/23456/23283/145430 |
| CP11-K439E+T366A | 49621/23282 | | |
| Ipi-D356K+L368I | 49125/23455 | 145491 | 49118/49644/23451/23277/145483 |
| CP11-K439E+L368I | 49651/23282 | | |
| Ipi-D356K+T394A | 49095/23455 | 145420 | 49091/49617/23452/23279/145433 |
| CP11-K439E+T394A | 49621/23282 | | |
| Ipi-D356K+T394S | 49111/23455 | 145452 | 49108/49634/23453/23280/145460 |
| CP11-K439E+T394S | 49637/23282 | | |
| Ipi-D356K+F405Y | 49141/23455 | 145513 | 49139/49664/23453/23279/145520 |
| CP11-K439E+F405Y | 49667/23282 | | |
| Ipi-D356K+K409Q | 49125/23455 | 145480 | 49128/49655/23457/23284/145488 |
| CP11-K439E+K409Q | 49651/23282 | | |
| Ipi-D356K+T411Y | 49187/23455 | 145605 | 49183/49709/23452/23279/145613 |
| CP11-K439E+T411Y | 49713/23282 | | |
| Ipi-D356K+Y349S | 49049/23455 | 145481 | 49042/49720/23451/23277/145483 |
| CP11-K439E+S354Y | 49727/23282 | | |
| Ipi-D356K+Y349C | 49065/23455 | 145435 | 49058/49660/23451/23277/145436 |
| CP11-K439E+S354C+L351I | 49667/23282 | | |
| Ipi-D356K+Y349C | 49065/23455 | 145405 | 49058/49630/23451/23277/145407 |
| CP11-K439E+S354C+T366A | 49637/23282 | | |
| Ipi-D356K+Y349C | 49065/23455 | 145437 | 49058/49660/23451/23277/145436 |
| CP11-K439E+S354C+L368I | 49667/23282 | | |
| Ipi-F405T | 49038/23455 | 145439 | 49031/49689/23451/23277/145442 |
| CP11-T394F | 49696/23282 | | |
| Ipi-Y349S | 49000/23451 | 145439 | 49001/49718/23451/23278/145438 |
| CP11-S364Y | 49718/23278 | | |
| Ipi-Y349C | 49044/23451 | 145499 | 49045/49734/23451/23278/145496 |
| CP11-S354C+S364Y | 49734/23278 | | |
| Ipi-Y349C | 49044/23451 | 145483 | 49045/49719/23451/23278/145479 |
| CP11-S354C+S364F | 49718/23278 | | |

| | | | |
|---|---|---|---|
| Note: The calculated heavy chain, light chain, and intact molecular weights in this table are theoretical values calculated by software with the post-translational modifications and redox states of the proteins taken into account. Therefore, the calculated molecular weights of the same light chain under different experimental conditions may be different. The results in Table 6 show that the calculated heavy chain, light chain, and intact molecular weights of the bispecific antibodies produced by the above combinations were very close to the actual heavy chain, light chain, and intact molecular weights measured by mass spectrometry, which indicates that the recombination and structures of the bispecific antibody molecules were in line with expectations. The mass spectra of the recombinant samples did not show peaks corresponding to the parental monoclonal antibodies, indicating that the above recombination reactions were relatively complete. | | | |

### Example 2.1: Source and Preparation of Anti-HER2 Monoclonal Antibodies Trastuzumab and Pertuzumab

The amino acid sequences of the heavy and light chains of trastuzumab and pertuzumab were from the Inxight Drugs database (trastuzumab entry: P188ANX8CK; pertuzumab entry: K16AIQ8CTM).

The amino acid sequence of the heavy chain of trastuzumab is shown below (SEQ ID NO: 15):

EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK

The amino acid sequence of the light chain of trastuzumab is shown below (SEQ ID NO: 16):

DIQMTQSPSSLSASVGDRVTITCRASQDVNTAVAWYQQKPGKAPKLLIYSASFLY
SGVPSRFSGSRSGTDFTLTISSLQPEDFATYYCQQHYTTPPTFGQGTKVEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

The amino acid sequence of the heavy chain of pertuzumab is shown below (SEQ ID NO: 17):

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK

The amino acid sequence of the light chain of pertuzumab is shown below (SEQ ID NO: 18):

DIQMTQSPSSLSASVGDRVTITCKASQDVSIGVAWYQQKPGKAPKLLIYSASYRY

TGVPSRFSGSGSGTDFTLTISSLQPEDFATYYCQQYYIYPYTFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

Note: The italicized regions are the constant regions of the antibody.

The DNAs encoding the above variable regions were synthesized by Suzhou Genewiz Biological Technology Co., Ltd. The heavy chain variable region of trastuzumab/pertuzumab (trastuzumab-VH/pertuzumab-VH) and its light chain variable region (trastuzumab-VL/pertuzumab-VL) were ligated to DNAs encoding the human IgG1 heavy chain constant region and the human kappa light chain constant region, respectively, to construct full-length heavy and light chain genes of trastuzumab/pertuzumab, designated trastuzumab-HC/pertuzumab-HC and trastuzumab-LC/pertuzumab-LC, respectively. The above heavy and light chain genes were cloned into two pcDNA3.4 expression vectors, respectively, and the expression vectors were simultaneously transfected into Expi293F^{™} cells (Thermo Fisher Scientific, Cat. No. A14527) using PEI (polyethylenimine) to express the antibody. The 293-F cells were cultured in a serum-free culture medium for 5 days. The cell supernatant was collected, and the antibody was purified by protein A affinity chromatography.

The purification procedure is described below: Impurities in the cell culture supernatant were removed by high-speed centrifugation, and the antibody in the supernatant was captured by MabSelect SuRe (Cytiva, Cat. No. 17543801) affinity chromatography. The MabSelect Sure affinity column was washed with 0.2 M NaOH, then rinsed with purified water, and then equilibrated with PBS. The supernatant was allowed to flow through the affinity column, and the affinity column was washed with PBS until A280 dropped to baseline. The target protein was eluted with a 0.1 M acetic acid buffer (pH 3.5), and the antibody solution was neutralized with 1 M Tris-HCl (pH 8.0). The antibody solution was properly concentrated by ultrafiltration. Then, the antibody was further purified using a HiLoad Superdex 200 gel chromatography column (Cytiva, Cat. No. 28989335). The antibody concentration was determined by ultraviolet spectrophotometry, and the antibody solution was sterilized by filtration and stored in a refrigerator (4 °C). This method was used to purify related monoclonal antibodies, and can also be used to purify other antibodies or recombinant proteins in the present disclosure.

### Example 2.2: Preparation of Trastuzumab and Pertuzumab Mutants

Gene mutations were introduced into the coding regions for the heavy chain CH3 domains of trastuzumab and pertuzumab by site-directed mutagenesis to mutate the amino acid residues at particular positions into particular amino acids. The positions of amino acid residues were identified using the Eu numbering scheme. Mutants of trastuzumab and pertuzumab were prepared according to the expression and purification methods described in Example 8. The antibody samples obtained were sterilized by filtration using a 0.2-µm filter head, and the concentrations of the antibodies were determined using a NanoDrop microspectrophotometer. The rule for naming the antibody mutants is described below: Illustratively, trastuzumab-E356K+T366A means that the E at position 356 of trastuzuamb was mutated into K, and the T at position 366 was mutated into A. The rule applies to other mutants. See Table 7. In the recombination reactions, trastuzuamb and pertuzumab are abbreviated as Tra and Per, respectively.

**Table 7. Parental monoclonal antibody mutations and corresponding recombination reactions**

| **No.** | **Trastuzumab CH3 mutation(s)** | **Pertuzumab CH3 mutation(s)** | **Recombination reaction** |
|---|---|---|---|
| 1 | F405L | K409R | Tra-F405L*Per-K409R |
| 2 | K409R | F405L | Tra-K409R*Per-F405L |
| 3 | E356K | K439E | Tra-E356K*Per-K439E |
| 4 | K439E | E356K | Tra-K439E*Per-E356K |
| 5 | E356K+Y349L | K439E+Y349L | Tra-E356K+Y349L*Per-K439E+Y349L |
| 6 | E356K+L351C | K439E+L351C | Tra-E356K+L351C*Per-K439E+L351C |
| 7 | E356K+L351V | K439E+L351V | Tra-E356K+L351V*Per-K439E+L351V |
| 8 | E356K+L351T | K439E+L351T | Tra-E356K+L351T*Per-K439E+L351T |
| 9 | E356K+L351I | K439E+L351I | Tra-E356K+L351I*Per-K439E+L351I |
| 10 | E356K+L351M | K439E+L351M | Tra-E356K+L351M*Per-K439E+L351M |
| 11 | E356K+L351F | K439E+L351F | Tra-E356K+L351F*Per-K439E+L351F |
| 12 | E356K+S364A | K439E+S364A | Tra-E356K+S364A*Per-K439E+S364A |
| 13 | E356K+S364T | K439E+S364T | Tra-E356K+S364T*Per-K439E+S364T |
| 14 | E356K+S364L | K439E+S364L | Tra-E356K+S364L*Per-K439E+S364L |
| 15 | E356K+T366G | K439E+T366G | Tra-E356K+T366G*Per-K439E+T366G |
| 16 | E356K+T366A | K439E+T366A | Tra-E356K+T366A*Per-K439E+T366A |
| 17 | E356K+T366S | K439E+T366S | Tra-E356K+T366S*Per-K439E+T366S |
| 18 | E356K+T366V | K439E+T366V | Tra-E356K+T366V*Per-K439E+T366V |
| 19 | E356K+T366L | K439E+T366L | Tra-E356K+T366L*Per-K439E+T366L |
| 20 | E356K+T366I | K439E+T366I | Tra-E356K+T366I*Per-K439E+T366I |
| 21 | E356K+T366H | K439E+T366H | Tra-E356K+T366H*Per-K439E+T366H |
| 22 | E356K+L368A | K439E+L368A | Tra-E356K+L368A*Per-K439E+L368A |
| 23 | E356K+L368V | K439E+L368V | Tra-E356K+L368V*Per-K439E+L368V |
| 24 | E356K+L368I | K439E+L368I | Tra-E356K+L368I*Per-K439E+L368I |
| 25 | E356K+L368M | K439E+L368M | Tra-E356K+L368M*Per-K439E+L368M |
| 26 | E356K+T394A | K439E+T394A | Tra-E356K+T394A*Per-K439E+T394A |
| 27 | E356K+T394S | K439E+T394S | Tra-E356K+T394S *Per-K439E+T394S |
| 28 | E356K+T394C | K439E+T394C | Tra-E356K+T394C*Per-K439E+T394C |
| 29 | E356K+T394V | K439E+T394V | Tra-E356K+T394V*Per-K439E+T394V |
| 30 | E356K+T394N | K439E+T394N | Tra-E356K+T394N*Per-K439E+T394N |
| 31 | E356K+V397T | K439E+V397T | Tra-E356K+V397T*Per-K439E+V397T |
| 32 | E356K+V397L | K439E+V397L | Tra-E356K+V397L*Per-K439E+V397L |
| 33 | E356K+V397I | K439E+V397I | Tra-E356K+V397I*Per-K439E+V397I |
| 34 | E356K+V397M | K439E+V397M | Tra-E356K+V397M*Per-K439E+V397M |
| 35 | E356K+F405L | K439E+F405L | Tra-E356K+F405L*Per-K439E+F405L |
| 36 | E356K+F405Y | K439E+F405Y | Tra-E356K+F405Y*Per-K439E+F405Y |
| 37 | E356K+Y407C | K439E+Y407C | Tra-E356K+Y407C*Per-K439E+Y407C |
| 38 | E356K+Y407V | K439E+Y407V | Tra-E356K+Y407V*Per-K439E+Y407V |
| 39 | E356K+Y407L | K439E+Y407L | Tra-E356K+Y407L*Per-K439E+Y407L |
| 40 | E356K+Y407H | K439E+Y407H | Tra-E356K+Y407H*Per-K439E+Y407H |
| 41 | E356K+Y407F | K439E+Y407F | Tra-E356K+Y407F*Per-K439E+Y407F |
| 42 | E356K+K409Q | K439E+K409Q | Tra-E356K+K409Q* Per-K439E+K409Q |
| 43 | E356K+K409R | K439E+K409R | Tra-E356K+K409R*Per-K439E+K409R |
| 44 | E356K+T411L | K439E+T411L | Tra-E356K+T411L*Per-K439E+T411L |
| 45 | E356K+T411Y | K439E+T411Y | Tra-E356K+T411Y*Per-K439E+T411Y |
| 46 | E356K+Y349S | K439E+S354Y | Tra-E356K+Y349S*Per-K439E+S354Y |
| 47 | E356K+Y349C | K439E+E357C | Tra-E356K+Y349C*Per-K439E+E357C |
| 48 | E356K+T366H | K439E+F405L | Tra-E356K+T366H*Per-K439E+F405L |
| 49 | E356K+T366H | K439E+Y407L | Tra-E356K+T366H*Per-K439E+Y407L |
| 50 | E356K+T366H | K439E+Y407H | Tra-E356K+T366H*Per-K439E+Y407H |
| 51 | E356K+F405T | K439E+T394F | Tra-E356K+F405T*Per-K439E+T394F |
| 52 | E356K+Y407L | K439E+T366H | Tra-E356K+Y407L*Per-K439E+T366H |
| 53 | E356K+Y407H | K439E+T366H | Tra-E356K+Y407H*Per-K439E+T366H |
| 54 | E356K+Y349C | K439E+S354C+L351I | Tra-E356K+Y349C*Per-K439E+S354C+L351I |
| 55 | E356K+Y349C | K439E+S354C+T366A | Tra-E356K+Y349C*Per-K439E+S354C+T366A |
| 56 | E356K+Y349C | K439E+S354C+L368I | Tra-E356K+Y349C*Per-K439E+S354C+L368I |
| 57 | Y349A | S354F | Tra-Y349A*Per-S354F |
| 58 | Y349S | S354F | Tra-Y349S*Per-S354F |
| 59 | Y349S | S354Y | Tra-Y349S*Per-S354Y |
| 60 | Y349V | S354F | Tra-Y349V*Per-S354F |
| 61 | Y349T | S354F | Tra-Y349T*Per-S354F |
| 62 | Y349G | S354W | Tra-Y349G*Per-S354W |
| 63 | Y349A | S354W | Tra-Y349A*Per-S354W |
| 64 | Y349S | S354W | Tra-Y349S*Per-S354W |
| 65 | Y349S | S364Y | Tra-Y349S*Per-S364Y |
| 66 | Y349C | E357C | Tra-Y349C*Per-E357C |
| 67 | Y349C | E357C+Q347E | Tra-Y349C*Per-E357C+Q347E |
| 68 | Y349C | S354C+L351I | Tra-Y349C*Per-S354C+L351I |
| 69 | Y349C | S354C+S364Y | Tra-Y349C*Per-S354C+S364Y |
| 70 | Y349C | S354C+S364F | Tra-Y349C*Per-S354C+S364F |
| 71 | Y349C | S354C+T366A | Tra-Y349C*Per-S354C+T366A |
| 72 | Y349C | S354C+L368I | Tra-Y349C*Per-S354C+L368I |
| 73 | Y349C | S354C+F405Y | Tra-Y349C*Per-S354C+F405Y |
| 74 | T366H | F405L | Tra-T366H*Per-F405L |
| 75 | T366H | Y407L | Tra-T366H*Per-Y407L |
| 76 | F405T | T394F | Tra-F405T*Per-T394F |
| 77 | F405L | T366H | Tra-F405L*Per-T366H |
| 78 | Y407L | T366H | Tra-Y407L*Per-T366H |
| 79 | F405T+S354C | T394F+Y349C | Tra-F405T+S354C*Per-T394F+Y349C |
| 80 | E356K+D399K | K439E+K409D | Tra-E356K+D399K*Per-K439E+K409D |
| 81 | E356K+K409D | K439E+D399K | Tra-E356K+K409D*Per-K439E+D399K |

| | | | |
|---|---|---|---|
| Note: * means that a recombination reaction was performed between the two monoclonal antibodies. | | | |

### Example 2.3: Recombination Method for Anti-HER2 Biparatopic Bispecific Antibodies

1700 mg of 2-MEA (Sigma-Aldrich, Cat. No./specification: 30078/100G) was weighed out and dissolved in 20 mL of PBS, and the pH was adjusted to 7.4 with 1 M NaOH. The amount-of-substance concentration of 2-MEA in this solution was about 750 mM. The concentrations of the trastuzumab and pertuzumab mutants prepared in the above examples were adjusted to the same amount-of-substance concentration (0.5-50 mg/mL) with PBS. An equal volume of a trastuzumab mutant was mixed well with a corresponding pertuzumab mutant, and a proper amount of the 2-MEA mother liquor was then added to and mixed well with the above antibody mixture, with the final concentration of 2-MEA being 75 mM. The mixture (containing the trastuzumab mutant, the pertuzumab mutant, and 2-MEA) was incubated in a water bath at 37 °C for 2.5 h. The mixture was transferred to Slide-A-Lyzer^{™} G3 Dialysis Cassettes (Thermo Fisher Scientific, Cat. No. A52971) and dialyzed against 5 L of PBS at room temperature. After 5 h, the PBS was refreshed, and the mixture was dialyzed in a cool store (4 °C). Samples were collected the following morning, and the concentrations were re-determined. The physicochemical properties of each sample and the corresponding parental monoclonal antibodies were assessed.

### Example 2.4: Recombination Efficiency of Anti-HER2 Biparatopic Bispecific Antibodies

Trastuzumab and pertuzumab bind to domains 4 and 2 of the extracellular segment of HER2, respectively. Based on these two monoclonal antibodies, anti-HER2 biparatopic bispecific antibodies can be prepared (reference: Weisser N E, Sanches M, Escobar-Cabrera E, et al. An anti-HER2 biparatopic antibody that induces unique HER2 clustering and complement-dependent cytotoxicity[J]. Nature Communications, 2023, 14(1): 1394). The bridging ELISA used in this example is described below: A microplate was coated with recombinant proteins corresponding to domains 1-3 of the extracellular segment of HER2 (purchased from ACROBiosystems, Cat: HE2-H52H9) and treated with a blocking solution (a phosphate buffer containing 1% bovine serum albumin and 0.05% Tween-20). The test antibodies were serially diluted and then transferred to the above microplate, and the plate was incubated at room temperature for half an hour. After the plate was washed, a recombinant protein corresponding to domain 4 of the extracellular segment of biotin-labeled HER2 (moderately diluted, purchased from Sino Biological, Cat: 10004-H08H4-B) was added, and the plate was incubated at room temperature for half an hour. After the plate was washed, streptavidin-peroxidase (moderately diluted, purchased from Sigma, Cat: S2438) was added, and the plate was incubated at room temperature for half an hour. After the plate was washed, 100 µL of a chromogenic solution with TMB (3,3',5,5'-tetramethylbenzidine) as a substrate was added to each well, and the plate was incubated at room temperature for 1-5 min. 50 µL of a stop solution (2 M H₂SO₄) was added to stop the reaction. OD450 was measured using a microplate reader (VERSA max). Plotting and data analysis were performed using GraphPad Prism 10, and EC₅₀ was calculated. Theoretically, a monoclonal antibody or a mixture of monoclonal antibodies cannot bind to domains 2 and 4 of the extracellular segment of HER2 simultaneously in this assay. In FIGs. 3A to 3K, the Top values represent the high plateaus of the fitted curves, and a larger Top value and a smaller EC₅₀ indicate stronger binding activity. (Tra+Per) Mix was a mixture of the monoclonal antibodies trastuzumab and pertuzumab (in an amount-of-substance ratio of 1:1) that did not undergo a recombination reaction. The binding parameters of the recombination reaction products to two epitopes of HER2 measured by bridging ELISA are shown in Table 8.

**Table 8. The ability of recombination reaction products to bind to two epitopes of HER2 simultaneously**

| **Figure No.** | **Control antibody or recombination reaction product** | **EC₅₀ (nM)** | **Top** |
|---|---|---|---|
| 3A | (Tra+Per) Mix | NA | NA |
| | Tra-F405L*Per-K409R | 0.58 | 2.99 |
| | Tra-K409R*Per-F405L | 0.51 | 3.06 |
| | Tra-E356K+V397L*Per-K439E+V397L | 0.40 | 2.88 |
| | Tra-E356K+V397M*Per-K439E+V397M | 0.54 | 2.90 |
| | Tra-E356K+V397T*Per-K439E+V397T | NA | NA |
| | Tra-E356K+V397I*Per-K439E+V397I | 0.70 | 2.24 |
| | Tra-E356K+F405Y*Per-K439E+F405Y | 0.54 | 3.19 |
| | Tra-E356K+F405L*Per-K439E+F405L | 0.64 | 3.05 |
| | Tra-E356K+T411L*Per-K439E+T411L | 0.83 | 2.95 |
| | Tra-E356K+T411Y*Per-K439E+T411Y | 0.57 | 3.02 |
| | Tra-E356K+L351C*Per-K439E+L351C | 0.97 | 2.89 |
| 3B | (Tra+Per) Mix | NA | NA |
| | Tra-F405L*Per-K409R | 0.53 | 2.71 |
| | Tra-K409R*Per-F405L | 0.44 | 2.67 |
| | Tra-E356K+T394C*Per-K439E+T394C | 0.62 | 1.70 |
| | Tra-E356K+Y349C*Per-K439E+E357C | 0.40 | 2.62 |
| | Tra-E356K+T366G*Per-K439E+T366G | 0.56 | 2.39 |
| | Tra-E356K+T366S*Per-K439E+T366S | NA | NA |
| | Tra-E356K+Y349L*Per-K439E+Y349L | 1.38 | 2.42 |
| | Tra-E356K+L351I*Per-K439E+L351I | 0.71 | 2.54 |
| | Tra-E356K+L351T*Per-K439E+L351T | 0.63 | 2.72 |
| | Tra-E356K+L351F*Per-K439E+L351F | 0.76 | 2.49 |
| 3C | (Tra+Per) Mix | NA | NA |
| | Tra-F405L*Per-K409R | 0.52 | 2.65 |
| | Tra-K409R*Per-F405L | 0.46 | 2.69 |
| | Tra-E356K+T394A*Per-K439E+T394A | 0.46 | 2.46 |
| | Tra-E356K+T394N*Per-K439E+T394N | 0.49 | 2.79 |
| | Tra-E356K+T366V*Per-K439E+T366V | 0.64 | 2.87 |
| | Tra-E356K+T366A*Per-K439E+T366A | 0.55 | 2.54 |
| | Tra-E356K+L368A*Per-K439E+L368A | 0.69 | 2.59 |
| | Tra-E356K+Y407L*Per-K439E+Y407L | 0.81 | 2.94 |
| | Tra-E356K+Y407V*Per-K439E+Y407V | 0.78 | 2.67 |
| | Tra-Y407L*Per-T366H | 0.95 | 2.31 |
| 3D | (Tra+Per) Mix | NA | NA |
| | Tra-F405L*Per-K409R | 0.42 | 2.66 |
| | Tra-K409R*Per-F405L | 0.42 | 2.64 |
| | Tra-K439E*Per-E356K | NA | NA |
| | Tra-E356K*Per-K439E | NA | NA |
| | Tra-E356K+Y407C*Per-K439E+Y407C | 0.50 | 2.64 |
| | Tra-E356K+S364T*Per-K439E+S364T | 0.39 | 2.45 |
| | Tra-E356K+T394V*Per-K439E+T394V | 0.43 | 2.62 |
| | Tra-E356K+K409R*Per-K439E+K409R | 0.38 | 2.72 |
| | Tra-E356K+K409Q* Per-K439E+K409Q | 0.34 | 2.69 |
| 3E | (Tra+Per) Mix | NA | NA |
| | Tra-F405L*Per-K409R | 0.42 | 2.38 |
| | Tra-K409R*Per-F405L | 0.51 | 2.56 |
| | Tra-E356K+T366L*Per-K439E+T366L | 0.62 | 2.72 |
| | Tra-E356K+T366I*Per-K439E+T366I | 0.64 | 2.79 |
| | Tra-E356K+L368V*Per-K439E+L368V | 0.77 | 2.85 |
| | Tra-E356K+Y407F*Per-K439E+Y407F | 0.64 | 2.60 |
| | Tra-E356K+Y407H*Per-K439E+Y407H | 0.78 | 2.94 |
| | Tra-E356K+T366H*Per-K439E+Y407H | 0.52 | 2.93 |
| | Tra-E356K+T366H*Per-K439E+Y407L | 0.37 | 2.92 |
| | Tra-F405L*Per-T366H | 0.59 | 2.45 |
| 3F | (Tra+Per) Mix | NA | NA |
| | Tra-F405L*Per-K409R | 0.48 | 2.16 |
| | Tra-K409R*Per-F405L | 0.40 | 2.23 |
| | Tra-E356K+L351V*Per-K439E+L351V | 0.41 | 2.15 |
| | Tra-E356K+S364A*Per-K439E+S364A | 0.39 | 2.01 |
| | Tra-E356K+S364L*Per-K439E+S364L | 0.56 | 2.33 |
| | Tra-E356K+T394S *Per-K439E+T394S | 0.48 | 2.00 |
| | Tra-E356K+L368I*Per-K439E+L368I | 0.72 | 2.27 |
| | Tra-E356K+L368M*Per-K439E+L368M | 0.90 | 0.83 |
| | Tra-E356K+T366H*Per-K439E+T366H | 1.04 | 2.38 |
| | Tra-E356K+Y407H*Per-K439E+T366H | 0.88 | 2.30 |
| | Tra-E356K+Y349S*Per-K439E+S354Y | 0.55 | 2.27 |
| 3G | (Tra+Per) Mix | NA | NA |
| | Tra-F405L*Per-K409R | 0.47 | 1.98 |
| | Tra-K409R*Per-F405L | 0.43 | 2.06 |
| | Tra-Y349S*Per-S354Y | 0.38 | 1.79 |
| | Tra-F405T*Per-T394F | 0.39 | 2.06 |
| | Tra-E356K+Y349C*Per-K439E+S354C+L368I | 0.43 | 1.99 |
| | Tra-E356K+Y349C*Per-K439E+S354C+L351I | 0.47 | 1.81 |
| | Tra-E356K+Y349C*Per-K439E+S354C+T366A | 0.62 | 1.90 |
| | Tra-Y349C*Per-S354C+T366A | 0.75 | 1.77 |
| | Tra-Y349C*Per-E357C | 0.90 | 1.90 |
| | Tra-Y349C*Per-E357C+Q347E | 0.71 | 1.95 |
| | Tra-E356K+Y407L*Per-K439E+T366H | 0.49 | 2.12 |
| 3H | (Tra+Per) Mix | NA | NA |
| | Tra-F405L*Per-K409R | 0.45 | 2.08 |
| | Tra-K409R*Per-F405L | 0.46 | 2.13 |
| | Tra-T366H*Per-Y407L | 0.61 | 2.02 |
| | Tra-E356K+T366H*Per-K439E+F405L | 0.50 | 2.49 |
| | Tra-T366H*Per-F405L | 0.49 | 1.67 |
| | Tra-E356K+D399K*Per-K439E+K409D | 0.57 | 2.30 |
| | Tra-E356K+K409D*Per-K439E+D399K | 0.86 | 2.43 |
| | Tra-Y349V*Per-S354F | 0.96 | 2.01 |
| | Tra-Y349T*Per-S354F | 1.15 | 2.17 |
| | Tra-Y349S*Per-S354F | 0.78 | 2.14 |
| 3I | (Tra+Per) Mix | NA | NA |
| | Tra-F405L*Per-K409R | 0.40 | 2.49 |
| | Tra-K409R*Per-F405L | 0.45 | 2.57 |
| | Tra-E356K+F405T*Per-K439E+T394F | 0.84 | 3.00 |
| | Tra-Y349C*Per-S354C+L351I | 0.71 | 1.47 |
| | Tra-Y349C*Per-S354C+F405Y | 0.63 | 2.42 |
| | Tra-E356K+L351M*Per-K439E+L351M | 0.38 | 1.08 |
| 3J | (Tra+Per) Mix | NA | NA |
| | Tra-F405L*Per-K409R | 0.83 | 2.24 |
| | Tra-K409R*Per-F405L | 0.89 | 2.36 |
| | Tra-Y349C*Per-S354C+L368I | 1.07 | 2.16 |
| | Tra-Y349A*Per-S354F | 0.85 | 2.07 |
| | Tra-Y349S*Per-S354W | 0.73 | 2.11 |
| | Tra-Y349A*Per-S354W | 0.77 | 2.19 |
| | Tra-Y349G*Per-S354W | 0.70 | 2.28 |
| 3K | (Tra+Per) Mix | NA | NA |
| | Tra-F405L*Per-K409R | 0.63 | 2.34 |
| | Tra-K409R*Per-F405L | 0.73 | 2.25 |
| | Tra-Y349S*Per-S364Y | 0.89 | 2.52 |
| | Tra-Y349C*Per-S354C+S364Y | 0.74 | 2.54 |
| | Tra-Y349C*Per-S354C+S364F | 0.71 | 2.41 |
| | Tra-F405T+S354C*Per-T394F+Y349C | 0.81 | 2.75 |

| | | | |
|---|---|---|---|
| Note: NA means that the binding signal was weak, and a curve could not be fitted accurately, so this parameter cannot be provided. | | | |

FIGs. 3A-3K and Table 8 show that the OD corresponding to (Tra+Per) Mix was close to the background value, indicating that it could not bind to two epitopes of HER2 simultaneously; the recombinant products Tra-F405L*Per-K409R and Tra-K409R*Per-F405L could bind to two epitopes of HER2 simultaneously; the signal curves of Tra-K439E*Per-E356K, Tra-E356K*Per-K439E, Tra-E356K+V397T*Per-K439E+V397T, and Tra-E356K+T366S*Per-K439E+T366S almost overlap with that of (Tra+Per) Mix, indicating that these four combinations could not produce bispecific antibodies under the conditions of this example; the recombination reaction products of most of the other combinations could bind to two epitopes of HER2 simultaneously and effectively, indicating that these recombination reactions yielded bispecific antibodies. The experimental data shown in this example can be used to roughly determine the bispecific antibody production efficiency of various recombination reactions.

### Example 2.5: Recombination Efficiency of Anti-HER2 Biparatopic Bispecific Antibodies

The physicochemical property analysis methods used in this example are described below.
1. The methods and conditions for HPLC-SEC and HPLC-IEC were the same as or similar to those in the previous examples.
2. High-performance liquid chromatography-reversed-phase (HPLC-RP)

An Agilent 1290 UPLC DAD chromatograph was used; the mobile phases were prepared as follows: mobile phase A was a 0.1% aqueous perchloric acid solution, and mobile phase B was acetonitrile; gradient elution B%: 0-15 min (20%-45%); 25 min (85%); 25.1-30 min (20%); injection amount: 3 µg; chromatography column: Agilent AdvanceBio RP-mAb Diphenyl, 2.1 × 100 mm, 3.5 µM; flow rate: 0.3 mL/min; elution time: 30 min; column temperature: 60 °C; sample chamber temperature: 15 °C; detection wavelength: 280 nm.

The parental monoclonal antibodies and corresponding recombination reaction products (containing bispecific antibodies) produced in the above examples were analyzed by HPLC-SEC, HPLC-IEC, and HPLC-RP. The obtained data on purity (here, the SEC purity was the proportion of the area of the main peak, the IEC purity was the sum of the proportions of the areas of primary peaks such as the main peak, the acidic peak, and the basic peak, and the RP purity was the proportion of the area of the main peak) are summarized in Table 9. The isoelectric points of the trastuzumab mutants with E356K and the pertuzumab mutants with K439E were significantly different (with a difference of around 0.5). Therefore, the parental monoclonal antibodies and the corresponding bispecific antibodies could be effectively separated by HPLC-IEC. However, due to their similar isoelectric points (with a difference of around 0.1 only), the trastuzumab mutants without E356K and the pertuzumab mutants without K439E could not be effectively separated by HPLC-IEC. Therefore, the remaining recombination reaction products were analyzed by HPLC-RP.

**Table 9. The SEC purity of parental monoclonal antibodies and the SEC and IEC purity of corresponding bispecific antibodies**

| **No.** | **Parental monoclonal antibody** | | **Recombination reaction product** | |
|---|---|---|---|---|
| | **Name** | **SEC (%)** | **SEC (%)** | **IEC/RP (%)** |
| 1 | Tra-E356K | 99.63 | 98.97 | NA |
| | Per-K439E | 99.65 | | |
| 2 | Tra-K439E | 99.62 | 99.24 | NA |
| | Per-E356K | 99.24 | | |
| 3 | Tra-E356K+L351C | 99.12 | 98.92 | 88.94 |
| | Per-K439E+L351C | 99.68 | | |
| 4 | Tra-E356K+L351V | 95.97 | 97.71 | 97.42 |
| | Per-K439E+L351V | 97.93 | | |
| 5 | Tra-E356K+L351I | 96.55 | 98.62 | 98.20 |
| | Per-K439E+L351I | 98.15 | | |
| 6 | Tra-E356K+L351M | 99.17 | 99.25 | 54.50 |
| | Per-K439E+L351M | 99.79 | | |
| 7 | Tra-E356K+L351F | 98.96 | 99.12 | 83.26 |
| | Per-K439E+L351F | 99.01 | | |
| 8 | Tra-E356K+S364A | 95.21 | 97.28 | 96.22 |
| | Per-K439E+S364A | 97.12 | | |
| 9 | Tra-E356K+S364T | 99.57 | 98.79 | 85.89 |
| | Per-K439E+S364T | 99.60 | | |
| 10 | Tra-E356K+S364L | 78.16 | 91.78 | 91.80 |
| | Per-K439E+S364L | 93.80 | | |
| 11 | Tra-E356K+T366G | 98.20 | 98.48 | 96.17 |
| | Per-K439E+T366G | 98.96 | | |
| 12 | Tra-E356K+T366A | 90.15 | 96.59 | 97.38 |
| | Per-K439E+T366A | 96.71 | | |
| 13 | Tra-E356K+T366V | 63.33 | 90.01 | 77.10 |
| | Per-K439E+T366V | 86.50 | | |
| 14 | Tra-E356K+T366L | 73.66 | 90.85 | 87.12 |
| | Per-K439E+T366L | 83.32 | | |
| 15 | Tra-E356K+T366I | 69.03 | 91.56 | 90.70 |
| | Per-K439E+T366I | 87.48 | | |
| 16 | Tra-E356K+T366H | 72.99 | 92.74 | 93.85 |
| | Per-K439E+T366H | 93.53 | | |
| 17 | Tra-E356K+L368A | 90.01 | 97.34 | 96.80 |
| | Per-K439E+L368A | 97.09 | | |
| 18 | Tra-E356K+L368V | 85.51 | 94.64 | 89.10 |
| | Per-K439E+L368V | 95.78 | | |
| 19 | Tra-E356K+L368M | 98.24 | 97.82 | 38.19 |
| | Per-K439E+L368M | 97.98 | | |
| 20 | Tra-E356K+T394A | 98.16 | 98.81 | 97.18 |
| | Per-K439E+T394A | 99.31 | | |
| 21 | Tra-E356K+T394S | 96.85 | 98.51 | 95.99 |
| | Per-K439E+T394S | 98.16 | | |
| 22 | Tra-E356K+T394C | 99.17 | 99.18 | 60.81 |
| | Per-K439E+T394C | 99.55 | | |
| 23 | Tra-E356K+T394V | 99.40 | 98.82 | 92.51 |
| | Per-K439E+T394V | 99.75 | | |
| 24 | Tra-E356K+T394N | 82.98 | 94.07 | 95.52 |
| | Per-K439E+T394N | 92.63 | | |
| 25 | Tra-E356K+V397I | 99.53 | 98.89 | 65.18 |
| | Per-K439E+V397I | 99.44 | | |
| 26 | Tra-E356K+V397M | 99.38 | 99.03 | 86.84 |
| | Per-K439E+V397M | 99.67 | | |
| 27 | Tra-E356K+Y407C | 73.40 | 95.77 | 93.25 |
| | Per-K439E+Y407C | 90.56 | | |
| 28 | Tra-E356K+Y407V | 81.14 | 94.47 | 94.61 |
| | Per-K439E+Y407V | 93.41 | | |
| 29 | Tra-E356K+Y407L | 75.74 | 89.35 | 96.13 |
| | Per-K439E+Y407L | 86.34 | | |
| 30 | Tra-E356K+Y407H | 58.30 | 67.50 | 69.88 |
| | Per-K439E+Y407H | 56.32 | | |
| 31 | Tra-E356K+Y407F | 98.05 | 98.65 | 94.24 |
| | Per-K439E+Y407F | 99.65 | | |
| 32 | Tra-E356K+T411L | 98.27 | 98.55 | 97.27 |
| | Per-K439E+T411L | 99.61 | | |
| 33 | Tra-E356K+Y349S | 93.13 | 95.82 | 95.53 |
| | Per-K439E+S354Y | 95.15 | | |
| 34 | Tra-E356K+Y349C | 85.28 | 94.75 | 91.50 |
| | Per-K439E+E357C | 81.59 | | |
| 35 | Tra-E356K+T366H | 72.40 | 84.29 | 83.56 |
| | Per-K439E+Y407L | 86.50 | | |
| 36 | Tra-E356K+T366H | 72.99 | 85.57 | 87.88 |
| | Per-K439E+F405L | 91.65 | | |
| 37 | Tra-E356K+T366H | 72.40 | 73.84 | 91.76 |
| | Per-K439E+Y407H | 56.32 | | |
| 38 | Tra-E356K+F405T | 60.88 | 93.11 | 94.73 |
| | Per-K439E+T394F | 93.21 | | |
| 39 | Tra-E356K+Y407L | 76.12 | 91.77 | 93.99 |
| | Per-K439E+T366H | 93.53 | | |
| 40 | Tra-E356K+Y407H | 57.72 | 90.86 | 83.92 |
| | Per-K439E+T366H | 93.53 | | |
| 41 | Tra-E356K+Y349C | 90.14 | 97.21 | 97.23 |
| | Per-K439E+S354C+L351I | 98.05 | | |
| 42 | Tra-E356K+Y349C | 90.14 | 95.90 | 97.06 |
| | Per-K439E+S354C+T366A | 91.66 | | |
| 43 | Tra-E356K+Y349C | 90.14 | 91.84 | 95.78 |
| | Per-K439E+S354C+L368I | 95.08 | | |
| 44 | Tra-Y349S | 96.05 | 97.50 | 88.02 |
| | Per-S354F | 95.59 | | |
| 45 | Tra-Y349S | 96.05 | 97.49 | 86.82 |
| | Per-S354Y | 95.83 | | |
| 46 | Tra-Y349V | 95.51 | 96.90 | 70.35 |
| | Per-S354F | 95.59 | | |
| 47 | Tra-Y349T | 93.55 | 96.71 | 69.78 |
| | Per-S354F | 95.59 | | |
| 48 | Tra-Y349G | 95.14 | 98.18 | 92.45 |
| | Per-S354W | 98.54 | | |
| 49 | Tra-Y349A | 97.37 | 97.30 | 93.06 |
| | Per-S354W | 98.54 | | |
| 50 | Tra-Y349S | 97.83 | 97.73 | 88.77 |
| | Per-S354W | 98.54 | | |
| 51 | Tra-Y349S | 97.10 | 97.19 | 94.43 |
| | Per-S364Y | 96.26 | | |
| 52 | Tra-Y349C | 94.86 | 96.74 | 94.66 |
| | Per-E357C | 74.05 | | |
| 53 | Tra-Y349C | 94.86 | 95.34 | 89.59 |
| | Per-E357C+Q347E | 67.14 | | |
| 54 | Tra-Y349C | 94.86 | 97.90 | 72.62 |
| | Per-S354C+L351I | 97.28 | | |
| 55 | Tra-Y349C | 95.20 | 93.37 | 92.92 |
| | Per-S354C+S364Y | 93.60 | | |
| 56 | Tra-Y349C | 95.20 | 96.53 | 95.69 |
| | Per-S354C+S364F | 94.20 | | |
| 57 | Tra-Y349C | 94.86 | 97.74 | 86.99 |
| | Per-S354C+T366A | 96.23 | | |
| 58 | Tra-Y349C | 97.53 | 98.72 | 77.04 |
| | Per-S354C+L368I | 96.18 | | |
| 59 | Tra-Y349C | 94.86 | 97.49 | 79.14 |
| | Per-S354C+F405Y | 93.98 | | |
| 60 | Tra-T366H | 91.31 | 96.50 | 70.05 |
| | Per-F405L | 93.20 | | |
| 61 | Tra-T366H | 91.31 | 94.19 | 63.63 |
| | Per-Y407L | 89.02 | | |
| 62 | Tra-F405L | 98.36 | 97.49 | 68.04 |
| | Per-T366H | 90.07 | | |
| 63 | Tra-Y407L | 92.38 | 96.21 | 64.15 |
| | Per-T366H | 90.03 | | |
| 64 | Tra-F405T+S354C | 78.20 | 87.83 | 89.63 |
| | Per-T394F+Y349C | 72.80 | | |
| 65 | Tra-E356K+D399K | 51.80 | 89.82 | 90.50 |
| | Per-K439E+K409D | 92.82 | | |
| 66 | Tra-E356K+K409D | 85.68 | 91.74 | 93.89 |
| | Per-K439E+D399K | 80.90 | | |

| | | | | |
|---|---|---|---|---|
| Note: NA means that it could not be determined accurately. | | | | |

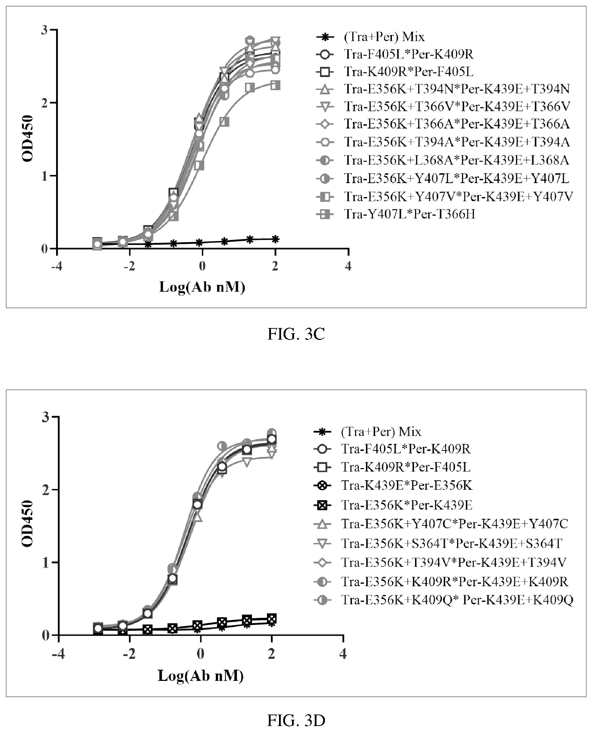

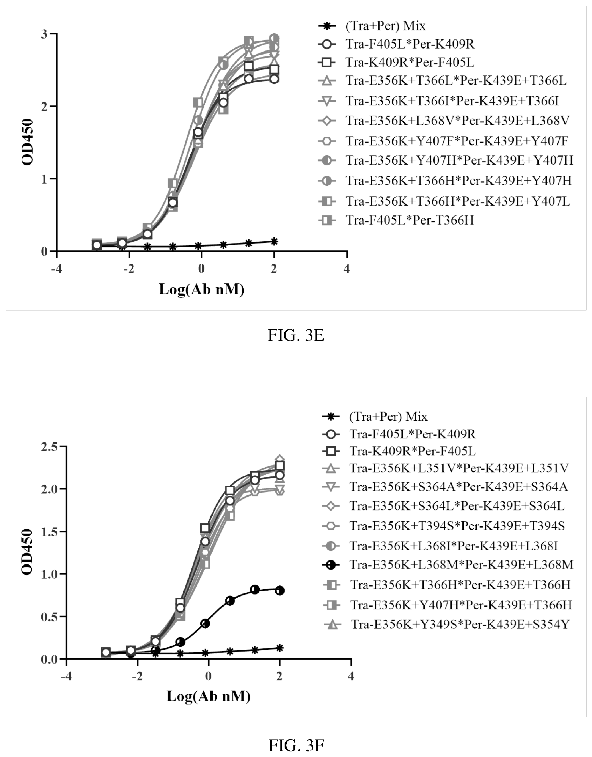

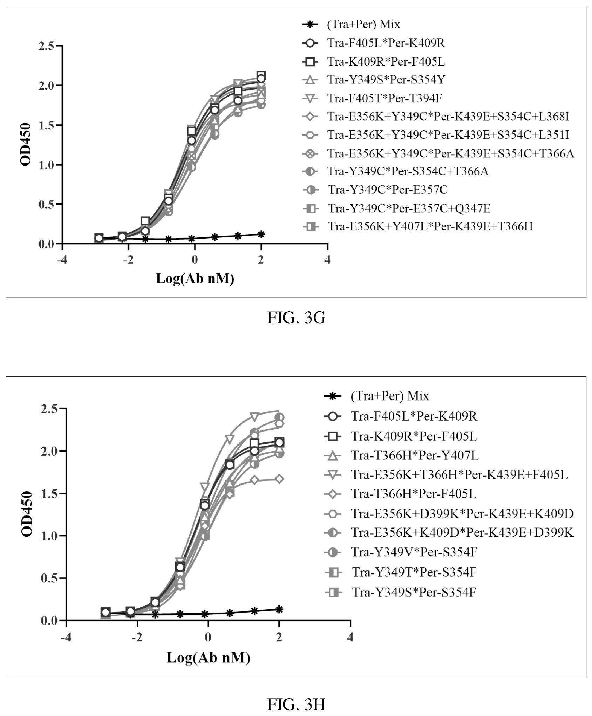

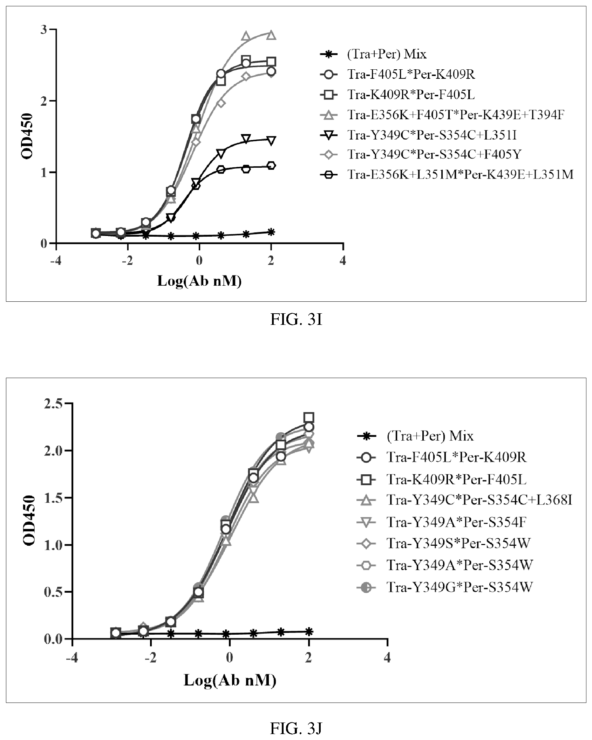

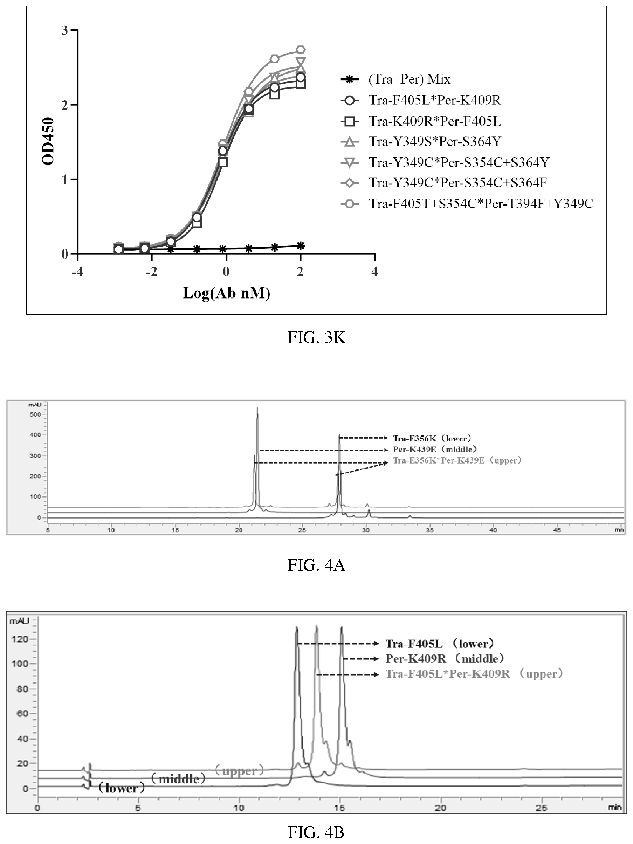

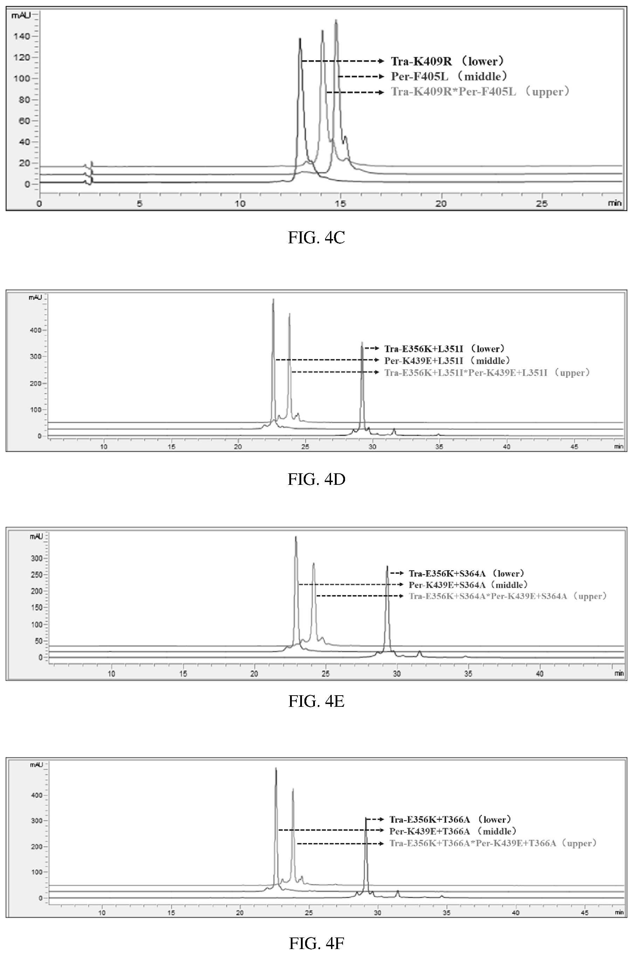

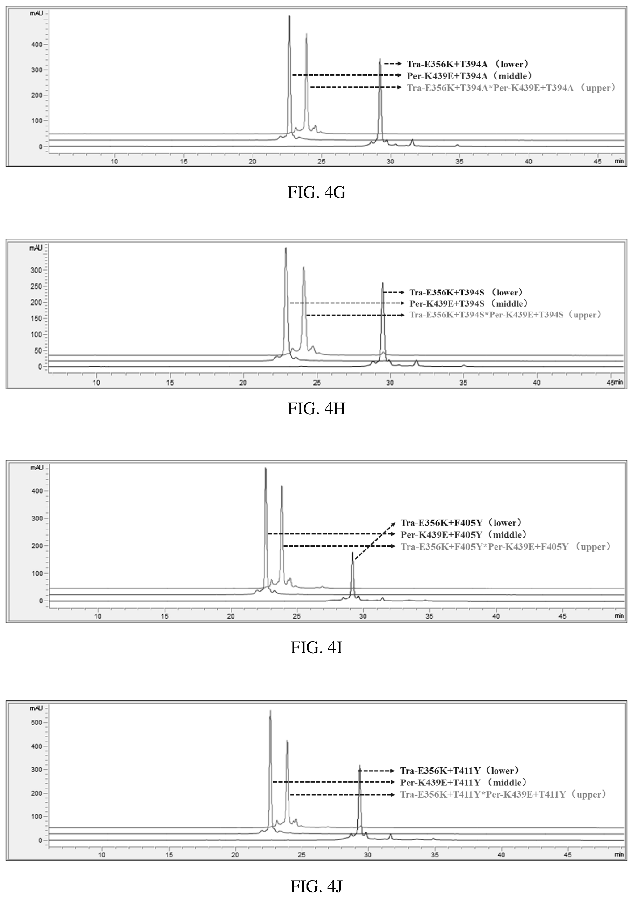

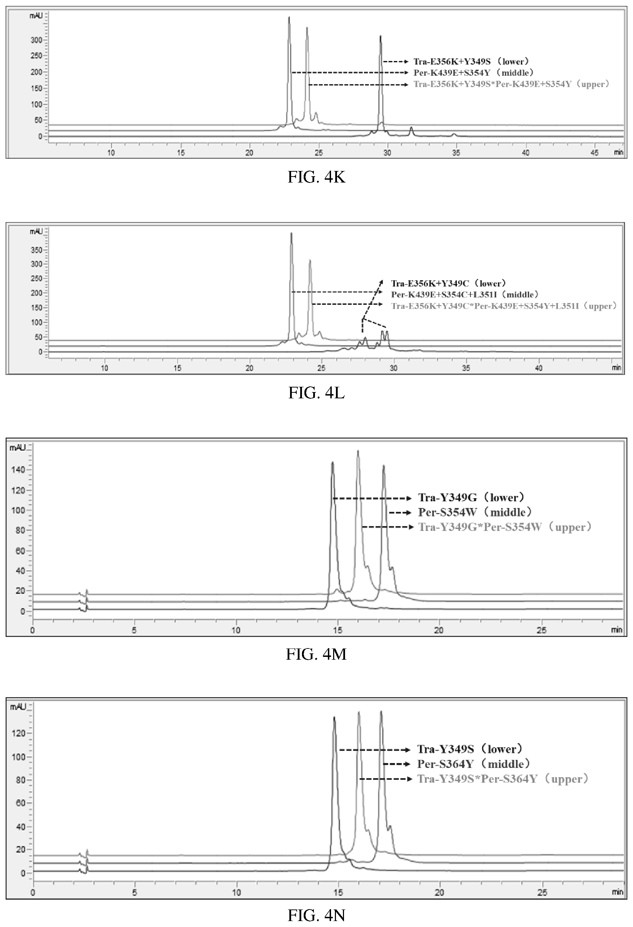

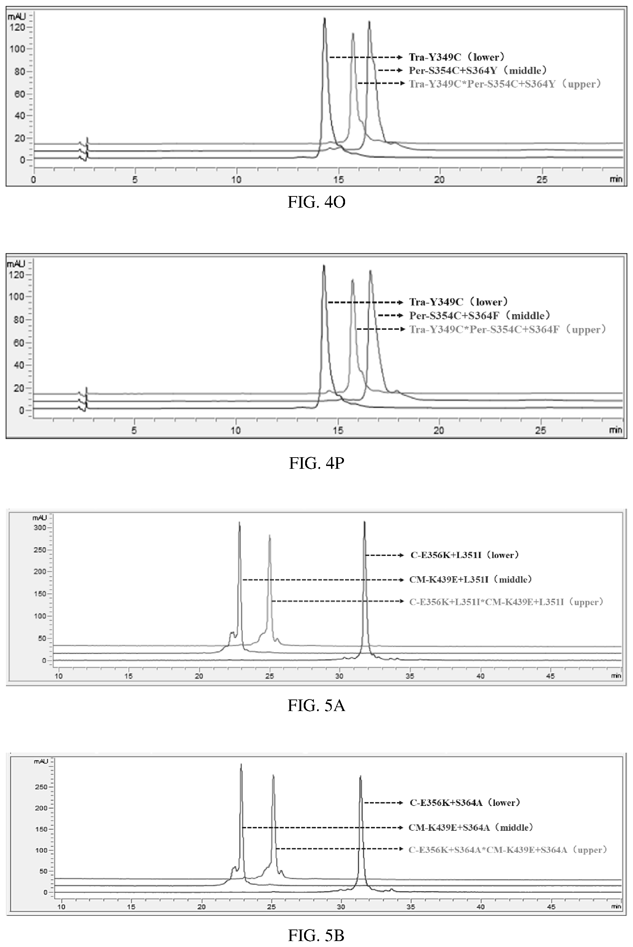

The data in FIG. 3, Table 8, and Table 9 were comprehensively analyzed. Preferably, the HPLC-IEC or HPLC-RP chromatograms of some parental monoclonal antibodies and corresponding recombination reaction products were superimposed. The selected combinations included Tra-E356K*Per-K439E (FIG. 4A), Tra-F405L*Per-K409R (FIG. 4B), Tra-K409R*Per-F405L (FIG. 4C), Tra-E356K+L351I*Per-K439E+L351I (FIG. 4D), Tra-E356K+S364A*Per-K439E+S364A (FIG. 4E), Tra-E356K+T366A*PerK439E+T366A (FIG. 4F), Tra-E356K+T394A*Per-K439E+T394A (FIG. 4G), Tra-E356K+T394S*Per-K439E+T394S (FIG. 4H), Tra-E356K+F405Y*Per-K439E+F405Y (FIG. 4I), Tra-E356K+T411Y*Per-K439E+T411Y (FIG. 4J), Tra-E356K+Y349S*PerK439E+S354Y (FIG. 4K), Tra-E356K+Y349C*Per-K439E+S354C+L3511 (FIG. 4L), Tra-Y349G*Per-S354W (FIG. 4M), Tra-Y349S*Per-S364Y (FIG. 4N), Tra-Y349C*Per-S354C+S364Y (FIG. 4O), and Tra-Y349C*Per-S354C+S364F (FIG. 4P).

If a recombination reaction yields a bispecific antibody, new peaks corresponding to the bispecific antibody will be found in the chromatogram, and the peaks at the positions corresponding to the parental monoclonal antibodies will relatively decrease. The percentage of the area of the peaks corresponding to the parental monoclonal antibodies is the amount that remains after the recombination reaction. FIG. 4A shows that no new peaks could be found in the chromatograms of Tra-E356K and Per-K439E under the conditions of this example, indicating that the reaction could not effectively cause recombination. FIGs. 4B to 4P show new peaks in the chromatograms of the recombination reaction products, indicating the generation of recombinant antibodies.

The formula for calculating the efficiency of a recombination reaction was: 100% - (percentage of area of peaks corresponding to parental monoclonal antibody A + percentage of area of peaks corresponding to parental monoclonal antibody B). The monoclonal antibodies used for recombination reactions were not subjected to fine purification. As a result, aggregates and a small amount of impurities could be present.

**Table 10. Recombination reaction efficiency calculations based on the amount of parental monoclonal antibody residues**

| **Parental monoclonal antibody** | | **Recombination reaction efficiency (%)** | **Figure No.** |
|---|---|---|---|
| **Name** | **Amount of residues (%)** | | |
| Tra-E356K | 49.0 | 0.3% | 4A |
| Per-K439E | 50.7 | | |
| Tra-F405L | 3.9 | 90.0 | 4B |
| Per-K409R | 6.1 | | |
| Tra-K409R | 3.3 | 88.8 | 4C |
| Per-F405L | 7.9 | | |
| Tra-E356K+L351I | 0.2 | 99.0 | 4D |
| Per-K439E+L351I | 0.9 | | |
| Tra-E356K+S364A | 0.3 | 97.4 | 4E |
| Per-K439E+S364A | 2.3 | | |
| Tra-E356K+T366A | 0.0 | 98.7 | 4F |
| Per-K439E+T366A | 1.3 | | |
| Tra-E356K+T394A | 0.0 | 98.6 | 4G |
| Per-K439E+T394A | 1.4 | | |
| Tra-E356K+T394S | 1.1 | 96.5 | 4H |
| Per-K439E+T394S | 2.5 | | |
| Tra-E356K+F405Y | 0.4 | 97.6 | 4I |
| Per-K439E+F405Y | 2.0 | | |
| Tra-E356K+T411Y | 1.6 | 96.5 | 4J |
| Per-K439E+T411Y | 1.9 | | |
| Tra-E356K+Y349S | 2.1 | 96.7 | 4K |
| Per-K439E+S354Y | 1.2 | | |
| Tra-E356K+Y349C | 1.4 | 97.0 | 4L |
| Per-K439E+S354C+L351I | 1.6 | | |
| Tra-Y349G | 3.2 | 92.5 | 4M |
| Per-S354W | 4.4 | | |
| Tra-Y349S | 1.9 | 95.0 | 4N |
| Per-S364Y | 3.0 | | |
| Tra-Y349C | 0.8 | 92.9 | 4O |
| Per-S354C+S364Y | 6.2 | | |
| Tra-Y349C | 1.8 | 95.7 | 4P |
| Per-S354C+S364F | 2.5 | | |

The above results (FIGs. 4A-4P and Table 10) show that in this example, the recombination reaction of Tra-E356K*Per-K439E was hardly detected, and the recombination reaction efficiency values of Tra-F405L*Per-K409R and Tra-K409R*Per-F405L were 90.0% and 88.8%, respectively, while the recombination reaction efficiency of all the other combinations in Table 10 was higher than 92.0%, with the highest efficiency being 99.0%.

3. The method and conditions for determining the molecular weights of deglycosylated antibodies by mass spectrometry were the same as those in the previous examples.

**Table 11. The results of mass spectrometry analysis of bispecific antibodies**

| **Parental monoclonal antibody** | | **Recombination reaction product** | |
|---|---|---|---|
| **Name** | **Calculated heavy chain/light chain molecular weight** | **Calculated intact molecular weight** | **Found heavy chain/light chain/intact molecular weight** |
| Tra-E356K+L351I | 49156/23443 | 145183 | 49154/49089/23442/23526/145185 |
| Per-K439E+L351I | 49090/23526 | | |
| Tra-E356K+S364A | 49140/23443 | 145151 | 49139/49073/23442/23526/145153 |
| Per-K439E+S364A | 49073/23526 | | |
| Tra-E356K+T366A | 49126/23443 | 145123 | 49124/49060/23442/23526/145126 |
| Per-K439E+T366A | 49060 /23526 | | |
| Tra-E356K+T394A | 49126/23443 | 145123 | 49123/49059/23442/23526/145125 |
| Per-K439E+T394A | 49060/23526 | | |
| Tra-E356K+T394S | 49142/23443 | 145155 | 49140/49076/23442/23526/145157 |
| Per-K439E+T394S | 49076/23526 | | |
| Tra-E356K+F405Y | 49172/23443 | 145215 | 49169/49106/23442/23526/145216 |
| Per-K439E+F405Y | 49106/23526 | | |
| Tra-E356K+T411Y | 49218/23443 | 145307 | 49217/49152/23442/23526/145309 |
| Per-K439E+T411Y | 49152/23526 | | |
| Tra-E356K+Y349S | 49081/23443 | 145183 | 49079/49165/23442/23526/145186 |
| Per-K439E+S354Y | 49165/23526 | | |
| Tra-E356K+Y349C | 49096/23443 | 145139 | 49098/49098/23442/23526/145141 |
| Per-K439E+S354C+L351I | 49106/23526 | | |
| Tra-F405T | 49111/23443 | 145183 | 49111/49134/23442/23256/145186 |
| Per-T394F | 49135/23526 | | |
| Tra-Y349G | 49043/23439 | 145176 | 49043/49181/23439/23522/145176 |
| Per-S354W | 49180/23522 | | |
| Tra-Y349S | 49073/23439 | 145183 | 49073/49157/23439/23522/145184 |
| Per-S364Y | 49157/23522 | | |
| Tra-Y349C | 49089/23439 | 145216 | 49088/49172/23439/23523/145214 |
| Per-S354C+S364Y | 49173/23522 | | |
| Tra-Y349C | 49089/23439 | 145199 | 49090/49157/23439/23520/145198 |
| Per-S354C+S364F | 49157/23522 | | |

| | | | |
|---|---|---|---|
| Note: The calculated heavy chain, light chain, and intact molecular weights in this table are theoretical values calculated by software with the post-translational modifications and redox states of the proteins taken into account. Therefore, the calculated molecular weights of the same light chain under different experimental conditions may be different. The results in Table 11 show that the calculated heavy chain, light chain, and intact molecular weights of the bispecific antibodies produced by the above combinations were very close to the actual heavy chain, light chain, and intact molecular weights measured by mass spectrometry, which indicates that the recombination and structures of the bispecific antibody molecules were in line with expectations. The mass spectra of the recombinant samples did not show peaks corresponding to the parental monoclonal antibodies, indicating that the above recombination reactions were relatively complete. | | | |

### Example 3.1: Source and Humanization of Mouse Anti-Human CD3 Monoclonal Antibody

The amino acid sequences of the heavy and light chain variable regions of the mouse anti-human CD3 monoclonal antibody (anti-CD3) prepared by hybridoma technology were from SEQ ID NOs: 2 and 4 in US 2009/0252683 A1. The amino acid sequences of the heavy chain variable region (VH) and light chain variable region (VL) of anti-CD3 were analyzed, and the framework regions (FRs) and complementarity determining regions (CDRs) of the VH and VL were determined according to the Kabat numbering scheme. The sequences of the heavy and light chain variable regions of anti-CD3 are shown below.
> The amino acid sequence of anti-CD3-VH (SEQ ID NO: 19): 
EVKLLESGGGLVQPKGSLKLSCAASGFTFNTYAMNWVRQAPGKGLEWVARIRS
KYNNYATYYADSVKDRFTISRDDSQSILYLQMNNLKTEDTAMYYCVRHGNFGN
SYVSWFAYWGQGTLVTVSA
> The amino acid sequence of anti-CD3-VL (SEQ ID NO: 20):

QAVVTQESALTTSPGETVTLTCRSSTGAVTTSNYANWVQEKPDHLFTGLIGGTN
KRAPGVPARFSGSLIGDKAALTITGAQTEDEAIYFCALWYSNLWVFGGGTKLTV

L

The process of humanizing the murine-derived anti-CD3 antibody is described below. By using the IgBlast tool, anti-CD3-VH and anti-CD3-VL were each compared with the NCBI human antibody variable region germline gene database. Through homology analysis, IGHV3-23*05 and IGLV7-43*01 were selected here as templates for the humanization of anti-CD3-VH and anti-CD3-VL, respectively. The CDRs of anti-CD3-VH and anti-CD3-VL were grafted into corresponding humanization templates, and WGQGTTVTVSS and FGGGTKLTVL were selected as the FR4 regions of the VH and VL, respectively, to form CDR-grafted VH and VL sequences with a primary structure of FR1-CDR1-FR2-CDR2-FR3-CDR3-FR4.

Here, amino acid residues of the CDR-grafted VH and VL were back-mutated or mutated otherwise.

The amino acid residues at positions 73, 94, 96, and 100 of the CDR-grafted VH were mutated, with a specific mutation combination of N73D, K94R, G96E, and N100Q, to obtain a humanized VH, designated anti-CD3-Hu-VH. The amino acid residues at positions 24, 33, 36, 46, 49, 57, 58, and 94 of the CDR-grafted VL were mutated, with a specific mutation combination of R24A, A33P, F36V, A46G, Y49G, W57G, T58V, and N94E, to obtain a humanized VL, designated anti-CD3-Hu-VL.

**Table 12. The CDR sequences of the anti-CD3-Hu antibody**

| Antibody | Heavy chain | | Light chain | |
|---|---|---|---|---|
| | HCDR1 | TYAMN (SEQ ID NO: 21) | LCDR1 | ASSTGAVTTSNYPN (SEQ ID NO: 24) |
| anti-CD3-Hu | HCDR2 | RIRSKYNNYATYYAD SVKD (SEQ ID NO: 22) | LCDR2 | GTNKRAP (SEQ ID NO: 25) |
| | HCDR3 | HENFGQSYVSWFAY (SEQ ID NO: 23) | LCDR3 | ALWYSELWV (SEQ ID NO: 26) |

> The amino acid sequence of anti-CD3-Hu-VH (SEQ ID NO: 27): 
EVQLLESGGGLVQPGGSLRLSCAASGFTFSTYAMNWVRQAPGKGLEWVSRIRS
KYNNYATYYADSVKDRFTISRDDSKNTLYLQMNSLRAEDTAVYYCARHENFGQ
SYVSWFAYWGQGTTVTVSS
> The amino acid sequence of anti-CD3-Hu-VL (SEQ ID NO: 28):

QTVVTQEPSLTVSPGGTVTLTCASSTGAVTTSNYPNWVQQKPGQAPRGLIGGTN
KRAPGVPARFSGSLLGGKAALTLSGVQPEDEAEYYCALWYSELWVFGGGTKLT
VL

Note: The portions in bold type are back mutations or other mutations.

DNAs encoding the above humanized heavy and light chain variable regions were synthesized. The anti-CD3-Hu-VH was recombined with a gene encoding a human IgG1 heavy chain constant region containing AAA mutations (L234A+L235A+G237A, silencing Fc-mediated effector functions) (SEQ ID NO: 29) to obtain a full-length humanized heavy chain gene, designated anti-CD3-Hu-HC (SEQ ID NO: 31). The anti-CD3-Hu-VL was ligated to a gene encoding the human lambda light chain constant region (SEQ ID NO: 30) to obtain a full-length humanized light chain gene, designated anti-CD3-Hu-LC (SEQ ID NO: 32).
> The amino acid sequence of the Fc-silenced human IgG1 heavy chain constant region (SEQ ID NO: 29): 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGAPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
> The amino acid sequence of the human lambda light chain constant region (SEQ ID NO: 30): 
GQPKAAPSVTLFPPSSEELQANKATLVCLISDFYPGAVTVAWKADSSPVKAGVET
TTPSKQSNNKYAASSYLSLTPEQWKSHRSYSCQVTHEGSTVEKTVAPTECS
> The amino acid sequence of anti-CD3-Hu-HC (SEQ ID NO: 31): 
EVQLLESGGGLVQPGGSLRLSCAASGFTFSTYAMNWVRQAPGKGLEWVSRIRS
KYNNYATYYADSVKDRFTISRDDSKNTLYLQMNSLRAEDTAVYYCARHENFGQ
SYVSWFAYWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPS
NTKVDKKVEPKSCDKTHTCPPCPAPEAAGAPSVFLFPPKPKDTLMISRTPEVTCV
VVDVSHEDPEVKFNWYVDGVEVHNAKTKPREEQYNSTYRVVSVLTVLHQDW
LNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGN
VFSCSVMHEALHNHYTQKSLSLSPGK
> The amino acid sequence of anti-CD3-Hu-LC (SEQ ID NO: 32):

QTVVTQEPSLTVSPGGTVTLTCASSTGAVTTSNYPNWVQQKPGQAPRGLIGGTN
KRAPGVPARFSGSLLGGKAALTLSGVQPEDEAEYYCALWYSELWVFGGGTKLT
VLGQPKAAPSVTLFPPSSEELQANKATLVCLISDFYPGAVTVAWKADSSPVKAG
VETTTPSKQSNNKYAASSYLSLTPEQWKSHRSYSCQVTHEGSTVEKTVAPTECS

The anti-CD3-Hu-HC and anti-CD3-Hu-LC genes were constructed into pcDNA3.4 expression vectors, respectively, and the resulting expression vectors of the heavy and light chains were simultaneously transfected into Expi293F^{™} cells (Thermo Fisher Scientific, Cat. No. A14527) by PEI transfection to express the antibody. The 293 cells were cultured in a serum-free culture medium for 5 days. The cell supernatant was collected, and the antibody was purified by protein A affinity chromatography. The antibody obtained was designated anti-CD3-Hu.

### Example 3.2: Sources of Anti-CEA and Anti-MUC1 Antibodies and Preparation of Corresponding Bispecific Antibodies

The amino acid sequences of the heavy and light chains of the humanized anti-CEA monoclonal antibody (Hu67-14) were from SEQ ID NOs: 84 and 85 in WO2021121204A1. The amino acid sequences of the heavy and light chains of the humanized anti-MUC1 monoclonal antibody (M6H2L2) were from SEQ ID NOs: 83 and 84 in WO2024213106A1.
> The amino acid sequence of the heavy chain of Hu67-14 (SEQ ID NO: 33): 
EVQLVQSGAEVKKPGESLKISCKGSGYSFTDYHMNWVKQMPGKGLEWMGDIN
PDIGGTSYNQNFKGKVTISVDKSISTAYLQWSSLKASDTAMYYCARWDFDSFAN
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK
> The amino acid sequence of the light chain of Hu67-14 (SEQ ID NO: 34): 
DIQMTQSPSSLSASVGDRVTITCRASESVSIIGTNLIHWYQQKPGKAPKLLIYHAS
NLETGVPSRFSGSGSGTDFTLTISSLQPEDFATYYCLQSRKIPYTFGGGTKVEIKRT
VAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
> The amino acid sequence of the heavy chain of M6H2L2 (SEQ ID NO: 35): 
EVQLVQSGAEVKKPGASVKVSCKASGYRFIPYWIEWVRQAPGQGLEWMGEILP
GTGRTNYNEKFKGRVTFTADTSTNTVYMELSSLRSEDTAVYYCARYGDDTSGG
YYAVDYWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQKSLSL
SPGK
> The amino acid sequence of the light chain of M6H2L2 (SEQ ID NO: 36):

EIVLTQSPATLSLSPGERATLSCRASESVNILGTNLIHWYQQKPGQAPKLLIYHAS
NLETGIPARFSGSGSRTDFTLTISSLEPEDFAVYYCLQSRKIPWTFGGGTKVEIKRT
VAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

Note: The italicized portions are the constant regions of the antibodies, and the portions underlined with wavy lines are the CH1 domains.

BLAST (Basic Local Alignment Search Tool) analysis showed that the similarity between the amino acid sequences of the light chain variable regions of Hu67-14 and M6H2L2 was relatively high (identities: 82%; positives: 94%). Comparison of the light chain CDRs of the two antibodies showed that the similarity between the corresponding CDRs was high. Here, the light chain of Hu67-14 was selected as a potential common light chain to construct a structurally symmetric tetravalent bispecific antibody. The anti-CEA*MUC1 bispecific antibody was constructed as follows: The VH and CH1 domains of M6H2L2 and the heavy chain of Hu67-14 were linked by 3 GGGGS adaptors connected in series to produce a long heavy chain. Mutation Q105E was introduced in the two VHs of the long heavy chain to down-regulate the isoelectric points of potential bispecific antibodies. The AAA mutations (L234A+L235A+G237A) were introduced into the Fc domain of the long heavy chain to silence Fc-mediated effector functions. The long heavy chain produced after the aforementioned engineering was designated anti-CEA*MUC1-HC (SEQ ID NO: 37). Mutation K42E was introduced into the light chain of Hu67-14 to further down-regulate the isoelectric points of potential bispecific antibodies, and the common light chain thus produced was designated anti-CEA*MUC1-LC (SEQ ID NO: 38).
> The amino acid sequence of anti-CEA*MUC1-HC is shown below (SEQ ID NO: 37): 
EVQLVQSGAEVKKPGASVKVSCKASGYRFIPYWIEWVRQAPGQGLEWMGEILP
GTGRTNYNEKFKGRVTFTADTSTNTVYMELSSLRSEDTAVYYCARYGDDTSGG
YYAVDYWGEGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKGGGGSGGGGSGGGGSEVQLVQSGAEVKKPGESLKISCKGS
GYSFTDYHMNWVKQMPGKGLEWMGDINPDIGGTSYNQNFKGKVTISVDKSIS
TAYLQWSSLKASDTAMYYCARWDFDSFANWGEGTLVTVSSASTKGPSVFPLAP
SSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPEAAGAPS
VFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK
> The amino acid sequence of anti-CEA*MUC1-LC is shown below (SEQ ID NO: 38):

DIQMTQSPSSLSASVGDRVTITCRASESVSIIGTNLIHWYQQKPGEAPKLLIYHAS
NLETGVPSRFSGSGSGTDFTLTISSLQPEDFATYYCLQSRKIPYTFGGGTKVEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

The anti-CEA*MUC1-HC and anti-CEA*MUC1-LC genes were constructed into pcDNA3.4 expression vectors, respectively, and the resulting expression vectors of the heavy and light chains were simultaneously transfected into Expi293F^{™} cells (Thermo Fisher Scientific, Cat. No. A14527) by PEI transfection to express the antibody. The 293 cells were cultured in a serum-free culture medium for 5 days. The cell supernatant was collected, and the antibody was purified by protein A affinity chromatography. The antibody obtained was designated anti-CEA*MUC1.

### Example 3.3: Preparation of Anti-CD3-Hu and Anti-CEA*MUC1 Mutants

Gene mutations were introduced into the heavy chain CH3 domains of the anti-CD3-Hu monoclonal antibody and the anti-CEA*MUC1 bispecific antibody by site-directed mutagenesis to mutate the amino acid residues at particular positions into other amino acid residues. Here, the positions of amino acid residues were identified using the Eu numbering scheme. Mutants of the anti-CD3-Hu monoclonal antibody and the anti-CEA*MUC1 bispecific antibody were prepared according to the expression and purification methods described in the previous examples. The antibody samples obtained were sterilized by filtration using a 0.2-µm filter head, and the concentrations of the antibodies were determined using a NanoDrop microspectrophotometer. The antibody mutants and corresponding recombination reactions are shown in Table 13. The rule for naming the antibody mutants is described below: Illustratively, E356K+L351I means that the E at position 356 was mutated into K, and the L at position 351 was mutated into I. The rule applies to other mutants. In the recombination reactions, anti-CD3-Hu and anti-CEA*MUC1 are abbreviated as C and CM, respectively.

**Table 13. Parental antibody mutants and corresponding recombination reactions**

| **No.** | **Anti-CD3-Hu CH3 mutation(s)** | **Anti-CEA*MUC1 CH3 mutation(s)** | **Recombination reaction** |
|---|---|---|---|
| 1 | E356K+L351I | K439E+L351I | C-E356K+L351I*CM-K439E+L351I |
| 2 | E356K+S364A | K439E+S364A | C-E356K+S364A*CM-K439E+S364A |
| 3 | E356K+T366A | K439E+T366A | C-E356K+T366A*CM-K439E+T366A |
| 4 | E356K+T394A | K439E+T394A | C-E356K+T394A*CM-K439E+T394A |
| 5 | E356K+T394S | K439E+T394S | C-E356K+T394S*CM-K439E+T394S |
| 6 | E356K+F405Y | K439E+F405Y | C-E356K+F405Y*CM-K439E+F405Y |
| 7 | E356K+T411Y | K439E+T411Y | C-E356K+T411Y*CM-K439E+T411Y |
| 8 | E356K+Y349S | K439E+S354Y | C-E356K+Y349S*CM-K439E+S354Y |
| 9 | E356K+Y349C | K439E+S354C+L351I | C-E356K+Y349C*CM-K439E+S354C+L351I |
| 10 | F405T | T394F | C-F405T*CM-T394F |
| 11 | Y349G | S354W | C-Y349G*CM-S354W |
| 12 | Y349S | S364Y | C-Y349S*CM-S364Y |
| 13 | Y349C | S354C+S364Y | C-Y349C*CM-S354C+S364Y |
| 14 | Y349C | S354C+S364F | C-Y349C*CM-S354C+S364F |

| | | | |
|---|---|---|---|
| Note: * means that a recombination reaction was performed between the two parental antibodies. | | | |

### Example 3.4: Preparation Method for Anti-CD3*CEA*MUC1 Trispecific Antibodies

1700 mg of 2-MEA (Sigma-Aldrich, Cat. No./specification: 30078/100G) was weighed out and dissolved in 20 mL of PBS, and the pH was adjusted to 7.4 with 1 M NaOH. The amount-of-substance concentration of 2-MEA in this solution was about 750 mM. The concentrations of the anti-CD3-Hu and anti-CEA*MUC1 antibody mutants prepared in the above examples were adjusted to the same amount-of-substance concentration (3.45 µM-690 µM) with PBS. An equal volume of an anti-CD3-Hu mutant was mixed well with a corresponding anti-CEA*MUC1 mutant, and a proper amount of the 2-MEA mother liquor was then added to and mixed well with the above antibody mixture, with the final concentration of 2-MEA being about 75 mM. The mixture (containing the anti-CD3-Hu mutant, the anti-CEA*MUC1 mutant, and 2-MEA) was incubated in a water bath at 37 °C for 2.5 h. The mixture was transferred to Slide-A-Lyzer^{™} G3 Dialysis Cassettes (Thermo Fisher Scientific, Cat. No. A52971) and dialyzed against 5 L of PBS at room temperature. After 5 h, the PBS was refreshed, and the mixture was further dialyzed in a cool store (4 °C). After overnight dialysis, samples were collected, and the antibody concentrations were re-determined. The physicochemical properties of the parental antibodies and corresponding recombination reaction products were assessed by HPLC-SEC and IEC.

### Example 3.5: Recombination Efficiency of Anti-CD3*CEA*MUC1 Trispecific Antibodies

The physicochemical property analysis methods used in this example were as shown in the previous examples.

The parental antibodies and corresponding recombination reaction products (containing trispecific antibodies) produced in the above examples were analyzed by HPLC-SEC and HPLC-IEC. The obtained data on purity (here, the SEC purity was the proportion of the area of the main peak, and the IEC purity was the sum of the proportions of the areas of primary peaks such as the main peak, the acidic peak, and the basic peak) are summarized in Table 14.

**Table 14. The SEC purity of parental antibodies and the SEC and IEC purity of corresponding recombination reaction products**

| **Parental antibody mutant** | | **Recombination reaction product** | |
|---|---|---|---|
| **Name** | **SEC (%)** | **SEC (%)** | **IEC (%)** |
| C-E356K+L351I | 96.19 | 94.33 | 97.30 |
| CM-K439E+L351I | 96.58 | | |
| C-E356K+S364A | 96.41 | 94.18 | 98.12 |
| CM-K439E+S364A | 96.65 | | |
| C-E356K+T366A | 94.78 | 93.29 | 100.00 |
| CM-K439E+T366A | 92.13 | | |
| C-E356K+T394A | 95.64 | 94.62 | 98.13 |
| CM-K439E+T394A | 94.72 | | |
| C-E356K+T394S | 96.43 | 93.27 | 96.42 |
| CM-K439E+T394S | 96.55 | | |
| C-E356K+F405Y | 88.26 | 89.52 | 94.70 |
| CM-K439E+F405Y | 90.71 | | |
| C-E356K+T411Y | 86.62 | 90.49 | 94.14 |
| CM-K439E+T411Y | 92.30 | | |
| C-E356K+Y349S | 91.46 | 91.49 | 97.31 |
| CM-K439E+S354Y | 93.72 | | |
| C-E356K+Y349C | 88.15 | 89.50 | 94.23 |
| CM-K439E+S354C+L351I | 94.26 | | |
| C-F405T | 92.83 | 85.17 | 90.02 |
| CM-T394F | 85.39 | | |
| C-Y349G | 84.49 | 90.70 | 96.37 |
| CM-S354W | 91.93 | | |
| C-Y349S | 96.63 | 93.57 | 96.20 |
| CM-S364Y | 94.70 | | |
| C-Y349C | 95.84 | 88.38 | 93.34 |
| CM-S354C+S364Y | 88.68 | | |
| C-Y349C | 95.84 | 90.96 | 90.13 |
| CM-S354C+S364F | 91.79 | | |

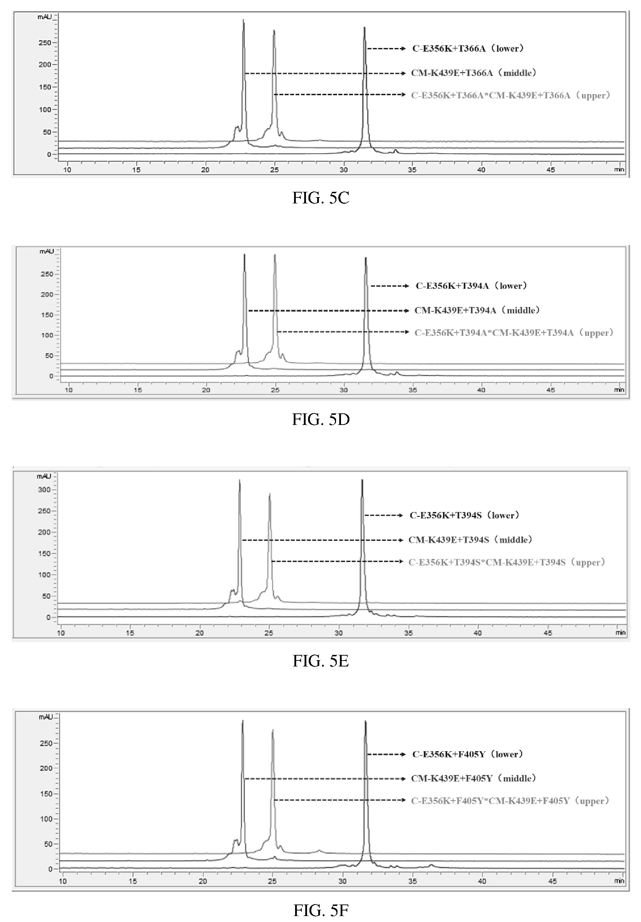

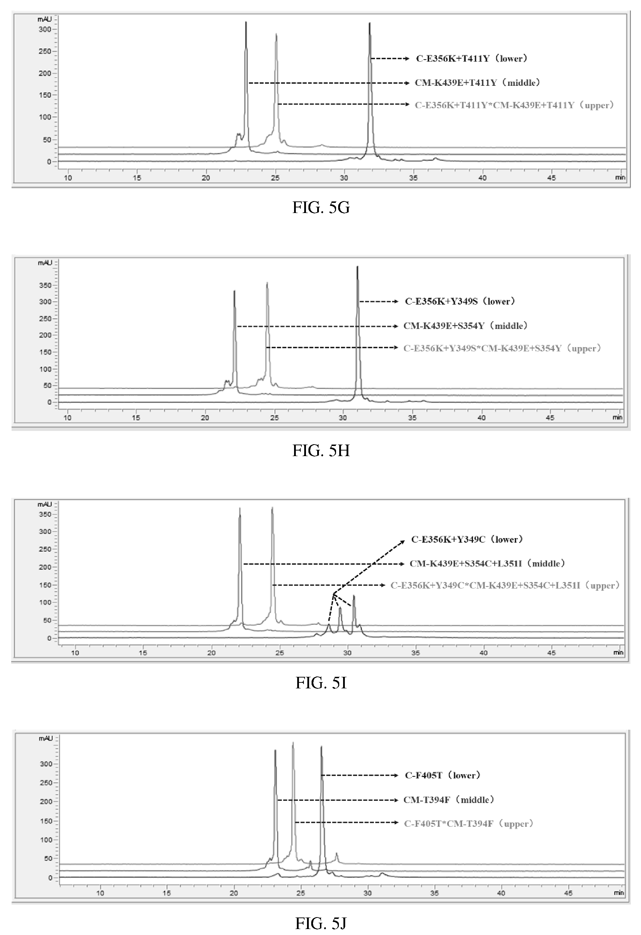

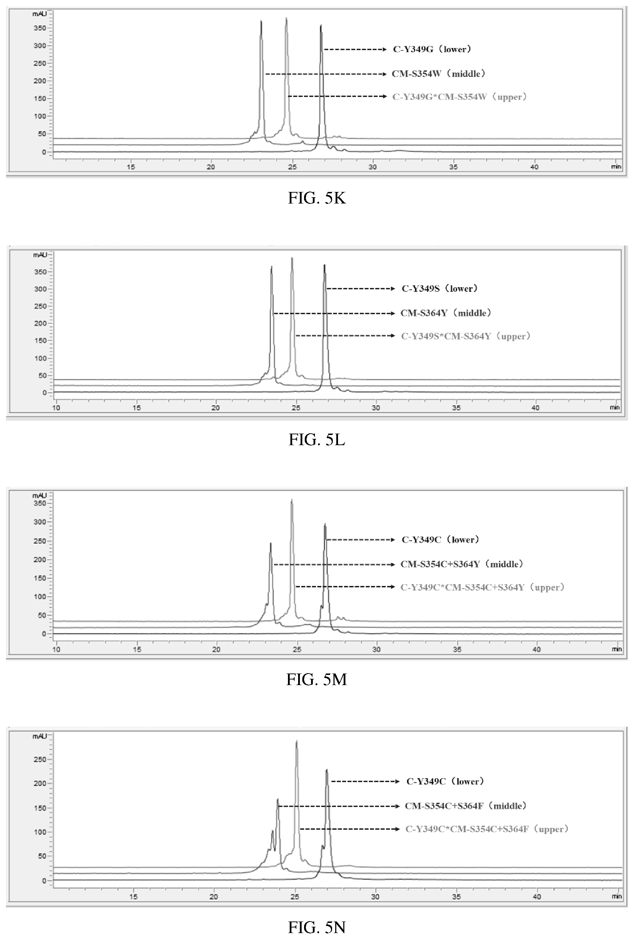

The HPLC-IEC results show that the main peaks of the parental antibody mutants of all combinations differed significantly in retention time, and the main peaks of the recombination reaction products (including trispecific antibodies) were between those of their two parental antibodies. Here, reactants and recombination reaction products could be effectively separated and identified by HPLC-IEC. The HPLC-IEC chromatograms of the parental mutants and corresponding recombination reaction products in Table 14 were superimposed and analyzed. The reaction combinations included C-E356K+L3511*CM-K439E+L3511 (FIG. 5A), C-E356K+S364A*CM-K439E+S364A (FIG. 5B), C-E356K+T366A*CM-K439E+T366A (FIG. 5C), C-E356K+T394A*CM-K439E+T394A (FIG. 5D), C-E356K+T394S*CM-K439E+T394S (FIG. 5E), C-E356K+F405Y*CM-K439E+F405Y (FIG. 5F), C-E356K+T411Y*CM-K439E+T411Y (FIG. 5G), C-E356K+Y349S*CM-K439E+S354Y (FIG. 5H), C-E356K+Y349C*CM-S354C+L3511 (FIG. 5I), C-F405T*CM-T394F (FIG. 5J), C-Y349G*CM-S354W (FIG. 5K), C-Y349S*CM-S364Y (FIG. 5L), C-Y349C*CM-S354C+S364Y (FIG. 5M), and C-Y349C*CM-S354C+S364F (FIG. 5N).

If a trispecific antibody is formed, new peaks corresponding to the trispecific antibody will be found in the chromatogram of the recombination reaction product, and the peaks at the positions corresponding to the parental antibodies will relatively decrease. The percentage of the area of the peaks corresponding to the parental antibodies is the amount that remains after the recombination reaction. FIGs. 5A-5N show significant new peaks in the chromatograms of the recombination reaction products, indicating the generation of recombinant antibodies. Here, the formula for calculating the efficiency of a recombination reaction was: 100% - (percentage of area of peaks corresponding to parental antibody A + percentage of area of peaks corresponding to parental antibody B).

**Table 15. The recombination reaction efficiency of various combinations of parental antibody mutants**

| **Combination No.** | **Parental antibody** | | **Recombination reaction efficiency** | **Figure No.** |
|---|---|---|---|---|
| | **Name** | **Amount of residues (%)** | | |
| 1 | C-E356K+L351I | 0.8 | 97.3 | 5A |
| | CM-K439E+L351I | 1.9 | | |
| 2 | C-E356K+S364A | 0.0 | 98.1 | 5B |
| | CM-K439E+S364A | 1.9 | | |
| 3 | C-E356K+T366A | 0.0 | 100.0 | 5C |
| | CM-K439E+T366A | 0.0 | | |
| 4 | C-E356K+T394A | 0.0 | 98.1 | 5D |
| | CM-K439E+T394A | 1.9 | | |
| 5 | C-E356K+T394S | 0.8 | 96.4 | 5E |
| | CM-K439E+T394S | 2.8 | | |
| 6 | C-E356K+F405Y | 5.3 | 94.7 | 5F |
| | CM-K439E+F405Y | 0.0 | | |
| 7 | C-E356K+T411Y | 5.9 | 94.1 | 5G |
| | CM-K439E+T411Y | 0.0 | | |
| 8 | C-E356K+Y349S | 1.8 | 97.3 | 5H |
| | CM-K439E+S354Y | 0.9 | | |
| 9 | C-E356K+Y349C | 3.4 | 94.3 | 5I |
| | CM-K439E+S354C+L351I | 2.3 | | |
| 10 | C-F405T | 10.0 | 90.0 | 5J |
| | CM-T394F | 0.0 | | |
| 11 | C-Y349G | 2.8 | 96.4 | 5K |
| | CM-S354W | 0.8 | | |
| 12 | C-Y349S | 2.5 | 96.2 | 5L |
| | CM-S364Y | 1.3 | | |
| 13 | C-Y349C | 6.4 | 93.4 | 5M |
| | CM-S354C+S364Y | 0.2 | | |
| 14 | C-Y349C | 5.1 | 90.1 | 5N |
| | CM-S354C+S364F | 4.8 | | |

The above results (FIGs. 5A to 5N, and Table 15) show that the recombination reaction efficiency of the different combinations of the present disclosure ranged from 90.0% to 100.0%, with 8 combinations exhibiting recombination reaction efficiency higher than 95%.

2. The method and conditions for determining the molecular weights of deglycosylated antibodies by mass spectrometry were the same as those in the previous examples.

**Table 16. The results of mass spectrometry analysis of recombination reaction products**

| **Parental antibody** | | **Recombination reaction product** | |
|---|---|---|---|
| **Name** | **Calculated heavy chain/light chain molecular weight** | **Calculated intact molecular weight** | **Found heavy chain/light chain/intact molecular weight** |
| C-E356K+L351I | 49980/22561 | 194255 | 49980/74215/22561/23769/194262 |
| CM-K439E+L351I | 74217/23770 | | |
| C-E356K+S364A | 49964/22561 | 194223 | 49959/74197/22560/23769/194222 |
| CM-K439E+S364A | 74201/23770 | | |
| C-E356K+T366A | 49950/22561 | 194195 | 49945/74183/22558/23769/194191 |
| CM-K439E+T366A | 74187/23770 | | |
| C-E356K+T394A | 49950/22561 | 194195 | 49944/74180/22556/23768/194191 |
| CM-K439E+T394A | 74187/23770 | | |
| C-E356K+T394S | 49966/22561 | 194227 | 49965/74201/22560/23768/194230 |
| CM-K439E+T394S | 74203/23770 | | |
| C-E356K+F405Y | 49996/22561 | 194287 | 49995/74231/22560/23768/194291 |
| CM-K439E+F405Y | 74233/23770 | | |
| C-E356K+T411Y | 50042/22561 | 194380 | 50042/74278/22560/23768/194379 |
| CM-K439E+T411Y | 74279/23770 | | |
| C-E356K+Y349S | 49904/22561 | 194255 | 49903/74291/22558/23768/194256 |
| CM-K439E+S354Y | 74293/23770 | | |
| C-E356K+Y349C | 49920/22561 | 194211 | 49919/74230/22560/23768/194209 |
| CM-K439E+S354C+L351I | 74233/23770 | | |
| C-F405T | 49935/22561 | 194255 | 49934/74260/22561/23768/194254 |
| CM-T394F | 74262/23770 | | |
| C-Y349G | 49875/22561 | 194248 | 49874/74313/22563/23768/194247 |
| CM-S354W | 74315/23770 | | |
| C-Y349S | 49905/22561 | 194255 | 49904/74290/22560/23768/194255 |
| CM-S364Y | 74292/23770 | | |
| C-Y349C | 49921/22561 | 194288 | 49921/74307/22560/23768/194287 |
| CM-S354C+S364Y | 74308/23770 | | |
| C-Y349C | 49921/22561 | 194272 | 49920/74295/22560/23769/194269 |
| CM-S354C+S364F | 74293/23770 | | |

Note: The calculated heavy chain, light chain, and intact molecular weights in this table are theoretical values calculated by software under conditions such as taking into account the post-translational modifications and redox states of the proteins. Therefore, the calculated molecular weights of the same light chain under different experimental conditions may not be completely identical.

The results in Table 16 show that the calculated heavy chain, light chain, and intact molecular weights of the trispecific antibodies produced by the above recombination reactions were very close to the actual heavy chain, light chain, and intact molecular weights measured by mass spectrometry, which indicates that the structures of the trispecific antibodies were in line with theoretical expectations. In addition, there were no significant peaks corresponding to the parental antibodies in the mass spectra of the recombination reaction products, indicating that the parental antibodies, as reactants, had been fully transformed into the recombinant products.

### Example 5: Binding Activity of Anti-CD3*CEA*MUC1 Trispecific Antibodies

The ability of recombination reaction products to bind to CD3 and CEA simultaneously, or to bind to CD3 and MUC1 simultaneously, was examined by bridging ELISA. The ELISA used in this example is described below:
A microplate was coated with human CD3 recombinant protein (purchased from ACROBiosystem, Cat: CDE-H5223) (100 ng per well) and treated with a blocking solution (a phosphate buffer containing 1% bovine serum albumin and 0.05% Tween-20). The test antibodies were serially diluted and then transferred to the above microplate, and the plate was incubated at room temperature for half an hour. After the plate was washed, biotinylated human CEA recombinant protein (moderately diluted, purchased from ACROBiosystems, Cat: CE5-H82E0) was added, and the plate was incubated at room temperature for half an hour. After the plate was washed, 100 µL of streptavidin-peroxidase (moderately diluted, purchased from Sigma, Cat: S2438) was added to each well. After the plate was washed, 100 µL of a chromogenic solution with TMB (3,3',5,5'-tetramethylbenzidine) as a substrate was added to each well, and the plate was incubated at room temperature for 1-5 min. 50 µL of a stop solution (2 M H₂SO₄) was added to stop the reaction. OD450 was measured using a microplate reader (VERSA max). Plotting and data analysis were performed using GraphPad Prism 10.0, and EC₅₀ and Top were calculated.

A microplate was coated with human MUC1 recombinant protein (purchased from ACROBiosystems, Cat: MU1-H52H9) (50 ng per well) and treated with a blocking solution. The test antibodies were serially diluted and then transferred to the above microplate, and the plate was incubated at room temperature for half an hour. After the plate was washed, biotinylated human CD3 recombinant protein (moderately diluted, purchased from ACROBiosystems, Cat: CDE-H82E1) was added, and the plate was incubated at room temperature for half an hour. After the plate was washed, 100 µL of streptavidin-peroxidase (moderately diluted) was added to each well. After the plate was washed, 100 µL of a chromogenic solution with TMB (3,3',5,5'-tetramethylbenzidine) as a substrate was added to each well, and the plate was incubated at room temperature for 1-5 min. 50 µL of a stop solution (2 M H₂SO₄) was added to stop the reaction. OD450 was measured using a microplate reader (VERSA max). Plotting and data analysis were performed using GraphPad Prism 10.0, and EC₅₀ and Top were calculated.

The Top values represent the high plateaus of the fitted curves, and a larger Top value and a smaller EC₅₀ indicate stronger binding activity. The binding parameters of the recombination reaction products (trispecific antibodies) measured by bridging ELISA are shown in Table 17. The results show that the parental antibodies, as controls, could not bind to CD3 and CEA simultaneously, nor could they bind to CD3 and MUC1 simultaneously; in contrast, the above recombination reaction products could bind to CD3 and CEA simultaneously, or bind to CD3 and MUC1 simultaneously. These results further indicate that the anti-CD3-Hu monoclonal antibody and the anti-CEA*MUC1 bispecific antibody formed an anti-CD3*CEA*MUC1 trispecific antibody through a half-antibody recombination reaction.

**Table 17. The antigen-binding activity of recombination reaction products**

| **Antigen** | **Parental antibody or recombination reaction product** | **EC₅₀ (nM)** | **Top** |
|---|---|---|---|
| CD3/CEA | C-E356K+L351I | NA | NA |
| | CM-K439E+L351I | NA | NA |
| | C-E356K+L351I*CM-K439E+L351I | 10.02 | 3.06 |
| | C-E356K+S364A*CM-K439E+S364A | 10.97 | 2.95 |
| | C-E356K+T366A*CM-K439E+T366A | 9.60 | 2.81 |
| | C-E356K+T394A*-CM-K439E+T394A | 9.56 | 2.82 |
| | C-E356K+T394S*CM-K439E+T394S | 8.58 | 2.96 |
| | E356K+F405Y*CM-K439E+F405Y | 11.07 | 3.42 |
| | C-E356K+T411Y*CM-K439E+T411Y | 9.22 | 3.21 |
| | C-E356K+Y349S*CM-K439E+S354Y | 10.44 | 3.17 |
| | C-E356K+Y349C*CM-K439E+S354C+L351I | 12.28 | 3.23 |
| | C-F405T*CM-T394F | 9.56 | 3.31 |
| | C-Y349G*CM-S354W | 9.26 | 2.92 |
| | C-Y349S*CM-S364Y | 10.57 | 3.06 |
| | C-Y349C*CM-S354C+S364Y | 11.67 | 3.32 |
| | C-Y349C*CM-S354C+S364F | 10.86 | 3.14 |
| CD3/MUC1 | C-E356K+L351I | NA | NA |
| | CM-K439E+L351I | NA | NA |
| | C-E356K+L351I*CM-K439E+L351I | 1.97 | 2.31 |
| | C-E356K+S364A*CM-K439E+S364A | 2.51 | 2.49 |
| | C-E356K+T366A*CM-K439E+T366A | 2.50 | 2.61 |
| | C-E356K+T394A*-CM-K439E+T394A | 2.29 | 2.49 |
| | C-E356K+T394S*CM-K439E+T394S | 1.63 | 2.15 |
| | E356K+F405Y*CM-K439E+F405Y | 2.00 | 2.78 |
| | C-E356K+T411Y*CM-K439E+T411Y | 1.92 | 2.72 |
| | C-E356K+Y349S*CM-K439E+S354Y | 2.07 | 2.57 |
| | C-E356K+Y349C*CM-K439E+S354C+L351I | 2.13 | 2.38 |
| | C-F405T*CM-T394F | 1.73 | 2.69 |
| | C-Y349G*CM-S354W | 2.53 | 2.52 |
| | C-Y349S*CM-S364Y | 2.28 | 2.42 |
| | C-Y349C*CM-S354C+S364Y | 1.93 | 2.61 |
| | C-Y349C*CM-S354C+S364F | 2.48 | 2.47 |

Although the above invention has been described in detail by means of drawings and examples for a clear understanding, the descriptions and examples should not be construed as limiting the scope of the present disclosure. The disclosures of all the patents and scientific documents cited herein are clearly incorporated by reference in their entirety.

## Claims

1. A method for preparing a heteromultimer, comprising the following steps:
a) a step of providing a molecule comprising a first polypeptide homomer,
b) a step of providing a molecule comprising a second polypeptide homomer,
c) a step of co-incubating the molecule comprising the first polypeptide homomer and the molecule comprising the second polypeptide homomer under reducing conditions, and
d) a step of obtaining a heteromultimer comprising the first polypeptide and the second polypeptide,
wherein the first polypeptide and the second polypeptide each comprise one CH3 domain;
the CH3 domains of the first polypeptide and the second polypeptide differ by at least one mutation site, and mutation sites in the CH3 domains are indicated by EU numbering, wherein:
1) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 351, 349, 364, 366, 368, 394, 405, 407, 409, and 411;
preferably, the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical amino acid mutation selected from the group consisting of positions 351, 364, 366, 368, 394, 405, 409, and 411;
more preferably, the CH3 domain of the first polypeptide comprises amino acid mutations at positions 356 and 351, and the CH3 domain of the second polypeptide comprises amino acid mutations at positions 439 and 351; or
2) the CH3 domain of the first polypeptide comprises an amino acid mutation at position 349, and the CH3 domain of the second polypeptide comprises an amino acid mutation at position 354, wherein the CH3 domain of the second polypeptide further comprises one amino acid mutation selected from the group consisting of positions 364, 351, 366, 368, and 405; or
the CH3 domain of the first polypeptide comprises an amino acid mutation at position 349, and the CH3 domain of the second polypeptide comprises amino acid mutations at positions 347 and 357;
preferably, the CH3 domain of the first polypeptide comprises an amino acid mutation at position 349, and the CH3 domain of the second polypeptide comprises amino acid mutations at positions 354 and 364; or
3) the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to iv):
i) 349;
ii) 354 and 351;
iii) 354 and 366; and
iv) 354 and 368;
preferably, the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368;
more preferably, the CH3 domain of the first polypeptide comprises amino acid mutations at positions 356 and 349, and the CH3 domain of the second polypeptide comprises amino acid mutations at positions 439, 354, and 351; or
4) the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394; or
the CH3 domain of the first polypeptide comprises an amino acid mutation at position 366, and the CH3 domain of the second polypeptide comprises an amino acid mutation at position 405 or 407;
preferably, the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394;
more preferably, the CH3 domain of the first polypeptide comprises an amino acid mutation at position 349, and the CH3 domain of the second polypeptide comprises an amino acid mutation at position 364; or
5) the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to vii):
i) 349;
ii) 354;
iii) 357;
iv) 366;
v) 394;
vi) 405; and
vii) 407;
preferably, the CH3 domain of the first polypeptide comprises amino acid mutations at positions 356 and 349, and the CH3 domain of the second polypeptide comprises an amino acid mutation at position 439, wherein the CH3 domain of the second polypeptide further comprises an amino acid mutation at position 354 or 357; or
the CH3 domain of the first polypeptide comprises amino acid mutations at positions 356 and 405, and the CH3 domain of the second polypeptide comprises amino acid mutations at positions 439 and 394; or
the CH3 domain of the first polypeptide comprises amino acid mutations at positions 356 and 366, and the CH3 domain of the second polypeptide comprises an amino acid mutation at position 439, wherein the CH3 domain of the second polypeptide further comprises an amino acid mutation at position 405 or 407; or
the CH3 domain of the first polypeptide comprises amino acid mutations at positions 356 and 407, and the CH3 domain of the second polypeptide comprises amino acid mutations at positions 439 and 366;
preferably, the CH3 domain of the first polypeptide comprises amino acid mutations at positions 356 and 349, and the CH3 domain of the second polypeptide comprises amino acid mutations at positions 439 and 354; or
6) the CH3 domain of the first polypeptide comprises an amino acid mutation at position 349, and the CH3 domain of the second polypeptide comprises an amino acid mutation at position 357; or
7) the CH3 domain of the first polypeptide comprises an amino acid mutation at position 354, and the CH3 domain of the second polypeptide comprises an amino acid mutation at position 349, wherein the CH3 domain(s) of the first polypeptide and/or the second polypeptide further comprise(s) at least one amino acid mutation selected from the group consisting of i) and ii):
i) 405; and
ii) 394;
preferably, the CH3 domain of the first polypeptide comprises amino acid mutations at positions 354 and 405, and the CH3 domain of the second polypeptide comprises amino acid mutations at positions 349 and 394;
optionally, wherein:
the amino acid at position 356 is mutated into Lys (K), Arg (R), or His (H); and/or
the amino acid at position 439 is mutated into Glu (E) or Asp (D); and/or
the amino acid at position 351 is mutated into Ile (I), Cys (C), Val (V), Thr (T), Phe (F), or Met (M); and/or
the amino acid at position 349 is mutated into Leu (L), Phe (F), Ser (S), Cys (C), Ala (A), Val (V), Thr (T), or Gly (G); and/or
the amino acid at position 364 is mutated into Ala (A), Val (V), Thr (T), Leu (L), Tyr (Y), or Phe (F); and/or
the amino acid at position 366 is mutated into Gly (G), Ser (S), Ala (A), Val (V), Leu (L), His (H), or Ile (I); and/or
the amino acid at position 368 is mutated into Val (V), Ile (I), Met (M), or Ala (A); and/or
the amino acid at position 394 is mutated into Phe (F), Ala (A), Ser (S), Cys (C), Val (V), or Asn (N); and/or
the amino acid at position 405 is mutated into Thr (T), Leu (L), or Tyr (Y); and/or
the amino acid at position 407 is mutated into Cys (C), Val (V), Leu (L), His (H), or Phe (F); and/or
the amino acid at position 409 is mutated into Gln (Q), Arg (R), or Asp (D); and/or
the amino acid at position 411 is mutated into Asn (N), Tyr (Y), or Leu (L); and/or
the amino acid at position 354 is mutated into Tyr (Y), Cys (C), Phe (F), or Trp (W); and/or
the amino acid at position 357 is mutated into Cys (C); and/or
the amino acid at position 347 is mutated into Glu (E).

2. The method according to claim 1, wherein:
1) the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of 351I, 351C, 351V, 351T, 351F, 351M, 349L, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y;
preferably, the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical or different amino acid mutation selected from the group consisting of 351I, 351C, 351V, 351T, 351F, 351M, 349L, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y;
further preferably, the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351I, 351T, 364A, 366A, 368I, 394A, 394S, 405Y, 409Q, and 411Y; more preferably, the CH3 domain of the first polypeptide comprises amino acid mutations of 356K and 351I, and the CH3 domain of the second polypeptide comprises amino acid mutations of 439E and 351I; or
2) the CH3 domain of the first polypeptide comprises an amino acid mutation of 349C, and the CH3 domain of the second polypeptide comprises an amino acid mutation of 354C, wherein the CH3 domain of the second polypeptide further comprises one amino acid mutation selected from the group consisting of 364Y, 364F, 351I, 366A, 368I, and 405Y; or
the CH3 domain of the first polypeptide comprises an amino acid mutation of 349C, and the CH3 domain of the second polypeptide comprises amino acid mutations of 347E and 357C;
preferably, the CH3 domain of the first polypeptide comprises an amino acid mutation of 349C, and the CH3 domain of the second polypeptide comprises an amino acid mutation of 354C, wherein the CH3 domain of the second polypeptide further comprises an amino acid mutation of 364Y or 364F;
more preferably, the CH3 domain of the first polypeptide comprises an amino acid mutation of 349C, and the CH3 domain of the second polypeptide comprises amino acid mutations of 354C and 364Y; or
3) the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to iv):
i) 349C;
ii) 354C and 351I;
iii) 354C and 366A; and
iv) 354C and 368I;
preferably, the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I;
more preferably, the CH3 domain of the first polypeptide comprises amino acid mutations of 356K and 349C, and the CH3 domain of the second polypeptide comprises amino acid mutations of 439E, 354C, and 351I; or
4) the CH3 domain of the first polypeptide comprises an amino acid mutation of 349S, and the CH3 domain of the second polypeptide comprises one amino acid mutation selected from the group consisting of 364Y, 354F, 354W, and 354Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 349A, and the CH3 domain of the second polypeptide comprises one amino acid mutation selected from the group consisting of 354F, 354Y, and 354W; or
the CH3 domain of the first polypeptide comprises an amino acid mutation of 349V, and the CH3 domain of the second polypeptide comprises an amino acid mutation of 354F; or
the CH3 domain of the first polypeptide comprises an amino acid mutation of 349T, and the CH3 domain of the second polypeptide comprises an amino acid mutation of 354F; or
the CH3 domain of the first polypeptide comprises an amino acid mutation of 405T, and the CH3 domain of the second polypeptide comprises an amino acid mutation of 394F; or
the CH3 domain of the first polypeptide comprises an amino acid mutation of 366H, and the CH3 domain of the second polypeptide comprises an amino acid mutation of 405L or 407L; or
preferably, the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 405T, and the CH3 domain of the second polypeptide comprises an amino acid mutation of 394F;
more preferably, the CH3 domain of the first polypeptide comprises an amino acid mutation of 349S, and the CH3 domain of the second polypeptide comprises an amino acid mutation of 364Y; or
5) the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to vii):
i) 349S or 349C;
ii) 354Y or 354C;
iii) 357C;
iv) 366H;
v) 394F;
vi) 405T or 405L; and
vii) 407L or 407H;
preferably, the CH3 domain of the first polypeptide comprises amino acid mutations of 356K and 349S, and the CH3 domain of the second polypeptide comprises amino acid mutations of 439E and 354Y; or
the CH3 domain of the first polypeptide comprises amino acid mutations of 356K and 349C, and the CH3 domain of the second polypeptide comprises amino acid mutations of 439E and 354C; or
the CH3 domain of the first polypeptide comprises amino acid mutations of 356K and 349C, and the CH3 domain of the second polypeptide comprises amino acid mutations of 439E and 357C; or
the CH3 domain of the first polypeptide comprises amino acid mutations of 356K and 405T, and the CH3 domain of the second polypeptide comprises amino acid mutations of 439E and 394F; or
the CH3 domain of the first polypeptide comprises amino acid mutations of 356K and 366H, and the CH3 domain of the second polypeptide comprises an amino acid mutation of 439E, wherein the CH3 domain of the second polypeptide further comprises one amino acid mutation selected from the group consisting of 405L, 407L, and 407H; or
the CH3 domain of the first polypeptide comprises amino acid mutations of 356K and 407L, and the CH3 domain of the second polypeptide comprises amino acid mutations of 439E and 366H; or
the CH3 domain of the first polypeptide comprises amino acid mutations of 356K and 407H, and the CH3 domain of the second polypeptide comprises amino acid mutations of 439E and 366H;
more preferably, the CH3 domain of the first polypeptide comprises amino acid mutations of 356K and 349S, and the CH3 domain of the second polypeptide comprises amino acid mutations of 439E and 354Y; or
6) the CH3 domain of the first polypeptide comprises an amino acid mutation of 349C, and the CH3 domain of the second polypeptide comprises an amino acid mutation of 357C; or
7) the CH3 domain of the first polypeptide comprises an amino acid mutation of 354C, and the CH3 domain of the second polypeptide comprises an amino acid mutation of 349C, wherein the CH3 domain(s) of the first polypeptide and/or the second polypeptide further comprise(s) at least one amino acid mutation selected from the group consisting of i) and ii):
i) 405T; and
ii) 394F;
preferably, the CH3 domain of the first polypeptide comprises amino acid mutations of 354C and 405T, and the CH3 domain of the second polypeptide comprises amino acid mutations of 349C and 394F.

3. The method according to claim 1 or 2, wherein the first polypeptide and the second polypeptide have different isoelectric points; preferably, the first polypeptide and the second polypeptide further comprise additional amino acid mutations such that the first polypeptide and the second polypeptide have different isoelectric points.

4. The method according to any one of claims 1 to 3, wherein the reducing conditions include adding one or more reductants selected from the group consisting of 2-mercaptoethylamine (2-MEA), dithiothreitol (DTT), dithioerythritol (DTE), glutathione (GSH), tris(2-carboxyethyl)phosphine (TCEP), L-cysteine, D-cysteine, and β-mercaptoethanol, as well as chemical derivatives thereof; preferably, the reductant is one or more selected from the group consisting of 2-MEA, glutathione, L-cysteine, dithiothreitol, β-mercaptoethanol, and TCEP; more preferably, the reductant is 2-MEA.

5. The method according to any one of claims 1 to 4, wherein the heteromultimer is a multispecific antibody or an Fc fusion protein.

6. The method according to claim 1, wherein the molecule comprising the first polypeptide homomer and the molecule comprising the second polypeptide homomer are selected from the group consisting of an Fc region, an antibody, a fusion protein comprising an Fc region (e.g., a receptor-, cytokine-, or hormone-fused Fc region), and an Fc region conjugated with a drug (e.g., a peptide or toxin);
preferably, the molecule comprising the first polypeptide homomer is a first parental antibody, and the molecule comprising the second polypeptide homomer is a second parental antibody.

7. A heteromultimer prepared by the method according to any one of claims 1 to 6.

8. A heteromultimer, comprising a first polypeptide and a second polypeptide, wherein the first polypeptide and the second polypeptide each comprise one CH3 domain, wherein:
1) the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical or different amino acid mutation selected from the group consisting of positions 351, 349, 364, 366, 368, 394, 405, 407, 409, and 411;
preferably, the CH3 domain of the first polypeptide comprises an amino acid mutation at position 356, and the CH3 domain of the second polypeptide comprises an amino acid mutation at position 439, wherein the CH3 domains of the first polypeptide and the second polypeptide each further comprise one identical amino acid mutation selected from the group consisting of positions 351, 364, 366, 368, 394, 405, 409, and 411; or
more preferably, the CH3 domain of the first polypeptide comprises amino acid mutations at positions 356 and 351, and the CH3 domain of the second polypeptide comprises amino acid mutations at positions 439 and 351; or
2) the CH3 domain of the first polypeptide comprises an amino acid mutation at position 349, and the CH3 domain of the second polypeptide comprises an amino acid mutation at position 354, wherein the CH3 domain of the second polypeptide further comprises one amino acid mutation selected from the group consisting of positions 364, 351, 366, 368, and 405; or
the CH3 domain of the first polypeptide comprises an amino acid mutation at position 349, and the CH3 domain of the second polypeptide comprises amino acid mutations at positions 347 and 357; or
preferably, the CH3 domain of the first polypeptide comprises an amino acid mutation at position 349, and the CH3 domain of the second polypeptide comprises amino acid mutations at positions 354 and 364; or
3) the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to iv):
i) 349;
ii) 354 and 351;
iii) 354 and 366; and
iv) 354 and 368;
preferably, the CH3 domain of the first polypeptide comprises amino acid mutations at positions 356 and 349, and the CH3 domain of the second polypeptide comprises amino acid mutations at positions 439 and 354, wherein the CH3 domain of the second polypeptide further comprises one amino acid mutation selected from the group consisting of positions 351, 366, and 368;
more preferably, the CH3 domain of the first polypeptide comprises amino acid mutations at positions 356 and 349, and the CH3 domain of the second polypeptide comprises amino acid mutations at positions 439, 354, and 351; or
4) the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394; or
the CH3 domain of the first polypeptide comprises an amino acid mutation at position 366, and the CH3 domain of the second polypeptide comprises an amino acid mutation at position 405 or 407;
preferably, the CH3 domain of the first polypeptide comprises an amino acid mutation at position 349, and the CH3 domain of the second polypeptide comprises an amino acid mutation at position 364 or 354; or
the CH3 domain of the first polypeptide comprises an amino acid mutation at position 405, and the CH3 domain of the second polypeptide comprises an amino acid mutation at position 394;
more preferably, the CH3 domain of the first polypeptide comprises an amino acid mutation at position 349, and the CH3 domain of the second polypeptide comprises an amino acid mutation at position 364; or
5) the CH3 domain of the first polypeptide comprises an amino acid mutation at position 356, and the CH3 domain of the second polypeptide comprises an amino acid mutation at position 439, wherein the CH3 domain(s) of the first polypeptide and/or the second polypeptide further comprise(s) at least one amino acid mutation selected from the group consisting of the following i) to vii):
i) 349;
ii) 354;
iii) 357;
iv) 366;
v) 394;
vi) 405; and
vii) 407;
preferably, the CH3 domain of the first polypeptide comprises amino acid mutations at positions 356 and 349, and the CH3 domain of the second polypeptide comprises an amino acid mutation at position 439, wherein the CH3 domain of the second polypeptide further comprises an amino acid mutation at position 354 or 357; or
the CH3 domain of the first polypeptide comprises amino acid mutations at positions 356 and 405, and the CH3 domain of the second polypeptide comprises amino acid mutations at positions 439 and 394; or
the CH3 domain of the first polypeptide comprises amino acid mutations at positions 356 and 366, and the CH3 domain of the second polypeptide comprises an amino acid mutation at position 439, wherein the CH3 domain of the second polypeptide further comprises an amino acid mutation at position 405 or 407; or
the CH3 domain of the first polypeptide comprises amino acid mutations at positions 356 and 407, and the CH3 domain of the second polypeptide comprises amino acid mutations at positions 439 and 366;
preferably, the CH3 domain of the first polypeptide comprises amino acid mutations at positions 356 and 349, and the CH3 domain of the second polypeptide comprises amino acid mutations at positions 439 and 354; or
6) the CH3 domain of the first polypeptide comprises an amino acid mutation at position 349, and the CH3 domain of the second polypeptide comprises an amino acid mutation at position 357; or
7) the CH3 domain of the first polypeptide comprises an amino acid mutation at position 354, and the CH3 domain of the second polypeptide comprises an amino acid mutation at position 349, wherein the CH3 domain(s) of the first polypeptide and/or the second polypeptide further comprise(s) at least one amino acid mutation selected from the group consisting of i) and ii):
i) 405; and
ii) 394;
preferably, the CH3 domain of the first polypeptide comprises amino acid mutations at positions 354 and 405, and the CH3 domain of the second polypeptide comprises amino acid mutations at positions 349 and 394;
optionally, wherein:
the amino acid at position 356 is mutated into Lys (K), Arg (R), or His (H); and/or
the amino acid at position 439 is mutated into Glu (E) or Asp (D); and/or
the amino acid at position 351 is mutated into Ile (I), Cys (C), Val (V), Thr (T), Phe (F), or Met (M); and/or
the amino acid at position 349 is mutated into Leu (L), Phe (F), Ser (S), Cys (C), Ala (A), Val (V), Thr (T), or Gly (G); and/or
the amino acid at position 364 is mutated into Ala (A), Val (V), Thr (T), Leu (L), Tyr (Y), or Phe (F); and/or
the amino acid at position 366 is mutated into Gly (G), Ser (S), Ala (A), Val (V), Leu (L), His (H), or Ile (I); and/or
the amino acid at position 368 is mutated into Val (V), Ile (I), Met (M), or Ala (A); and/or
the amino acid at position 394 is mutated into Phe (F), Ala (A), Ser (S), Cys (C), Val (V), or Asn (N); and/or
the amino acid at position 405 is mutated into Thr (T), Leu (L), or Tyr (Y); and/or
the amino acid at position 407 is mutated into Cys (C), Val (V), Leu (L), His (H), or Phe (F); and/or
the amino acid at position 409 is mutated into Gln (Q), Arg (R), or Asp (D); and/or
the amino acid at position 411 is mutated into Asn (N), Tyr (Y), or Leu (L); and/or
the amino acid at position 354 is mutated into Tyr (Y), Cys (C), Phe (F), or Trp (W); and/or
the amino acid at position 357 is mutated into Cys (C); and/or
the amino acid at position 347 is mutated into Glu (E);
the CH3 domains of the first polypeptide and the second polypeptide differ by at least one mutation site, and mutation sites in the CH3 domains are indicated by EU numbering.

9. The heteromultimer according to claim 8, wherein:
1) the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domains of the first polypeptide and the second polypeptide each further comprise one identical or different amino acid mutation selected from the group consisting of 351I, 351C, 351V, 351T, 351F, 351M, 349L, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y;
preferably, the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351I, 351C, 351V, 351T, 351F, 351M, 349L, 364A, 364T, 364L, 366G, 366A, 366V, 366L, 366H, 366I, 368V, 368I, 368A, 368M, 394A, 394S, 394C, 394V, 394N, 405L, 405Y, 407C, 407V, 407L, 407H, 407F, 409Q, 409R, 411L, and 411Y;
further preferably, the CH3 domain of the first polypeptide comprises an amino acid mutation of 356K, and the CH3 domain of the second polypeptide comprises an amino acid mutation of 439E, wherein the CH3 domains of the first polypeptide and the second polypeptide each further comprise one identical amino acid mutation selected from the group consisting of 351I, 351T, 364A, 366A, 368I, 394A, 394S, 405Y, 409Q, and 411Y;
more preferably, the CH3 domain of the first polypeptide comprises amino acid mutations of 356K and 351I, and the CH3 domain of the second polypeptide comprises amino acid mutations of 439E and 351I; or
2) the CH3 domain of the first polypeptide comprises an amino acid mutation of 349C, and the CH3 domain of the second polypeptide comprises an amino acid mutation of 354C, wherein the CH3 domain of the second polypeptide further comprises one amino acid mutation selected from the group consisting of 364Y, 364F, 351I, 366A, 368I, and 405Y; or
the CH3 domain of the first polypeptide comprises an amino acid mutation of 349C, and the CH3 domain of the second polypeptide comprises amino acid mutations of 347E and 357C;
preferably, the CH3 domain of the first polypeptide comprises an amino acid mutation of 349C, and the CH3 domain of the second polypeptide comprises an amino acid mutation of 354C, wherein the CH3 domain of the second polypeptide further comprises an amino acid mutation of 364Y or 364F;
more preferably, the CH3 domain of the first polypeptide comprises an amino acid mutation of 349C, and the CH3 domain of the second polypeptide comprises amino acid mutations of 354C and 364Y; or
3) the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to iv):
i) 349C;
ii) 354C and 351I;
iii) 354C and 366A; and
iv) 354C and 368I;
preferably, the CH3 domain of the first polypeptide comprises amino acid mutations of 356K and 349C, and the CH3 domain of the second polypeptide comprises amino acid mutations of 439E and 354C, wherein the CH3 domain of the second polypeptide further comprises one amino acid mutation selected from the group consisting of 351I, 366A, and 368I;
more preferably, the CH3 domain of the first polypeptide comprises amino acid mutations of 356K and 349C, and the CH3 domain of the second polypeptide comprises amino acid mutations of 439E, 354C, and 351I; or
4) the CH3 domain of the first polypeptide comprises an amino acid mutation of 349S, and the CH3 domain of the second polypeptide comprises one amino acid mutation selected from the group consisting of 364Y, 354F, 354W, and 354Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 349A, and the CH3 domain of the second polypeptide comprises one amino acid mutation selected from the group consisting of 354F, 354Y, and 354W; or
the CH3 domain of the first polypeptide comprises an amino acid mutation of 349V, and
the CH3 domain of the second polypeptide comprises an amino acid mutation of 354F; or
the CH3 domain of the first polypeptide comprises an amino acid mutation of 349T, and the CH3 domain of the second polypeptide comprises an amino acid mutation of 354F; or
the CH3 domain of the first polypeptide comprises an amino acid mutation of 405T, and the CH3 domain of the second polypeptide comprises an amino acid mutation of 394F; or
the CH3 domain of the first polypeptide comprises an amino acid mutation of 366H, and the CH3 domain of the second polypeptide comprises an amino acid mutation of 405L or 407L;
preferably, the CH3 domain of the first polypeptide comprises an amino acid mutation of 349S, and the CH3 domain of the second polypeptide comprises an amino acid mutation of 364Y; or
the CH3 domain of the first polypeptide comprises an amino acid mutation of 349G, and the CH3 domain of the second polypeptide comprises an amino acid mutation of 354W; or
the CH3 domain of the first polypeptide comprises an amino acid mutation of 405T, and the CH3 domain of the second polypeptide comprises an amino acid mutation of 394F;
more preferably, the CH3 domain of the first polypeptide comprises an amino acid mutation of 349S, and the CH3 domain of the second polypeptide comprises an amino acid mutation of 364Y; or
5) the CH3 domain of the first polypeptide comprises an amino acid mutation selected from the group consisting of 356K, 356R, and 356H, and the CH3 domain of the second polypeptide comprises an amino acid mutation selected from the group consisting of 439E and 439D, wherein the CH3 domain(s) of the first polypeptide and/or the second polypeptide further comprise(s) at least one amino acid mutation selected from the group consisting of the following i) to vii):
i) 349S or 349C;
ii) 354Y or 354C;
iii) 357C;
iv) 366H;
v) 394F;
vi) 405T or 405L; and
vii) 407L or 407H;
preferably, the CH3 domain of the first polypeptide comprises amino acid mutations of 356K and 349S, and the CH3 domain of the second polypeptide comprises amino acid mutations of 439E and 354Y; or
the CH3 domain of the first polypeptide comprises amino acid mutations of 356K and 349C, and the CH3 domain of the second polypeptide comprises amino acid mutations of 439E and 354C; or
the CH3 domain of the first polypeptide comprises amino acid mutations of 356K and 349C, and the CH3 domain of the second polypeptide comprises amino acid mutations of 439E and 357C; or
the CH3 domain of the first polypeptide comprises amino acid mutations of 356K and 405T, and the CH3 domain of the second polypeptide comprises amino acid mutations of 439E and 394F; or
the CH3 domain of the first polypeptide comprises amino acid mutations of 356K and 366H, and the CH3 domain of the second polypeptide comprises an amino acid mutation of 439E, wherein the CH3 domain of the second polypeptide further comprises one amino acid mutation selected from the group consisting of 405L, 407L, and 407H; or
the CH3 domain of the first polypeptide comprises amino acid mutations of 356K and 407L, and the CH3 domain of the second polypeptide comprises amino acid mutations of 439E and 366H; or
the CH3 domain of the first polypeptide comprises amino acid mutations of 356K and 407H, and the CH3 domain of the second polypeptide comprises amino acid mutations of 439E and 366H;
more preferably, the CH3 domain of the first polypeptide comprises amino acid mutations of 356K and 349S, and the CH3 domain of the second polypeptide comprises amino acid mutations of 439E and 354Y; or
6) the CH3 domain of the first polypeptide comprises an amino acid mutation of 349C, and the CH3 domain of the second polypeptide comprises an amino acid mutation of 357C; or
7) the CH3 domain of the first polypeptide comprises an amino acid mutation of 354C, and the CH3 domain of the second polypeptide comprises an amino acid mutation of 349C, wherein the CH3 domain(s) of the first polypeptide and/or the second polypeptide further comprise(s) at least one amino acid mutation selected from the group consisting of i) and ii):
i) 405T; and
ii) 394F;
preferably, the CH3 domain of the first polypeptide comprises amino acid mutations of 354C and 405T, and the CH3 domain of the second polypeptide comprises amino acid mutations of 349C and 394F.

10. The heteromultimer according to any one of claim 8 or 9, wherein the first polypeptide and the second polypeptide have different isoelectric points; preferably, the first polypeptide and the second polypeptide further comprise additional amino acid mutations such that the first polypeptide and the second polypeptide have different isoelectric points.

11. The heteromultimer according to any one of claims 8 to 10, wherein the heteromultimer is a multispecific antibody or an Fc fusion protein.

12. A heteromultimer prepared by an extracellular Fab-arm exchange reaction, comprising a first polypeptide and a second polypeptide, wherein:
(1) the first polypeptide comprises one CH3 domain, wherein the CH3 domain of the first polypeptide comprises amino acid mutations of 356K and 351I, and the second polypeptide comprises one CH3 domain, wherein the CH3 domain of the second polypeptide comprises amino acid mutations of 439E and 351I; the mutation sites are indicated by EU numbering;
preferably, the first polypeptide comprises one CH3 domain, wherein the CH3 domain of the first polypeptide comprises amino acid mutations of D356K and L351I, and the second polypeptide comprises one CH3 domain, wherein the CH3 domain of the second polypeptide comprises amino acid mutations of K439E and L351I; the mutation sites are indicated by EU numbering; or
the first polypeptide comprises one CH3 domain, wherein the CH3 domain of the first polypeptide comprises amino acid mutations of E356K and L351I, and the second polypeptide comprises one CH3 domain, wherein the CH3 domain of the second polypeptide comprises amino acid mutations of K439E and L351I; the mutation sites are indicated by EU numbering; or
(2) the first polypeptide comprises one CH3 domain, wherein the CH3 domain of the first polypeptide comprises an amino acid mutation of 349C, and the second polypeptide comprises one CH3 domain, wherein the CH3 domain of the second polypeptide comprises amino acid mutations of 354C and 364Y; the mutation sites are indicated by EU numbering;
preferably, the first polypeptide comprises one CH3 domain, wherein the CH3 domain of the first polypeptide comprises an amino acid mutation of Y349C, and the second polypeptide comprises one CH3 domain, wherein the CH3 domain of the second polypeptide comprises amino acid mutations of S354C and S364Y; the mutation sites are indicated by EU numbering; or
(3) the first polypeptide comprises one CH3 domain, wherein the CH3 domain of the first polypeptide comprises amino acid mutations of 356K and 349C, and the second polypeptide comprises one CH3 domain, wherein the CH3 domain of the second polypeptide comprises amino acid mutations of 439E, 354C, and 351I; the mutation sites are indicated by EU numbering;
preferably, the first polypeptide comprises one CH3 domain, wherein the CH3 domain of the first polypeptide comprises amino acid mutations of D356K and Y349C, and the second polypeptide comprises one CH3 domain, wherein the CH3 domain of the second polypeptide comprises amino acid mutations of K439E, S354C, and L351I; the mutation sites are indicated by EU numbering; or
the first polypeptide comprises one CH3 domain, wherein the CH3 domain of the first polypeptide comprises amino acid mutations of E356K and Y349C, and the second polypeptide comprises one CH3 domain, wherein the CH3 domain of the second polypeptide comprises amino acid mutations of K439E, S354C, and L351I; the mutation sites are indicated by EU numbering; or
(4) the first polypeptide comprises one CH3 domain, wherein the CH3 domain of the first polypeptide comprises an amino acid mutation of 349S, and the second polypeptide comprises one CH3 domain, wherein the CH3 domain of the second polypeptide comprises an amino acid mutation of 364Y; the mutation sites are indicated by EU numbering;
preferably, the first polypeptide comprises one CH3 domain, wherein the CH3 domain of the first polypeptide comprises an amino acid mutation of Y349S, and the second polypeptide comprises one CH3 domain, wherein the CH3 domain of the second polypeptide comprises an amino acid mutation of S364Y; the mutation sites are indicated by EU numbering.

13. A pharmaceutical composition, comprising the heteromultimer according to any one of claims 8 to 12 and one or more pharmaceutically acceptable carriers, diluents, or excipients.

14. An immunoconjugate, comprising the heteromultimer according to any one of claims 8 to 12 and an effector, wherein the effector is conjugated to the heteromultimer; preferably, the effector is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

15. One or more isolated nucleic acids, encoding the first polypeptide and/or the second polypeptide of the heteromultimer according to any one of claims 8 to 12.

16. One or more host cells, comprising the one or more isolated nucleic acids according to claim 15.

17. A method for preparing the heteromultimer according to any one of claims 8 to 12, comprising expressing the one or more isolated nucleic acids according to claim 15 or culturing the one or more host cells according to claim 16 to produce the heteromultimer.
